# EUROPÄISCHE PATENTSCHRIFT

(11) **EP 3 898 888 B1**
(45) Veröffentlichungstag und Bekanntmachung des Hinweises auf die Patenterteilung: **03.08.2022**
(21) Anmeldenummer: 19829492.8
(22) Anmeldetag: 17.12.2019
(51) Int. Cl.: C09K 11/06, H01L 51/50, C07D 471/04, C07D 519/00

(54) **MATERIALIEN FÜR ORGANISCHE ELEKTROLUMINESZENZVORRICHTUNGEN**
MATERIALS FOR ORGANIC ELECTROLUMINESCENT DEVICES
MATIÈRES POUR DISPOSITIFS ÉLECTROLUMINESCENTS ORGANIQUES

(30) Priorität: 19.12.2018 EP 18213861
(43) Veröffentlichungstag der Anmeldung: 27.10.2021
(73) Patentinhaber: Merck Patent GmbH, 64293 Darmstadt (DE)
(72) Erfinder: PARHAM, Amir, 60486 FRANKFURT AM MAIN (DE); KROEBER, Jonas, 60311 FRANKFURT AM MAIN (DE); EHRENREICH, Christian, 64285 DARMSTADT (DE); ENGELHART, Jens, 64285 DARMSTADT (DE); EICKHOFF, Christian, 68259 MANNHEIM (DE); PFALZGRAF, Jens, 64853 OTZBERG (DE)
(86) Internationale Anmeldenummer: PCT/EP2019/085506
(87) Internationale Veröffentlichungsnummer: WO 2020/127165

(56) Entgegenhaltungen:
- WO-A1-2014/094964
- WO-A1-2018/189134
- DE-A1-102010 019 306
- DE-A1-102014 008 722

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig phosphoreszierende metallorganische Komplexe eingesetzt. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien können somit auch zu Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter, aber auch als Elektronentransportmaterialien, Lochblockiermaterialien bwz. Exzitonenblockiermaterialien und welche dort insbesondere zu einer verbesserten Lebensdauer führen.

Überraschend wurde gefunden, dass bestimmte, unten näher beschriebene Verbindungen diese Aufgabe lösen und sich gut für die Verwendung in OLEDs eignen. Dabei weisen die OLEDs insbesondere eine deutlich verbesserte Lebensdauer, eine hohe Effizienz und eine geringe Betriebsspannung auf. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus WO 2011/137951 ist die Verwendung von Lactamen als Matrixmaterialien für phosphoreszierende Emitter bekannt. Lactamderivate mit einem Substitutionsmuster, wie nachfolgend beschrieben, sind nicht offenbart. Weitere Lactamderivate sind aus WO2014094964 bekannt.

Gegenstand der vorliegenden Erfindung ist eine Verbindung gemäß Formel (1), 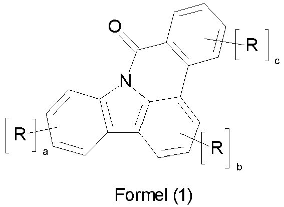 mit der Maßgabe, dass mindestens ein Rest R vorhanden ist, der für eine Gruppe Y steht, und wobei für die verwendeten Symbole und Indizes gilt:
- Y: ist bei jedem Auftreten gleich oder verschieden eine Gruppe der folgenden Formel (2), 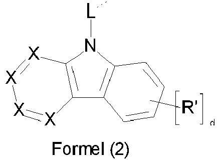 wobei die gestrichelte Bindung die Verknüpfung dieser Gruppe in der Formel (1) kennzeichnet;
- X: ist bei jedem Auftreten gleich oder verschieden CR' oder zwei benachbarte X stehen für eine Gruppe der folgenden Formel (3), und die verbleibenden X sind bei jedem Auftreten gleich oder verschieden CR', 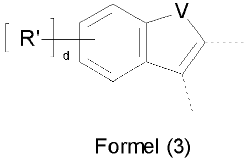 wobei die gestrichelten Bindungen die Verknüpfung dieser Gruppe in der Formel (2) kennzeichnen;
- V: ist NR', C(R')₂, O oder S;
- L: ist ein aromatisches oder heteroaromatisches Ringsystem mit 5 bis 30 aromatischen Ringatomen, das mit einem oder mehreren Resten R' substituiert sein kann;
- R, R': ist bei jedem Auftreten gleich oder verschieden H, D, F, Cl, Br, I, N(Ar')₂, N(R¹)₂, OAr', SAr', CN, NO₂, OR¹, SR¹, COOR¹, C(=O)N(R¹)₂, Si(R')₃, B(OR')₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches, heteroaliphatisches, aromatisches oder heteroaromatisches Ringsystem bilden; weiterhin können zwei Reste R' auch miteinander ein aliphatisches, heteroaliphatisches, aromatisches oder heteroaromatisches Ringsystem bilden; mit der Maßgabe, dass mindestens ein Rest R für eine Gruppe Y steht;
- Ar': ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
- R¹: ist bei jedem Auftreten gleich oder verschieden H, D, F, Cl, Br, I, N(R²)₂, CN, NO₂, OR², SR², Si(R²)₃, B(OR²)₂, COOR², C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können und wobei ein oder mehrere H-Atome in der Alkyl-, Alkenyl- oder Alkinylgruppe durch D, F, Cl, Br, I oder CN ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein aliphatisches, heteroaliphatisches, aromatisches oder heteroaromatisches Ringsystem bilden;
- R²: ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
- a: ist 0, 1, 2, 3 oder 4;
- b: ist bei jedem Auftreten gleich oder verschieden 0, 1, 2 oder 3;
- c: ist 0, 1, 2, 3 oder 4;
- d: ist bei jedem Auftreten gleich oder verschieden 0, 1, 2, 3 oder 4;
mit der Maßgabe, dass a + b + c ≥ 1 ist;
dabei ist die folgende Verbindung von der Erfindung ausgenommen:

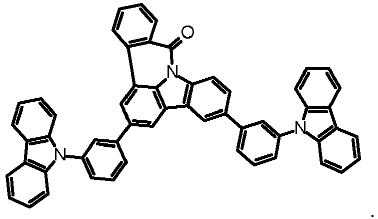

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome, bevorzugt 6 bis 40 C-Atome im Ringsystem. Ein heteroaromatisches Ringsystem im Sinne dieser Erfindung enthält 2 bis 60 C-Atome, bevorzugt 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. Ebenso sollen hierunter Systeme verstanden werden, in denen zwei oder mehr Aryl- bzw. Heteroarylgruppen direkt miteinander verknüpft sind, wie z. B. Biphenyl, Terphenyl, Bipyridin oder Phenylpyridin. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugte aromatische bzw. heteroaromatische Ringsysteme sind einfache Aryl- bzw. Heteroarylgruppen sowie Gruppen, in denen zwei oder mehr Aryl- bzw. Heteroarylgruppen direkt miteinander verknüpft sind, beispielsweise Biphenyl oder Bipyridin, sowie Fluoren oder Spirobifluoren.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe OR¹ mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe SR¹ mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, besonders bevorzugt F oder CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht:

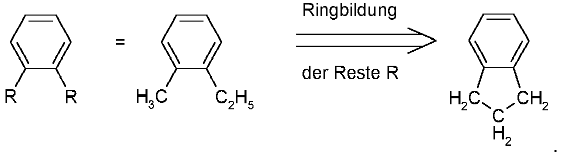

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

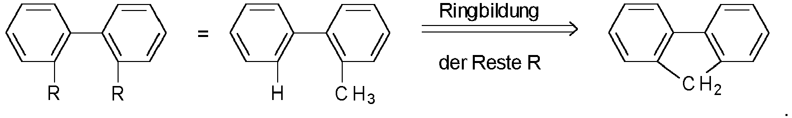

Je nach Position der Bindung der Gruppe Y entstehen verschiedene Isomere. Diese sind nachfolgend durch die Formeln (4) bis (6) dargestellt, 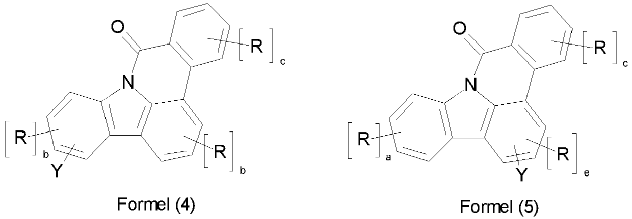 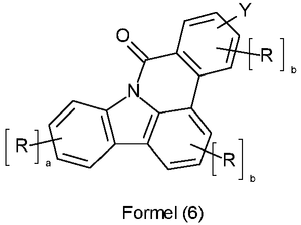 wobei die verwendeten Symbole und Indizes die oben genannten Bedeutungen aufweisen und weiterhin gilt:
- e: ist 0, 1 oder 2.

Bevorzugt sind die Verbindungen der Formel (4) oder (5), besonders bevorzugt die Verbindungen der Formel (4).

Bevorzugte Ausführungsformen der Verbindungen der Formeln (4) bis (6) sind die Verbindungen der folgenden Formeln (4a) bis (6a), 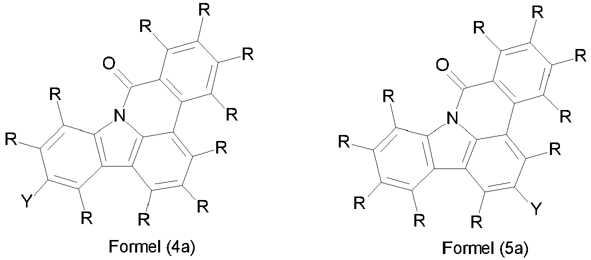 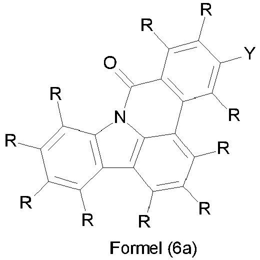 wobei die verwendeten Symbole die oben genannten Bedeutungen aufweisen.

Besonders bevorzugt sind die Verbindungen der folgenden Formeln (4b) bis (6b), 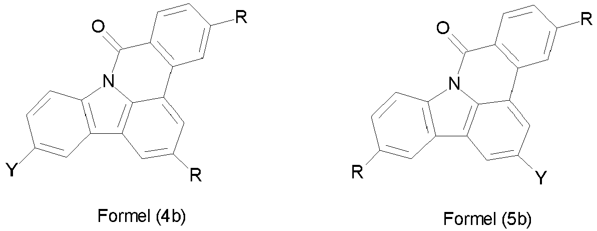 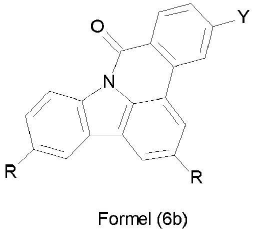 wobei die verwendeten Symbole die oben genannten Bedeutungen aufweisen.

In einer weiteren bevorzugten Ausführungsform der Erfindung stehen in der Verbindung der Formel (1) genau ein oder zwei Reste R für eine Gruppe Y, und besonders bevorzugt steht genau ein Rest R für eine Gruppe Y.

Im Folgenden werden bevorzugte Ausführungsformen der Gruppe Y beschrieben.

In einer bevorzugten Ausführungsform der Erfindung steht L gleich oder verschieden bei jedem Auftreten für ein aromatisches oder heteroaromatisches Ringsystem mit 6 bis 24 aromatischen Ringatomen, welches durch einen oder mehrere Reste R' substituiert sein kann. Wenn L für ein heteroaromatisches Ringsystem steht, dann enthält es bevorzugt keine elektronenarmen Heteroarylgruppen, wobei elektronenarme Heteroarylgruppen dadurch gekennzeichnet sind, dass sie einen heteroaromatischen Sechsring mit mindestens einem Stickstoffatom und/oder einen heteroaromatischen Fünfring mit mindestens zwei Heteroatomen, von denn mindestens eines ein Stickstoffatom ist, enthalten, wobei an diese Gruppen auch noch weitere aromatische oder heteroaromatische Gruppen ankondensiert sein können. Besonders bevorzugt steht L gleich oder verschieden bei jedem Auftreten für ein aromatisches Ringsystem mit 6 bis 24 aromatischen Ringatomen oder für Dibenzofuran, Dibenzothiophen oder Carbazol, wobei diese Gruppen jeweils durch einen oder mehrere Reste R' substituiert sein können. Ganz besonders bevorzugt steht L gleich oder verschieden bei jedem Auftreten für ein aromatisches Ringsystem mit 6 bis 12 aromatischen Ringatomen, insbesondere für Phenylen, wobei diese Gruppen jeweils durch einen oder mehrere Reste R' substituiert sein können, bevorzugt aber unsubstituiert sind.

Beispiele für bevorzugte Gruppen L sind bei jedem Auftreten gleich oder verschieden ausgewählt aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, Spirobifluoren, Naphthalin, Carbazol, Dibenzofuran, Dibenzothiophen, Indenocarbazol, Indolocarbazol, Phenanthren, Triphenylen oder einer Kombination aus zwei oder drei dieser Gruppen, welche jeweils mit einem oder mehreren Resten R', bevorzugt nicht-aromatischen Resten R', substituiert sein können.

Beispiele für geeignete Gruppen L sind die Strukturen der folgenden Formeln (L-1) bis (L-67), wobei die gestrichelten Bindungen die Verknüpfung der Gruppe andeuten und die Strukturen jeweils durch einen oder mehrere Reste R' substituiert sein können,

| | | |
|---|---|---|
| 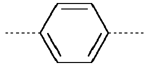 | 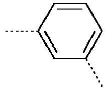 | 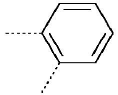 |
| (L-1) | (L-2) | (L-3) |
| 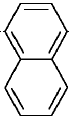 | 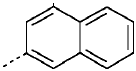 | 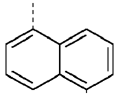 |
| (L-4) | (L-5) | (L-6) |
| 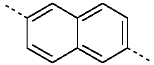 | 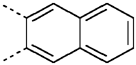 | 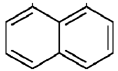 |
| (L-7) | (L-8) | (L-9) |
| 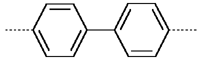 | 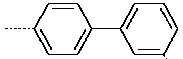 | 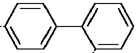 |
| (L-10) | (L-11) | (L-12) |
| 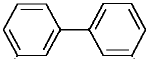 | 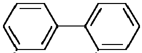 | 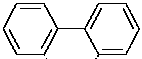 |
| (L-13) | (L-14) | (L-15) |
| 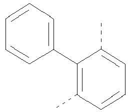 | 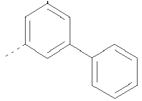 | 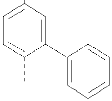 |
| (L-16) | (L-17) | (L-18) |
| 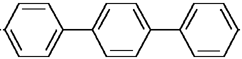 | 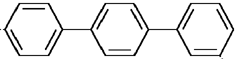 | |
| (L-19) | (L-20) | |
| 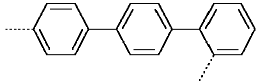 | 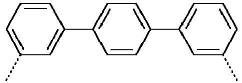 | |
| (L-21) | (L-22) | |
| 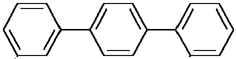 | 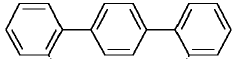 | |
| (L-23) | (L-24) | |
| 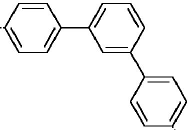 | 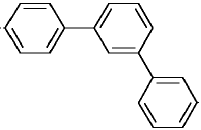 | |
| (L-25) | (L-26) | |
| 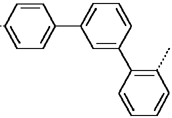 | 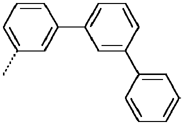 | |
| (L-27) | (L-28) | |
| 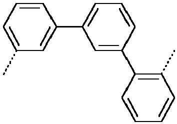 | 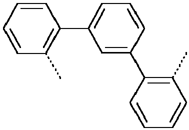 | |
| (L-29) | (L-30) | |
| 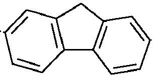 | 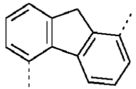 | 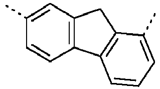 |
| (L-31) | (L-32) | (L-33) |
| 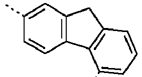 | 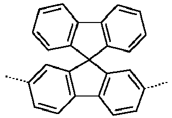 | 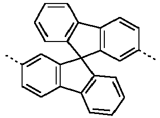 |
| (L-34) | (L-35) | (L-36) |
| 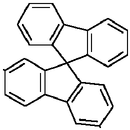 | 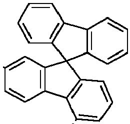 | 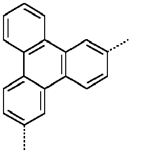 |
| (L-37) | (L-38) | (L-39) |
| 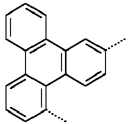 | 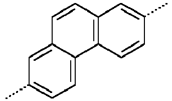 | 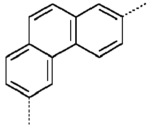 |
| (L-40) | (L-41) | (L-42) |
| 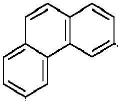 | 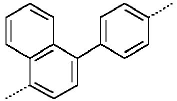 | 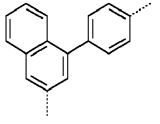 |
| (L-43) | (L-44) | (L-45) |
| 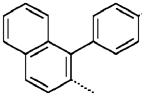 | 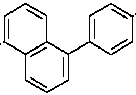 | 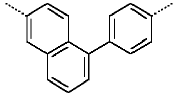 |
| (L-46) | (L-47) | (L-48) |
| 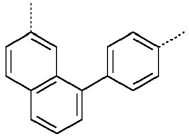 | 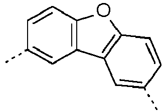 | 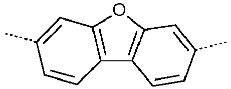 |
| (L-49) | (L-50) | (L-51) |
| 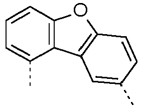 | 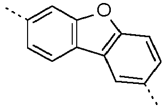 | 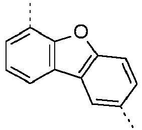 |
| (L-52) | (L-53) | (L-54) |
| 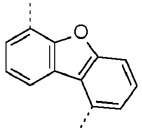 | 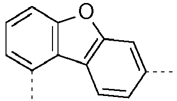 | 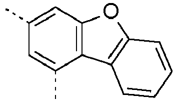 |
| (L-55) | (L-56) | (L-57) |
| 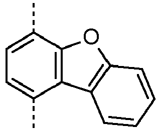 | 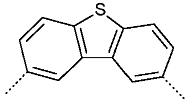 | 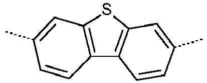 |
| (L-58) | (L-59) | (L-60) |
| 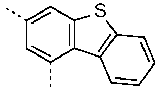 | 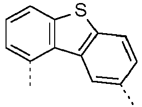 | 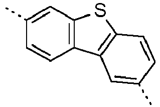 |
| (L-61) | (L-62) | (L-63) |
| 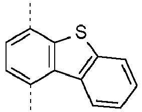 | 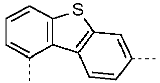 | 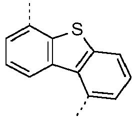 |
| (L-64) | (L-65) | (L-66) |
| 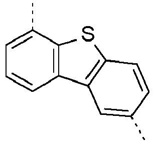 | | |
| (L-67) | | |

Besonders bevorzugt sind die Strukturen (L-1) bis (L-3), (L-10) bis (L-18), (L-50), (L-52), (L-59) und (L-62).

Die Gruppe Y ist bevorzugt gewählt aus den Gruppen der folgenden Formeln (Y-1) bis (Y-7), 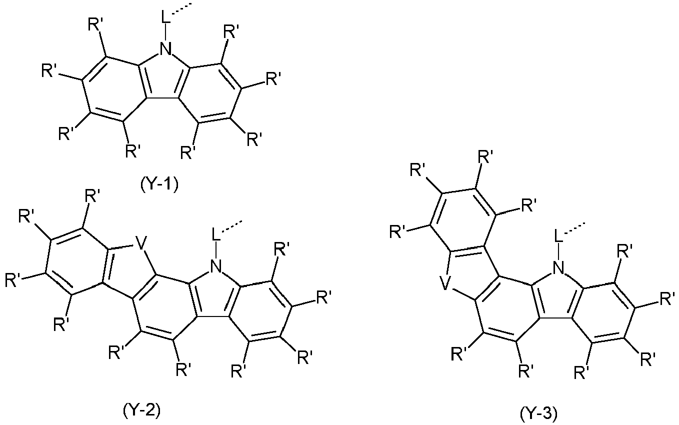 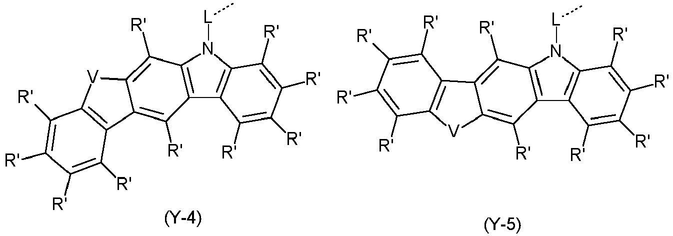 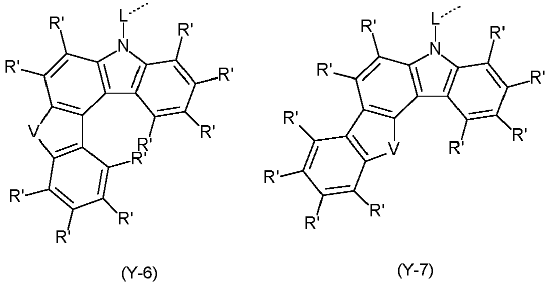 wobei die verwendeten Symbole die oben genannten Bedeutungen aufweisen.

Besonders bevorzugt ist die Gruppe Y gewählt aus den Strukturen der folgenden Formeln (Y-1a) bis (Y-7a), 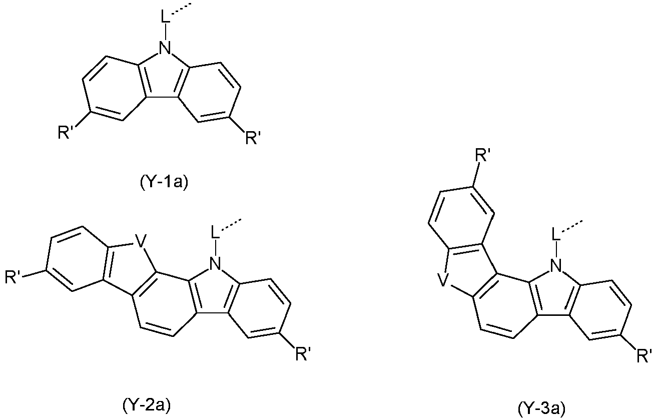 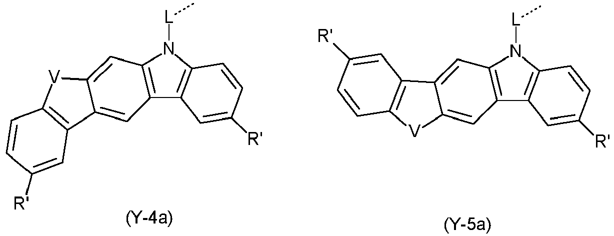 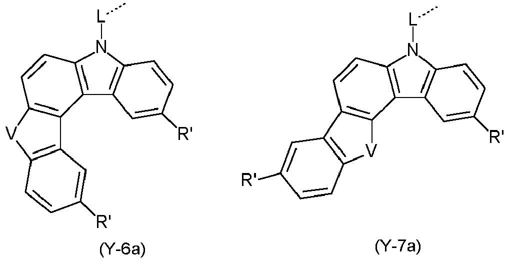 wobei die verwendeten Symbole die oben genannten Bedeutungen aufweisen.

Dabei stehen in den Formeln (Y-2) bis (Y-7) bzw. (Y-2a) bis (Y-7a) bevorzugt alle Reste R' für H.

Weiterhin steht in Formel (Y-1) bzw. (Y-1a) bevorzugt mindestens ein Rest R', der in para-Position zum Stickstoffatom gebunden ist, für eine Gruppe ungleich Wasserstoff. Dabei sind diese Reste R' bevorzugt gleich oder verschieden bei jeden Auftreten ein aromatisches oder heteroaromatisches Ringsystem mit 6 bis 24 aromatischen Ringatomen, besonders bevorzugt mit 6 bis 13 aromatischen Ringatomen, welches jeweils mit einem oder mehreren Resten R¹ substiutiert sein kann.

Besonders bevorzugt steht die Gruppe (Y-1) für eine Gruppe der folgenden Formel (Y-8), und die Gruppe (Y-1a) steht besonders bevorzugt für eine Gruppe der folgenden Formel (Y-8a), 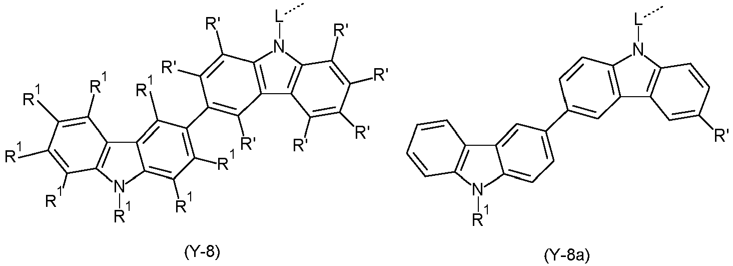 wobei der an das Stickstoffatom gebundene Rest R¹ bevorzugt für ein aromatisches oder heteroaromatisches Ringsystem mit 6 bis 30 aromatischen Ringatomen steht, welches mit einem oder mehreren Resten R² substituiert sein kann, und wobei R' in Formel (Y-8a) bevorzugt für H steht.

In einer bevorzugten Ausführungsform der Erfindung stehen außer dem Rest, der für die Gruppe Y steht, insgesamt maximal drei der Reste R und R', besonders bevorzugt insgesamt maximal zwei Reste R und R' und ganz besonders bevorzugt insgesamt maximal ein Rest R oder R' in der Verbindung der Formel (1) bzw. den oben aufgeführten bevorzugten Strukturen für eine Gruppe ungleich Wasserstoff.

Im Folgenden werden bevorzugte Substituenten R, R' Ar', R¹ und R² an den erfindungsgemäßen Verbindungen beschrieben. In einer besonders bevorzugten Ausführungsform der Erfindung treten die nachfolgend genannten Bevorzugungen für R, R', Ar', R¹ und R² gleichzeitig auf und gelten für die Strukturen der Formel (1), sowie für alle oben aufgeführten bevorzugten Ausführungsformen.

In einer bevorzugten Ausführungsform der Erfindung sind R bzw. R' bei jedem Auftreten gleich oder verschieden ausgewählt aus der Gruppe bestehend aus H, D, F, N(Ar')₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden; weiterhin können zwei Reste R' auch miteinander ein aliphatisches oder aromatisches Ringsystem bilden. Besonders bevorzugt sind R bzw. R' bei jedem Auftreten gleich oder verschieden ausgewählt aus der Gruppe bestehend aus H, N(Ar')₂, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R¹, bevorzugt nicht-aromatische Reste R¹, substituiert sein kann. Ganz besonders bevorzugt sind R bzw. R' bei jedem Auftreten gleich oder verschieden ausgewählt aus der Gruppe bestehend aus H oder einem aromatischen oder heteroaromatischen Ringsystem mit 6 bis 24 aromatischen Ringatomen, bevorzugt mit 6 bis 13 aromatischen Ringatomen, das jeweils durch einen oder mehrere Reste R¹, bevorzugt nicht-aromatische Reste R¹, substituiert sein kann. Weiterhin kann es bevorzugt sein, wenn R oder R' für eine Triaryl- bzw. -heteroarylamingruppe steht, welche durch einen oder mehrere Reste R¹ substituiert sein kann. Diese Gruppe ist eine Ausführungsform eines aromatischen oder heteroaromatischen Ringsystems, wobei dann mehrere Aryl- bzw. Heteroarylgruppen durch ein Stickstoffatom miteinander verknüpft sind. Wenn R bzw. R' für eine Triaryl- bzw. -heteroarylamingruppe steht, weist diese Gruppe bevorzugt 18 bis 30 aromatische Ringatome auf und kann durch einen oder mehrere Reste R¹, bevorzugt nicht-aromatische Reste R¹, substituiert sein.

In einer weiteren bevorzugten Ausführungsform der Erfindung ist Ar' ein aromatisches oder heteroaromatisches Ringsystem mit 6 bis 30 aromatischen Ringatomen, das durch einen oder mehrere Reste R¹ substituiert sein kann. In einer besonders bevorzugten Ausführungsform der Erfindung ist Ar' ein aromatisches oder heteroaromatisches Ringsystem mit 6 bis 24 aromatischen Ringatomen, insbesondere mit 6 bis 13 aromatischen Ringatomen, das durch einen oder mehrere, bevorzugt nicht-aromatische, Reste R¹ substituiert sein kann.

In einer weiteren bevorzugten Ausführungsform der Erfindung ist R¹ gleich oder verschieden bei jedem Auftreten ausgewählt aus der Gruppe bestehend aus H, D, F, CN, OR², einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R² substituiert sein kann und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R² substituiert sein kann; dabei können zwei oder mehrere Reste R¹ miteinander ein aliphatisches Ringsystem bilden. In einer besonders bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R² substituiert sein kann, bevorzugt aber unsubstituiert ist.

In einer weiteren bevorzugten Ausführungsform der Erfindung ist R² gleich oder verschieden bei jedem Auftreten H, F, eine Alkylgruppe mit 1 bis 4 C-Atomen oder eine Arylgruppe mit 6 bis 10 C-Atomen, welche mit einer Alkylgruppe mit 1 bis 4 C-Atomen substituiert sein kann, bevorzugt aber unsubstituiert ist.

Geeignete aromatische bzw. heteroaromatische Ringsysteme R, R' bzw. Ar' sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R¹ substituiert sein können. Wenn R bzw. R' bzw. Ar' für eine Heteroarylgruppe, insbesondere für Triazin, Pyrimidin, Chinazolin oder Carbazol steht, können auch aromatische oder heteroaromatische Reste R¹ an dieser Heteroarylgruppe bevorzugt sein.

Dabei sind die Gruppen R bzw. R', wenn sie für ein aromatisches bzw. heteroaromatisches Ringsystem stehen, bzw. Ar' bevorzugt gewählt aus den Gruppen der folgenden Formeln R-1 bis R-83, 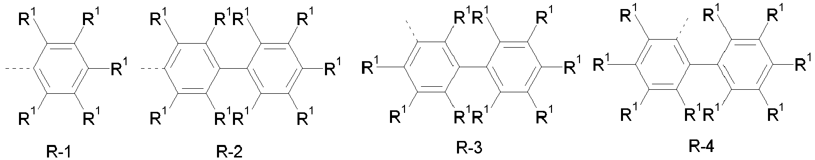 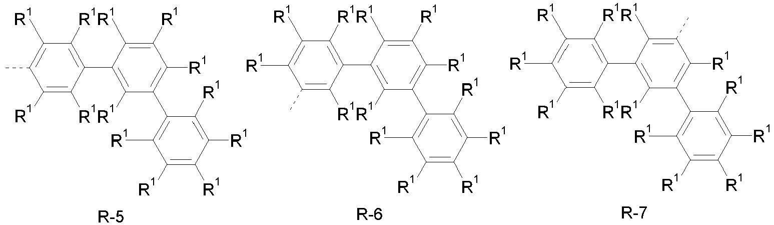 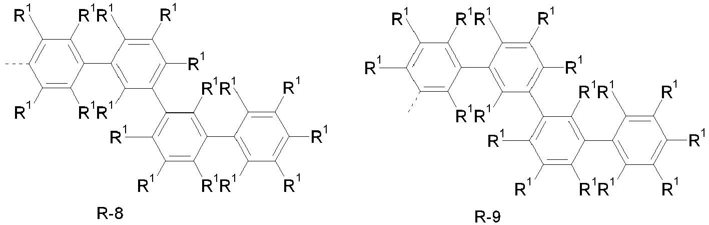 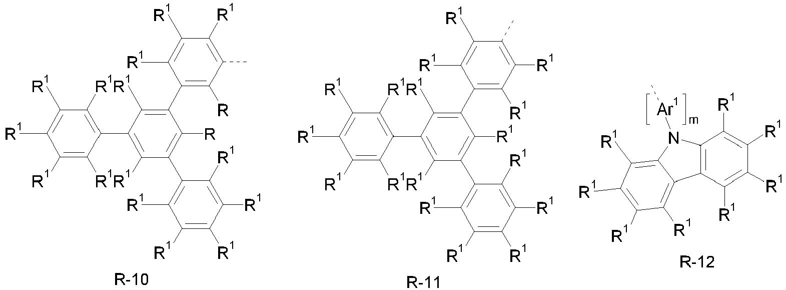 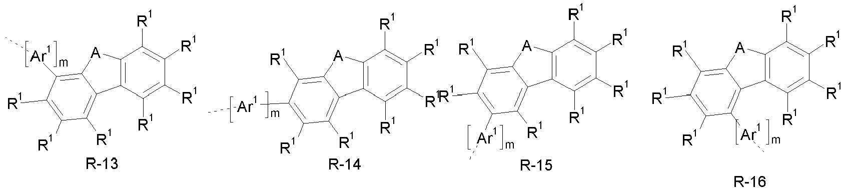 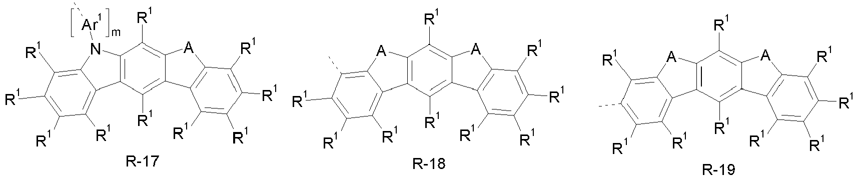 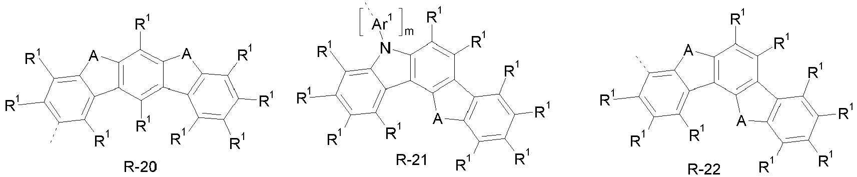 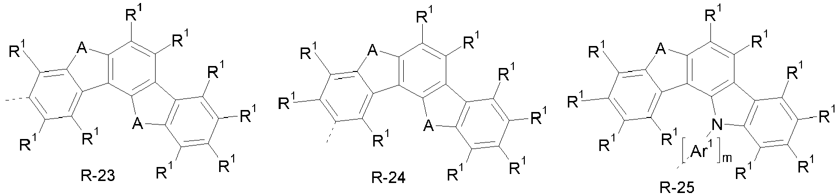 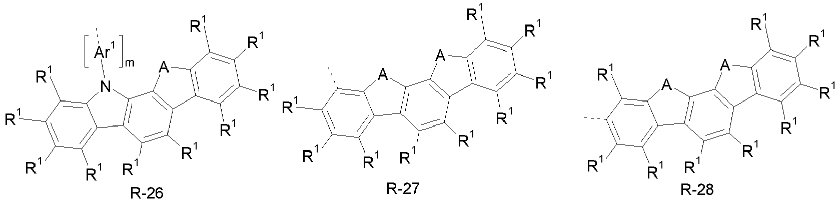 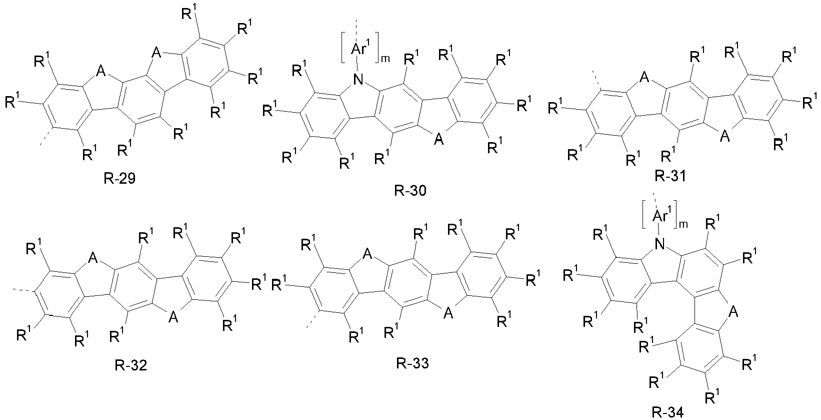 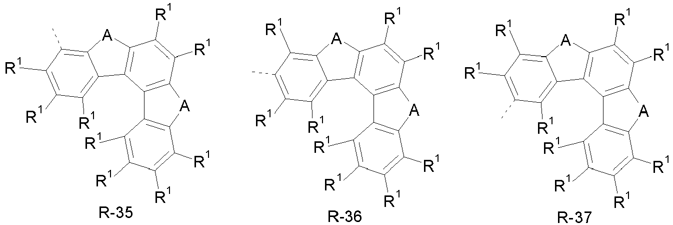 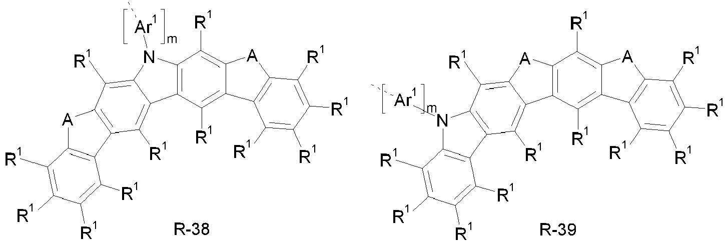 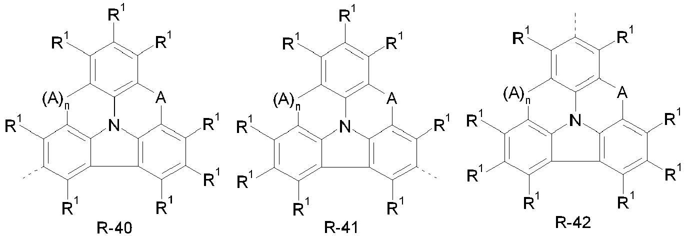 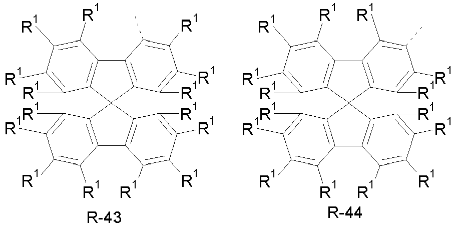 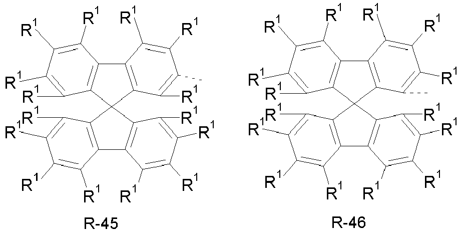 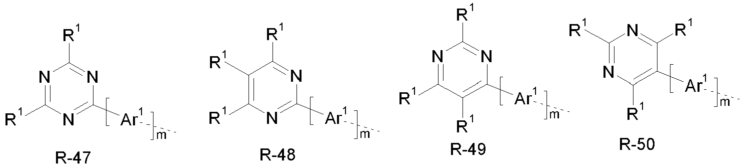 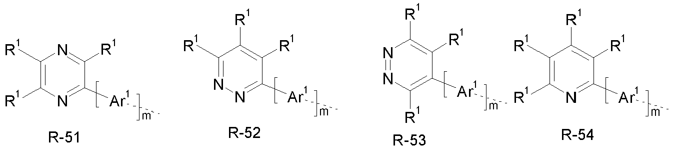 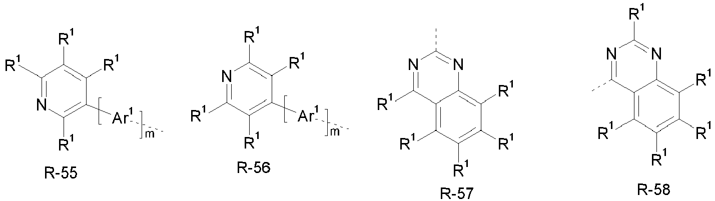 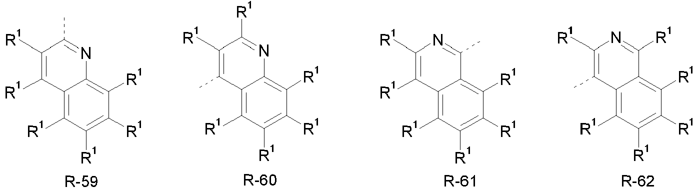 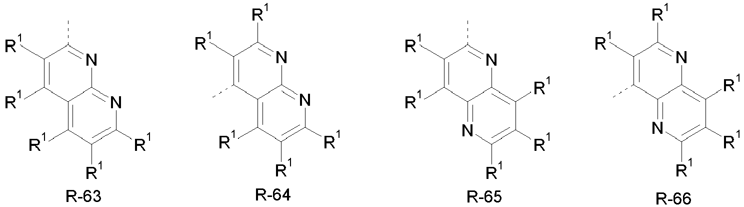 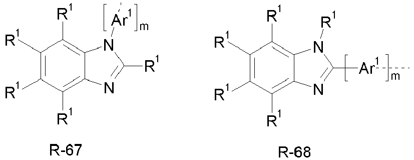 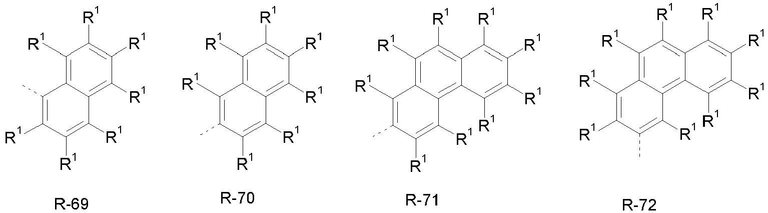 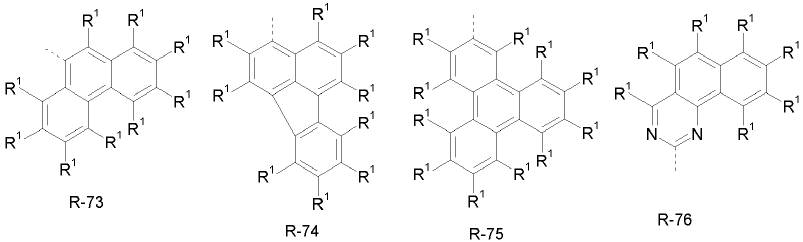 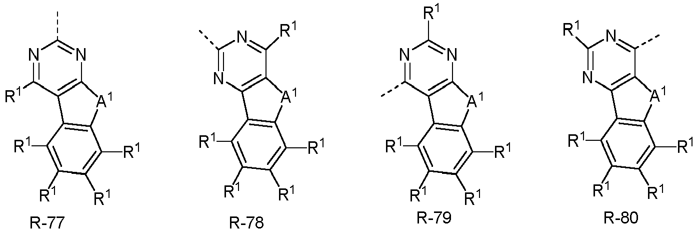 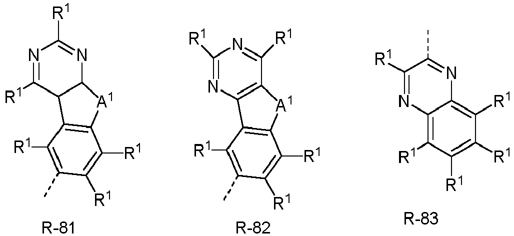 wobei R¹ die oben genannten Bedeutungen aufweist, die gestrichelte Bindung die Bindung an ein Kohlenstoffatom des Grundgerüsts in Formel (1), (2) und (3) bzw. in den bevorzugten Ausführungsformen bzw. an das Stickstoffatom in der Gruppe N(Ar')₂ darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- n: ist 0 oder 1, wobei n = 0 bedeutet, dass an dieser Position keine Gruppe A gebunden ist und an den entsprechenden Kohlenstoffatomen statt dessen Reste R¹ gebunden sind;
- m: ist 0 oder 1, wobei m = 0 bedeutet, dass die Gruppe Ar¹ nicht vorhanden ist und dass die entsprechende aromatische bzw. heteroaromatische Gruppe direkt an ein Kohlenstoffatom des Grundgerüsts in Formel (1) bzw. in den bevorzugten Ausführungsformen bzw. an das Stickstoffatom in der Gruppe N(Ar')₂ gebunden ist; mit der Maßgabe, dass m = 1 ist für die Strukturen (R-12), (R-17), (R-21), (R-25), (R-26), (R-30), (R-34), (R-38) und (R-39), wenn es sich bei diesen Gruppen um Ausführungsformen von Ar' handelt.

Wenn die oben genannten Gruppen R-1 bis R-83 für R, R' bzw. Ar' mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen. In einer besonders bevorzugten Ausführungsform der Erfindung steht in Gruppen Ar, R bzw. Ar', die mehrere Gruppen A aufweisen, mindestens eine Gruppe A für C(R¹)₂ oder für NR¹.

Wenn A für NR¹ steht, steht der Substituent R¹, der an das Stickstoffatom gebunden ist, bevorzugt für ein aromatisches oder heteroaromatisches Ringsystem mit 5 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, bevorzugt mit 6 bis 12 aromatischen Ringatomen, welches keine kondensierten Arylgruppen oder Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R² substituiert sein kann. Besonders bevorzugt sind Phenyl, Biphenyl, Terphenyl und Quaterphenyl mit Verknüpfungsmustern, wie vorne für R-1 bis R-11 aufgeführt, wobei diese Strukturen durch einen oder mehrere Reste R² substituiert sein können, bevorzugt aber unsubstituiert sind.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Weitere geeignete Gruppen R, R' bzw. Ar' sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 60 und bevorzugt maximal 40.

Dabei können Ar⁴ und Ar² miteinander und/oder Ar² und Ar³ miteinander auch durch eine Gruppe, ausgewählt aus C(R¹)₂, NR¹, O oder S, verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, insbesondere mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe. Dies gilt insbesondere, wenn Ar⁴ mit Ar² durch einen Einfachbindung verbunden ist.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar² und Ar³ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben die Alkylgruppen in Verbindungen, die durch Vakuumverdampfung verarbeitet werden, bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste R, R', Ar', R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren, Triphenylen und Chinazolin, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

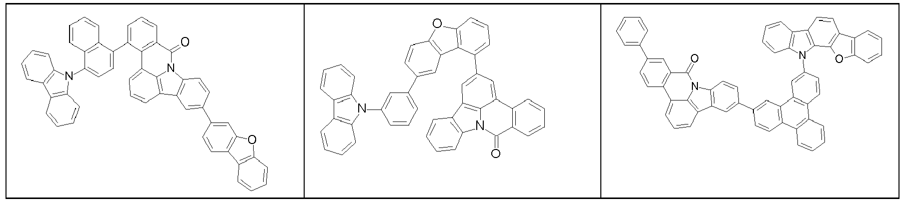

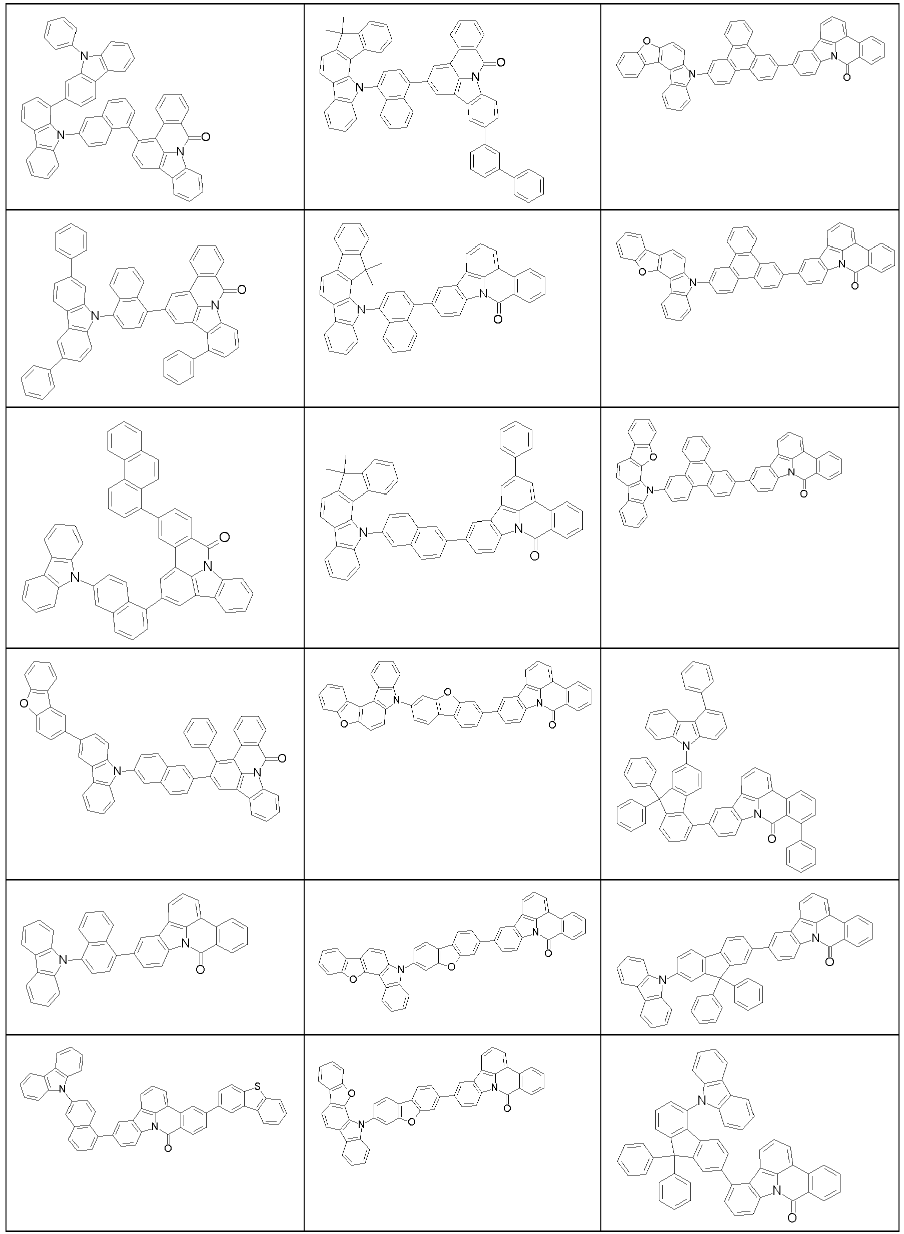

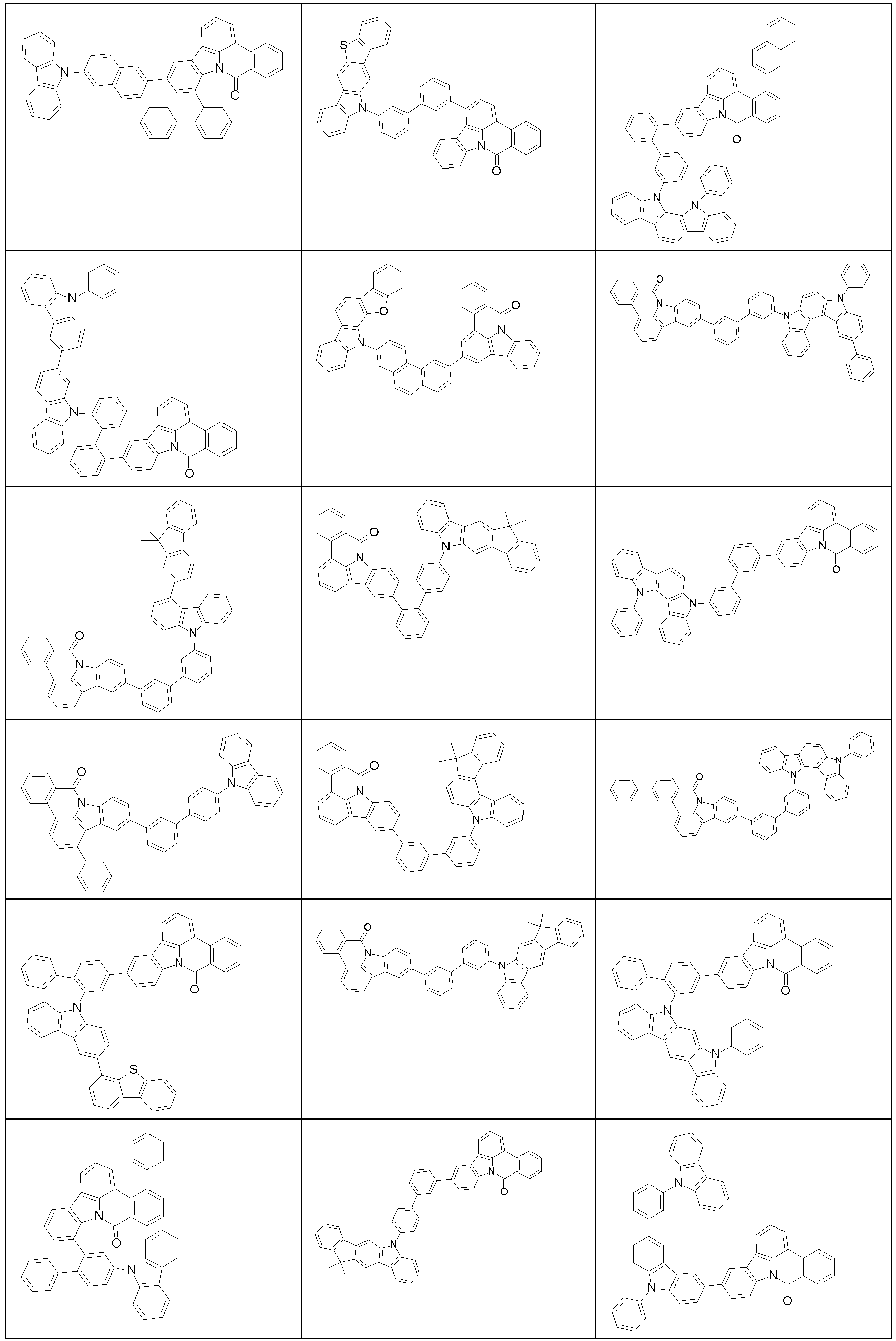

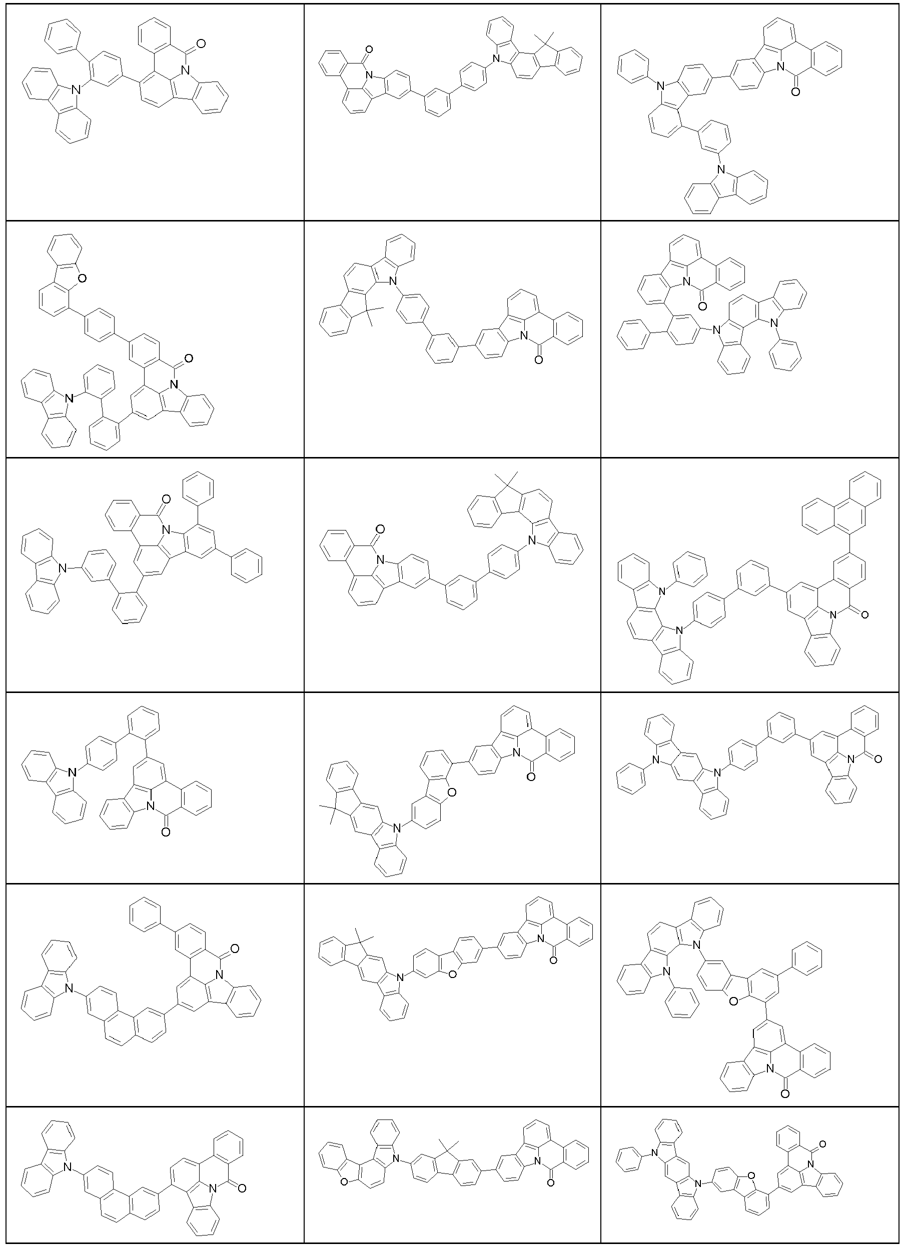

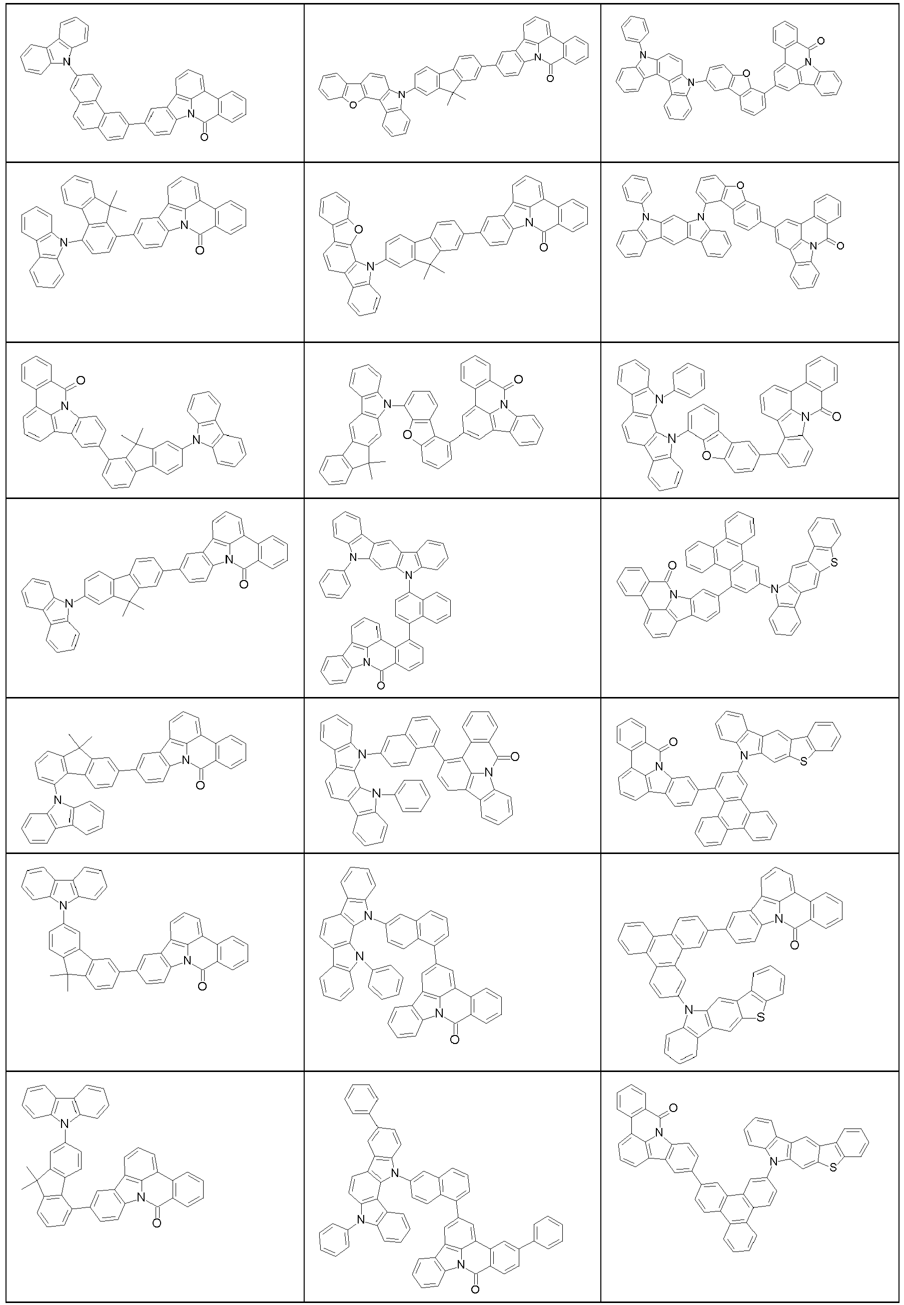

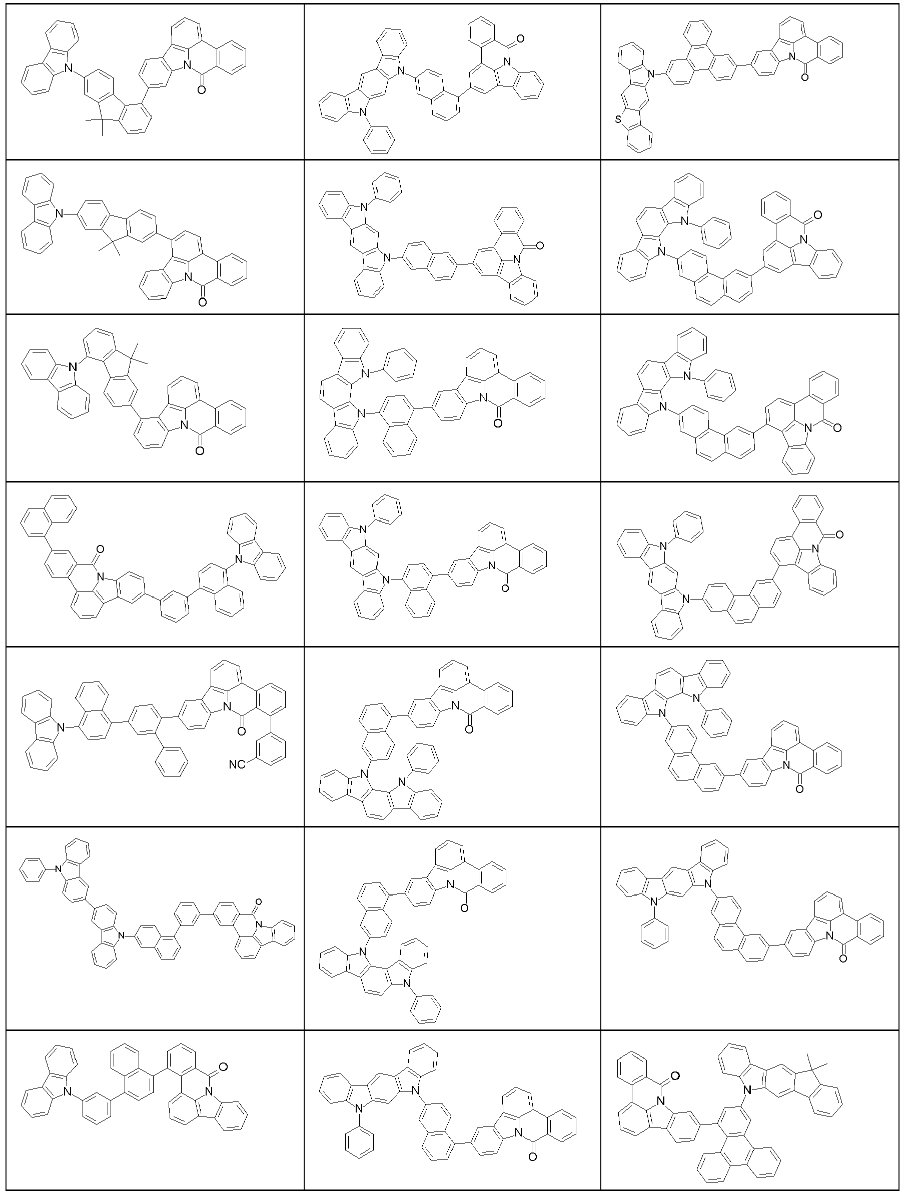

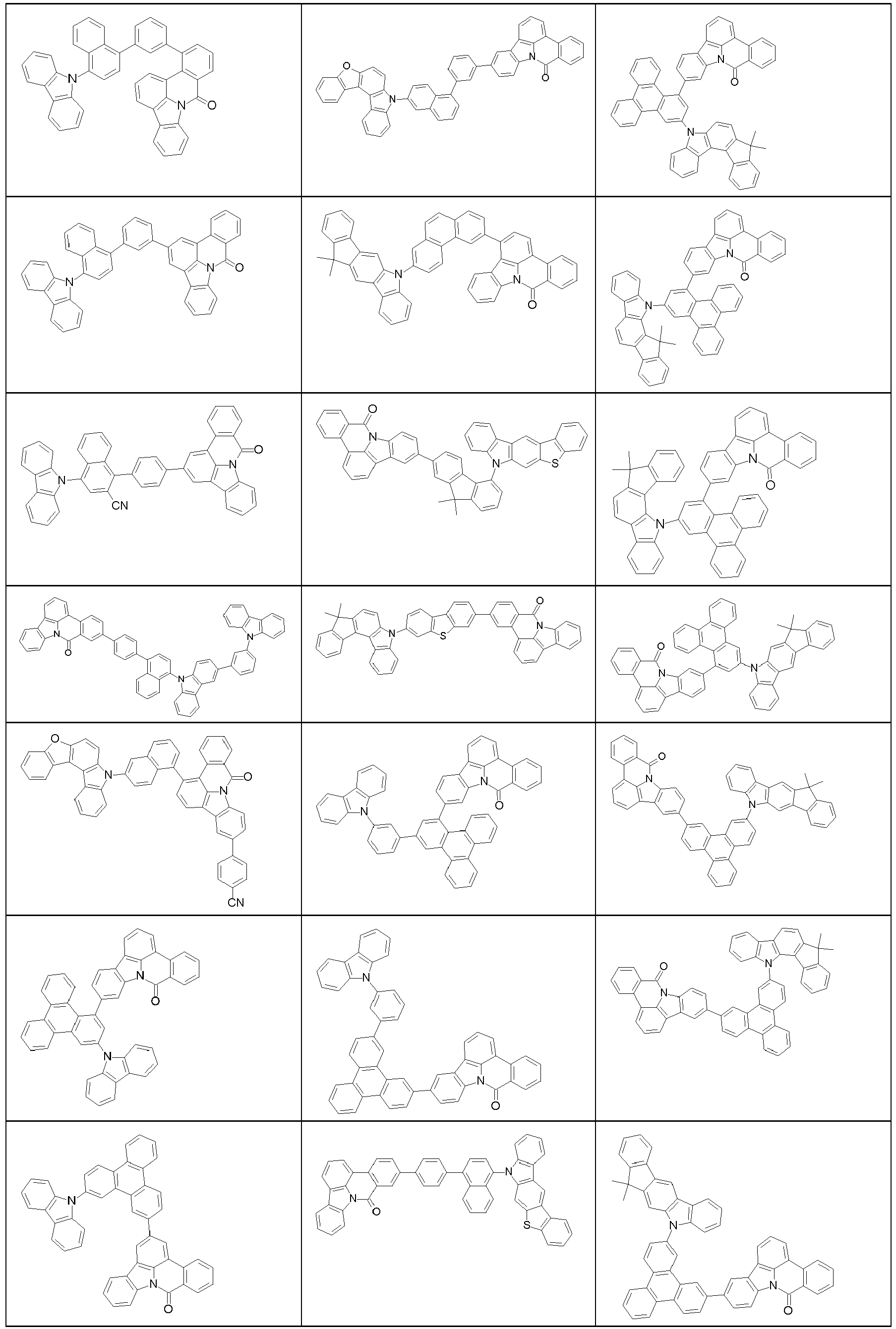

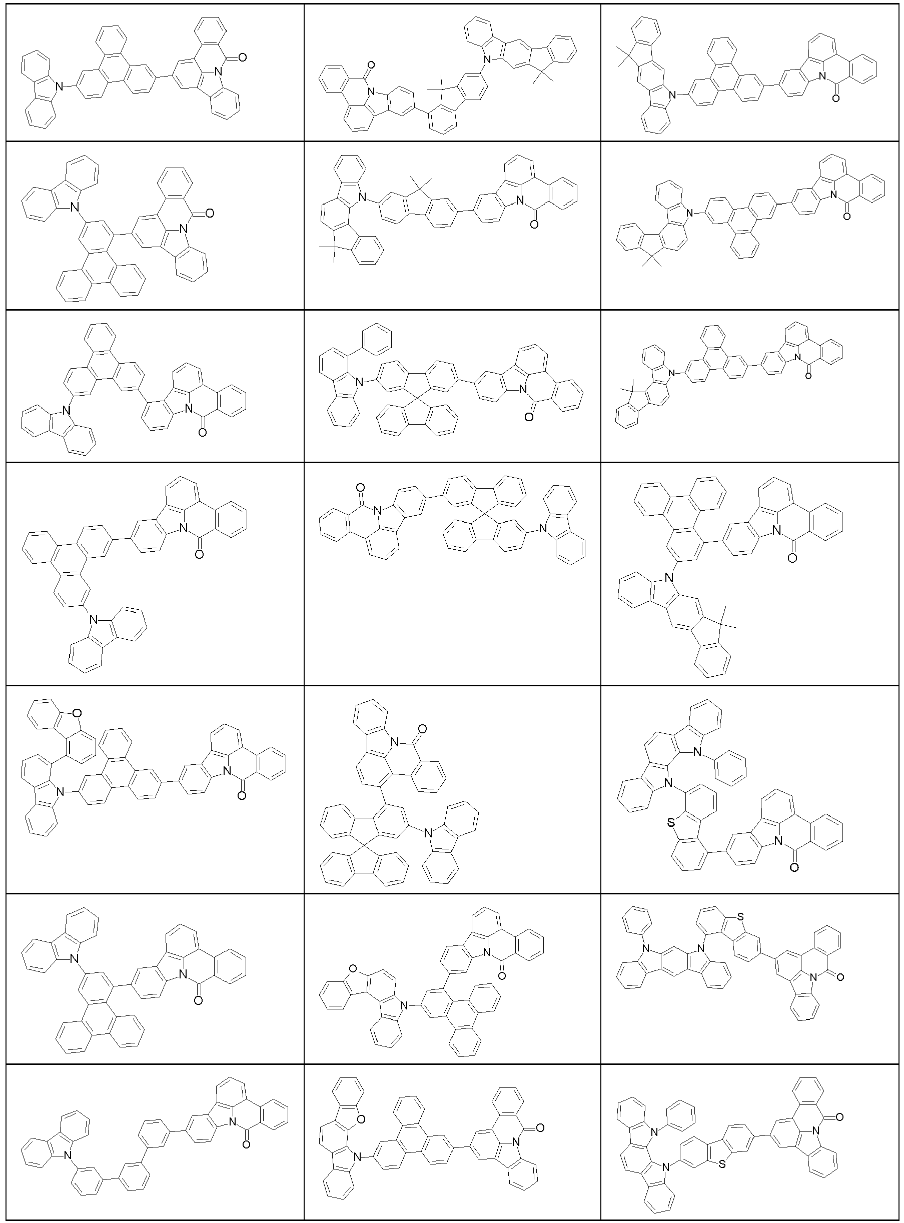

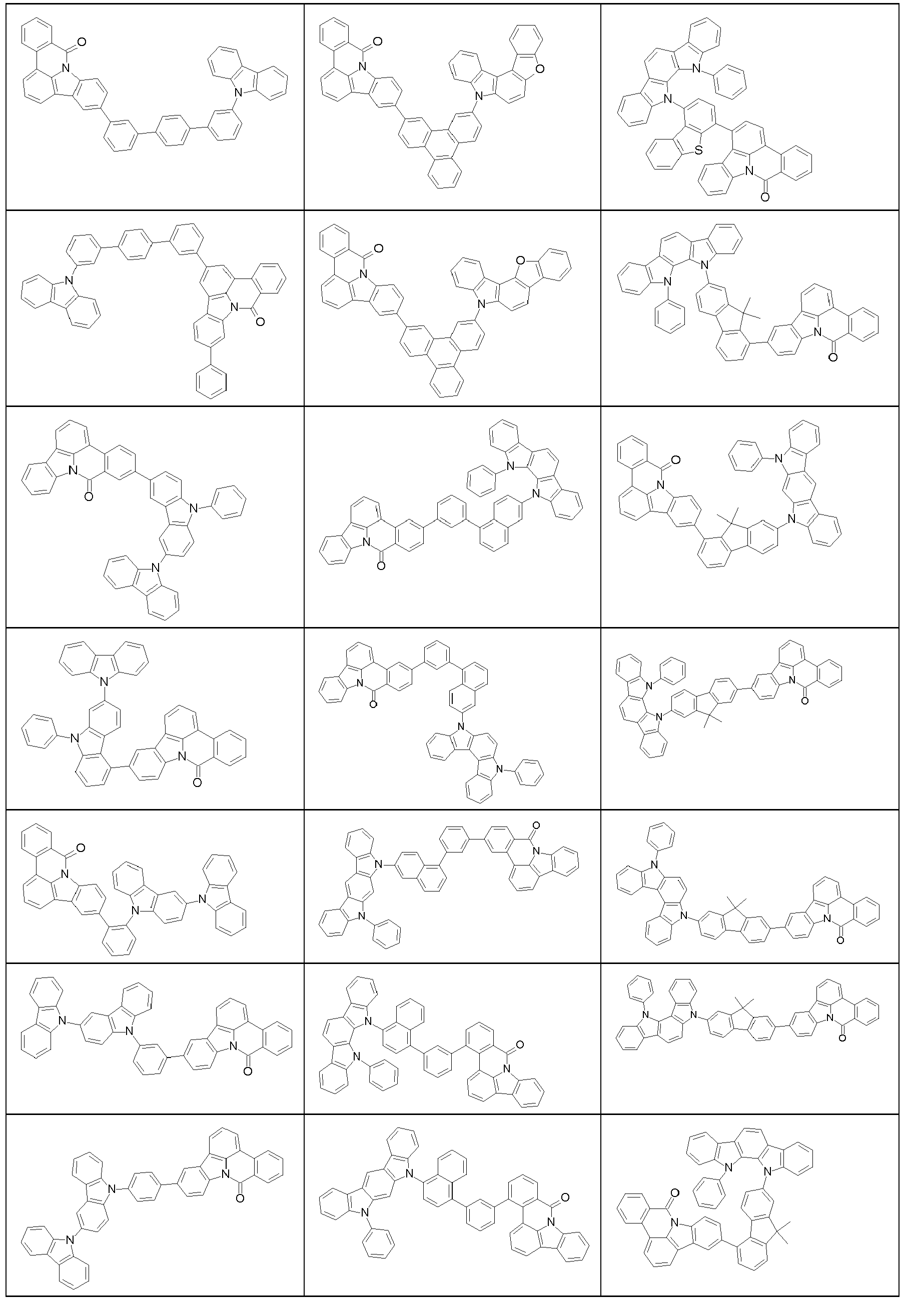

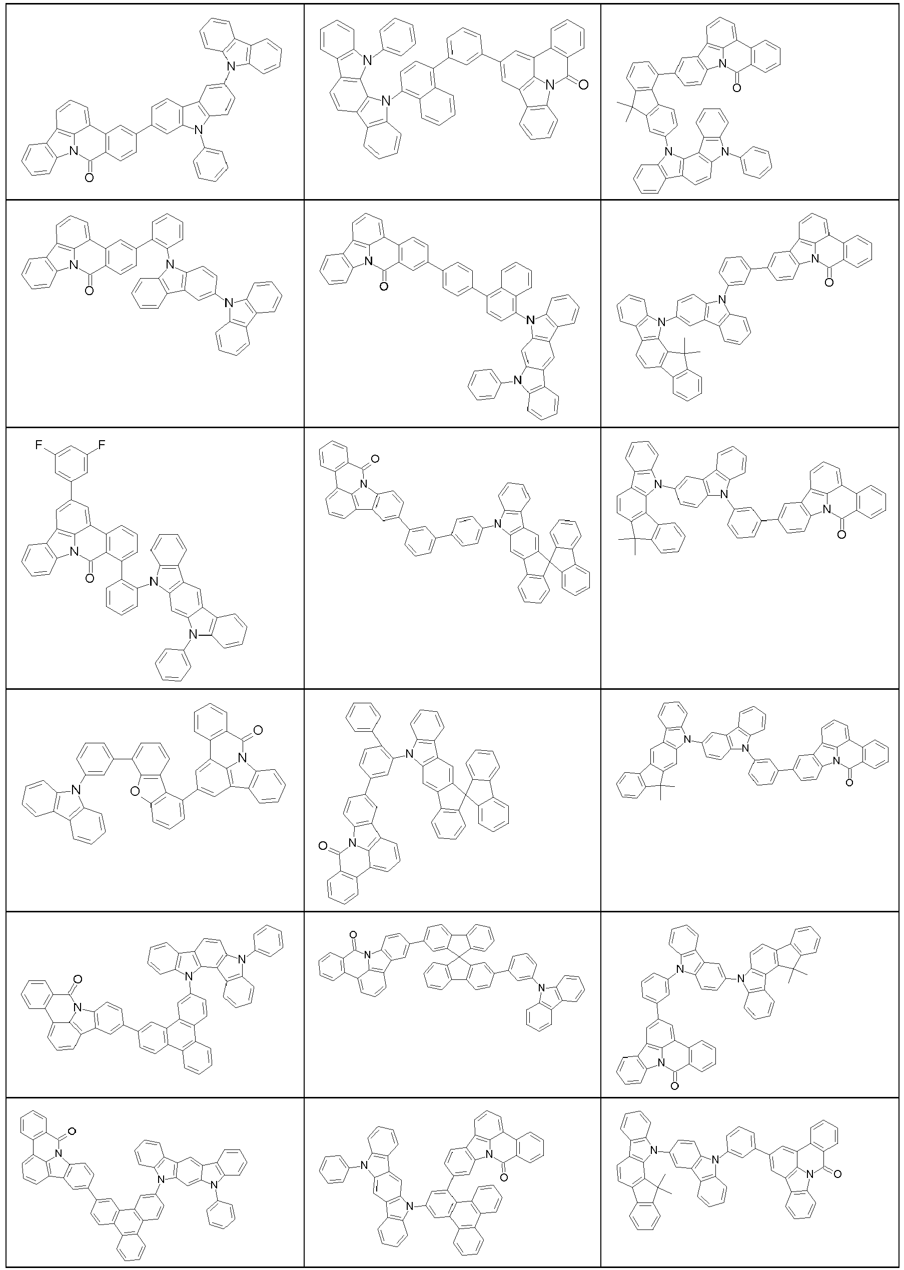

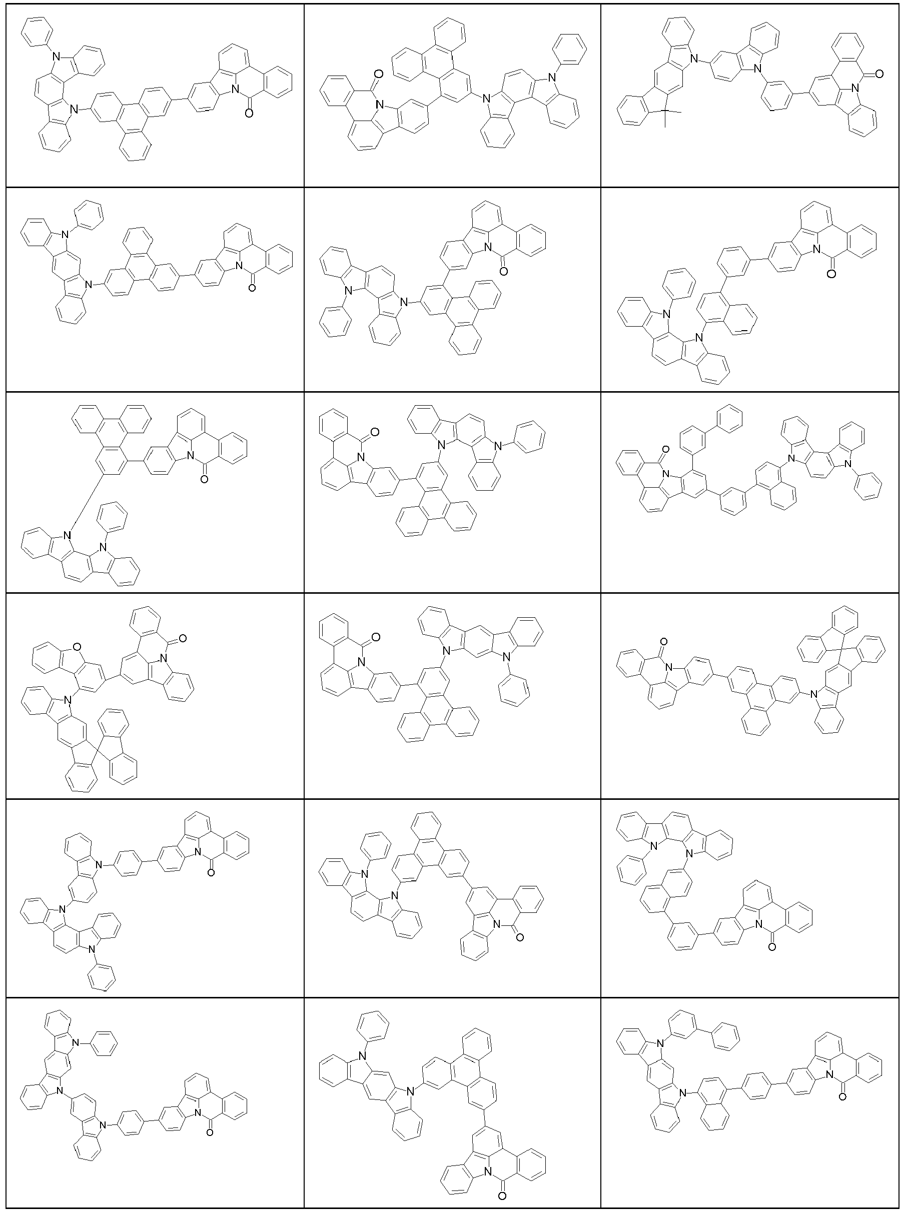

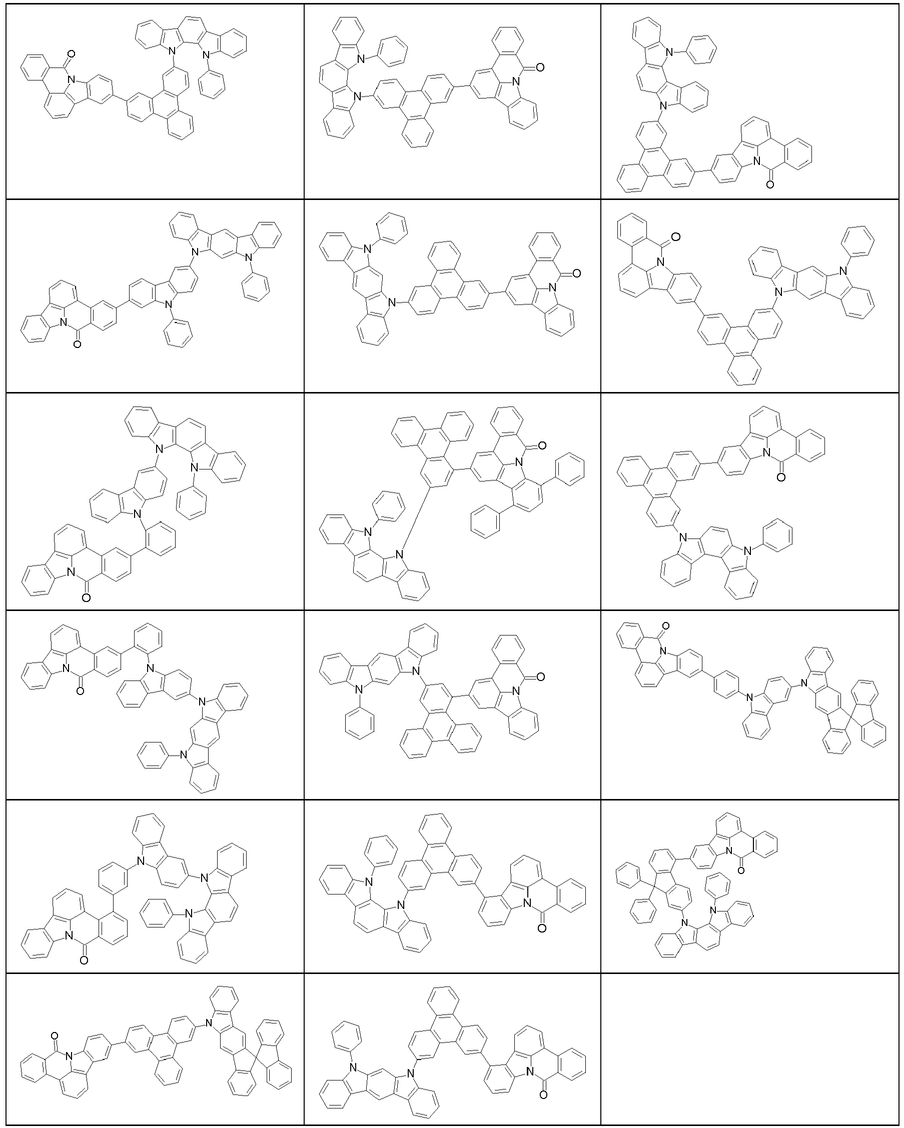

Die Grundstruktur der erfindungsgemäßen Verbindungen kann nach dem in Schema 1 bis 3 skizzierten Weg dargestellt werden.

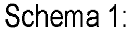

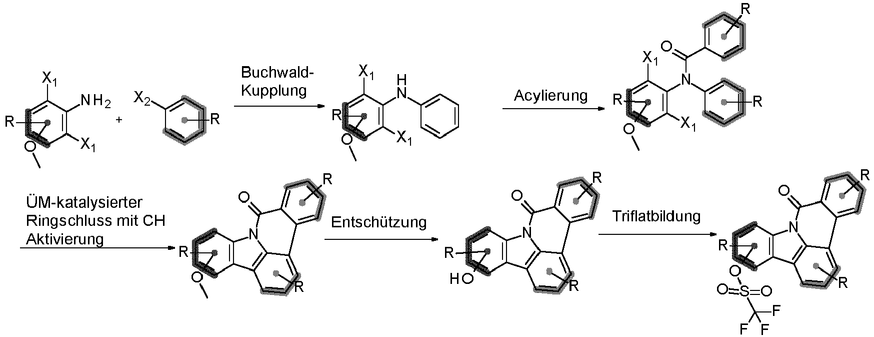

Analog können auch die beiden anderen Grundstrukturen dargestellt werden (Schema 2 und 3).

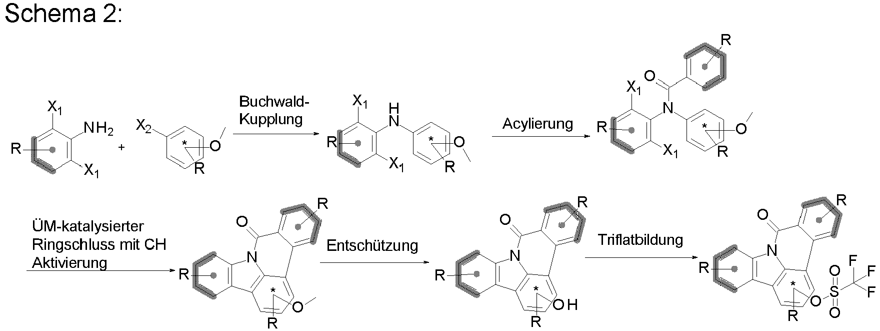

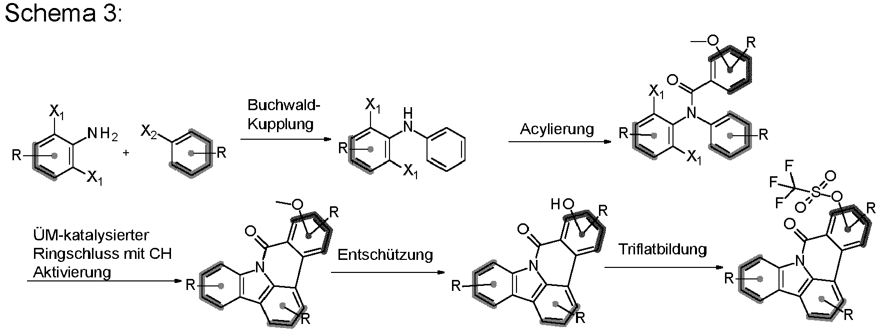

Dabei gilt in Schema 1 bis 3 X₁ = Cl oder Br und X₂ = Br oder I, mit der Bedingung, dass für X₁ = Cl X₂ = Br ist und dass für X₁ = Br X₂ = I ist. Ein weiterer Gegenstand der vorliegenden Erfindung ist daher ein Verfahren zur Herstellung der erfindungsgemäßen Verbindungen, gekennzeichnet durch die folgenden Syntheseschritte:
a) Synthese des Lactamgrundgerüsts, welches statt der Gruppe Y eine reaktive Abgangsgruppe trägt, beispielsweise Triflat oder ein Halogen;
und
b) Einführung der Gruppe Y durch eine Kupplungsreaktion, beispielsweise eine Suzuki-Kupplung.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind. Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochblockierschicht und/oder in einer Lochtransportschicht und/oder in einer Exzitonenblockierschicht eingesetzt werden.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben. Es handelt sich bei solchen Materialien bevorzugt um reine Kohlenwasserstoffe. Beispiele für solche Materialien finden sich beispielsweise in WO 2006/130598, WO 2009/021126, WO 2009/124627 und WO 2010/006680.

Bevorzugte Matrixmaterialien, welche als Mischung zusammen mit den erfindungsgemäßen Verbindungen eingesetzt werden können, sind die Verbindungen der folgenden Formeln (7) und (8), 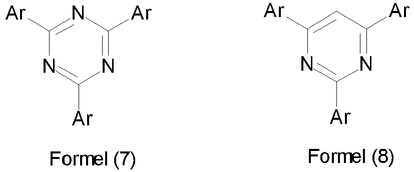 wobei Ar gleich oder verschieden bei jedem Auftreten für ein aromatisches oder heteroaromatisches Ringsystem mit 5 bis 40 aromatischen Ringatomen steht, welches mit einem oder mehreren Resten R substituiert sein kann, wobei R die oben genannten Bedeutungen aufweist.

Besonders bevorzugt sind die Triazinderivate der Formel (7).

In einer bevorzugten Ausführungsform der Erfindung sind die Gruppe Ar bei jedem Auftreten gleich oder verschieden ausgewählt aus der Gruppe bestehend aus einem aromatischen oder heteroaromatischen Ringsystem mit 6 bis 30 aromatischen Ringatomen, das jeweils durch einen oder mehrere Reste R substituiert sein kann. Besonders bevorzugt sind die Gruppen Ar bei jedem Auftreten gleich oder verschieden ausgewählt aus der Gruppe bestehend aus einem aromatischen oder heteroaromatischen Ringsystem mit 6 bis 24 aromatischen Ringatomen, bevorzugt mit 6 bis 20 aromatischen Ringatomen, das jeweils durch einen oder mehrere Reste R, bevorzugt nicht-aromatische Reste R, substituiert sein kann.

Geeignete aromatische bzw. heteroaromatische Ringsysteme Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, welches über die 1- oder 2-Position verknüpft sein kann, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Chinazolin, Benzimidazol, Phenanthren, Triphenylen oder einer Kombination aus zwei oder drei dieser Gruppen, welche jeweils mit einem oder mehreren Resten R substituiert sein können.

Dabei sind die Gruppen Ar bevorzugt gewählt aus den Gruppen der folgenden Formeln Ar-1 bis Ar-83, 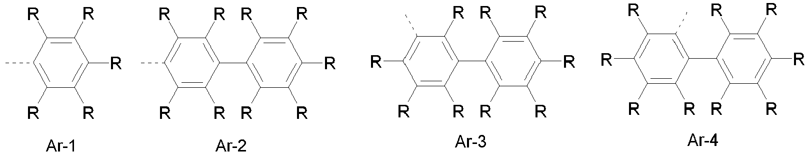 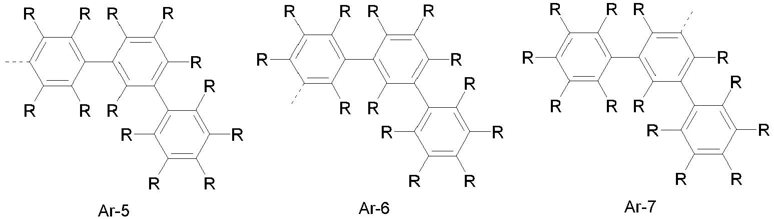 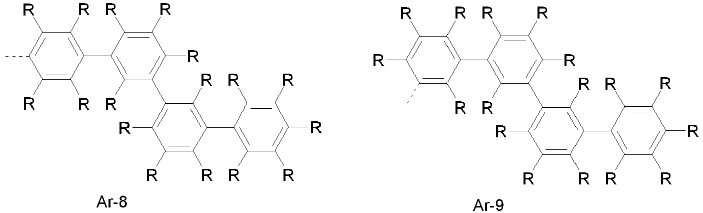 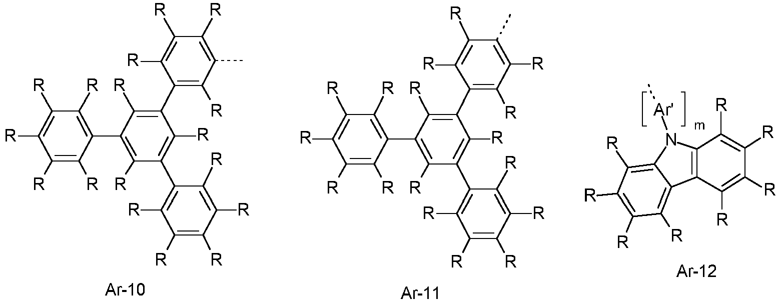 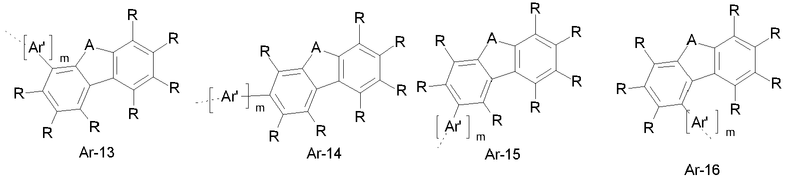 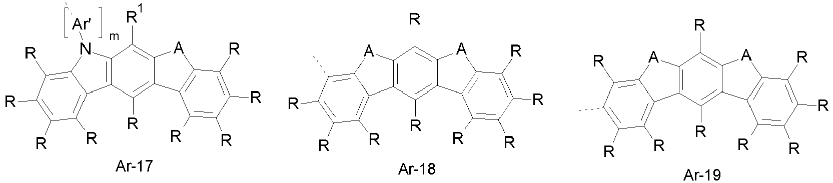 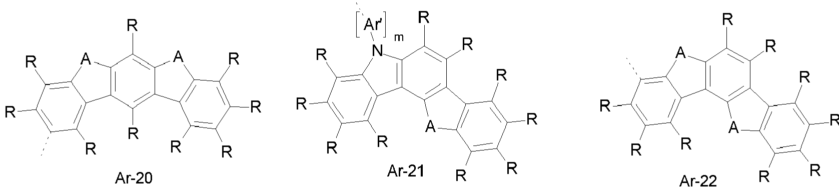 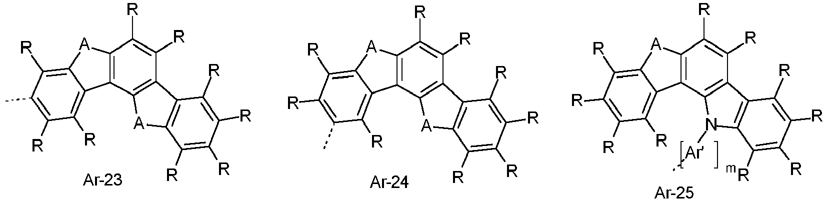 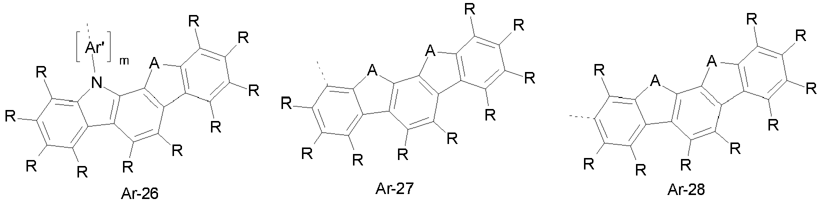 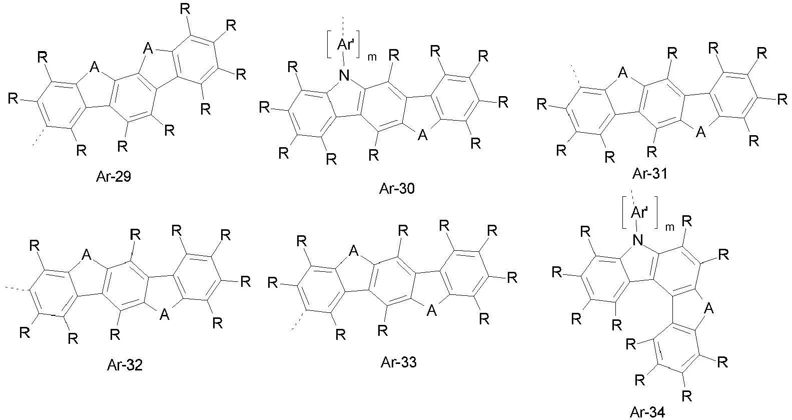 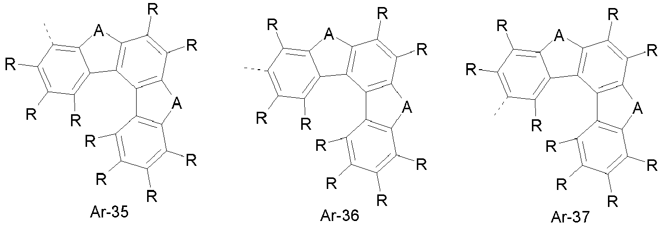 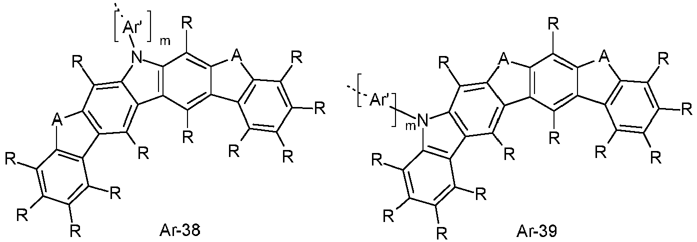 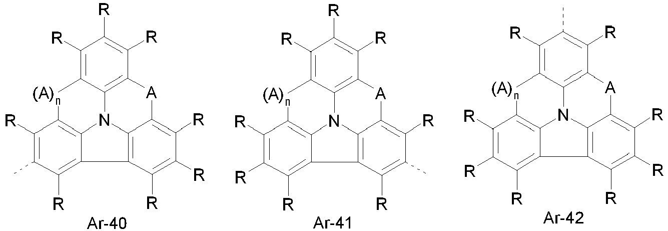 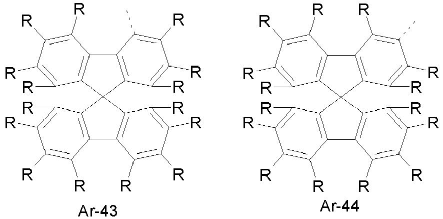 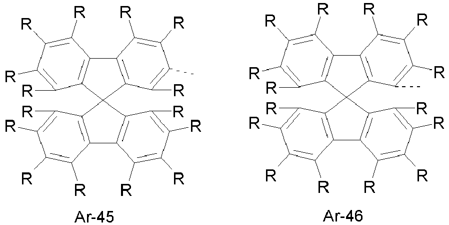 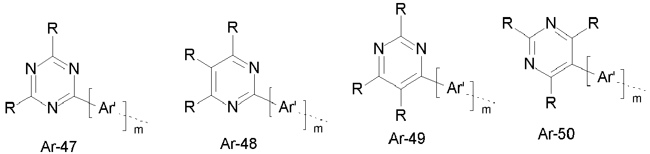 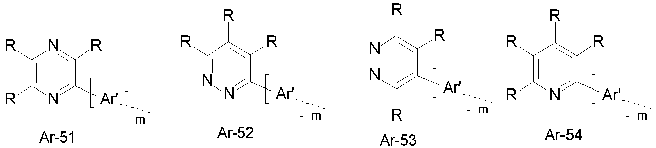 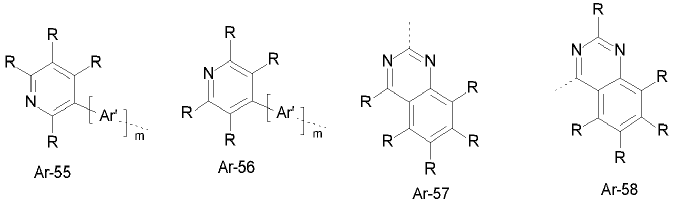 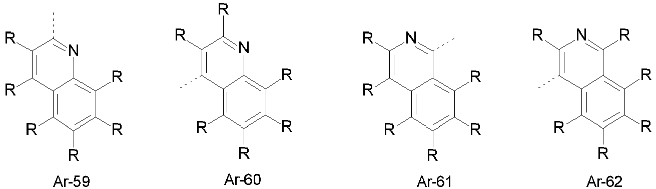 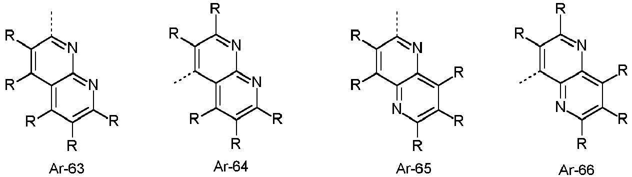 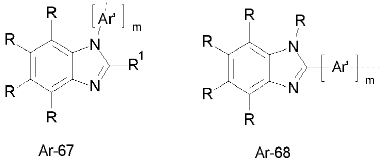 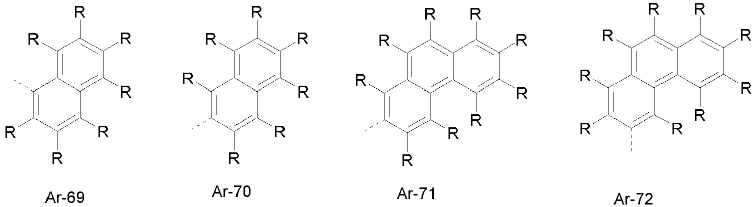 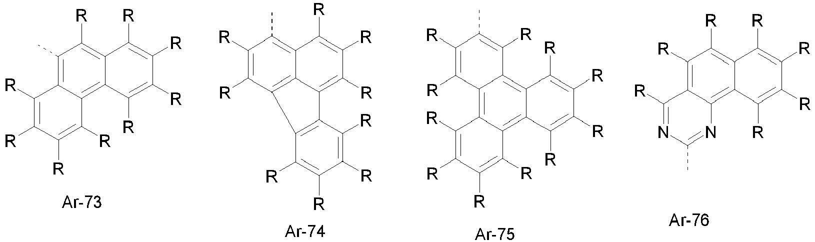 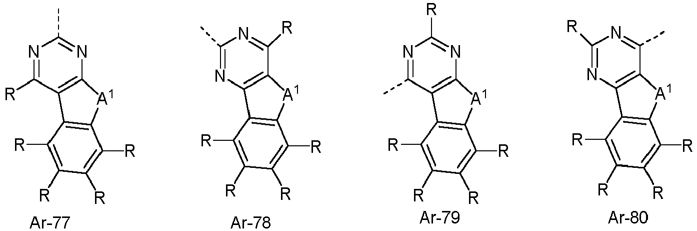 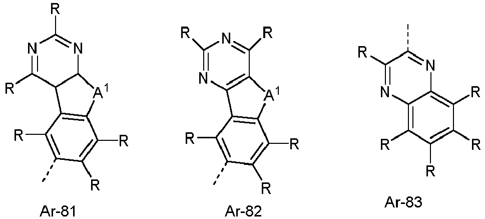 wobei R die oben genannten Bedeutungen aufweist, die gestrichelte Bindung die Bindung an das Triazin bzw. Pyrimidin darstellt und weiterhin gilt:
- Ar': ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- A: ist bei jedem Auftreten gleich oder verschieden CR₂, NR, O oder S;
- n: ist 0 oder 1, wobei n = 0 bedeutet, dass an dieser Position keine Gruppe A gebunden ist und an die entsprechenden Kohlenstoffatome statt dessen Reste R gebunden sind;
- m: ist 0 oder 1, wobei m = 0 bedeutet, dass die Gruppe Ar' nicht vorhanden ist und dass die entsprechende aromatische bzw. heteroaromatische Gruppe direkt an das Triazin bzw. Pyrimidin gebunden ist.

In der folgenden Tabelle sind Beispiele für geeignete elektronentransportierende Matrixmaterialien abgebildet, die zusammen mit den erfindungsgemäßen Materialien verwendet werden können.

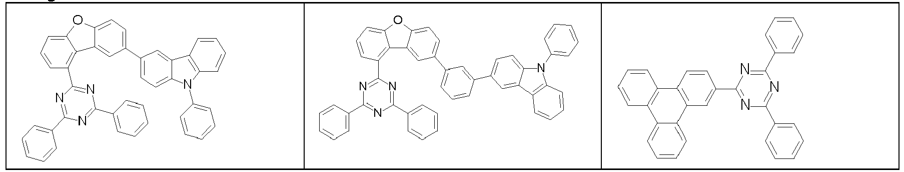

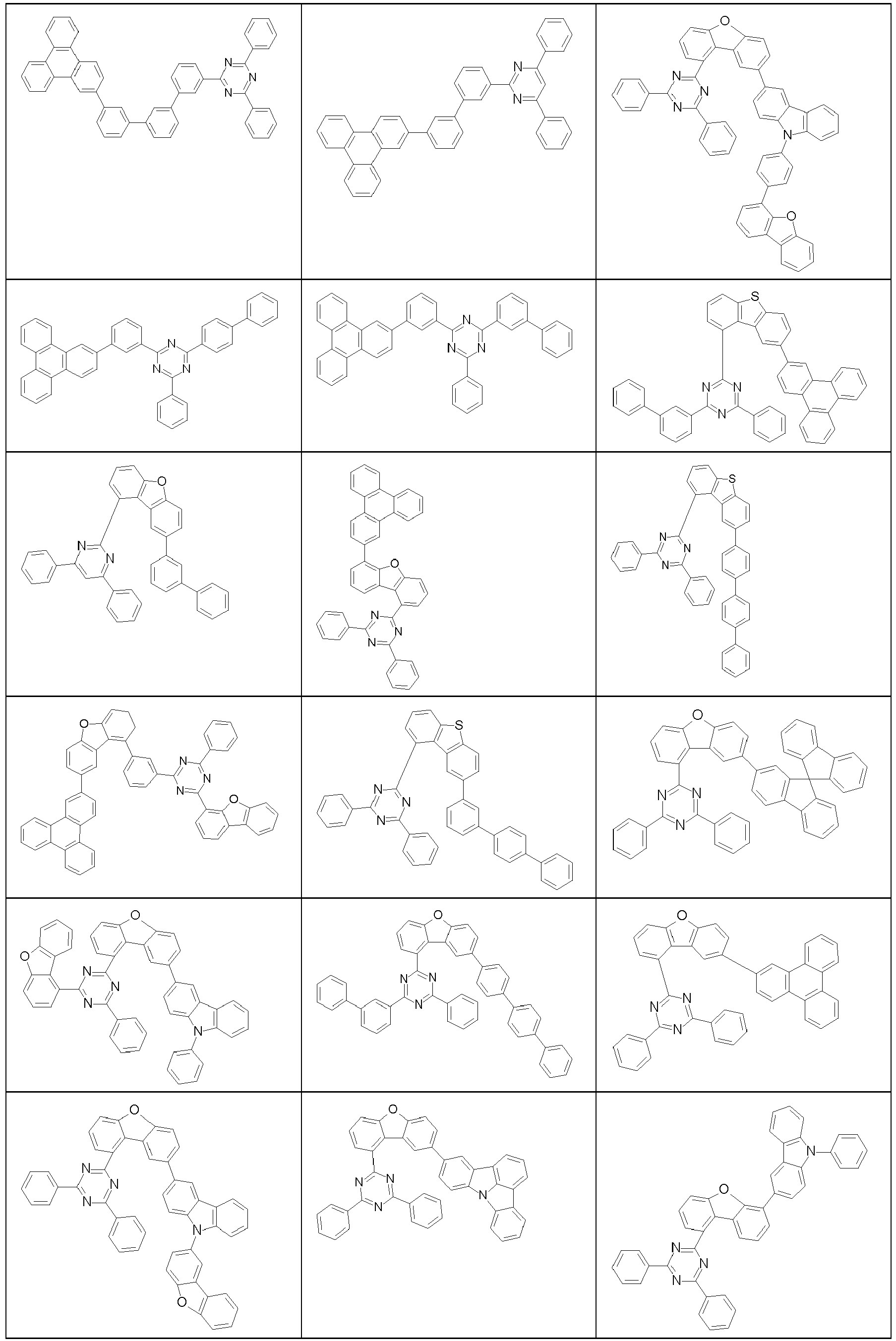

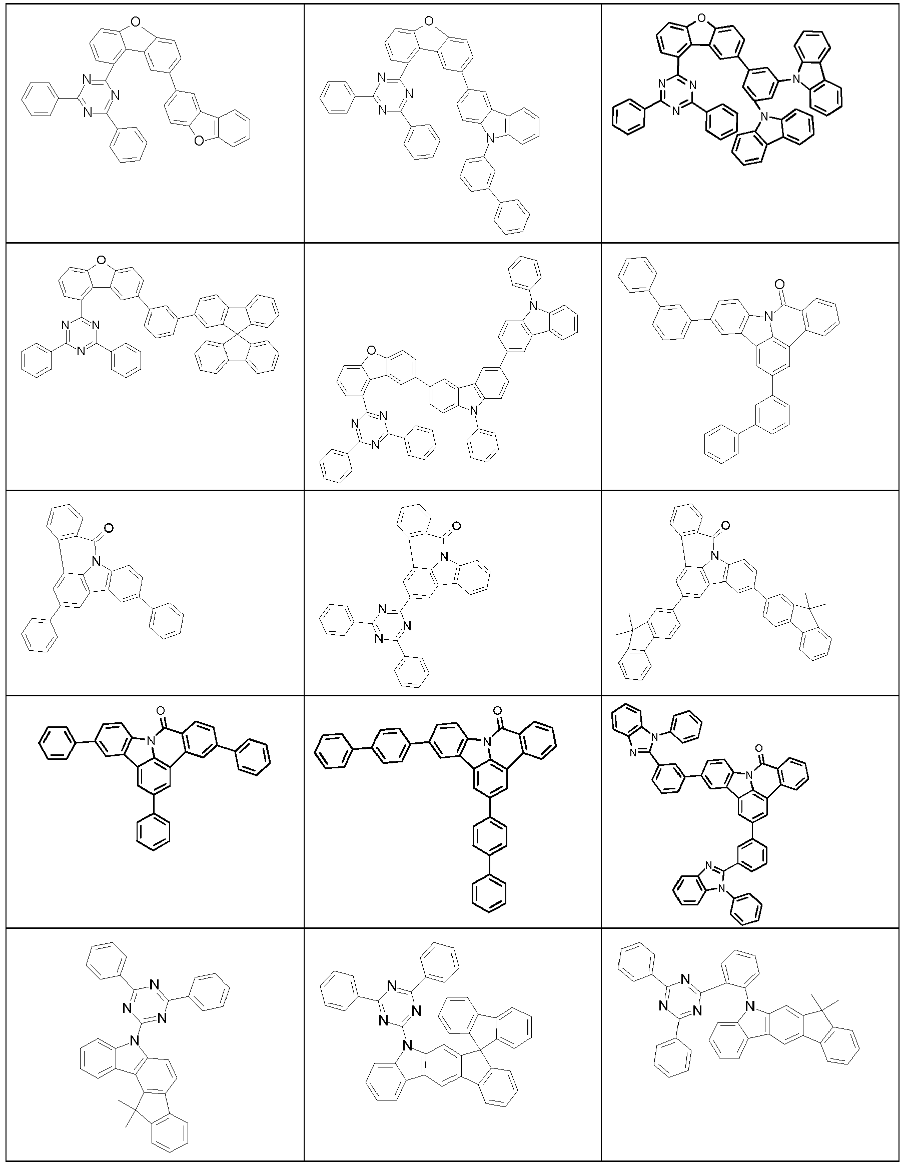

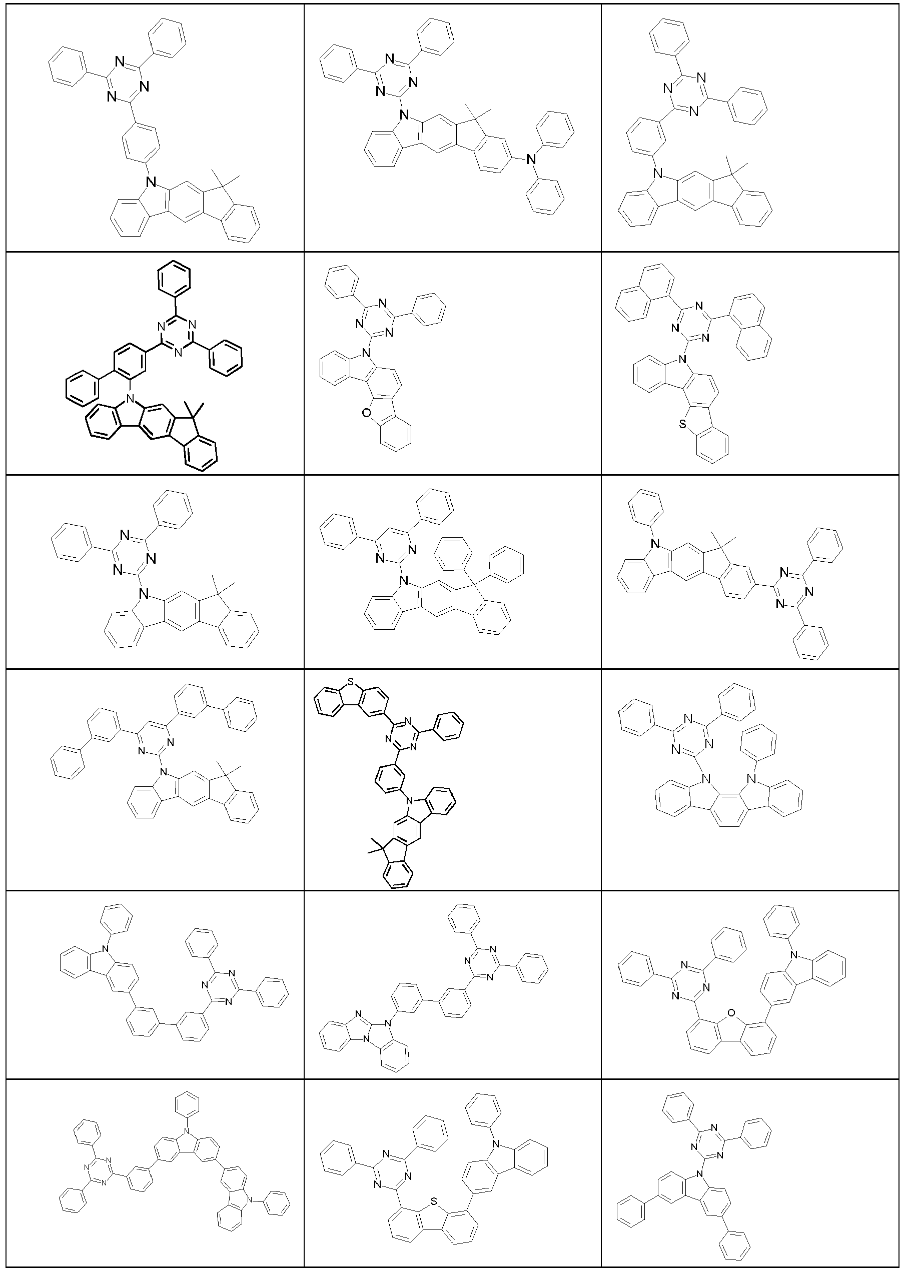

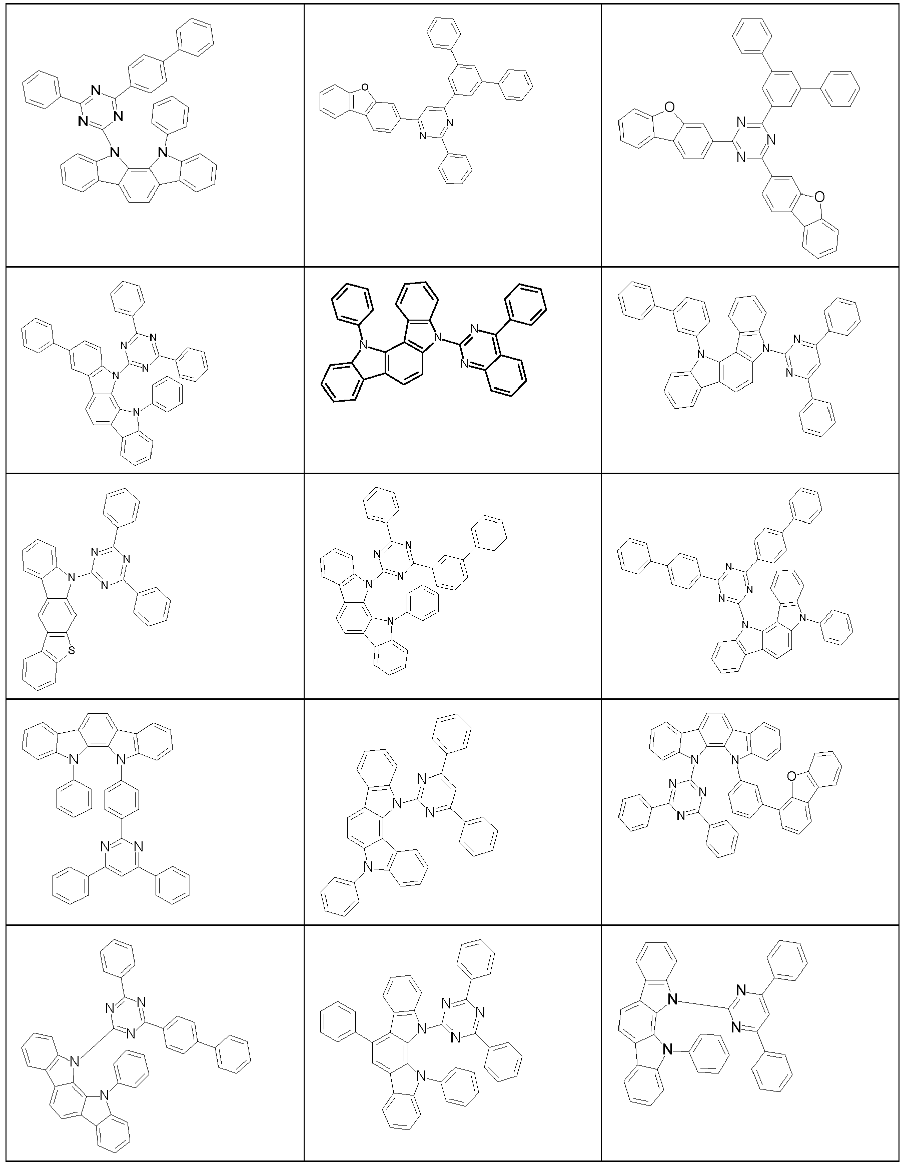

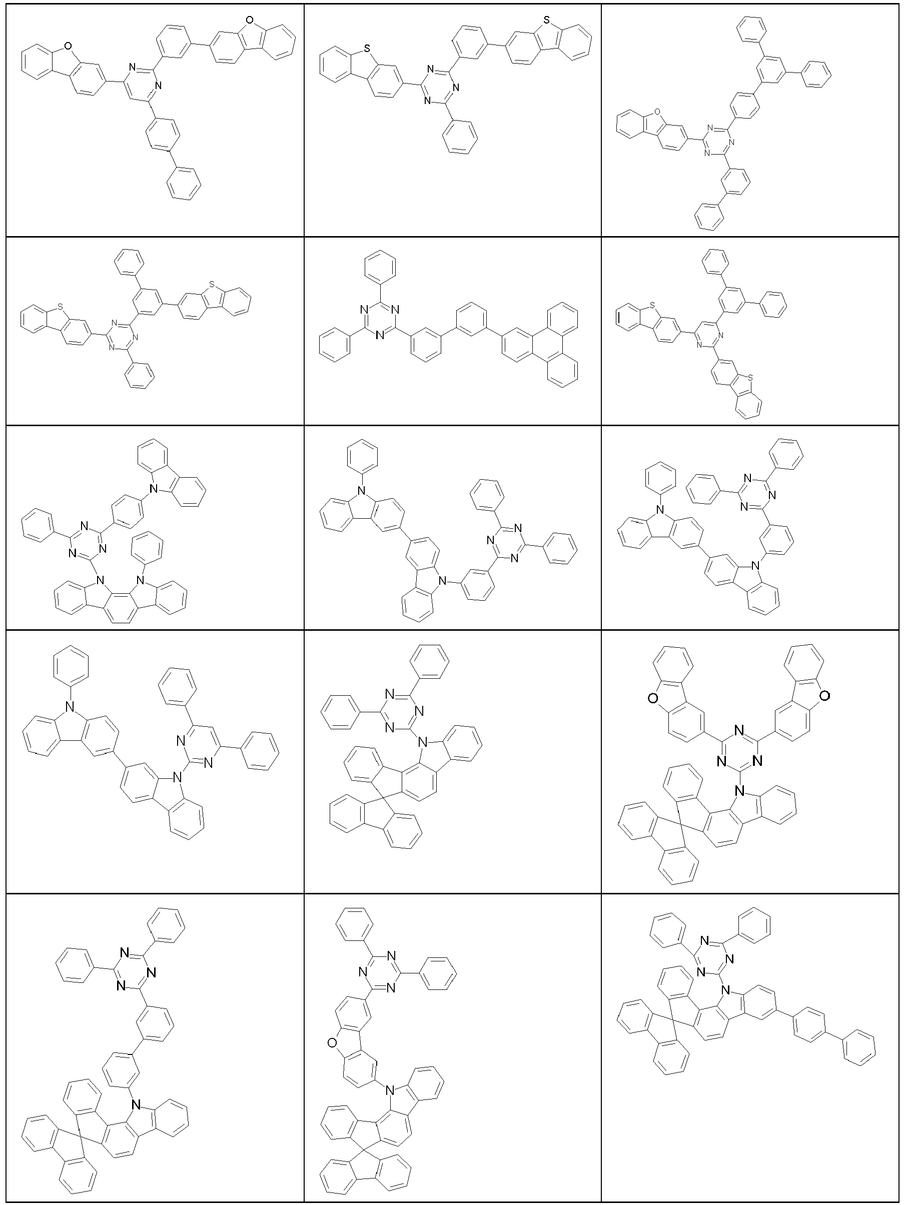

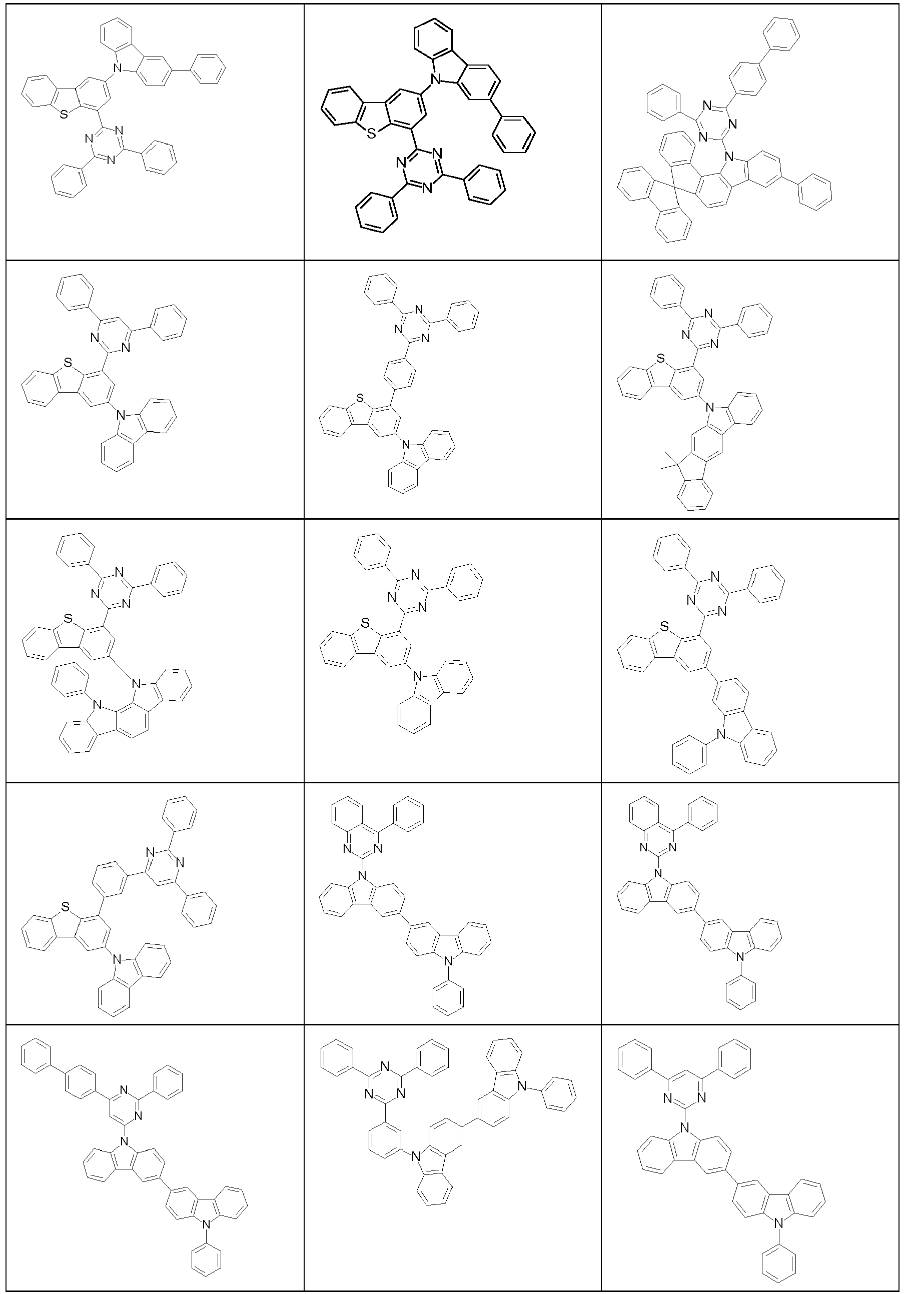

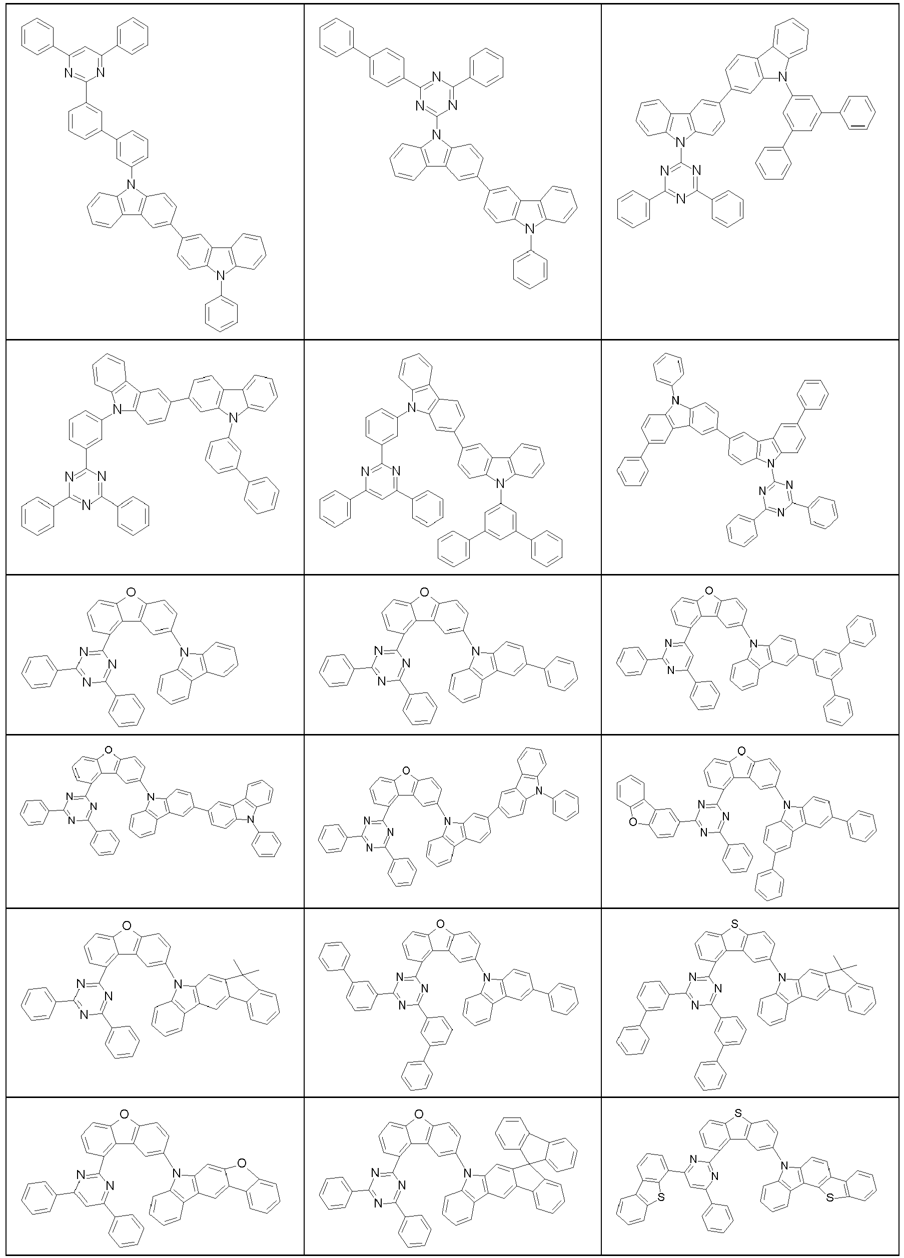

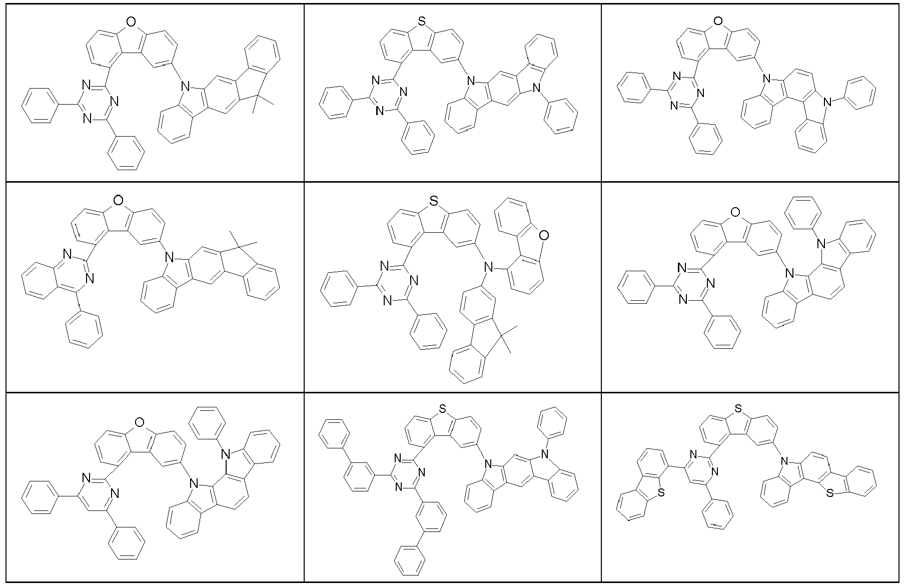

In der folgenden Tabelle sind Beispiele für geeignete lochtransportierende Matrixmaterialien abgebildet, die zusammen mit den erfindungsemäßen Materialien verwendet werden können.

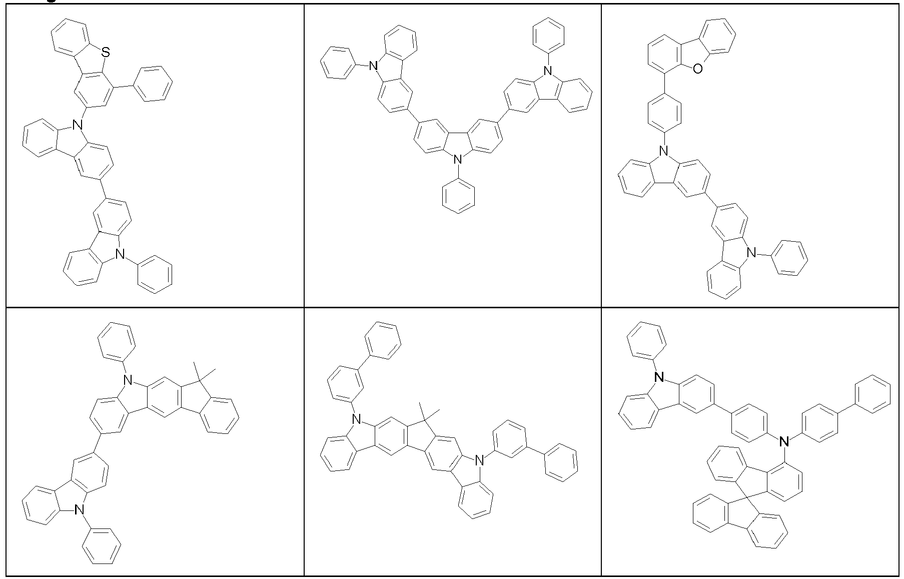

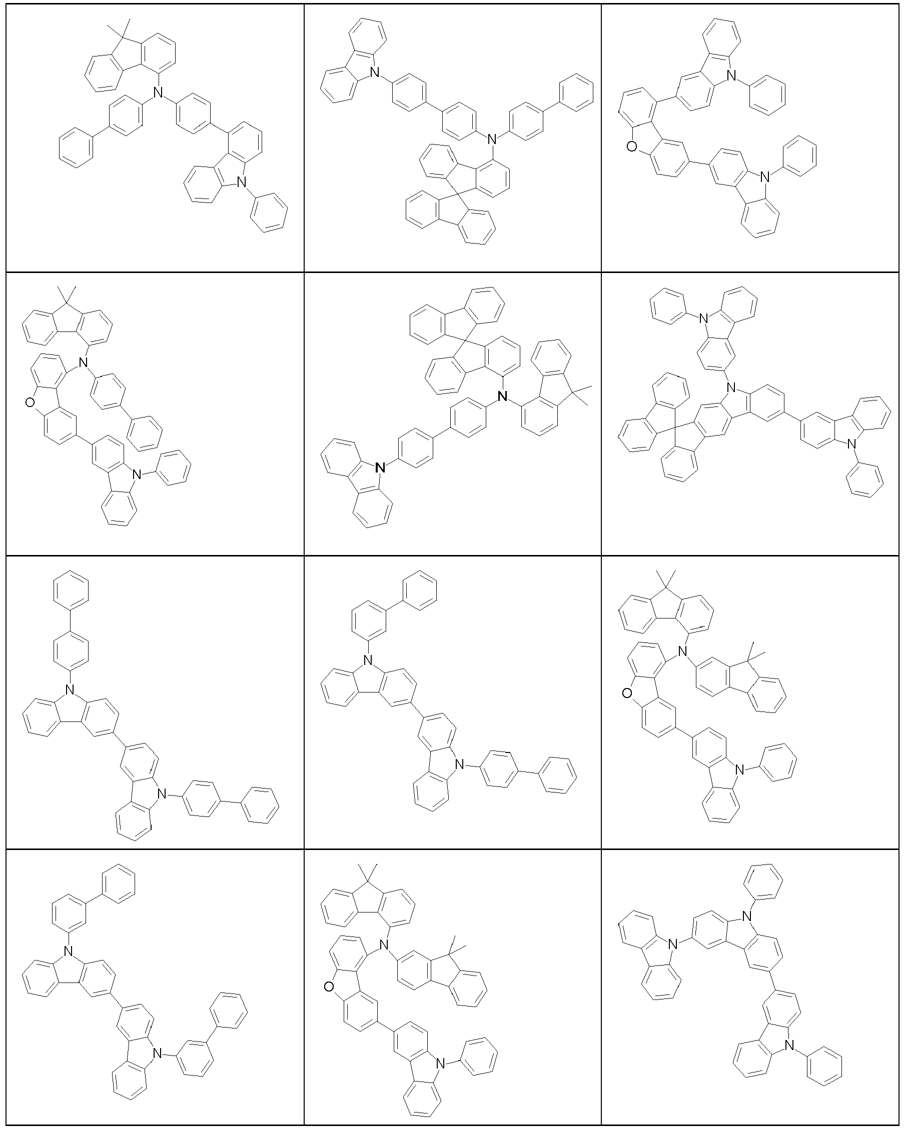

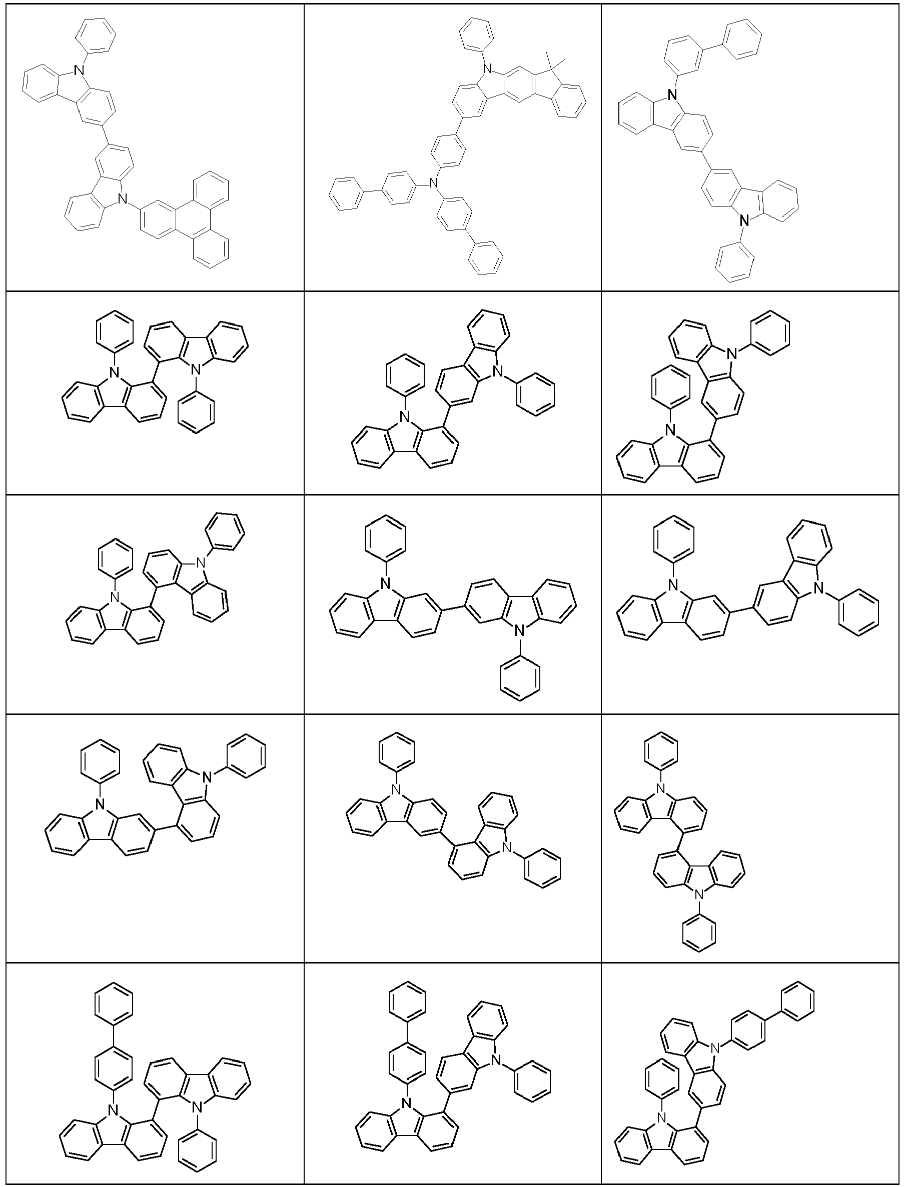

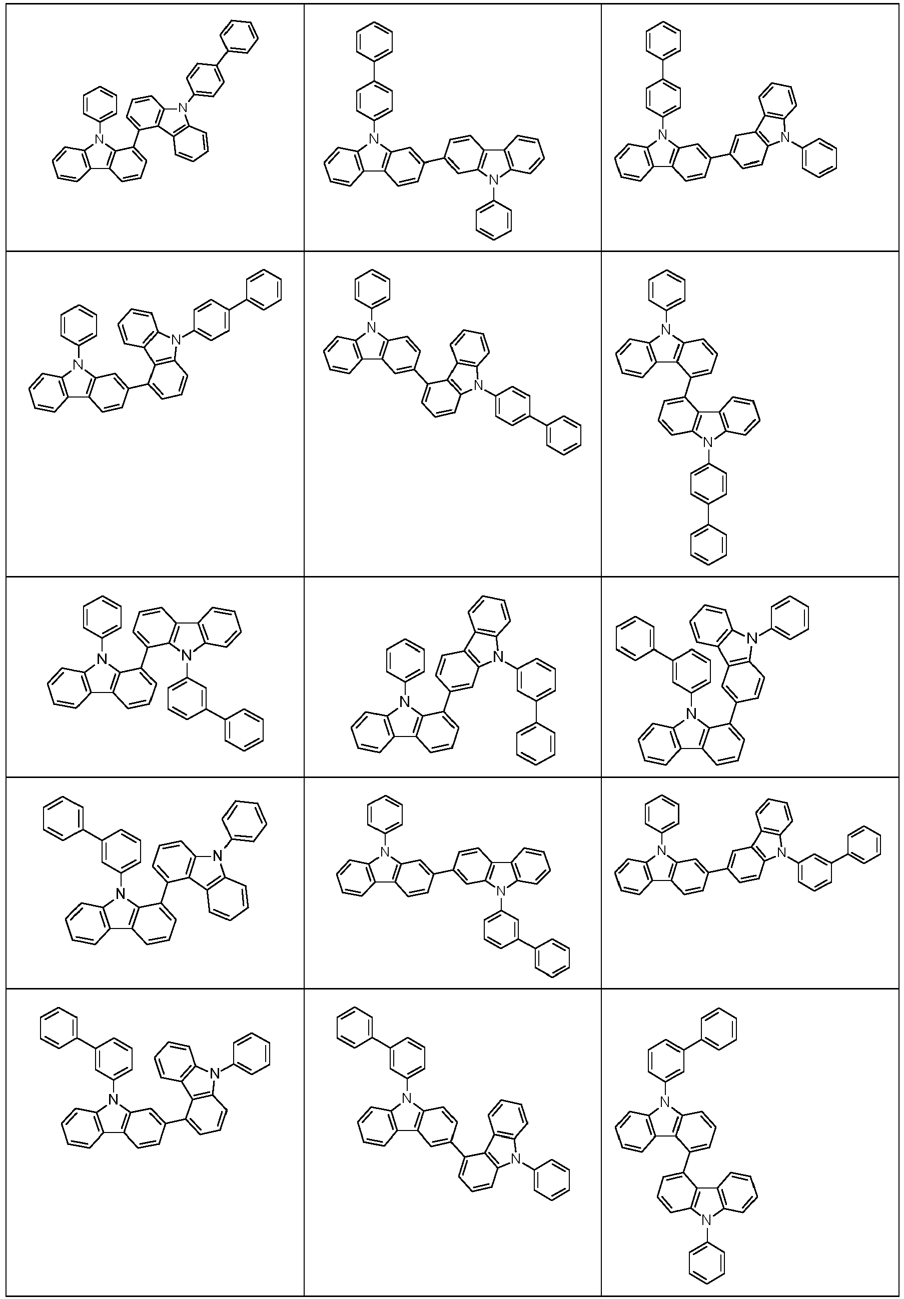

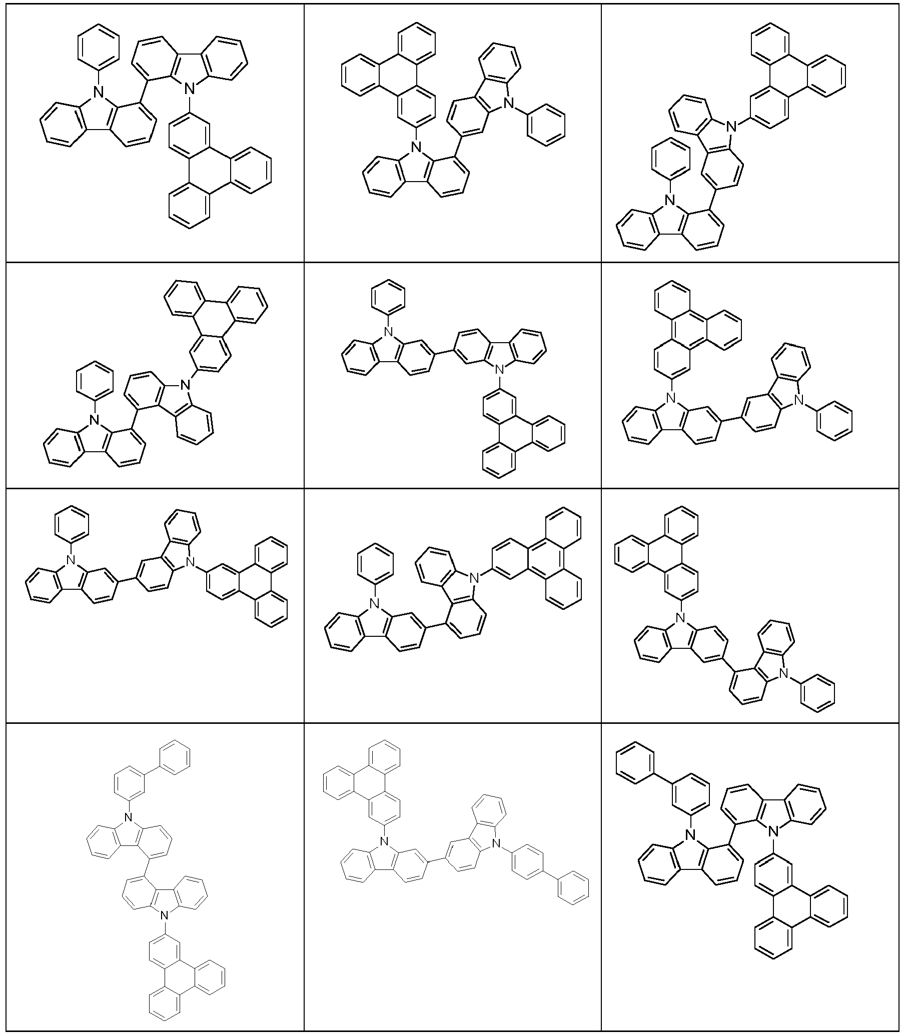

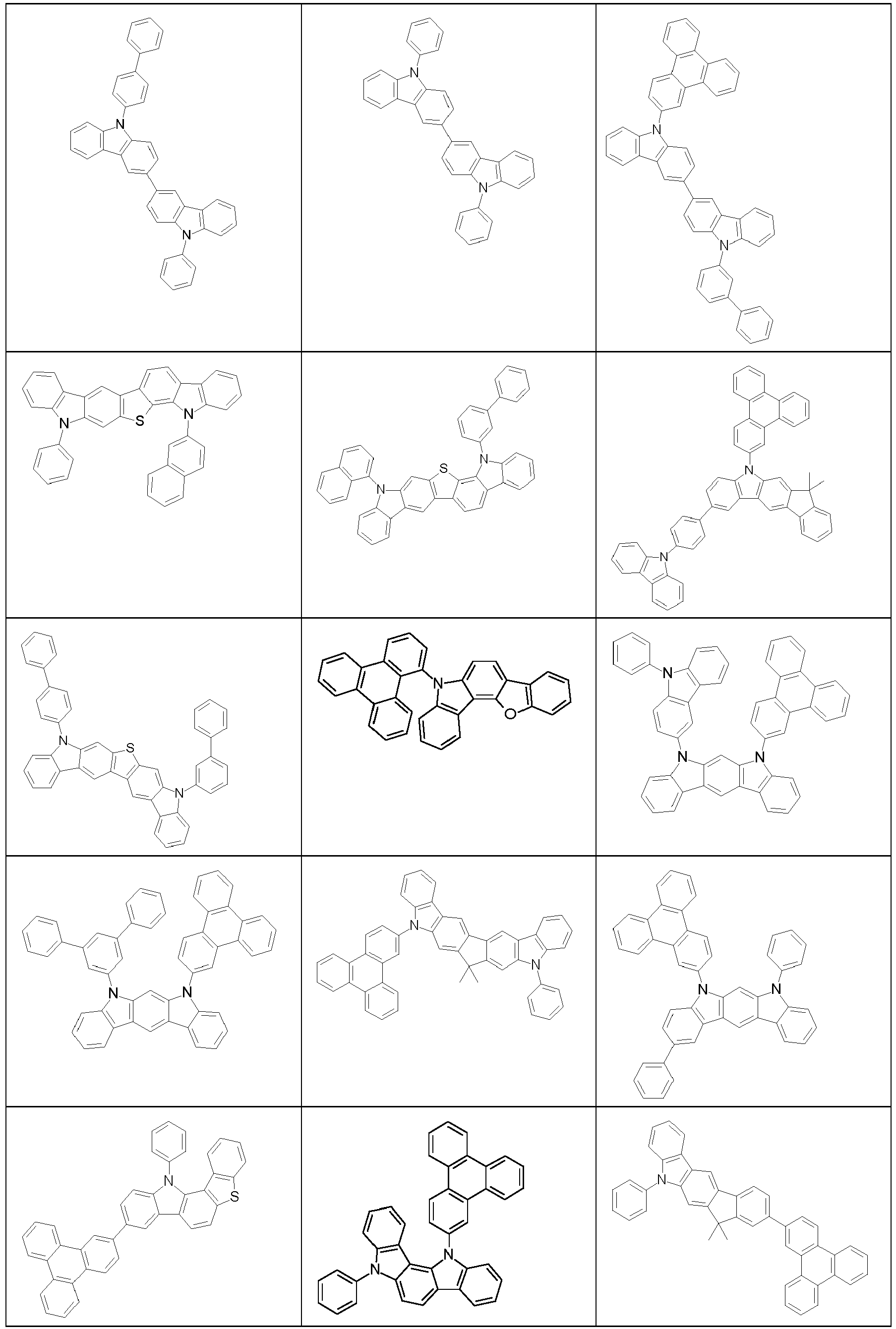

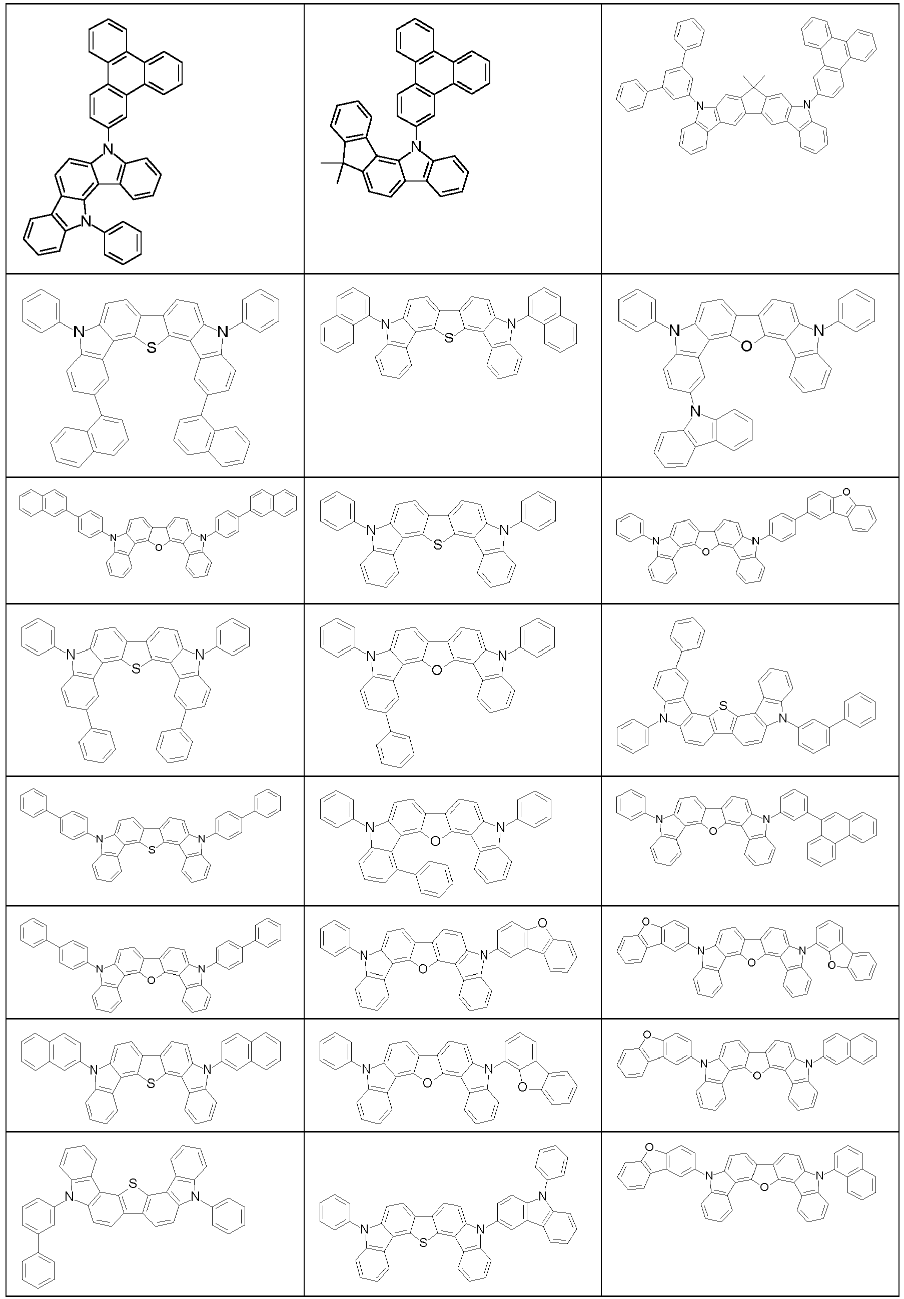

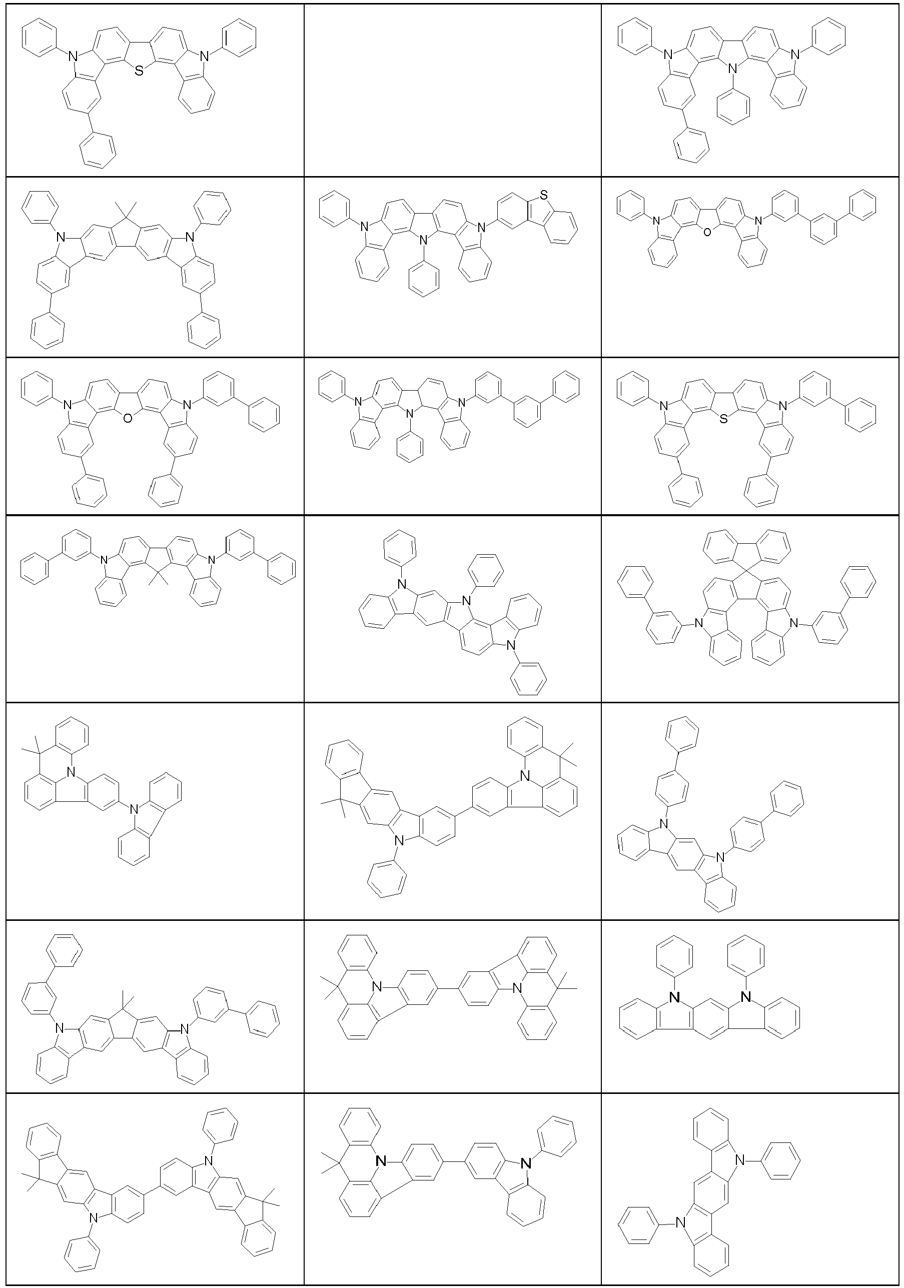

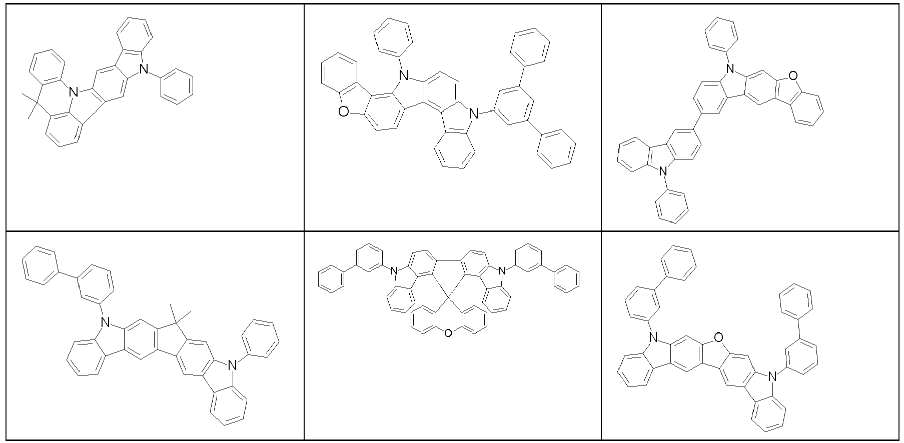

In der folgenden Tabelle sind Beispiele für geeignete Wide Bandgap Matrixmaterialien abgebildet, die zusammen mit den erfindungsgemäßen Materialien verwendet werden können.

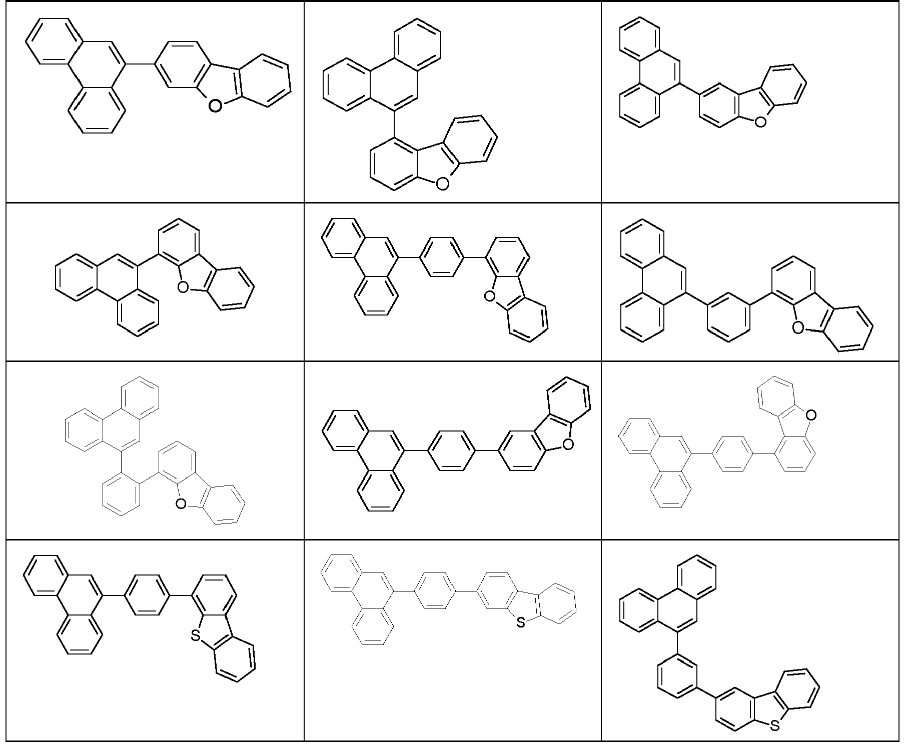

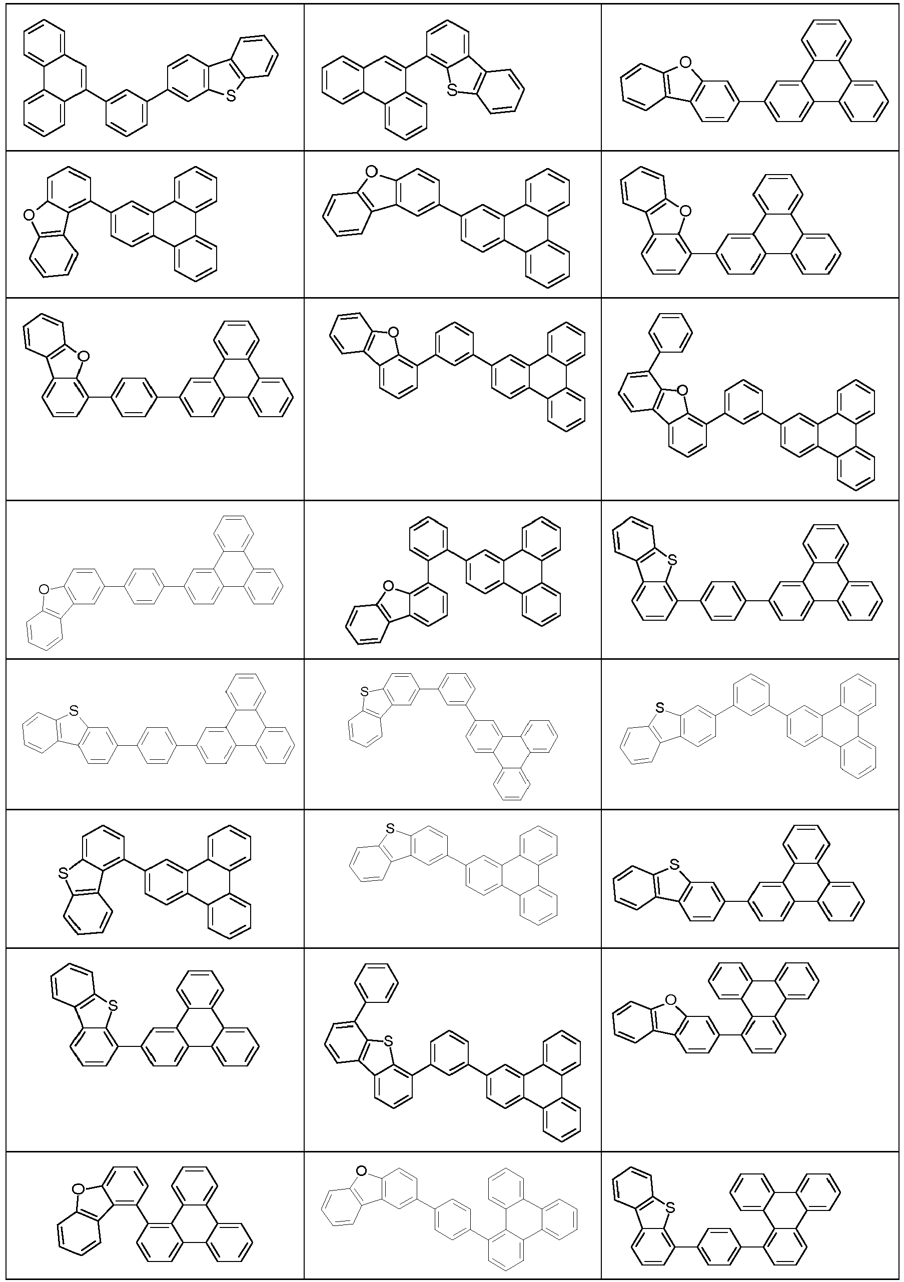

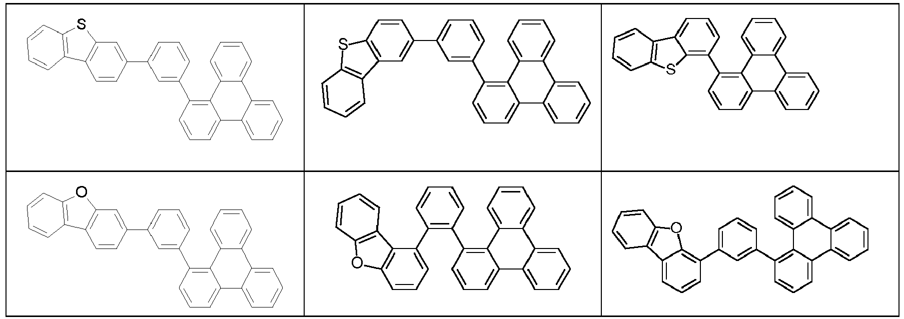

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2018/011186 und WO 2018/041769, sowie den noch nicht offen gelegten Patentanmeldungen EP 17182995.5, EP 17205103.9, EP 17206950.2 und EP 18156388.3 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Beispiele für phosphoreszierende Dotanden sind nachfolgend aufgeführt.

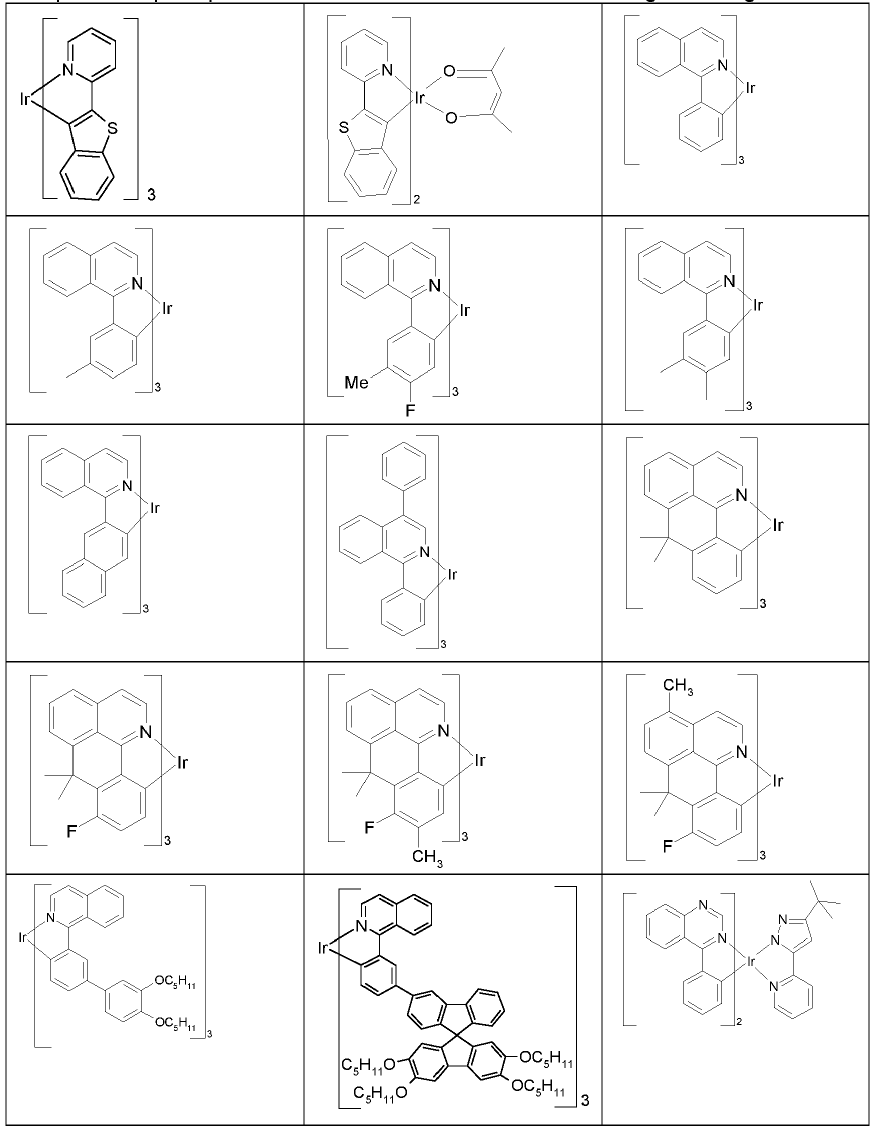

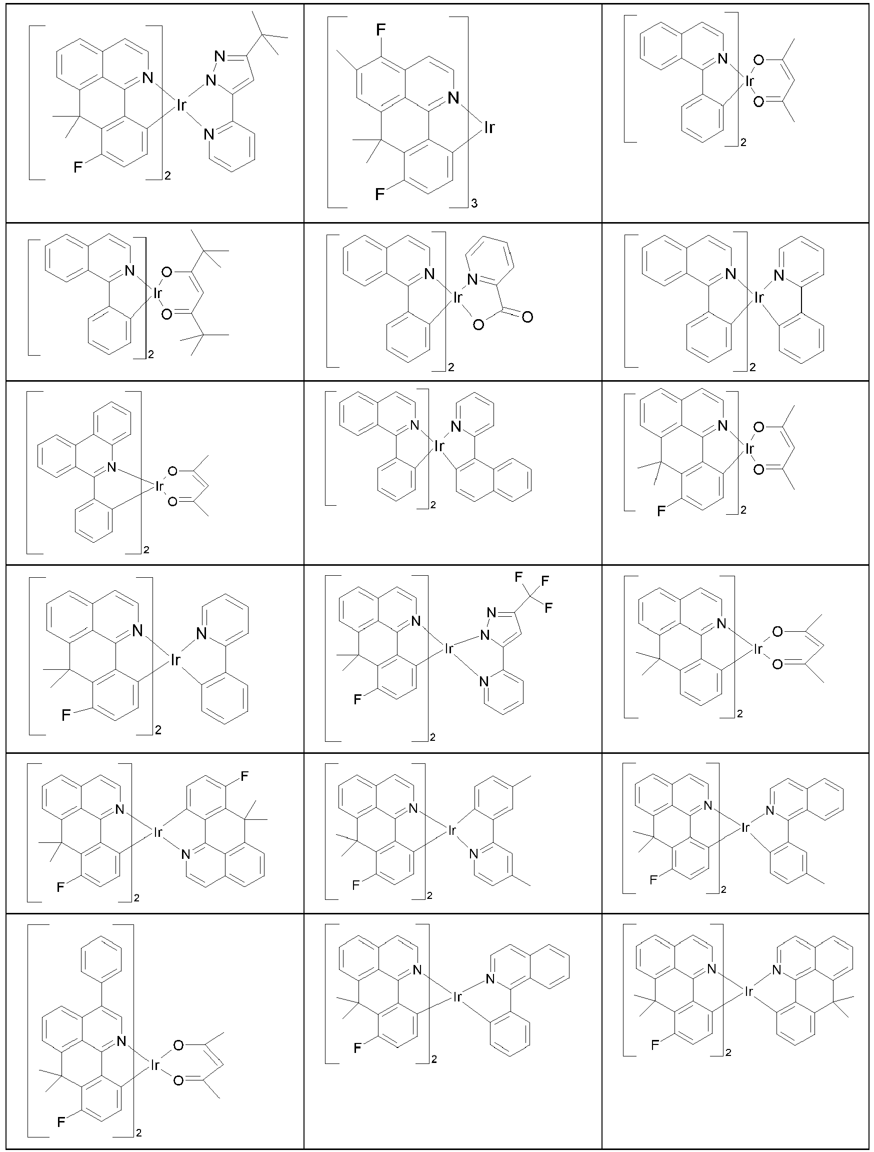

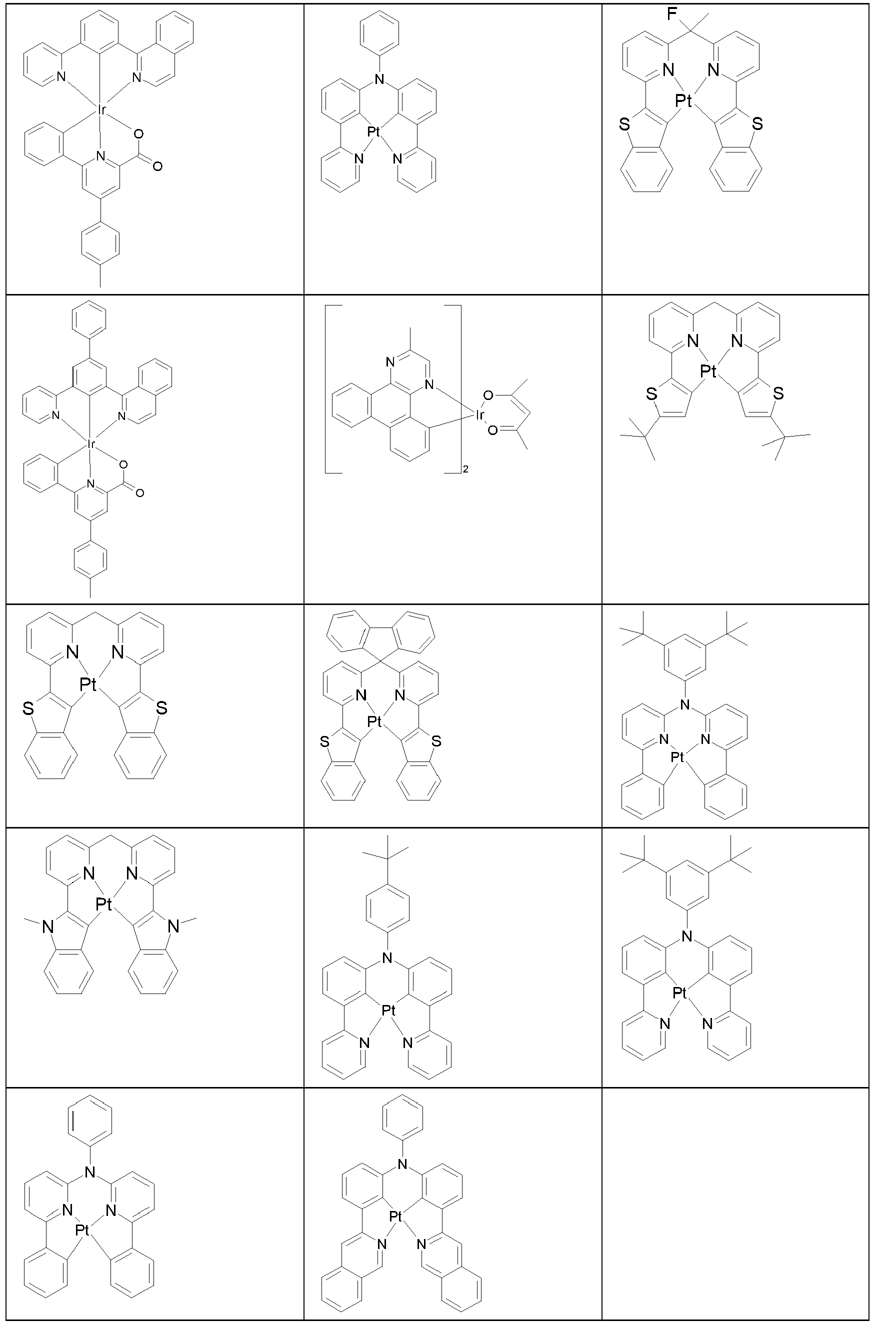

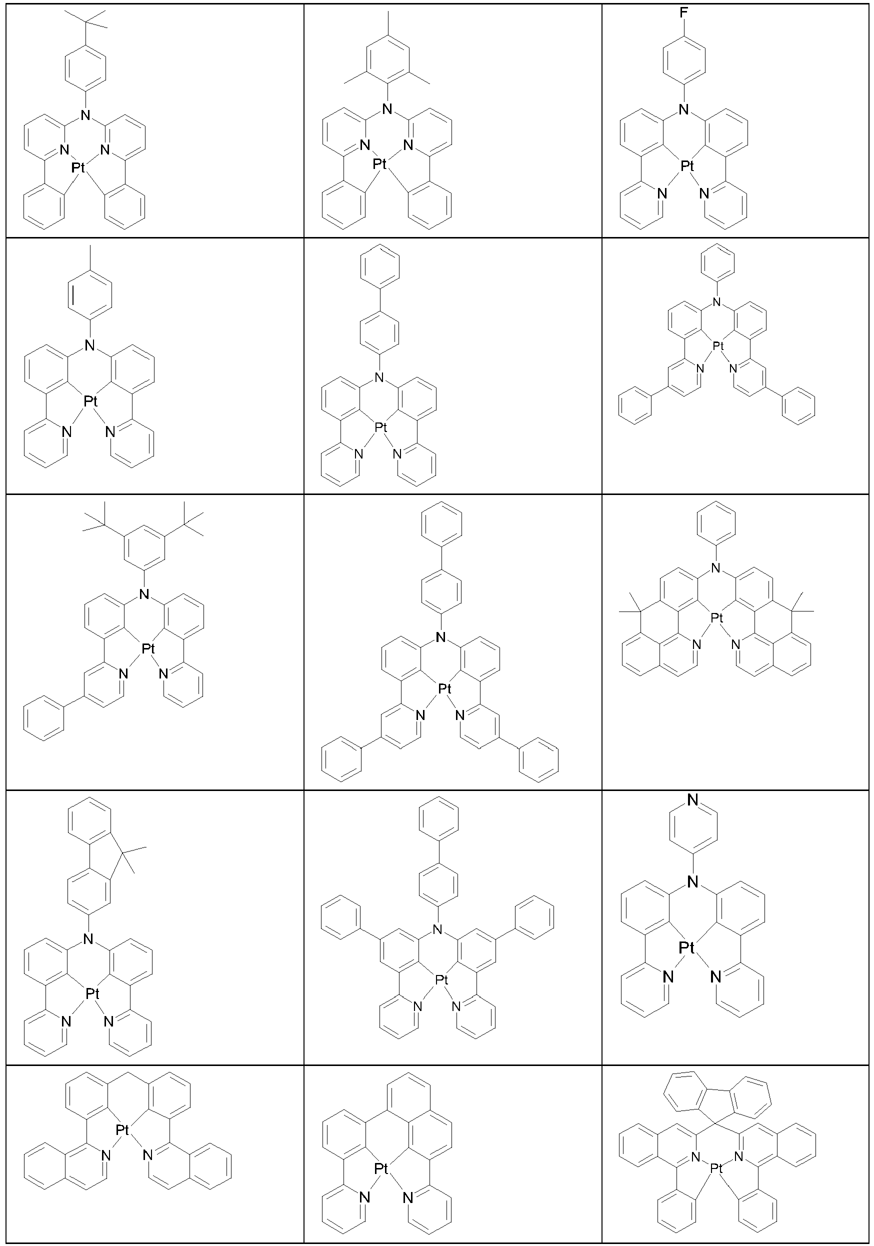

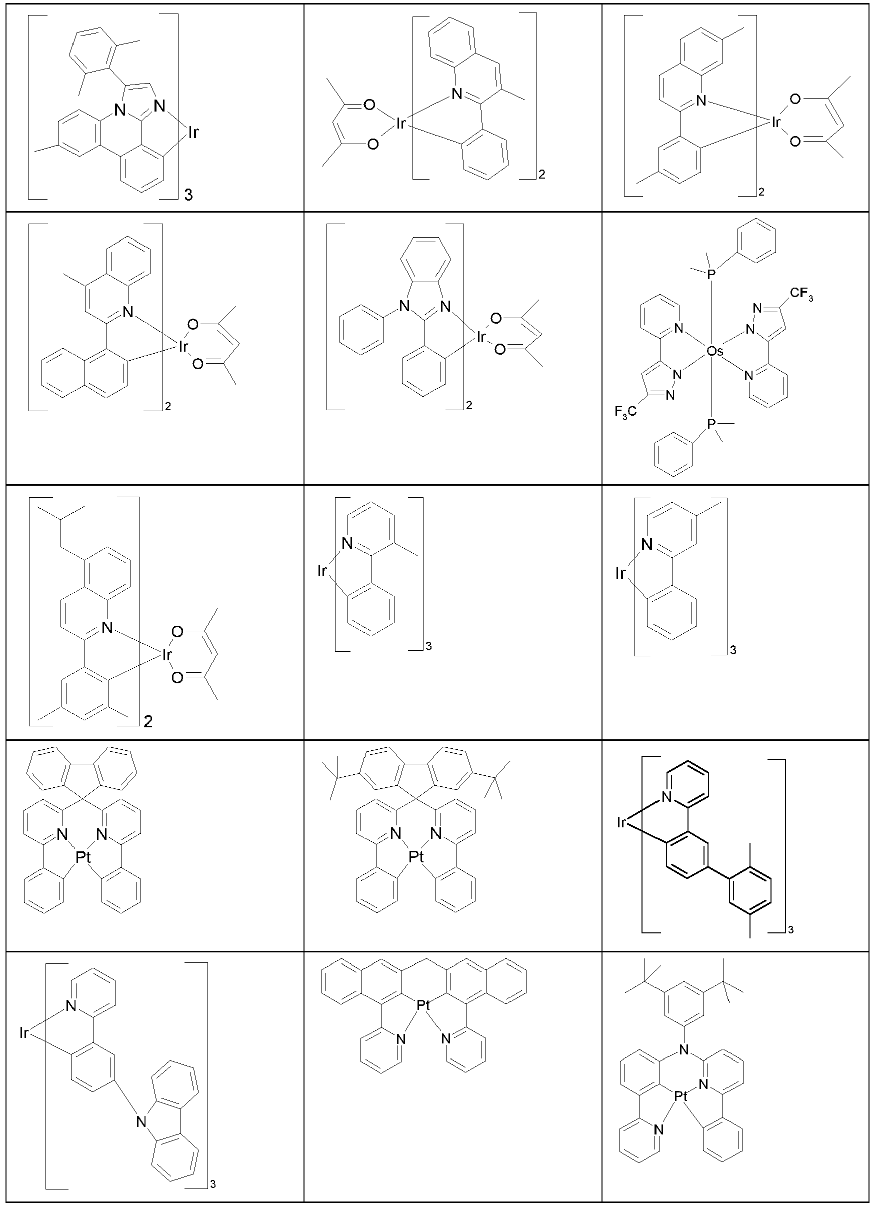

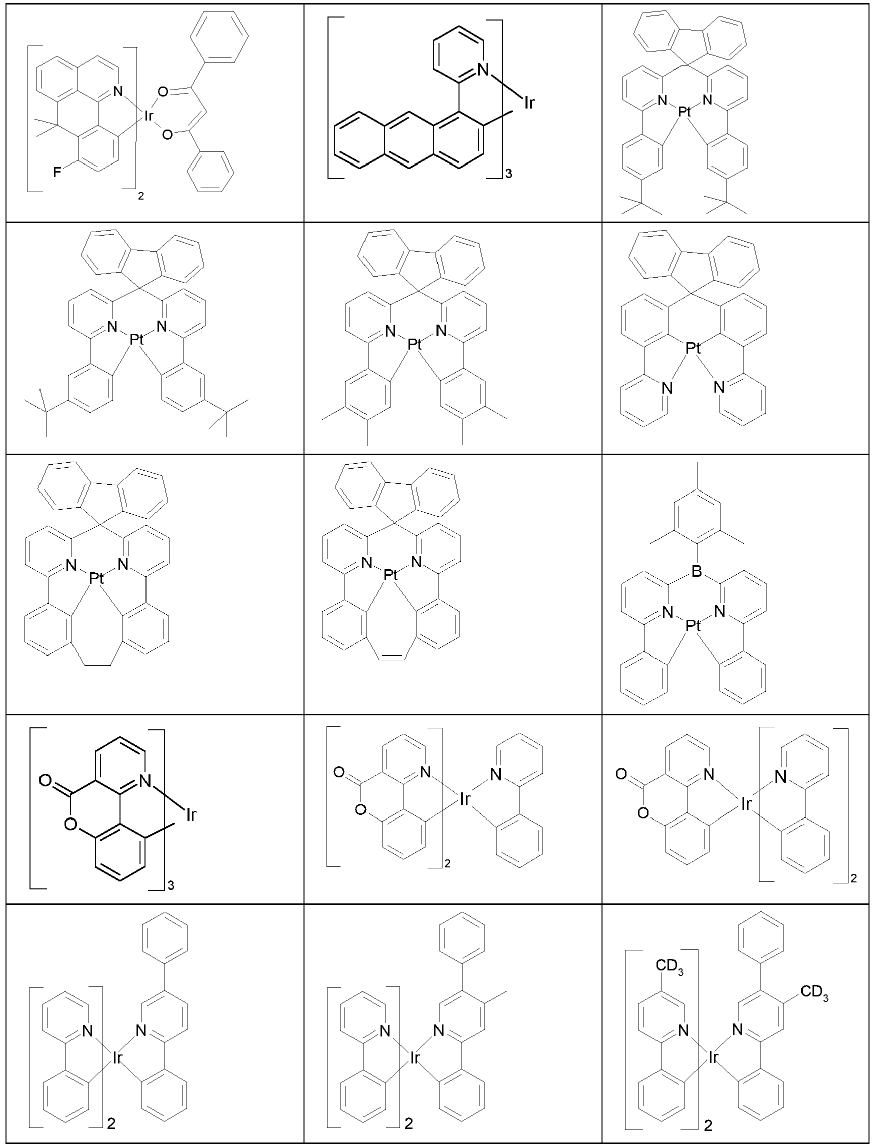

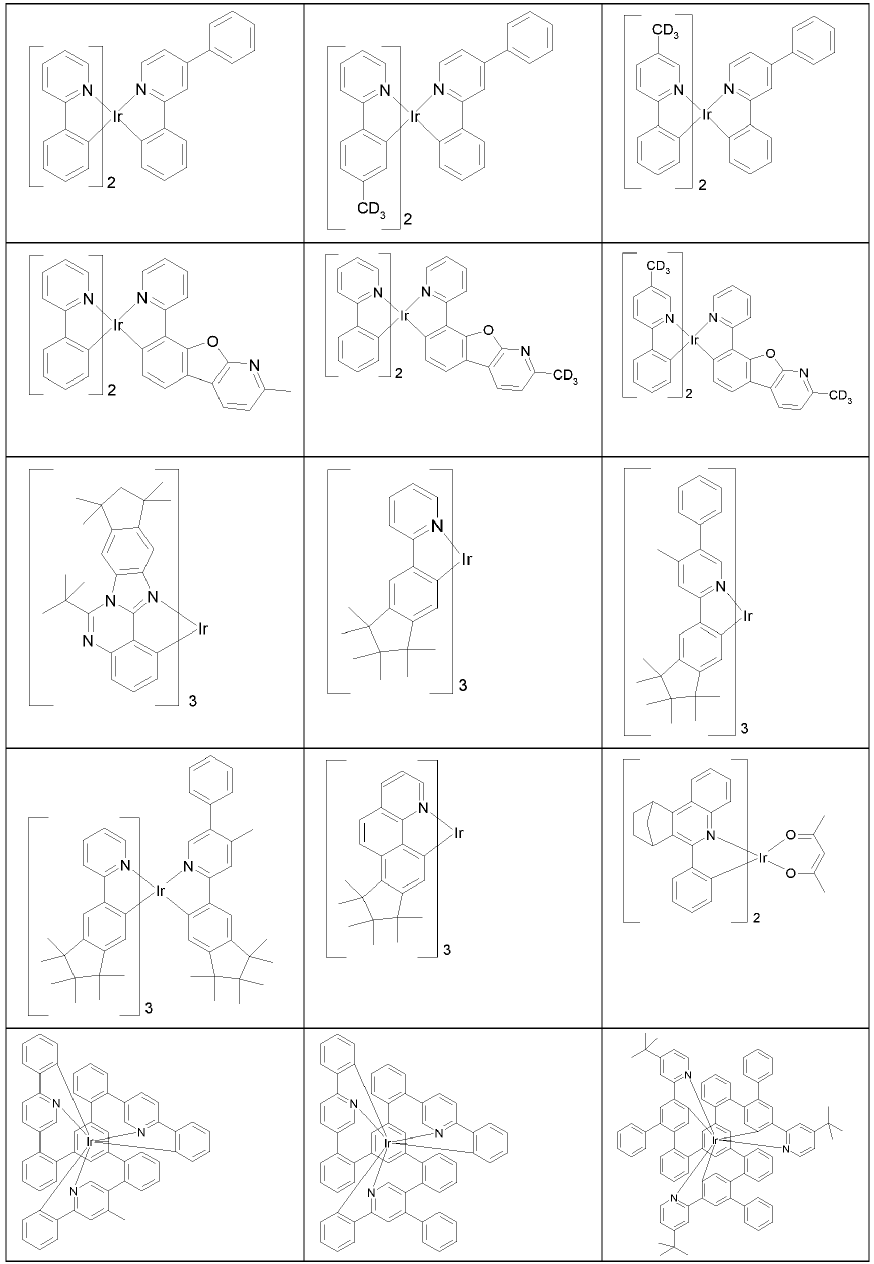

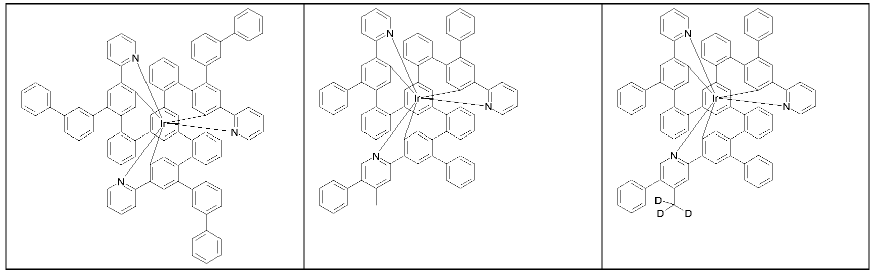

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die Verwendung der erfindungsgemäßen Verbindungen als Matrixmaterial für phosphoreszierende Emitter führt zu einer Verbesserung der Lebensdauer. Dies gilt insbesondere gegenüber dem Einsatz von Lactamen als Matrixmaterial, die zwar eine ähnliche Struktur wie die erfindungsgemäßen Verbindungen aufweisen, jedoch keine Gruppe Y enthalten.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

### Synthesebeispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Darstellung der Synthone:

**S1:**

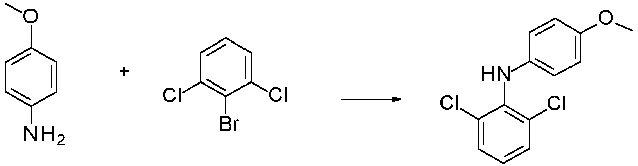

Para-Anisidin [104-94-9] (136.2 g, 1.11 mol), 1-Brom-2,6-dichlorbenzol [19393-92-1] (250.2 g, 1.11 mol) und Natrium-tert-butylat (214.7 g, 2.23 mol) werden mit Toluol (1500 mL) in einem 4 L Vierhalskolben vorgelegt und 30 min mit Argon inertisiert. Anschließend wird Pd(dppf)Cl₂ x DCM [95464-05-4] (4.51 g, 5.53 mmol) zugegeben und das Reaktionsgemisch für 18 h unter Rückfluss gerührt. Anschließend wird der Ansatz extraktiv mit Wasser und Toluol aufgearbeitet, die organische Phase über Na₂SO₄ getrocknet und über ein Kieselgelbett filtriert. Das Filtrat wird einrotiert und das erhaltene Rohprodukt über Vakuum-Destillation weiter aufgereinigt. Ausbeute: 218.3 g (813 mmol, 74%), gelber teilkristalliner öliger Feststoff.

Analog können folgende Verbindungen dargestellt werden. Hierbei kann als Katalysatorsystem statt Pd(dppf)Cl₂ x DCM auch Pd(OAc)₂/S-Phos [657408-07-6] verwendet werden. Zur Aufreinigung kann neben Destillation auch Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 40% und 85%.

| Edukt 1 | Edukt 2 | Produkt |
|---|---|---|
| 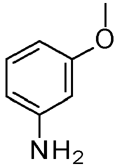 CAS-536-90-3 | 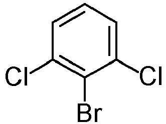 CAS-19393-92-1 | 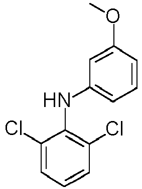 S2 |
| 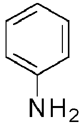 CAS-62-53-3 | 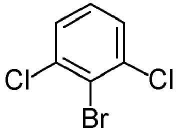 CAS-19393-92-1 | 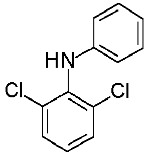 S3 |
| 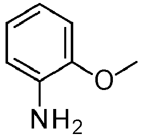 CAS-90-04-0 | 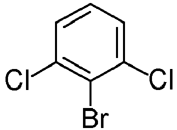 CAS-19393-92-1 | 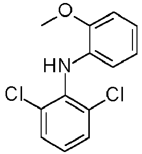 S4 |
| 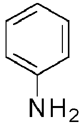 CAS-62-53-3 | 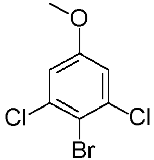 CAS-174913-20-3 | 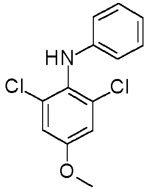 S5 |
| 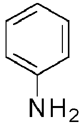 CAS-62-53-3 | 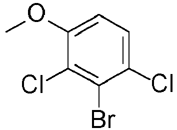 CAS-174913-16-7 | 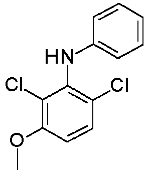 S6 |
| 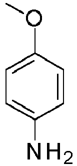 CAS-104-94-9 | 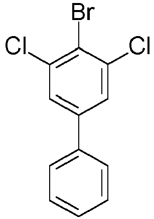 | 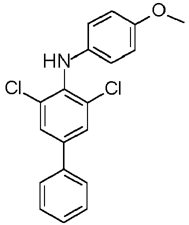 S7 |
| 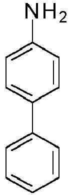 CAS-92-67-1 | 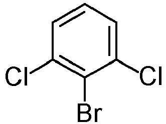 CAS-19393-92-1 | 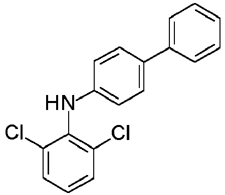 S8 |
| 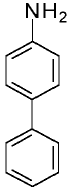 CAS-92-67-1 | 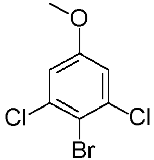 CAS-174913-20-3 | 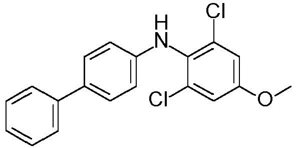 S9 |
| 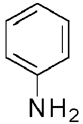 CAS-62-53-3 | 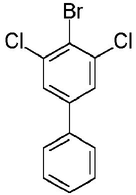 | 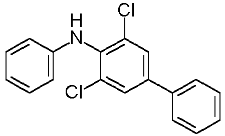 S10 |

**S25:**

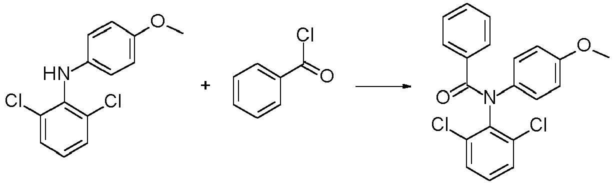

S1 (217.7 g, 812 mmol) wird in Pyridin (1000 mL) vorgelegt und mit Argon inertisiert. Anschließend wird Benzoylchlorid [98-88-4] (150 mL, 1.29 mol) tropfenweise zugegeben. Nach beendeter Zugabe wird der Ansatz 15 h unter Rückfluss gerührt. Man lässt den Ansatz auf Raumtemperatur abkühlen, saugt den ausgefallenen Feststoff ab und wäscht diesen viermal mit Wasser. Das Rohprodukt wird in Ethanol suspendiert und 3 h unter Rückfluss gerührt. Nach dem Abkühlen saugt man den ausgefallenen Feststoff ab und wäscht diesen mit Ethanol. Ausbeute: 284 g (763 mmol, 94%) weißer Feststoff; 98%ig nach ¹H-NMR.

Analog können folgende Verbindungen dargestellt werden. Zur Aufreinigung kann auch Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4 Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 40% und 85%.

| Edukt 1 | Edukt 2 | Produkt |
|---|---|---|
| 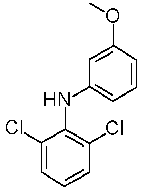 S2 | 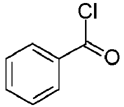 | 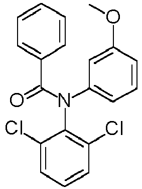 S26 |
| 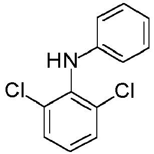 S3 | 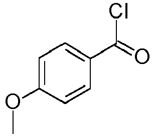 CAS-100-07-2 | 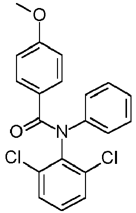 S27 |
| 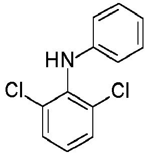 S3 | 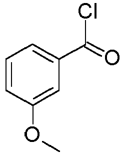 CAS-1711-05-3 | 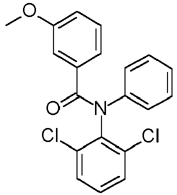 S28 |
| 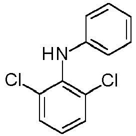 S3 | 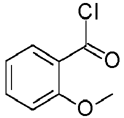 CAS-21615-34-9 | 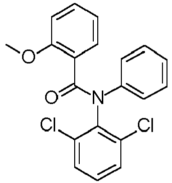 S29 |
| 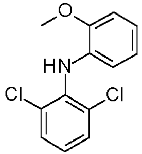 S4 | 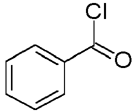 | 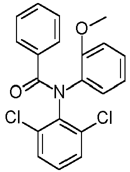 S30 |
| 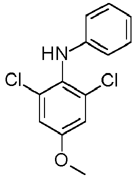 S5 | 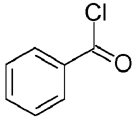 | 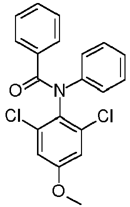 S31 |
| 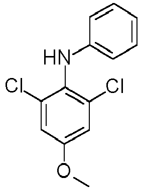 S5 | 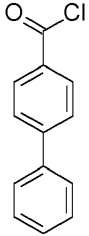 CAS-14002-51-8 | 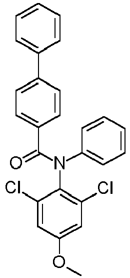 S32 |
| 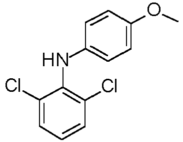 S1 | 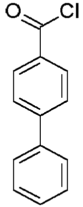 CAS-14002-51-8 | 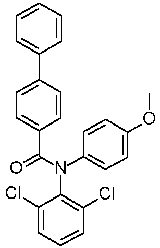 S33 |
| 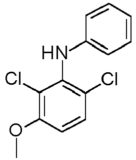 S6 | 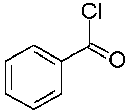 | 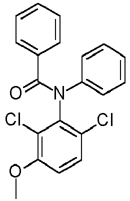 S34 |
| 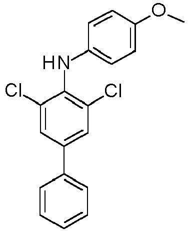 S7 | 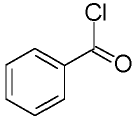 | 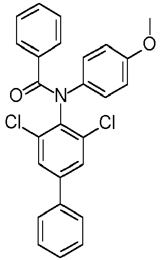 S35 |
| 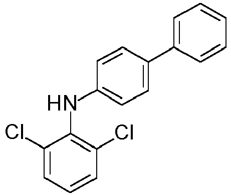 S8 | 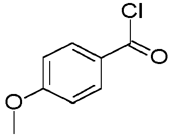 CAS-100-07-2 | 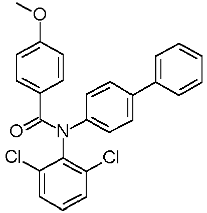 S36 |
| 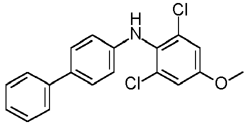 S9 | 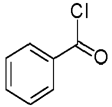 | 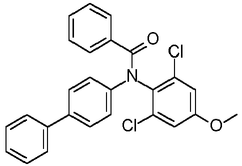 S37 |
| 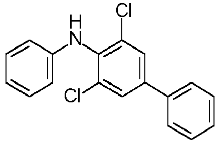 S10 | 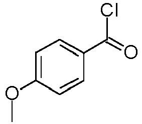 CAS-100-07-2 | 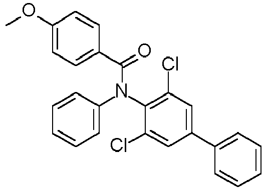 S38 |

**S50:**

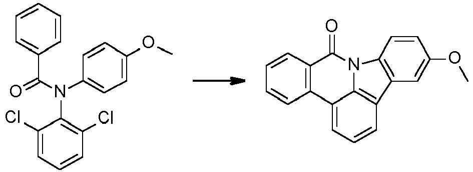

S25 (181.2 g, 486.8 mmol) und Kaliumcarbonat (202.5 g, 1.46 mol) werden in N,N-Dimethylacetamid (1800 mL) vorgelegt und 30 min mit Argon inertisiert. Anschließend werden 1,3-Bis(2,6-diisopropylphenyl)-imidazoliumchlorid [CAS-250285-32-6] (8.55 g, 19.47 mmol) und Palladium(II)acetat (2.18 g, 9.69 mmol) zugegeben, das Reaktionsgemisch auf Rückfluss erhitzt und 72 h bei dieser Temperatur gerührt. Nach Abkühlen auf Raumtemperatur wird das Lösungsmittel am Rotationsverdampfer abgezogen, der Rückstand mit Ethanol/Wasser (1:1; 1000 mL) verrührt, der Feststoff abgesaugt und mit Wasser und Ethanol gewaschen. Das Rohprodukt wird aus Ethylacetat umkristallisiert. Ausbeute: 49.4 g (165 mmol; 34%) brauner Feststoff; ca. 95%ig nach ¹H-NMR.

Analog können folgende Verbindungen dargestellt werden. Hierbei kann als Ligand statt 1,3-Bis(2,6-diisopropylphenly)imidazoliumchlorid [CAS-250285-32-6] auch 1,3-Bis(2,4,6-trimethylphenyl)imidazoliumchlorid [141556-45-8], Tricyclohexylphosphin [2622-14-2] oder Tri-tert-butyl-phosphin [13716-12-6] verwendet werden. Zur Aufreinigung kann neben Destillation auch Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 10% und 50%.

| Edukt | Produkt(e) |
|---|---|
| 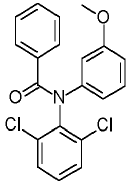 S26 | 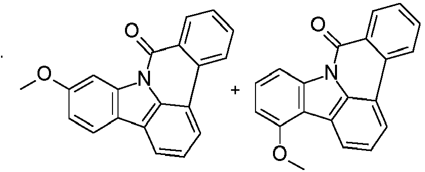 S51 S52 Trennung der Isomere über Säulenchromatorgraphie |
| 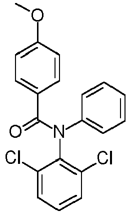 S27 | 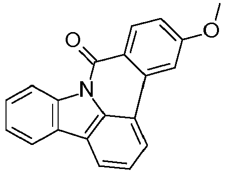 S53 |
| 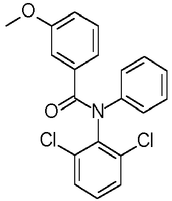 S28 | 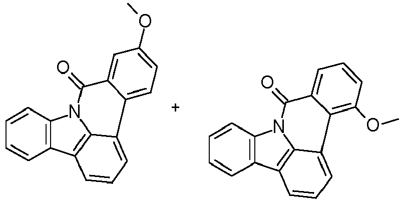 S54 S55 Trennung der Isomere über Säulenchromatorgraphie |
| 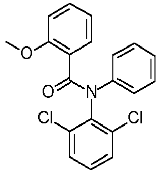 S29 | 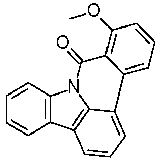 S56 |
| 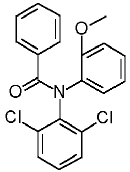 S30 | 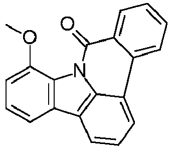 S57 |
| 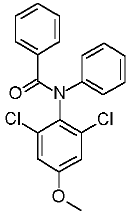 S31 | 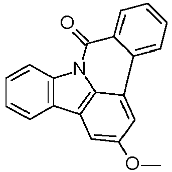 S58 |
| 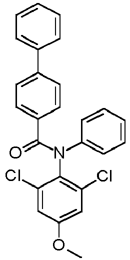 S32 | 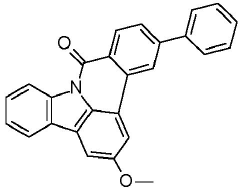 S59 |
| 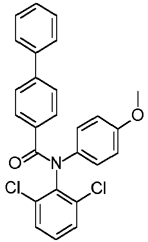 S33 | 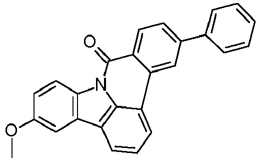 S60 |
| 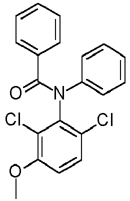 S34 | 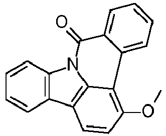 S61 |
| 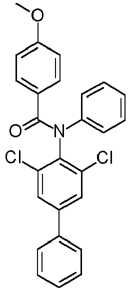 S38 | 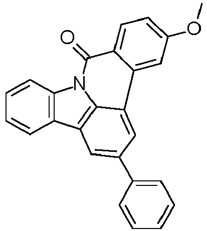 S62 |
| 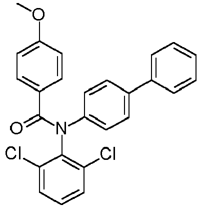 S36 | 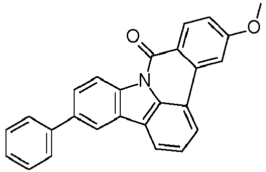 S63 |
| 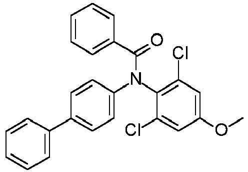 S37 | 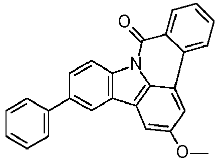 S64 |
| 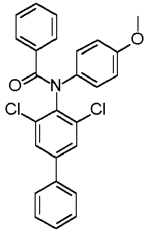 S35 | 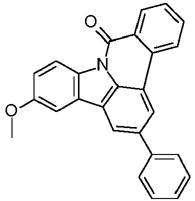 S65 |

**S75:** 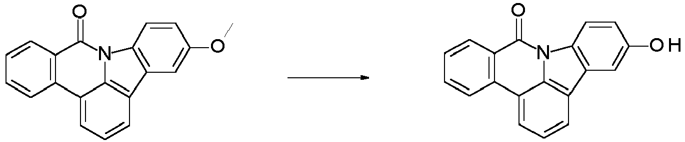 S50 (36.84 g, 123.1 mmol) wird in einem Kolben unter einer Argonatmosphäre in Dichlormethan (1200 mL) vorgelegt und im Eisbad auf 0 °C gekühlt. Anschließend wird Bortribromid [10294-33-4] (24.0 mL, 252.9 mmol) tropfenweise zugegeben, und anschließend wird der Ansatz langsam auf Raumtemperatur erwärmen gelassen. Anschließend wird der Ansatz tropfenweise mit Methanol gequencht und das Lösungsmittel am Rotationsverdampfer abgezogen. Es wird noch zweimal Methanol (300 mL) zugegeben und wieder abrotiert. Der braune Feststoff wird mit 400 mL Methanol versetzt und unter Rückfluss erhitzt. Nach Abkühlen wird der Feststoff abgesaugt und mit Methanol gewaschen. Das Rohprodukt wird mit n-Butylacetat heißextrahiert.

Ausbeute: 28.5 g (100 mmol; 81%) beiger Feststoff; 95%ig nach ¹H-NMR.

Analog können folgende Verbindungen dargestellt werden. Zur Aufreinigung kann auch Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 50% und 90%.

| Edukt | Produkt(e) |
|---|---|
| 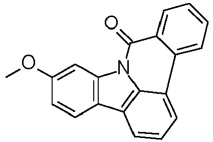 S51 | 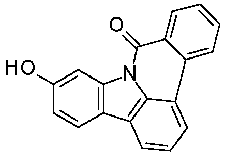 S76 |
| 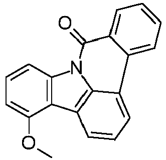 S52 | 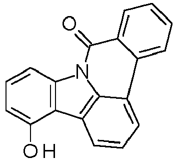 S77 |
| 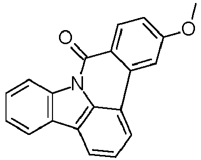 S53 | 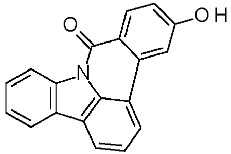 S78 |
| 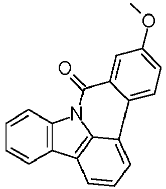 S54 | 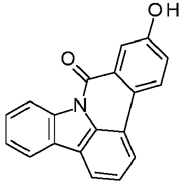 S79 |
| 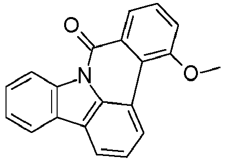 S55 | 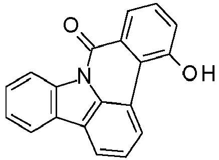 S80 |
| 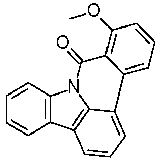 S56 | 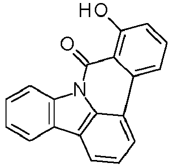 S81 |
| 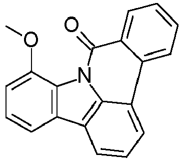 S57 | 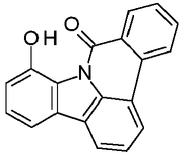 S82 |
| 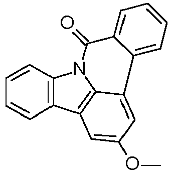 S58 | 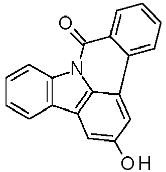 S83 |
| 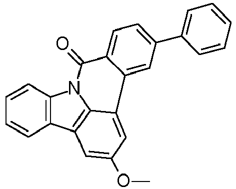 S59 | 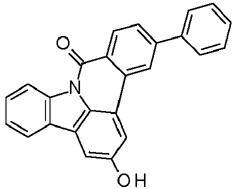 S84 |
| 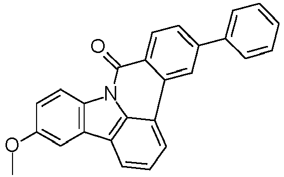 S60 | 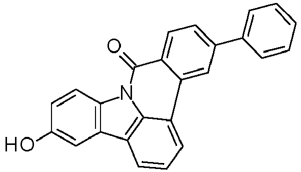 S85 |
| 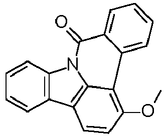 S61 | 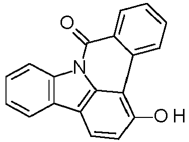 S86 |
| 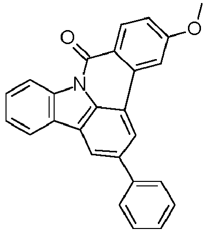 S62 | 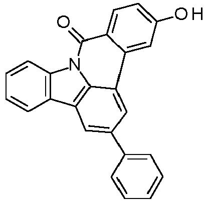 S87 |
| 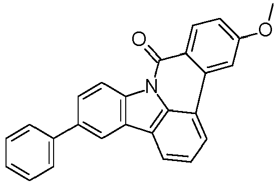 S63 | 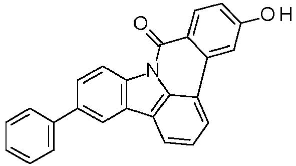 S88 |
| 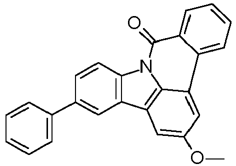 S64 | 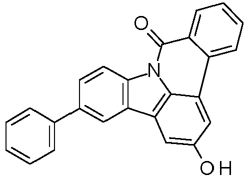 S89 |
| 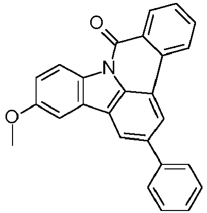 S65 | 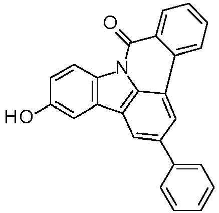 S90 |

**S100:**

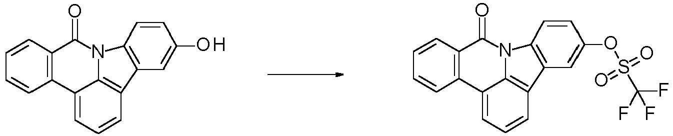

S75 (27.84 g, 97.6 mmol) wird in Pyridin (500 mL) vorgelegt und im Eisbad auf 0 °C gekühlt. Anschließend wird Trifluormethansulfonsäureanhydrid [358-23-6] (50.4 mL, 293.4 mmol) langsam zugetropft, so dass die Innentemperatur 10 °C nicht übersteigt. Anschließend lässt man den Ansatz über Nacht auf Raumtemperatur erwärmen. Das Pyridin wird am Rotationsverdampfer abgezogen und der Rückstand extraktiv mit Dichlormethan und 1 mol/L HCl aufgearbeitet. Die organische Phase wird viermal mit Wasser gewaschen, über Na₂SO₄ getrocknet und auf 300 mL eingeengt. Der ausgefallene Feststoff wird abgesaugt und mit Dichlormethan und Ethanol gewaschen. Ausbeute: 38.7 g (92.7 mmol, 95%) beige Feststoff; 95%ig nach ¹H-NMR.

Analog können folgende Verbindungen dargestellt werden. Zur Aufreinigung kann auch Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 55% und 97%.

| Edukt | Produkt(e) |
|---|---|
| 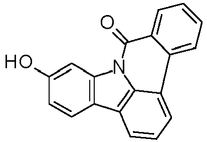 S76 | 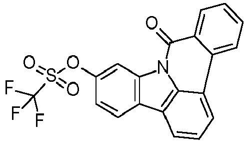 S101 |
| 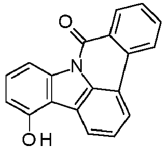 S77 | 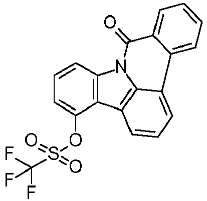 S102 |
| 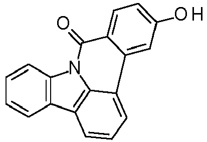 S78 | 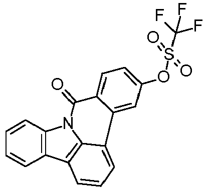 S103 |
| 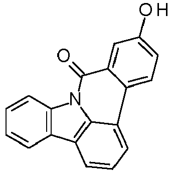 S79 | 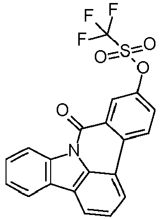 S104 |
| 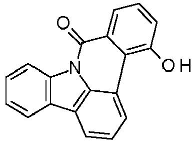 S80 | 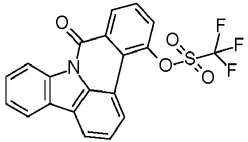 S105 |
| 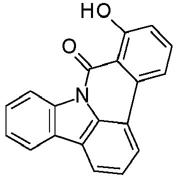 S81 | 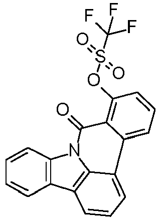 S106 |
| 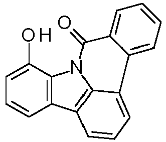 S82 | 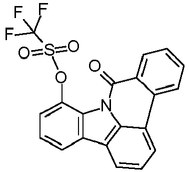 S107 |
| 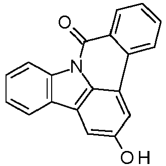 S83 | 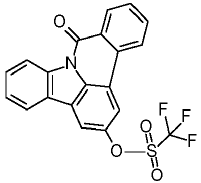 S108 |
| 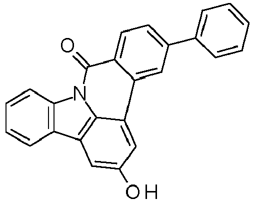 S84 | 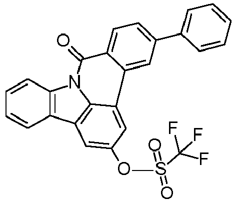 S109 |
| 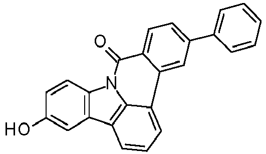 S85 | 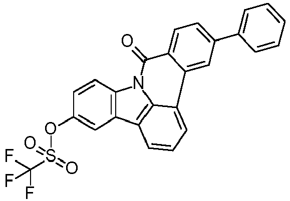 S110 |
| 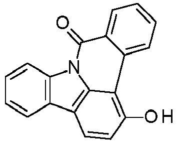 S86 | 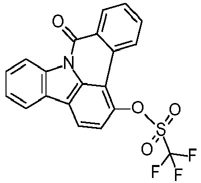 S111 |
| 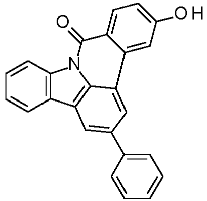 S87 | 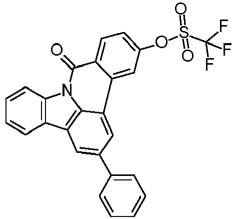 S112 |
| 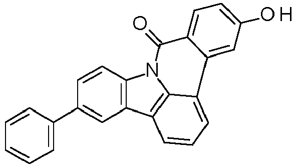 S88 | 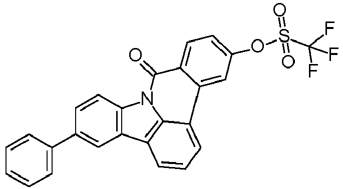 S113 |
| 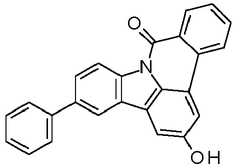 S89 | 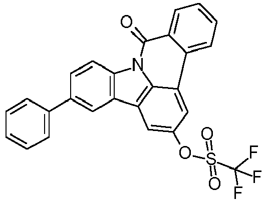 S114 |
| 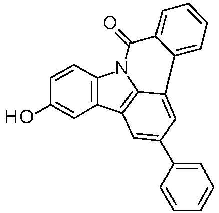 S90 | 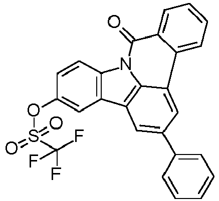 S115 |

**S125:**

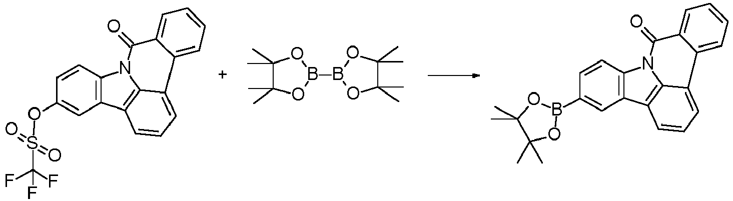

S1 (40.2 g, 96.2 mmol), Bis(pinacolato)diboron [73183-34-3] (26.93 g, 105.0 mmol) und Kaliumacetat (28.82 g, 293.6 mmol) werden in 1,4-Dioxan (600 mL) vorgelegt und 2 min mit Argon inertisiert. Anschließend werden X-Phos [564483-18-7] (456 mg 0.96 mmol) und Pd₂(dba)₃ [51364-51-3] (435 mg, 0.48 mmol) zugegeben und das Reaktionsgemisch für 16 h unter Rückfluss gerührt. Nach dem Abkühlen wird das Lösungsmittel abrotiert und der Rückstand extraktiv mit Dichlormethan/Wasser aufgearbeitet. Die organische Phase wird über Na₂SO₄ getrocknet, mit Ethylacetat versetzt und das Dichlormethan am Rotationsverdampfer bis 500 mbar abrotiert. Der ausgefallene Feststoff wird abgesaugt und mit Ethylacetat gewaschen. Ausbeute: 30.8 g (77.9 mmol, 81%) beige Feststoff; 98%ig nach ¹H-NMR.

Analog können folgende Verbindungen dargestellt werden. Hierbei kann als Ligand statt X-Phos auch S-Phos verwendet werden. Zur Aufreinigung kann Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 60% und 95%.

| Edukt | Produkt(e) |
|---|---|
| 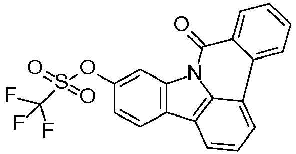 S101 | 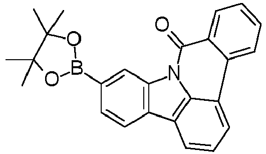 S126 |
| 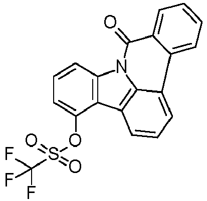 S102 | 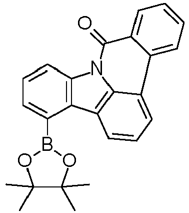 S127 |
| 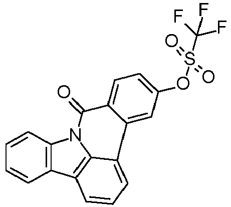 S103 | 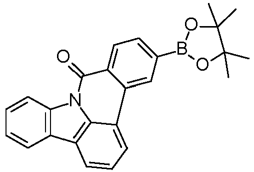 S128 |
| 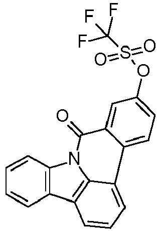 S104 | 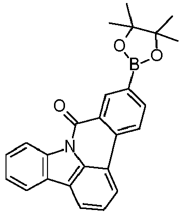 S129 |
| 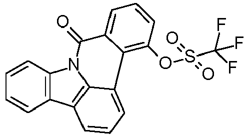 S105 | 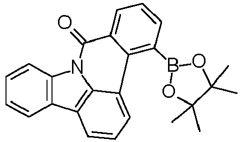 S130 |
| 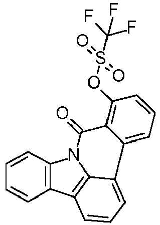 S106 | 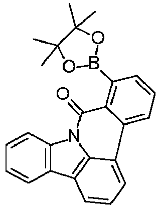 S131 |
| 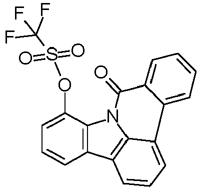 S107 | 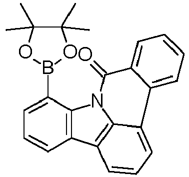 S132 |
| 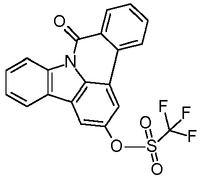 S108 | 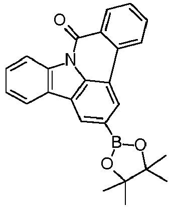 S133 |
| 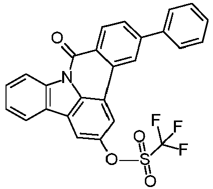 S109 | 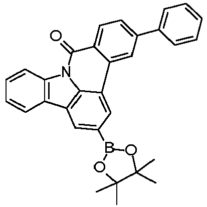 S134 |
| 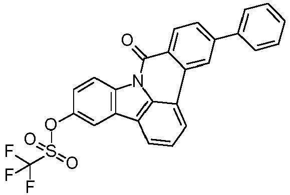 S110 | 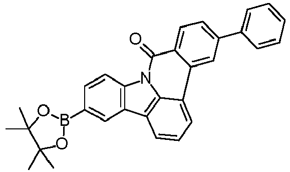 S135 |
| 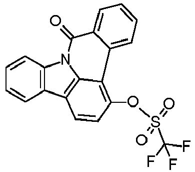 S111 | 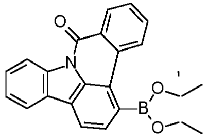 S136 |
| 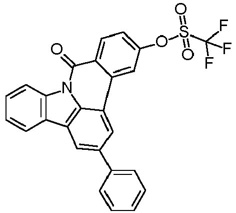 S112 | 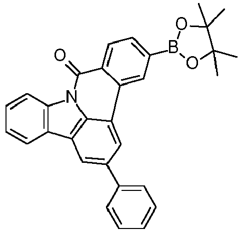 S137 |
| 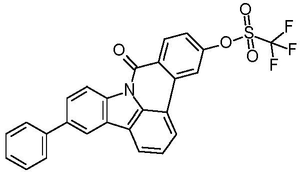 S113 | 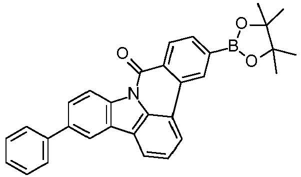 S138 |
| 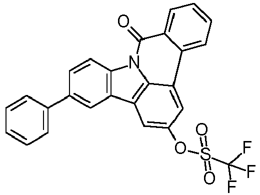 S114 | 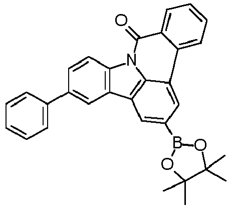 S139 |
| 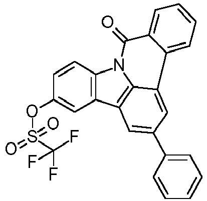 S115 | 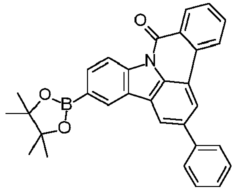 S140 |

**S150**

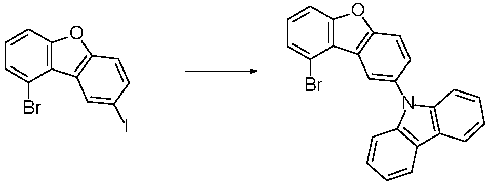

Unter inerter Atmosphäre werden 1-Brom-8-iod-dibenzofuran [CAS-1822311-11-4] (37.28 g, 100 mmol), Carbazol [86-74-8] (16.71 g, 100 mmol), Kaliumcarbonat (34.55 g, 250 mmol) und Kupferpulver (1.27 g, 20.0 mmol) in DMF (350 mL) vorgelegt, weitere 15 min mit Argon inertisiert und anschließend 32 h bei 130 °C gerührt. Man lässt den Ansatz auf Raumtemperatur abkühlen, filtriert über ein Celite-Bett, wäscht zweimal mit 200 mL DMF nach und rotiert das Filtrat bis zur Trockene am Rotationsverdampfer ein. Der Rückstand wird extraktiv mit Dichlormethan / Wasser aufgearbeitet, die organische Phase zweimal mit Wasser und einmal mit gesättigter NaCl-Lösung gewaschen und über Na₂SO₄ getrocknet. Es werden 150 mL Ethanol zugegeben, das Dichlormethan wird am Rotationsverdampfer bis 500 mbar abgezogen, der ausgefallende Festoff abgesaugt und mit Ethanol gewaschen. Ausbeute: 29.2 g (71.1 mmol, 71%) grauer Feststoff; 97%ig nach ¹H-NMR.

Analog können folgende Verbindungen dargestellt werden. Zur Aufreinigung kann Säulenchromatographie verwendet werden, oder zur Umkristallisation können gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan, Dimethylsulfoxid, N,N-Dimethylformamid, N,N-Dimethylacetamid, N-Methylpyrrolidon, etc. verwendet werden. Die Ausbeuten liegen typischerweise im Bereich zwischen 20% und 80%.

| Edukt 1 | Edukt 2 | Produkt |
|---|---|---|
| 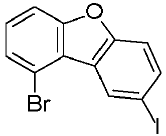 CAS-1822311-11-4 | 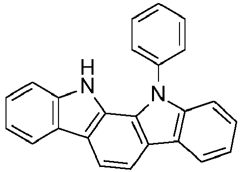 CAS-1024598-06-8 | 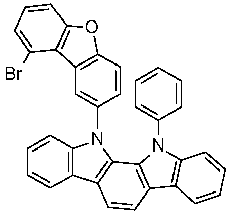 S151 |
| 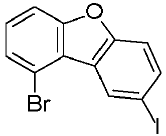 CAS-1822311-11-4 | 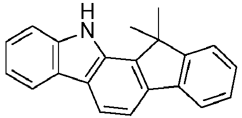 CAS-1329054-41-2 | 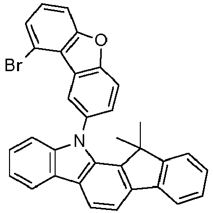 S152 |
| 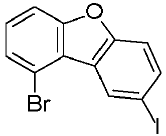 CAS-1822311-11-4 | 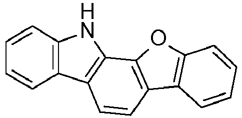 CAS-1338919-70-2 | 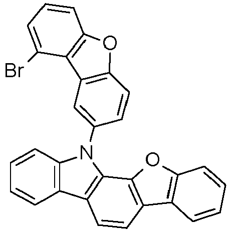 S153 |
| 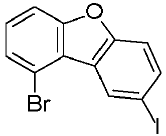 CAS-1822311-11-4 | 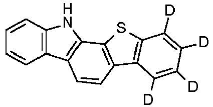 CAS-1447708-61-3 | 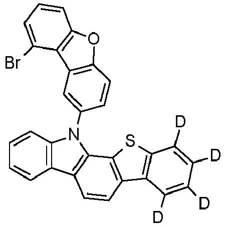 S154 |
| 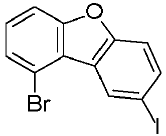 CAS-1822311-11-4 | 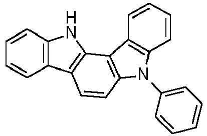 CAS-1247053-55-9 | 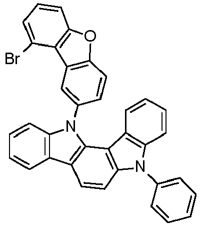 S155 |
| 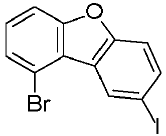 CAS-1822311-11-4 | 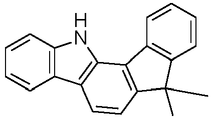 CAS-1616231-39-0 | 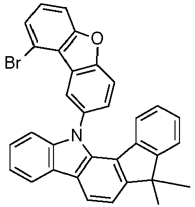 S156 |
| 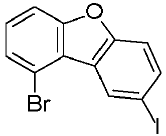 CAS-1822311-11-4 | 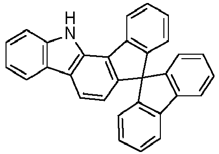 CAS-1615703-28-0 | 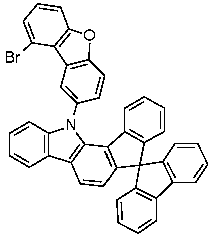 S157 |
| 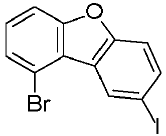 CAS-1822311-11-4 | 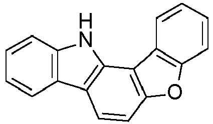 CAS-1246308-85-9 | 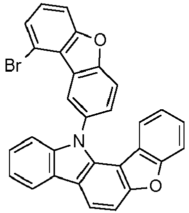 S158 |
| 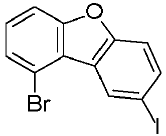 CAS-1822311-11-4 | 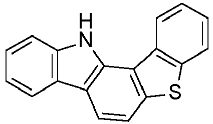 CAS-206447-68-9 | 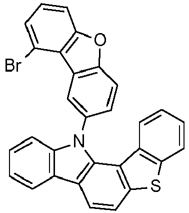 S159 |
| 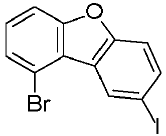 CAS-1822311-11-4 | 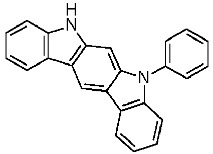 CAS-1448296-00-1 | 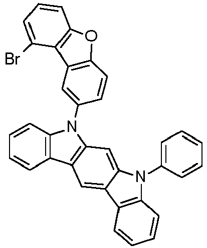 S160 |
| 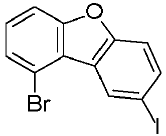 CAS-1822311-11-4 | 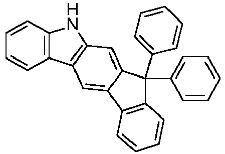 CAS-1257220-52-2 | 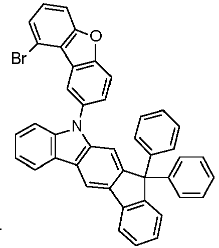 S161 |
| 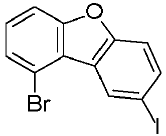 CAS-1822311-11-4 | 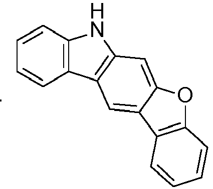 CAS-1246308-83-7 | 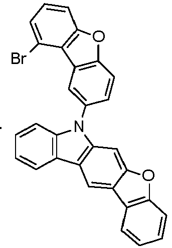 S162 |
| 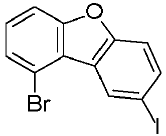 CAS-1822311-11-4 | 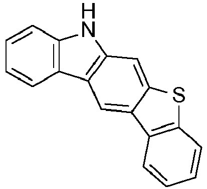 CAS-1255309-04-6 | 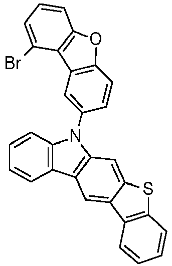 S163 |
| 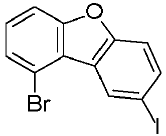 CAS-1822311-11-4 | 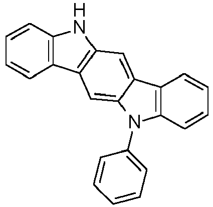 CAS-1316311-27-9 | 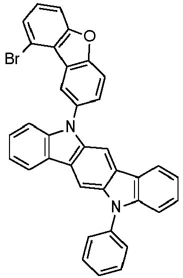 S164 |
| 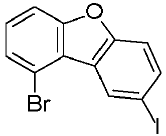 CAS-1822311-11-4 | 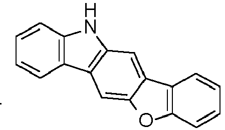 CAS-1199350-22-5 | 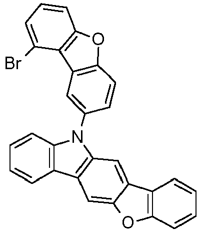 S165 |
| 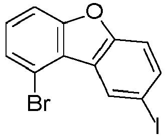 CAS-1822311-11-4 | 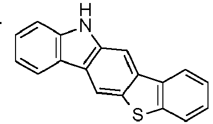 CAS-1255309-10-4 | 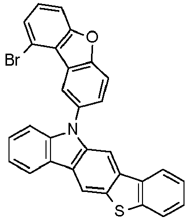 S166 |
| 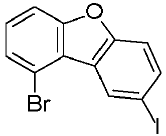 CAS-1822311-11-4 | 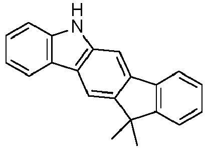 CAS-1260228-95-2 | 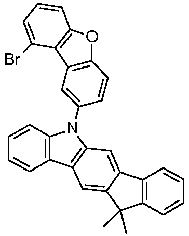 S167 |
| 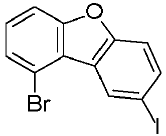 CAS-1822311-11-4 | 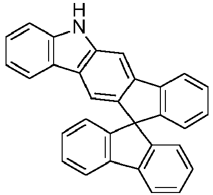 CAS-1219841-59-4 | 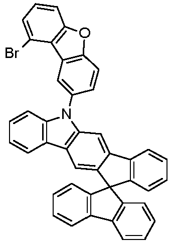 S168 |
| 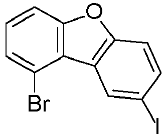 CAS-1822311-11-4 | 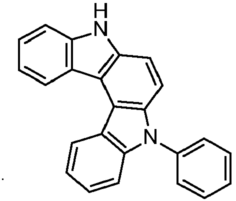 CAS-1637752-63-6 | 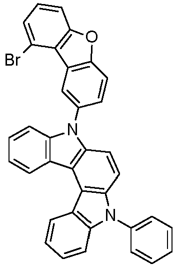 S169 |
| 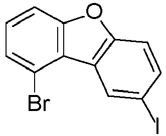 CAS-1822311-11-4 | 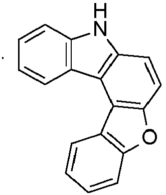 CAS-1622290-43-0 | 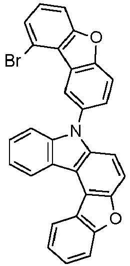 S170 |
| 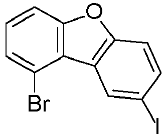 CAS-1822311-11-4 | 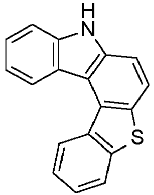 CAS-1255309-17-1 | 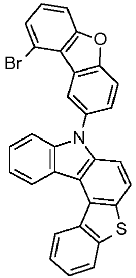 S171 |
| 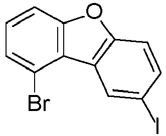 CAS-1822311-11-4 | 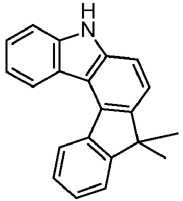 CAS-1623813-70-6 | 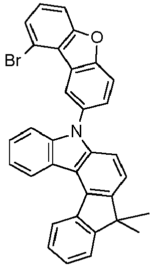 S172 |
| 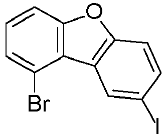 CAS-1822311-11-4 | 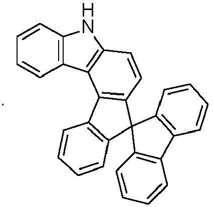 CAS-1936530-01-6 | 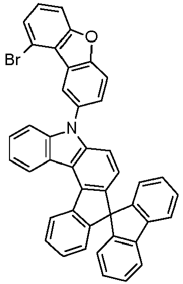 S173 |
| 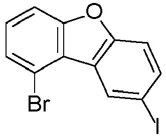 CAS-1822311-11-4 | 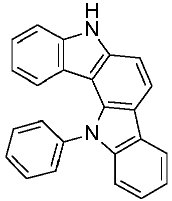 CAS-1346571-68-3 | 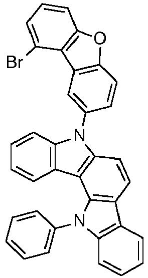 S174 |
| 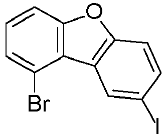 CAS-1822311-11-4 | 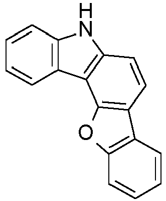 CAS-1199616-66-4 | 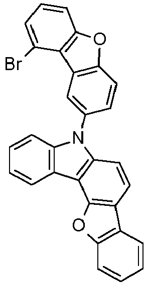 S175 |
| 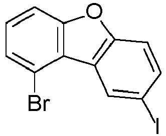 CAS-1822311-11-4 | 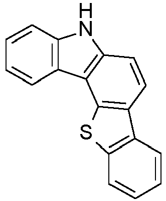 CAS-1255308-97-4 | 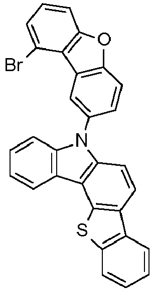 S176 |
| 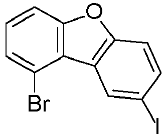 CAS-1822311-11-4 | 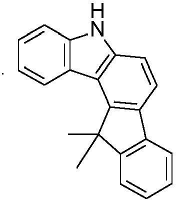 CAS-1346645-54-2 | 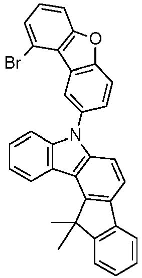 S177 |
| 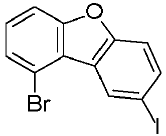 CAS-1822311-11-4 | 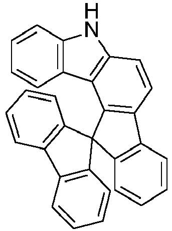 CAS-2055578-08-8 | 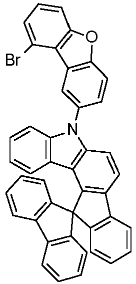 S178 |
| 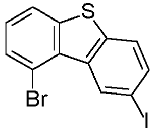 CAS-1822311-12-5 | 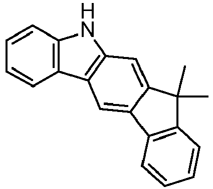 CAS-1257220-47-5 | 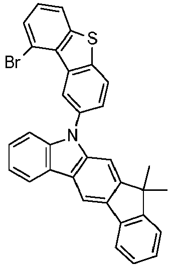 S179 |
| 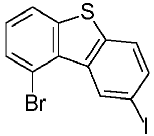 CAS-1822311-12-5 | 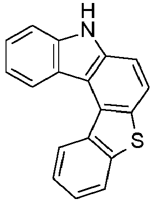 CAS-1255309-17-1 | 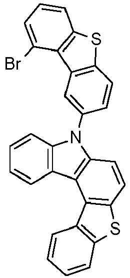 S180 |
| 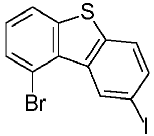 CAS-1822311-12-5 | 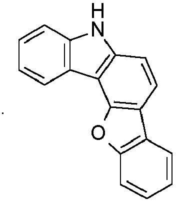 CAS-1199616-66-4 | 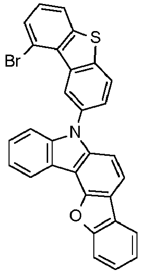 S181 |
| 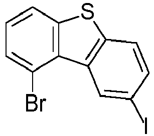 CAS-1822311-12-5 | 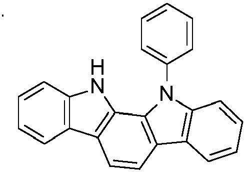 CAS-1024598-06-8 | 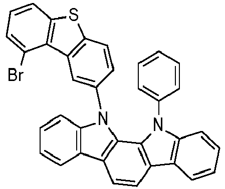 S182 |
| 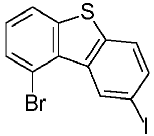 CAS-1822311-12-5 | 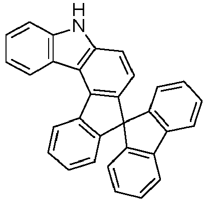 CAS-1936530-01-6 | 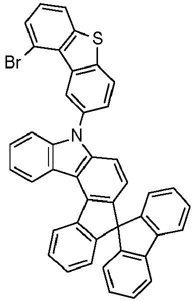 S183 |
| 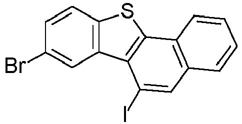 CAS-1883821-22-4 | 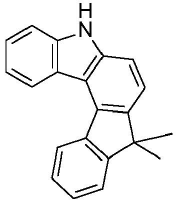 CAS-1623813-70-6 | 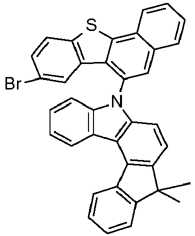 S184 |
| 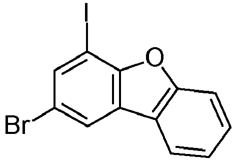 CAS-1401068-25-4 | 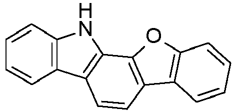 CAS-1338919-70-2 | 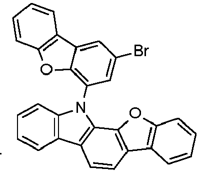 S185 |
| 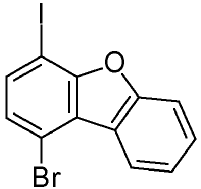 CAS- 1923736-35-9 | 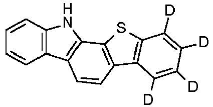 CAS-1447708-61-3 | 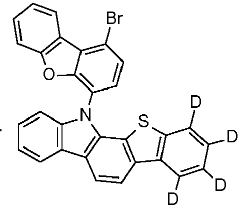 S186 |
| 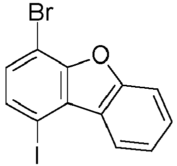 CAS- 2096506-51-1 | 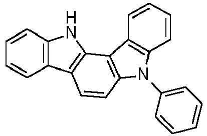 CAS-1247053-55-9 | 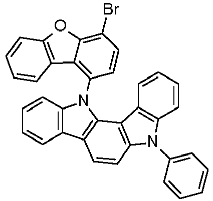 S187 |
| 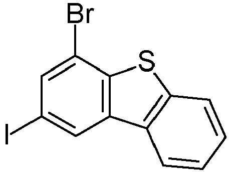 CAS- 1913276-15-9 | 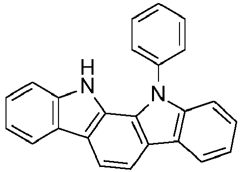 CAS-1024598-06-8 | 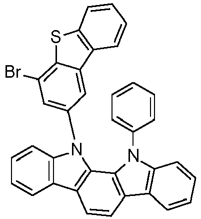 S188 |
| 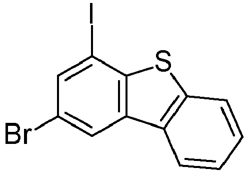 CAS- 1923736-34-8 | 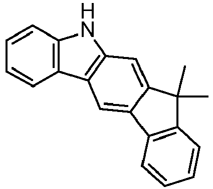 CAS-1257220-47-5 | 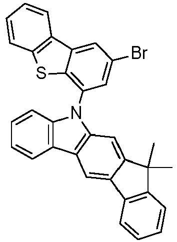 S189 |
| 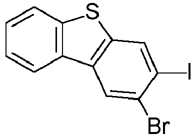 CAS-1549979-42-1 | 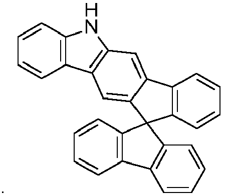 CAS-1219841-59-4 | 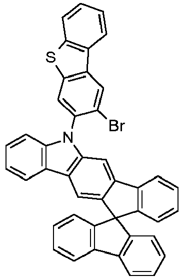 S190 |
| 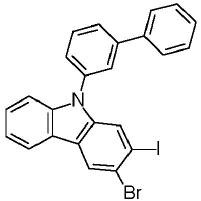 CAS-2086719-53-9 | 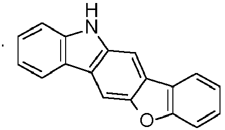 CAS-1199350-22-5 | 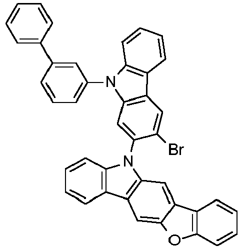 S191 |
| 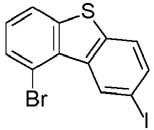 CAS-1822311-12-5 | 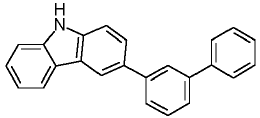 CAS-1643526-99-1 | 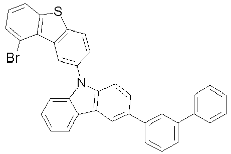 S192 |
| 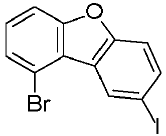 CAS-1822311-11-4 | 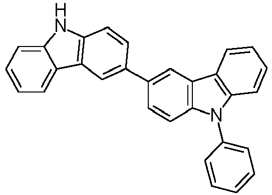 CAS-1060735-14-9 | 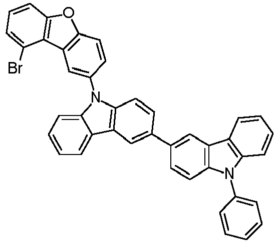 S193 |
| 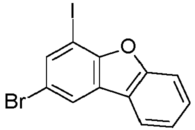 CAS-1401068-25-4 | 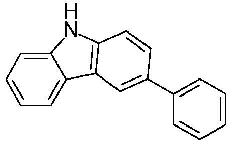 CAS-103012-26-6 | 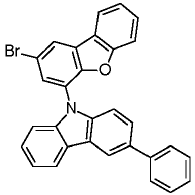 S194 |
| 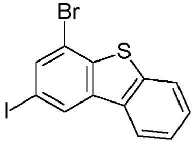 CAS- 1913276-15-9 | 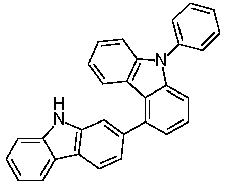 CAS-2160600-08-6 | 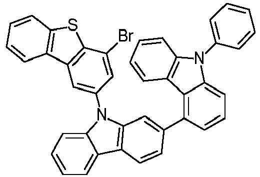 S195 |
| 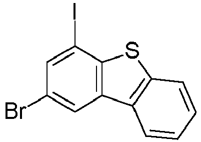 CAS- 1923736-34-8 | 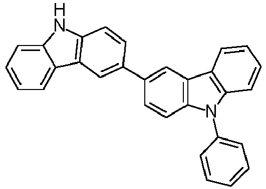 CAS-1060735-14-9 | 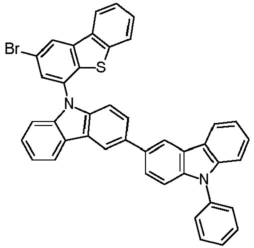 S196 |

### Darstellung der Produkte:

**P1:**

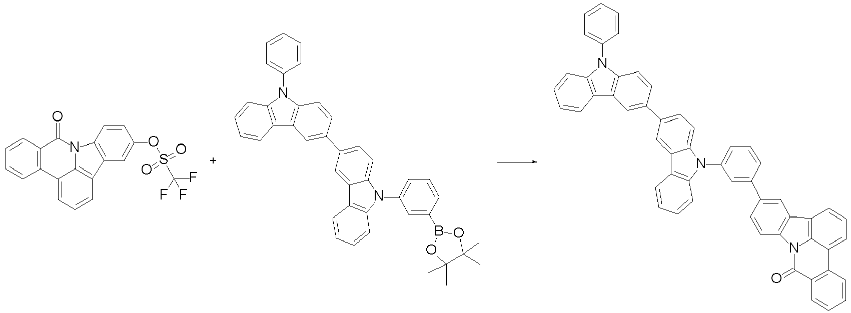

S100 (15.02 g, 36.0 mmol), 9-Phenyl-9'-[3-(4,4,5,5-tetramethyl-[1,3,2]-dioxaborolan-2-yl)-phenyl]-9H,9'H-[3,3']bicarbazolyl [2088364-11-6] (26.41 g, 43.3 mmol) und Trikaliumphosphat (15.54 g) werden in einem Kolben vorgelegt, mit Tetrahydrofuran (400 mL) und Wasser (100 mL) versetzt und 30 min mit Argon inertisiert. Anschließend werden Palladium(II)-acetat [3375-31-3] (204.3 mg) und X-Phos [564483-18-7] (905 mg) zugegeben und der Ansatz 20 h unter Rückfluss erhitzt. Nach Abkühlen saugt man den ausgefallenen Feststoff ab und wäscht diesen zweimal mit Wasser und zweimal mit THF. Das Rohprodukt wird viermal mit Toluol heißextrahiert und abschließend im Hochvakuum sublimiert. Ausbeute: 15.6 g (20.7 mmol, 58%) gelber Feststoff; Reinheit > 99.9% nach HPLC.

Analog können folgende Verbindungen dargestellt werden. Hierbei kann als Phosphinligand statt X-Phos auch S-Phos [657408-07-6] oder als Katalysatorsystem (Palladiumquelle und Ligand) Bis(triphenylphosphin)-palladiumchlorid [13965-03-2] verwendet werden. Zur Aufreinigung kann auch Säulenchromatographie verwendet werden, oder zur Umkristallisation oder Heißextraktion können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden. Die Ausbeuten liegen typischerweise im Bereich zwischen 15% und 75%.

| Edukt 1 | Edukt 2 | Produkt |
|---|---|---|
| S100 | 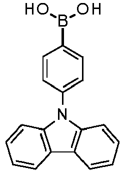 CAS-419536-33-7 | 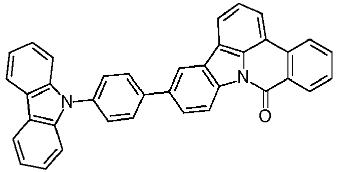 P2 |
| S100 | 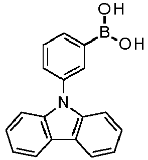 CAS-864377-33-3 | 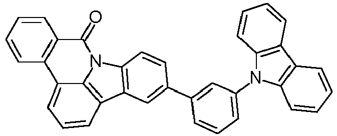 P3 |
| S100 | 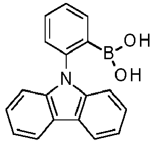 CAS-1189047-28-6 | 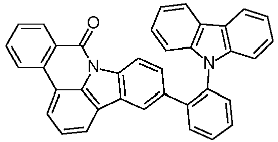 P4 |
| S100 | 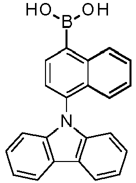 CAS-1246021-50-0 | 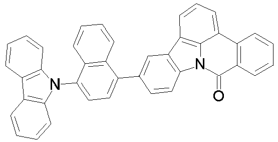 P5 |
| S100 | 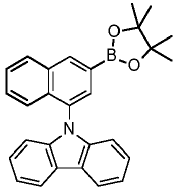 CAS-1819346-26-3 | 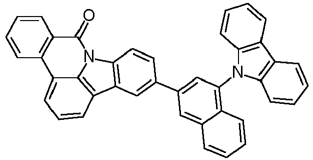 P6 |
| S100 | 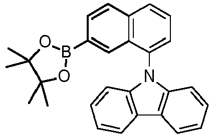 CAS-1221685-92-2 | 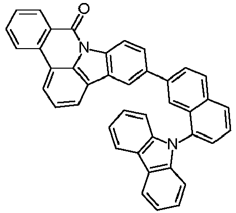 P7 |
| S100 | 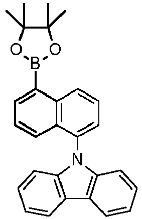 CAS-1820789-13-6 | 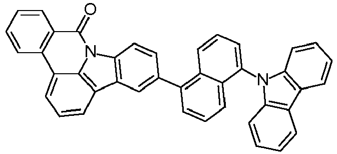 P8 |
| S100 | 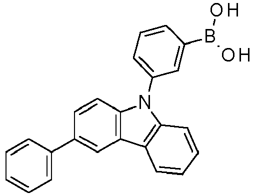 CAS-1801285-73-3 | 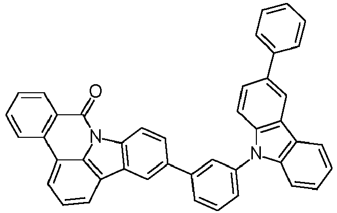 P9 |
| S100 | 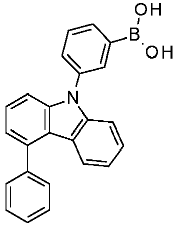 CAS-1801285-68-6 | 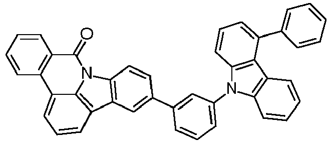 P10 |
| S100 | 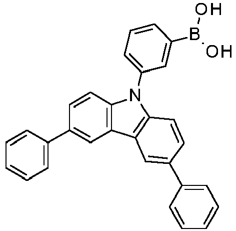 CAS-1332748-66-9 | 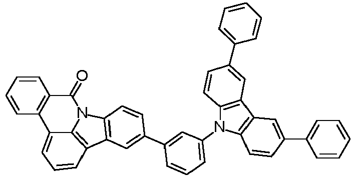 P11 |
| S100 | 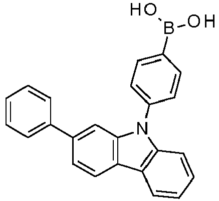 CAS-2075740-65-5 | 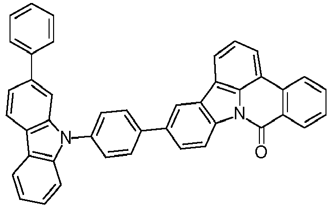 P12 |
| S100 | 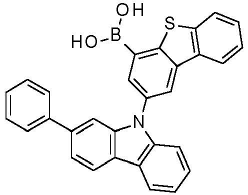 CAS-2177306-79-3 | 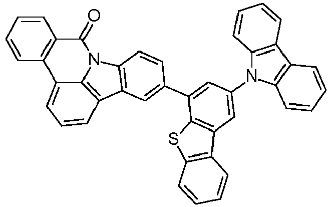 P13 |
| S100 | 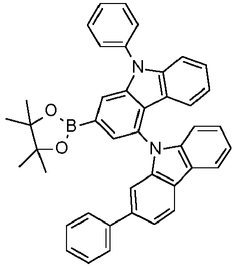 CAS-2231201-17-3 | 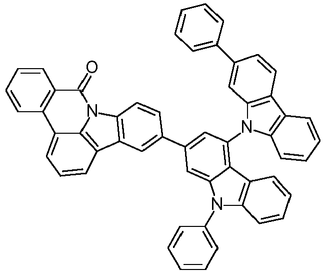 P14 |
| S100 | 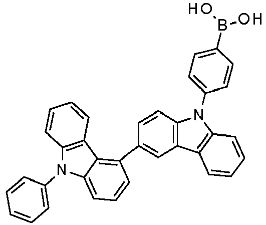 CAS-1885113-59-6 | 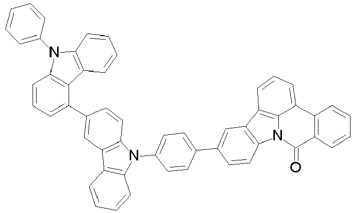 P15 |
| S100 | 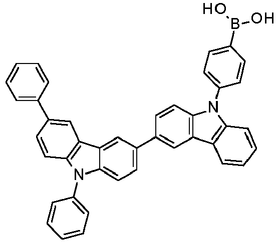 CAS-1398395-86-2 | 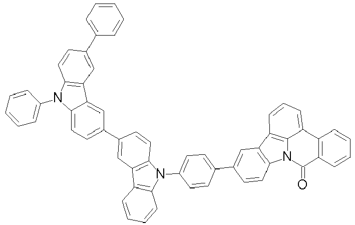 P16 |
| S100 | 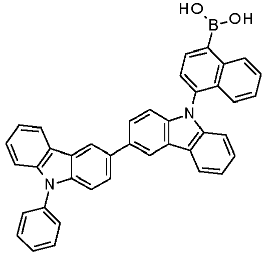 CAS-1398395-43-1 | 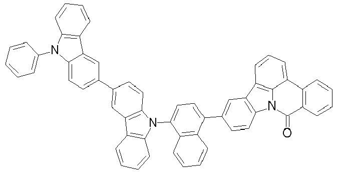 P17 |
| S100 | 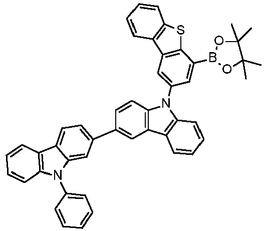 CAS-2079874-17-0 | 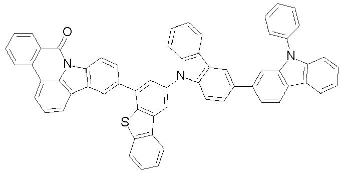 P18 |
| S100 | 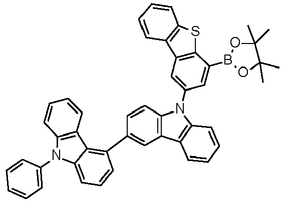 CAS-2079874-18-1 | 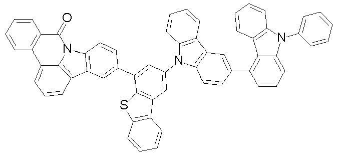 P19 |
| S100 | 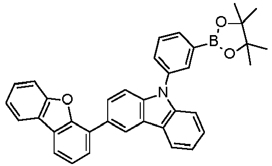 CAS-1829586-22-2 | 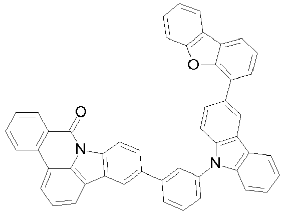 P20 |
| S100 | 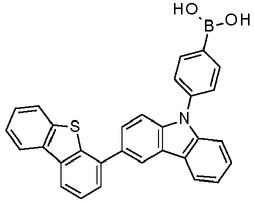 CAS-1894228-52-4 | 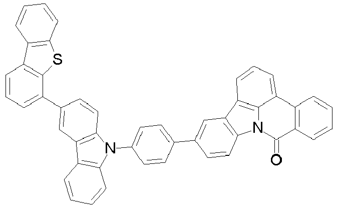 P21 |
| S101 | 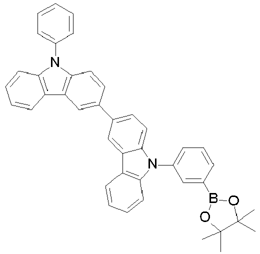 CAS-2088364-11-6 | 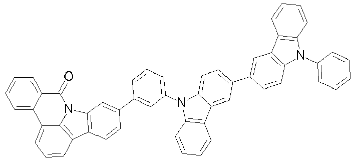 P22 |
| S102 | 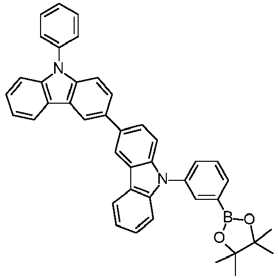 CAS-2088364-11-6 | 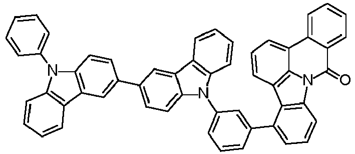 P23 |
| S103 | 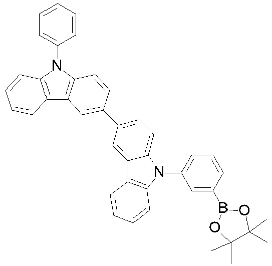 CAS-2088364-11-6 | 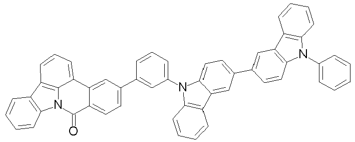 P24 |
| S104 | 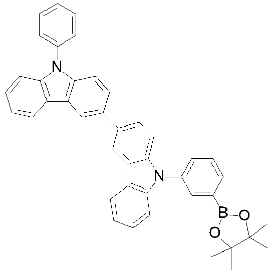 CAS-2088364-11-6 | 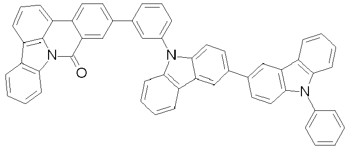 P25 |
| S105 | 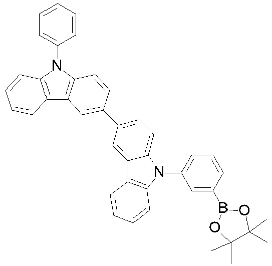 CAS-2088364-11-6 | 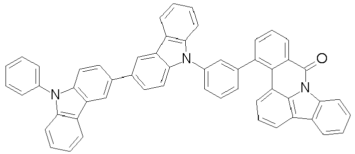 P26 |
| S106 | 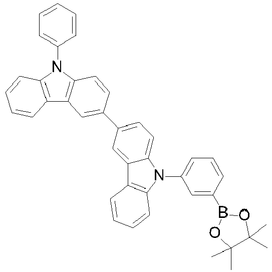 CAS-2088364-11-6 | 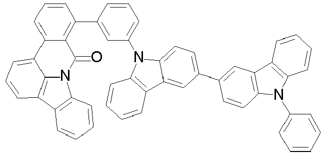 P27 |
| S107 | 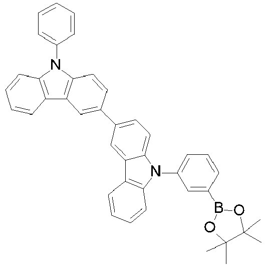 CAS-2088364-11-6 | 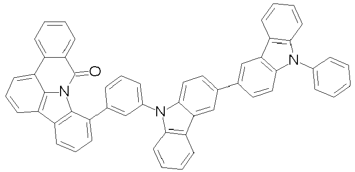 P28 |
| S108 | 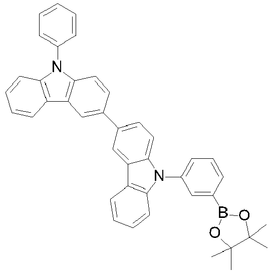 CAS-2088364-11-6 | 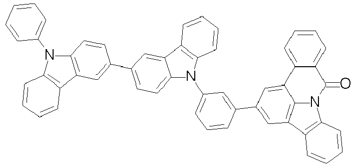 P29 |
| S109 | 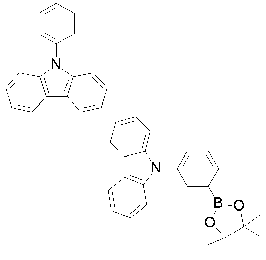 CAS-2088364-11-6 | 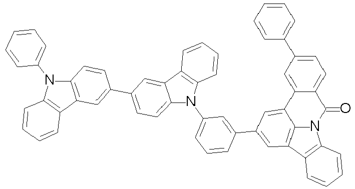 P30 |
| S110 | 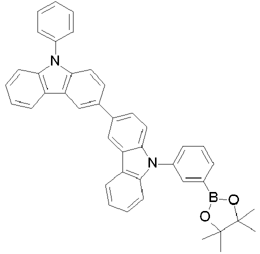 CAS-2088364-11-6 | 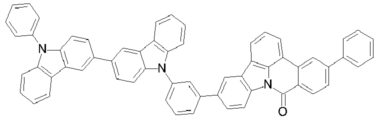 P31 |
| S111 | 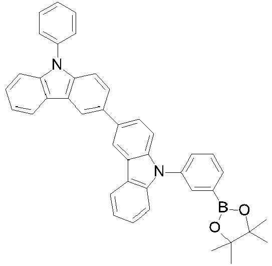 CAS-2088364-11-6 | 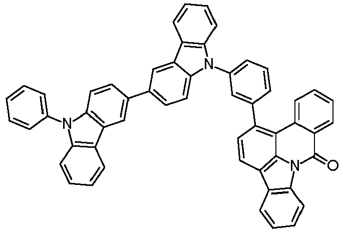 P32 |
| S112 | 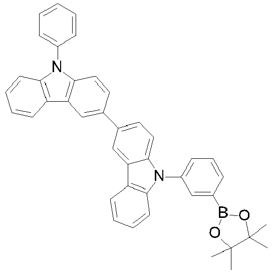 CAS-2088364-11-6 | 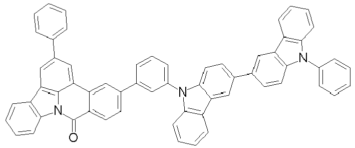 P33 |
| S113 | 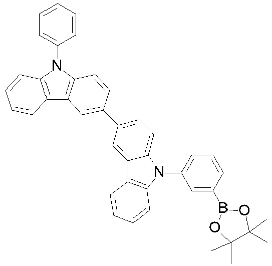 CAS-2088364-11-6 | 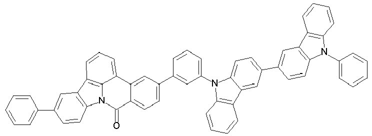 P34 |
| S114 | 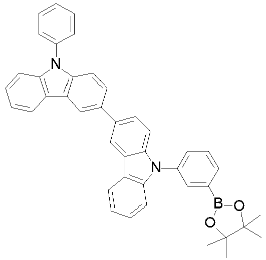 CAS-2088364-11-6 | 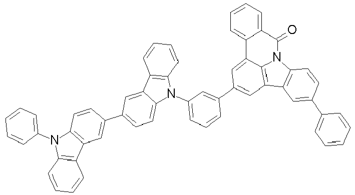 P35 |
| S115 | 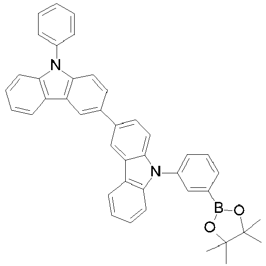 CAS-2088364-11-6 | 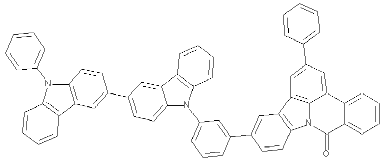 P36 |
| S101 | 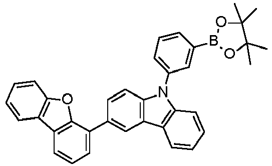 CAS-1829586-22-2 | 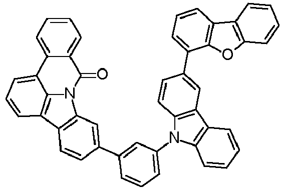 P37 |
| S102 | 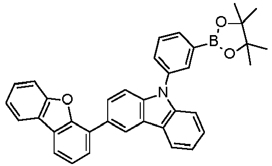 CAS-1829586-22-2 | 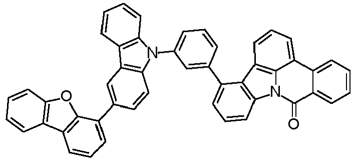 P38 |
| S103 | 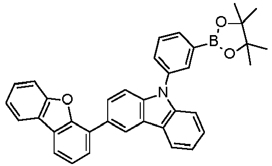 CAS-1829586-22-2 | 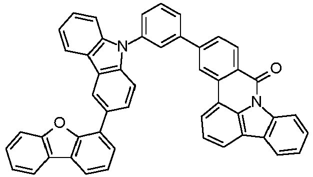 P39 |
| S104 | 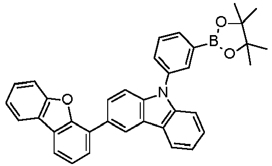 CAS-1829586-22-2 | 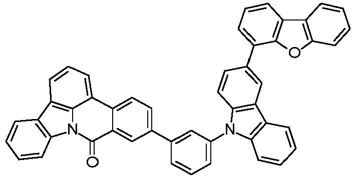 P40 |
| S105 | 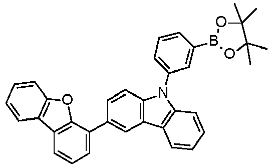 CAS-1829586-22-2 | 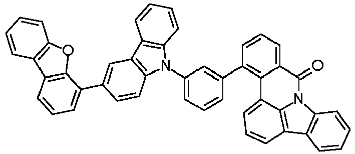 P41 |
| S106 | 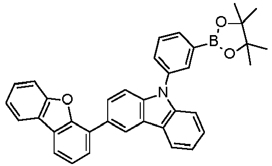 CAS-1829586-22-2 | 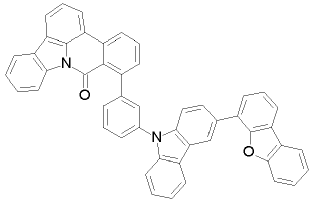 P42 |
| S107 | 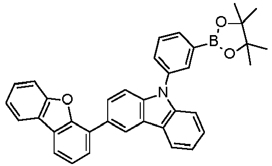 CAS-1829586-22-2 | 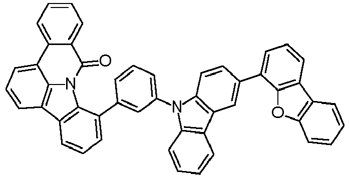 P43 |
| S108 | 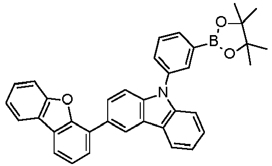 CAS-1829586-22-2 | 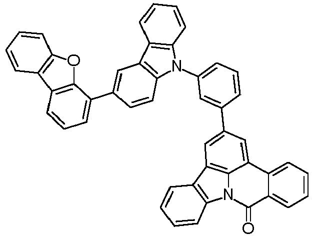 P44 |
| S109 | 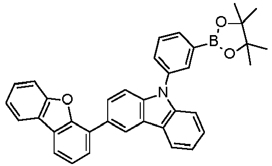 CAS-1829586-22-2 | 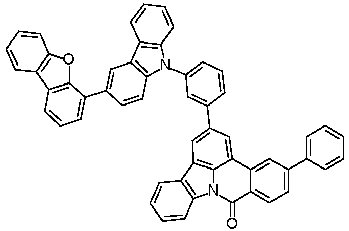 P45 |
| S110 | 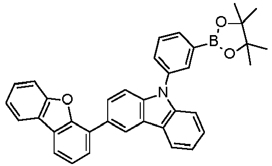 CAS-1829586-22-2 | 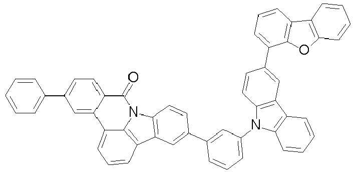 P46 |
| S111 | 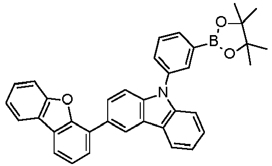 CAS-1829586-22-2 | 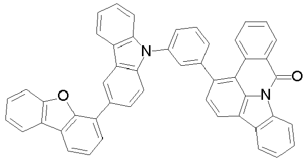 P47 |
| S112 | 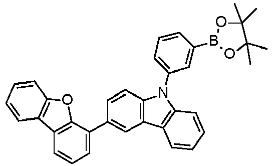 CAS-1829586-22-2 | 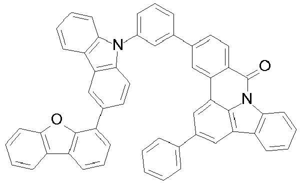 P48 |
| S113 | 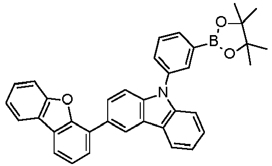 CAS-1829586-22-2 | 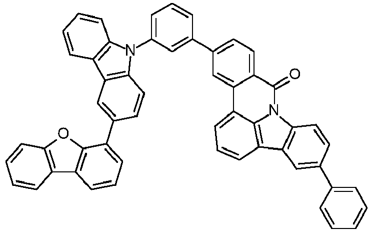 P49 |
| S114 | 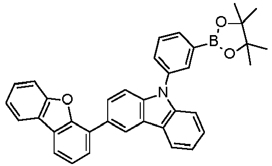 CAS-1829586-22-2 | 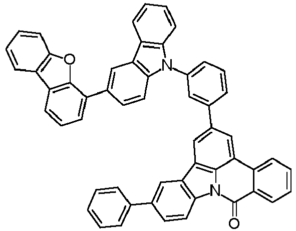 P50 |
| S115 | 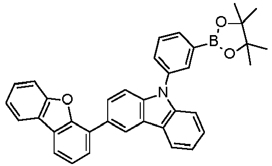 CAS-1829586-22-2 | 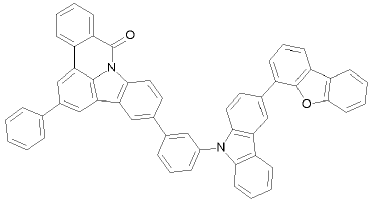 P51 |
| S103 | 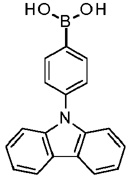 CAS-419536-33-7 | 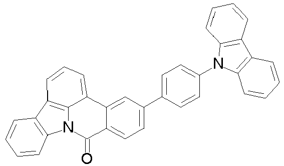 P52 |
| S103 | 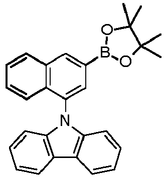 CAS-1819346-26-3 | 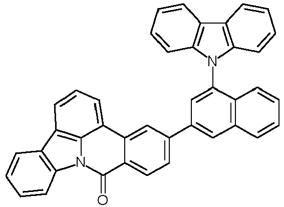 P53 |
| S103 | 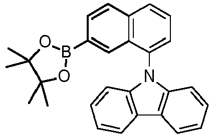 CAS-1221685-92-2 | 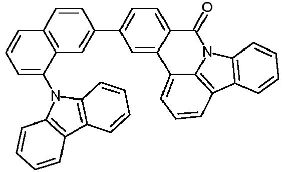 P54 |
| S103 | 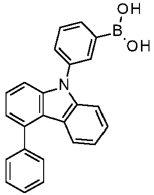 CAS-1801285-68-6 | 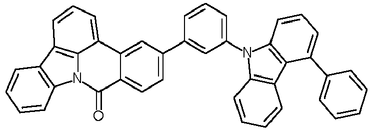 P55 |
| S103 | 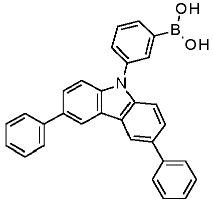 CAS-1332748-66-9 | 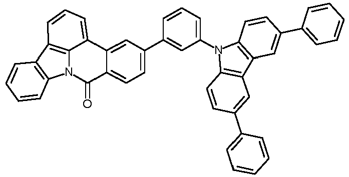 P56 |
| S103 | 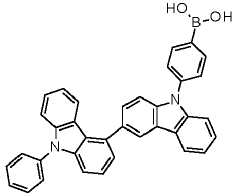 CAS-1885113-59-6 | 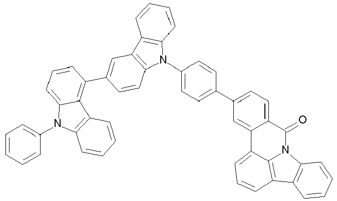 P57 |
| S103 | 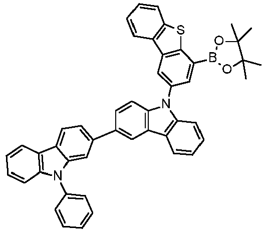 CAS-2079874-17-0 | 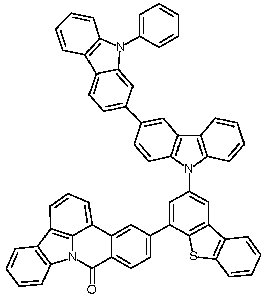 P58 |
| S103 | 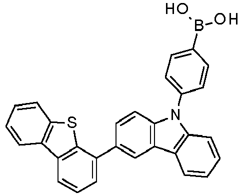 CAS-1894228-52-4 | 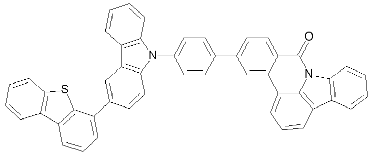 P59 |
| S108 | 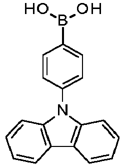 CAS-419536-33-7 | 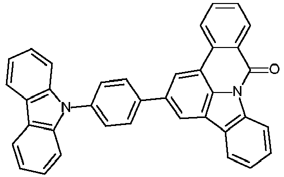 P60 |
| S108 | 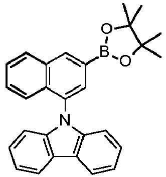 CAS-1819346-26-3 | 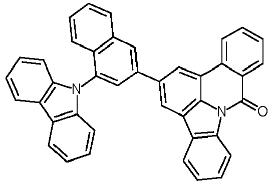 P61 |
| S108 | 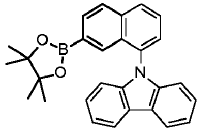 CAS-1221685-92-2 | 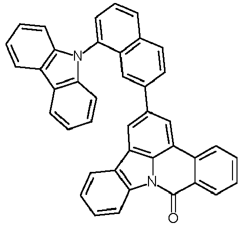 P62 |
| S108 | 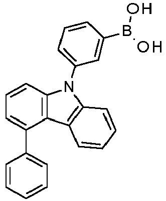 CAS-1801285-68-6 | 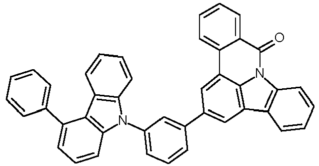 P63 |
| S108 | 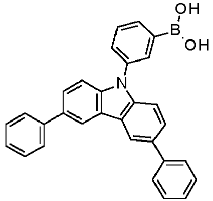 CAS-1332748-66-9 | 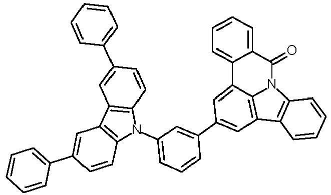 P64 |
| S108 | 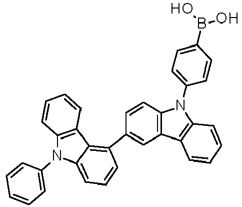 CAS-1885113-59-6 | 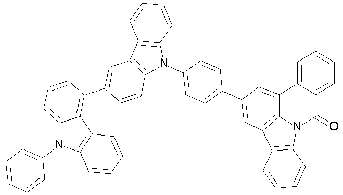 P65 |
| S108 | 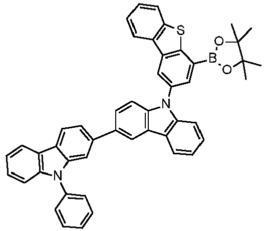 CAS-2079874-17-0 | 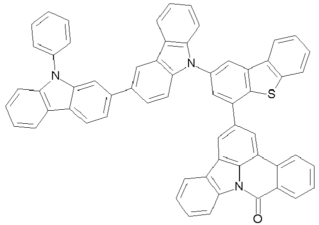 P66 |
| S108 | 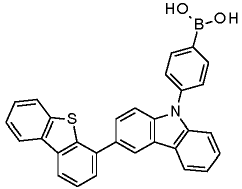 CAS-1894228-52-4 | 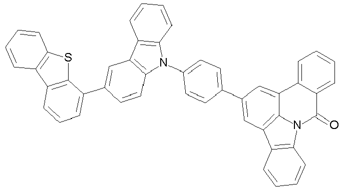 P67 |
| S110 | 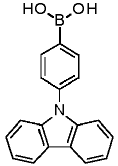 CAS-419536-33-7 | 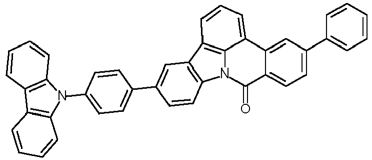 P68 |
| S110 | 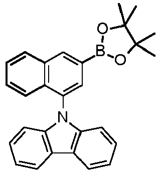 CAS-1819346-26-3 | 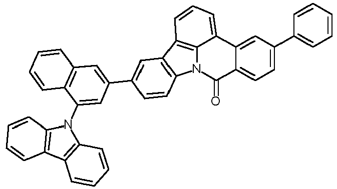 P69 |
| S110 | 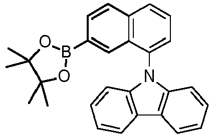 CAS-1221685-92-2 | 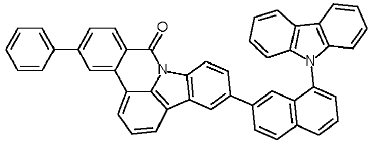 P70 |
| S110 | 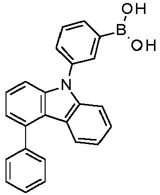 CAS-1801285-68-6 | 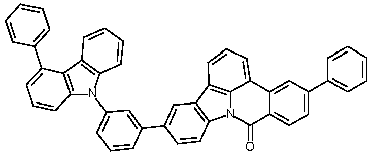 P71 |
| S110 | 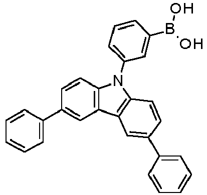 CAS-1332748-66-9 | 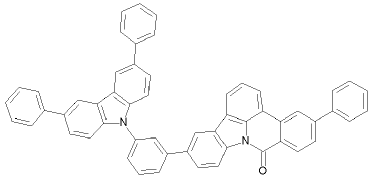 P72 |
| S110 | 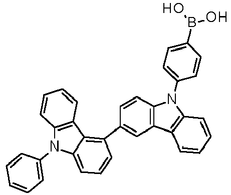 CAS-1885113-59-6 | 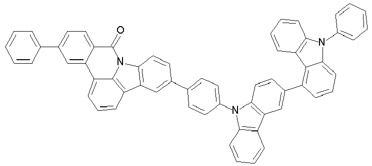 P73 |
| S110 | 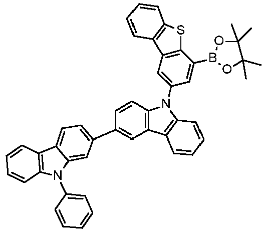 CAS-2079874-17-0 | 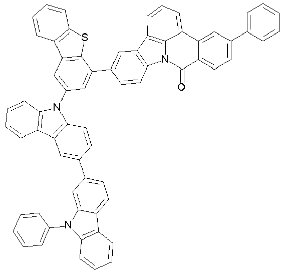 P74 |
| S110 | 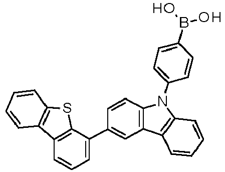 CAS-1894228-52-4 | 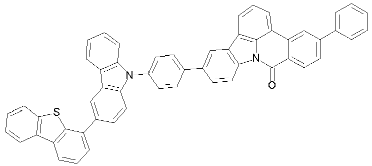 P75 |
| S114 | 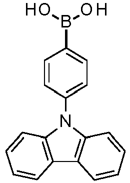 CAS-419536-33-7 | 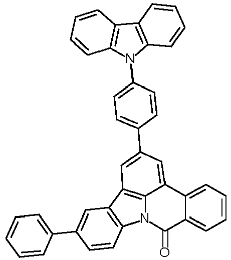 P76 |
| S114 | 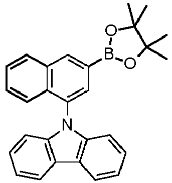 CAS-1819346-26-3 | 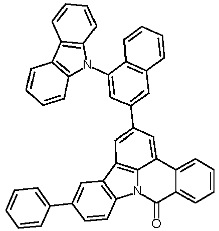 P77 |
| S114 | 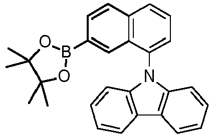 CAS-1221685-92-2 | 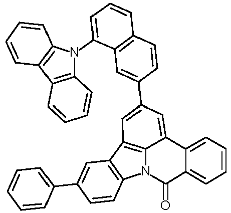 P78 |
| S114 | 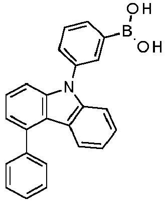 CAS-1801285-68-6 | 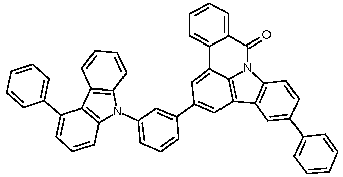 P79 |
| S114 | 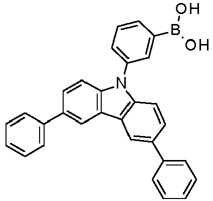 CAS-1332748-66-9 | 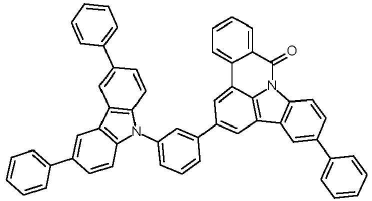 P80 |
| S114 | 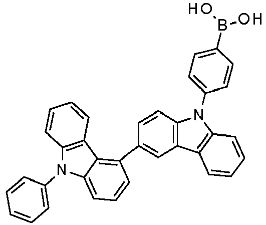 CAS-1885113-59-6 | 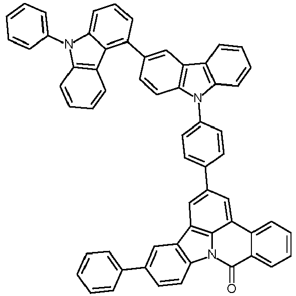 P81 |
| S114 | 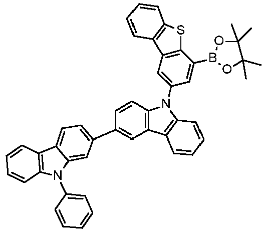 CAS-2079874-17-0 | 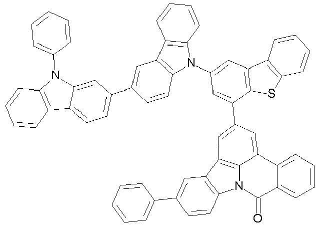 P82 |
| S114 | 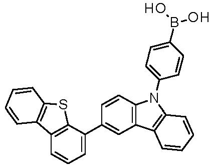 CAS-1894228-52-4 | 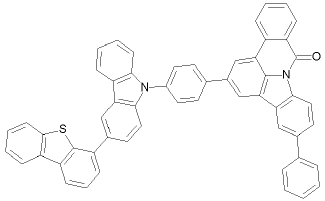 P83 |
| S115 | 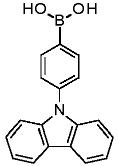 CAS-419536-33-7 | 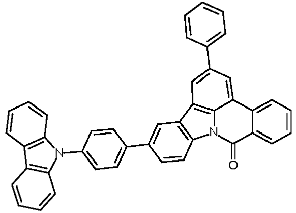 P84 |
| S115 | 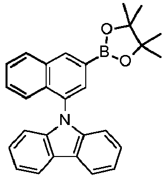 CAS-1819346-26-3 | 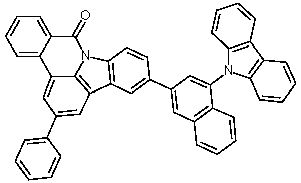 P85 |
| S115 | 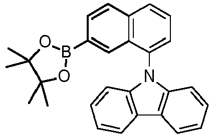 CAS-1221685-92-2 | 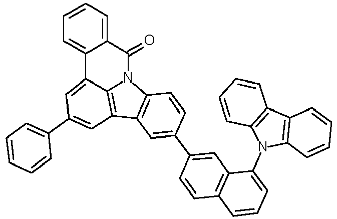 P86 |
| S115 | 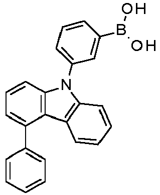 CAS-1801285-68-6 | 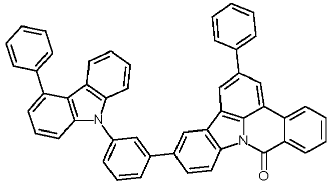 P87 |
| S115 | 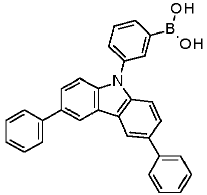 CAS-1332748-66-9 | 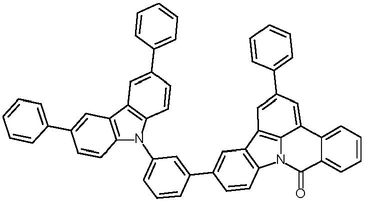 P88 |
| S115 | 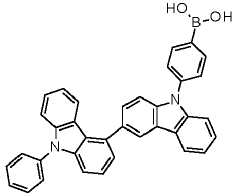 CAS-1885113-59-6 | 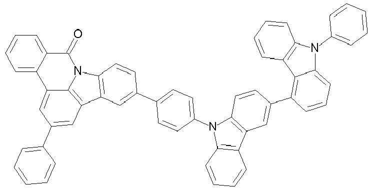 P89 |
| S115 | 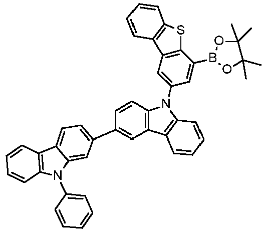 CAS-2079874-17-0 | 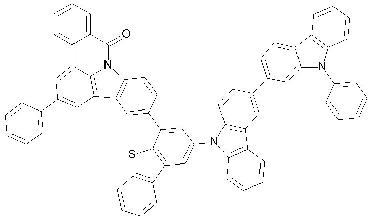 P90 |
| S115 | 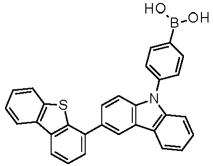 CAS-1894228-52-4 | 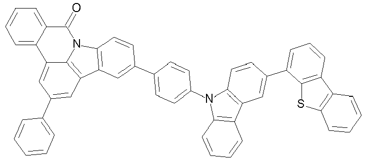 P91 |

**P200**

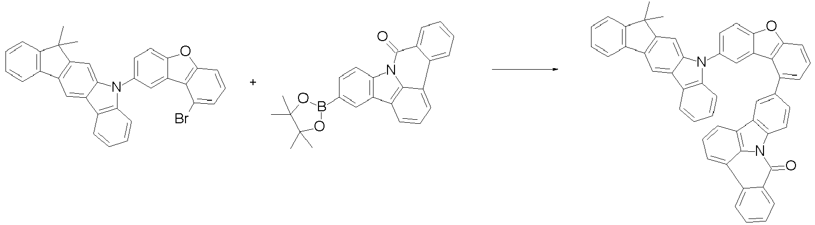

5-(9-Brom-2-dibenzofuranyl)-5,7-dihydro-7,7-dimethyl-indeno[2,1-b]-carbazol [2226483-41-4] (21.24 g, 40.2 mmol), S125 (15.89 g, 40.2 mmol) und Natriumcarbonat (8.51 g, 80.3 mmol) werden in Toluol (200 mL), 1,4-Dioxan (200 mL) und Wasser (100 mL) vorgelegt und 20 min mit Argon inertisiert. Anschließend wird Tetrakis(triphenylphosphin)-palladium(0) (928 mg, 0.80 mmol) zugegeben und das Reaktionsgemisch 32 h unter Rückfluss gerührt. Nach Abkühlen wird der ausgefallene Feststoff abgesaugt und mit Ethanol gewaschen. Das Rohprodukt wird zweimal mit Toluol heißextrahiert, anschließend dreimal aus Dimethylacetamid umkristallisiert und abschließend im Hochvakuum sublimiert. Ausbeute: 15.8 g (22.1 mmol, 55%) gelber Feststoff; Reinheit > 99.9% nach HPLC.

Katalysatorsystem für die Umsetzung von Cl statt Br:
Für die Umsetzung von Cl statt Brom wird statt Tetrakis(triphenylphosphin)-palladium(0) X-Phos [564483-18-7] oder S-Phos [657408-07-6] als Phosphinligand und Pd(OAc)₂ [3375-31-3] oder Pd₂(dba)₃ [51364-51-3] als Palladiumquelle verwendet werden. Alternativ kann auch das Katalysatorsystem Pd-X-Phos-G3 [1445085-55-1] verwendet werden. Es kann auch für die Umsetzung von Brom vorteilhaft sein, eines der letztgenannten Katalysatorsysteme zu verwenden.

Zur Aufreinigung kann auch Säulenchromatographie verwendet werden, oder zur Umkristallisation oder Heißextraktion können andere gängige Lösungsmittel wie Ethanol, Butanol, Aceton, Ethylacetat, Acetonitril, Toluol, Xylol, Dichlormethan, Methanol, Tetrahydrofuran, n-Butylacetat, 1,4-Dioxan oder zur Umkristallisation Hochsieder wie Dimethylsulfoxid, N,N-Dimethylformamid, N,N-Dimethylacetamid, N-Methylpyrrolidon, etc. verwendet werden. Die Ausbeuten liegen typischerweise im Bereich zwischen 13% und 75%.

| Edukt 1 | Edukt 2 | Produkt |
|---|---|---|
| S125 | 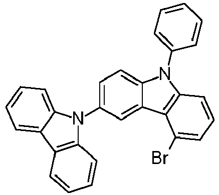 CAS-1801420-73-4 | 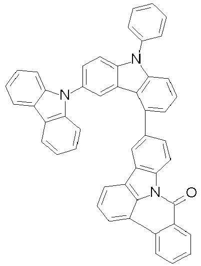 P201 |
| S125 | 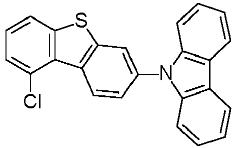 CAS-1622440-74-7 | 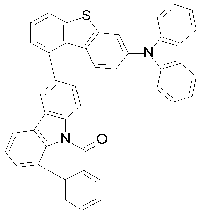 P202 |
| S125 | 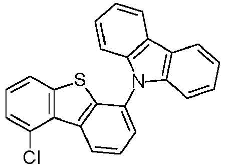 CAS-1622440-71-4 | 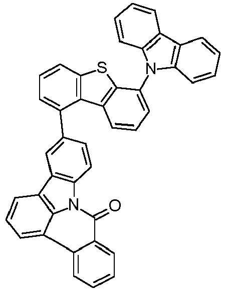 P203 |
| S125 | 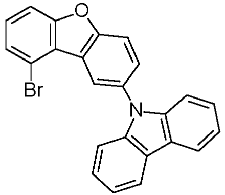 | 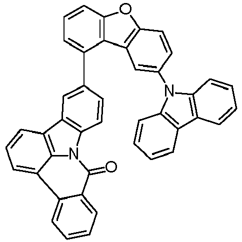 P204 |
| S125 | 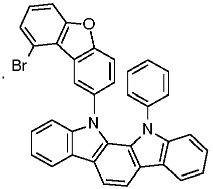 S151 | 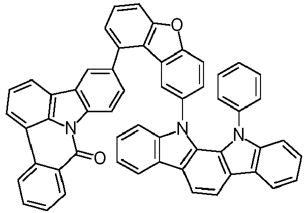 P205 |
| S125 | 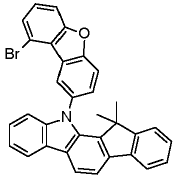 S152 | 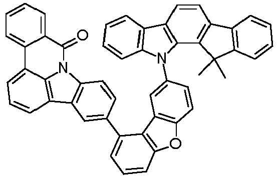 P206 |
| S125 | 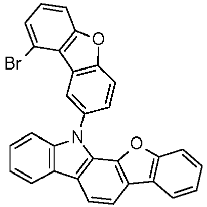 S153 | 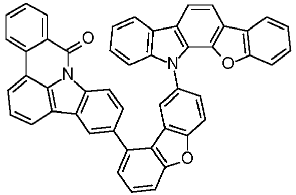 P207 |
| S125 | 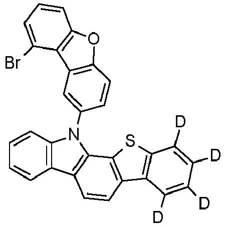 S154 | 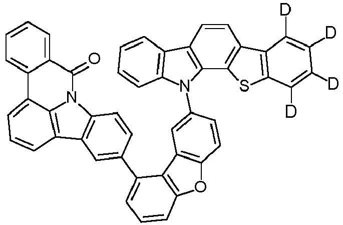 P208 |
| S125 | 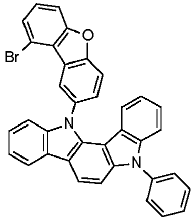 S155 | 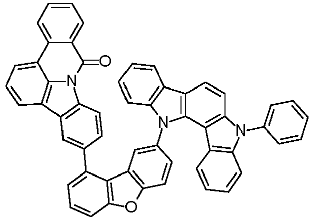 P209 |
| S125 | 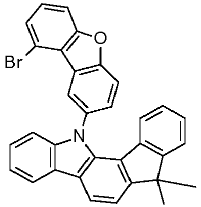 S156 | 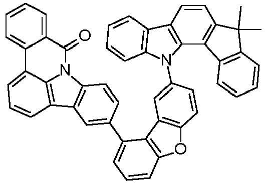 P210 |
| S125 | 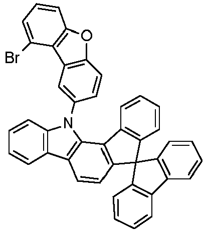 S157 | 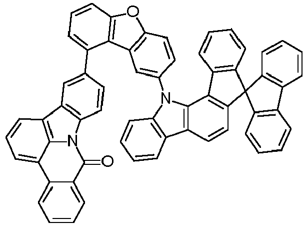 P211 |
| S125 | 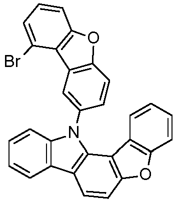 S158 | 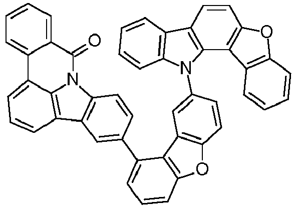 P212 |
| S125 | 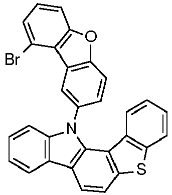 S159 | 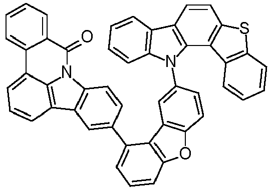 P213 |
| S125 | 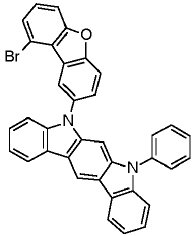 S160 | 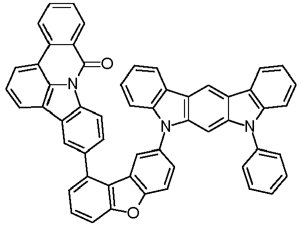 P214 |
| S125 | 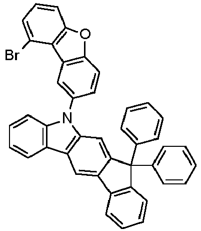 S161 | 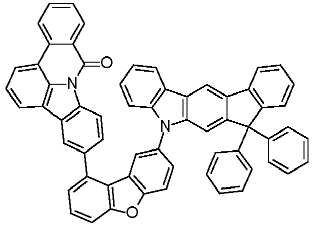 P215 |
| S125 | 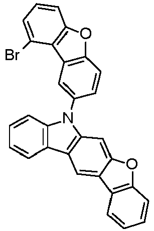 S162 | 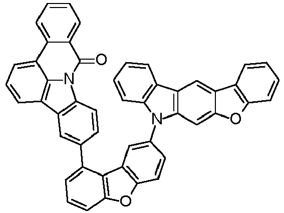 P216 |
| S125 | 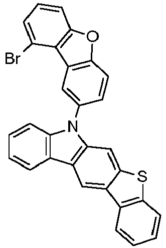 S163 | 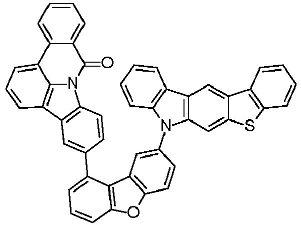 P217 |
| S125 | 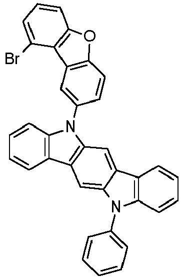 S164 | 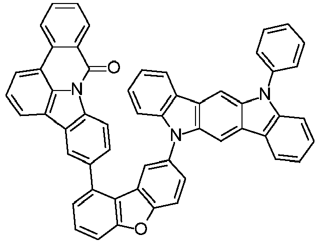 P218 |
| S125 | 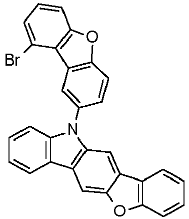 S165 | 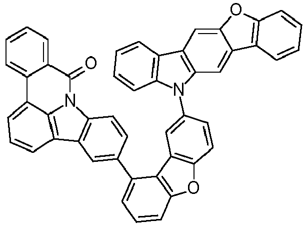 P219 |
| S125 | 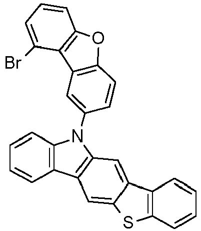 S166 | 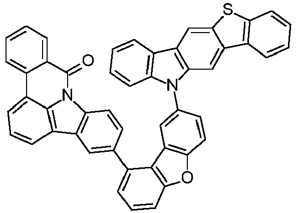 P220 |
| S125 | 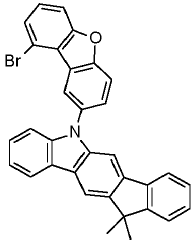 S167 | 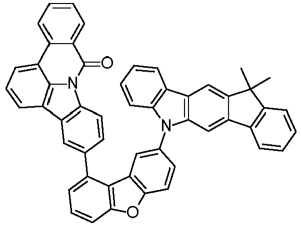 P221 |
| S125 | 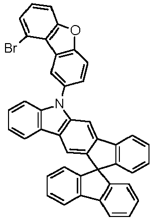 S168 | 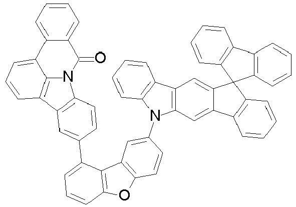 P222 |
| S125 | 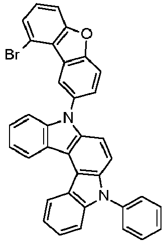 S169 | 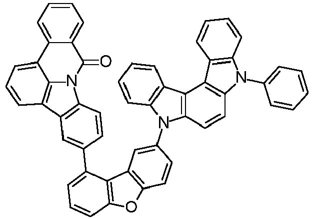 P223 |
| S125 | 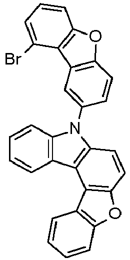 S170 | 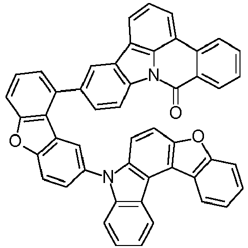 P224 |
| S125 | 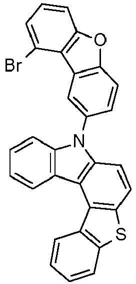 S171 | 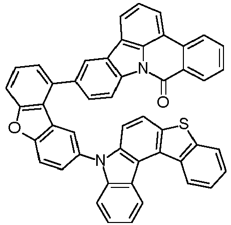 P225 |
| S125 | 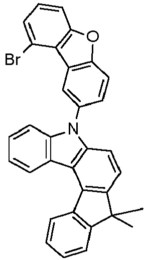 S172 | 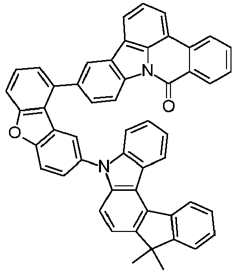 P226 |
| S125 | 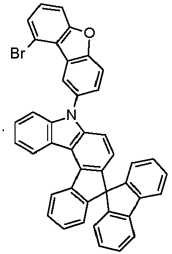 S173 | 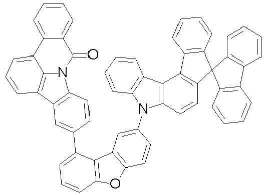 P227 |
| S125 | 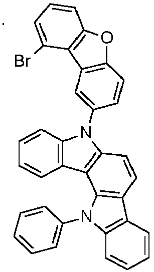 S174 | 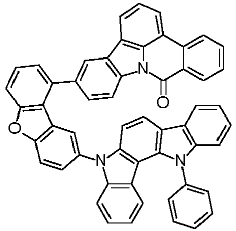 P228 |
| S125 | 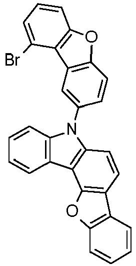 S175 | 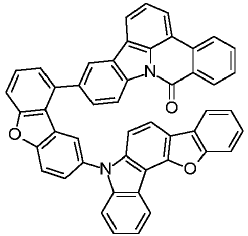 P229 |
| S125 | 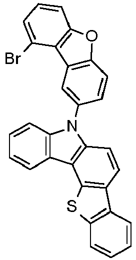 S176 | 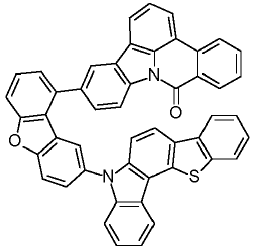 P230 |
| S125 | 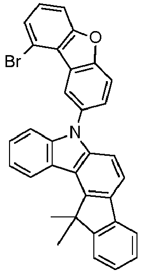 S177 | 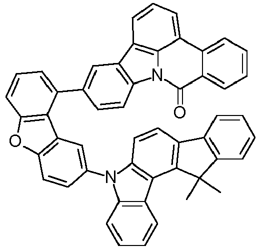 P231 |
| S125 | 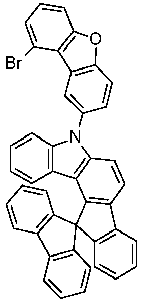 S178 | 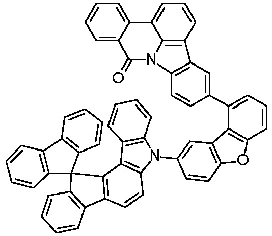 P232 |
| S125 | 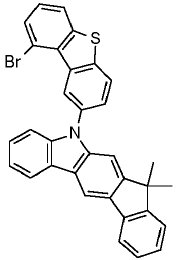 S179 | 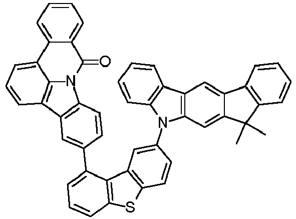 P233 |
| S125 | 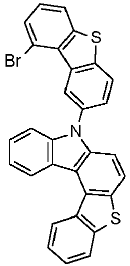 S180 | 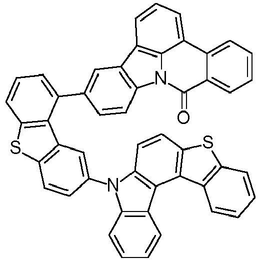 P234 |
| S125 | 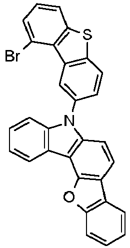 S181 | 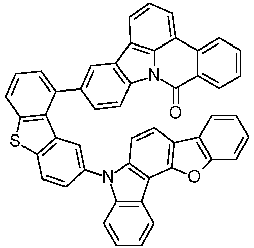 P235 |
| S125 | 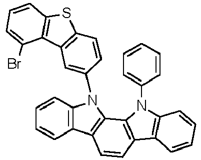 S182 | 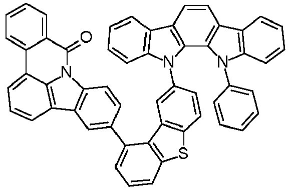 P236 |
| S125 | 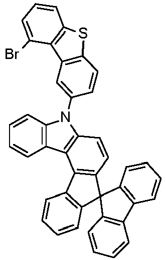 S183 | 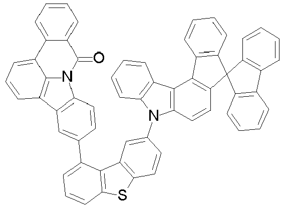 P237 |
| S125 | 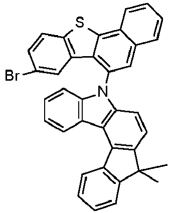 S184 | 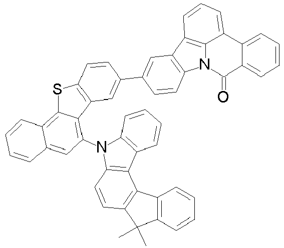 P238 |
| S125 | 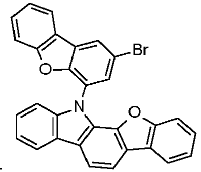 S185 | 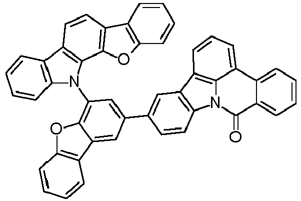 P239 |
| S125 | 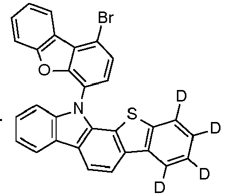 S186 | 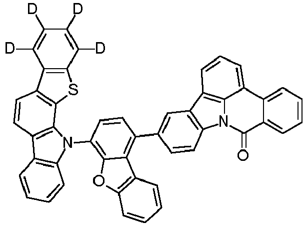 P240 |
| S125 | 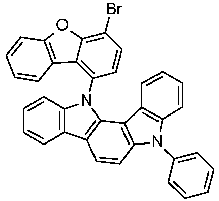 S187 | 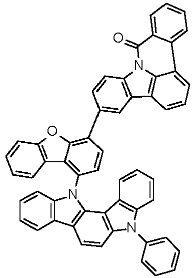 P241 |
| S125 | 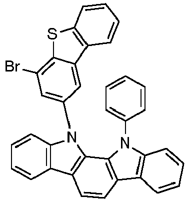 S188 | 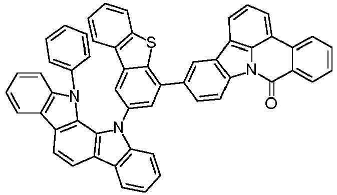 P242 |
| S125 | 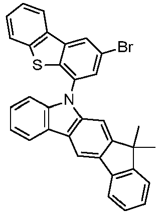 S189 | 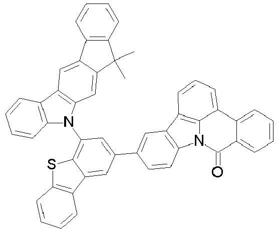 P243 |
| S125 | 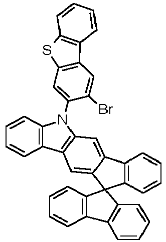 S190 | 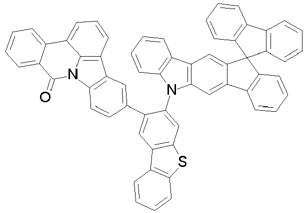 P244 |
| S125 | 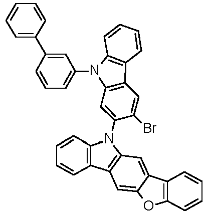 S191 | 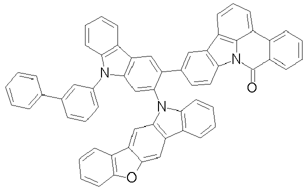 P245 |
| S125 | 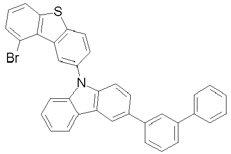 S192 | 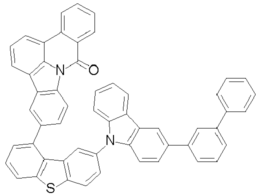 P246 |
| S125 | 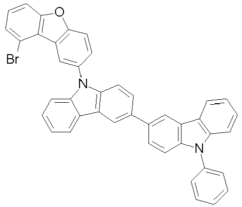 S193 | 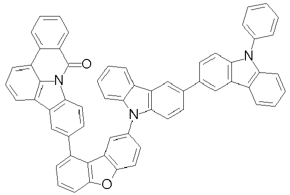 P247 |
| S125 | 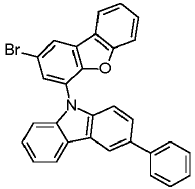 S194 | 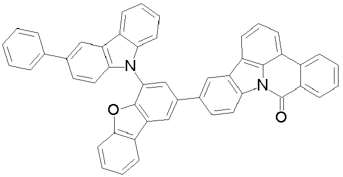 P248 |
| S125 | 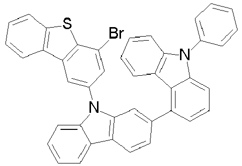 S195 | 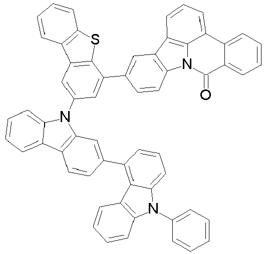 P249 |
| S125 | 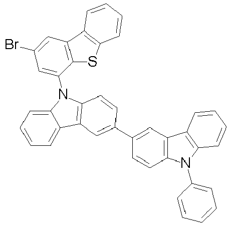 S196 | 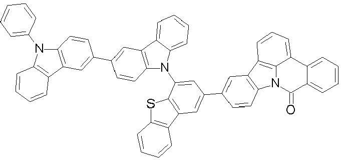 P250 |
| S126 | 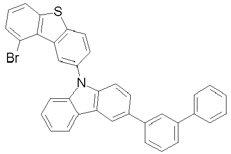 S192 | 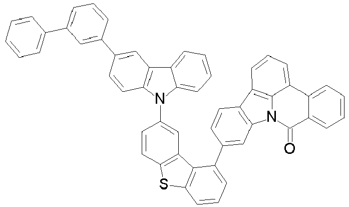 P251 |
| S127 | 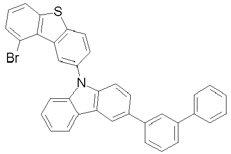 S192 | 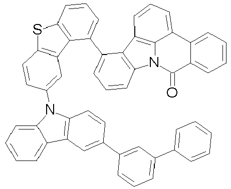 P252 |
| S128 | 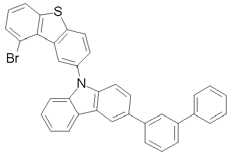 S192 | 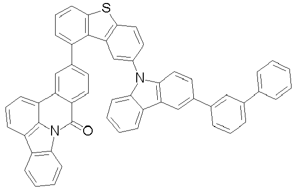 P253 |
| S129 | 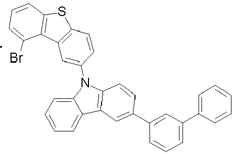 S192 | 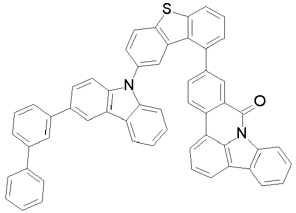 P254 |
| S130 | 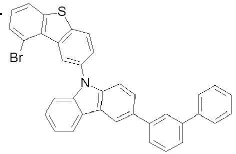 S192 | 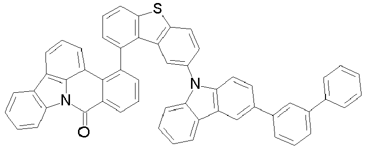 P255 |
| S131 | 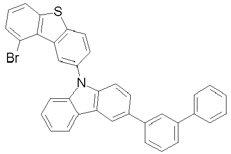 S192 | 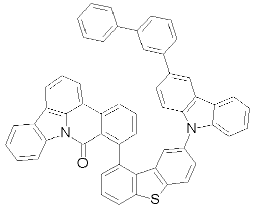 P256 |
| S132 | 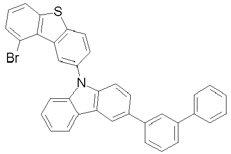 S192 | 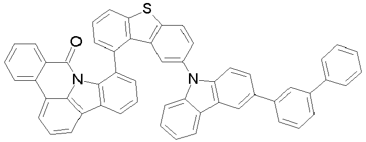 P257 |
| S133 | 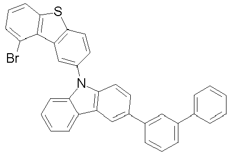 S192 | 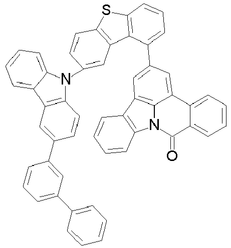 P258 |
| S134 | 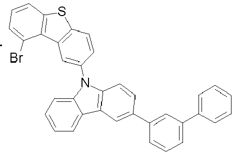 S192 | 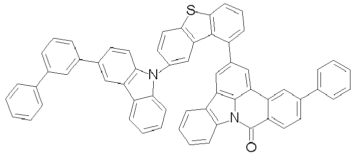 P259 |
| S135 | 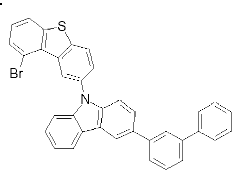 S192 | 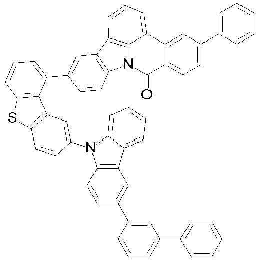 P260 |
| S136 | 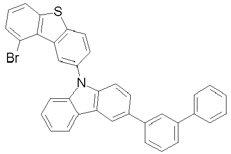 S192 | 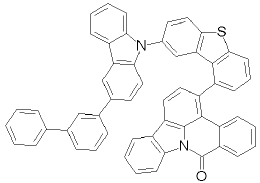 P261 |
| S137 | 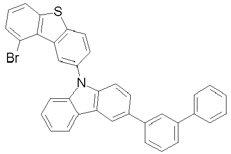 S192 | 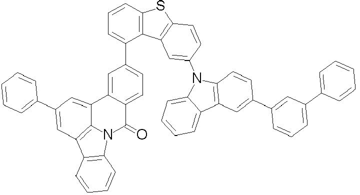 P262 |
| S138 | 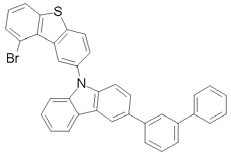 S192 | 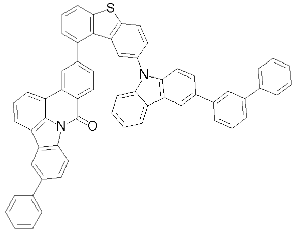 P263 |
| S139 | 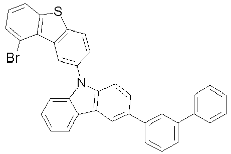 S192 | 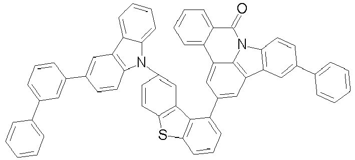 P264 |
| S140 | 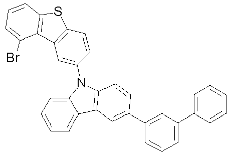 S192 | 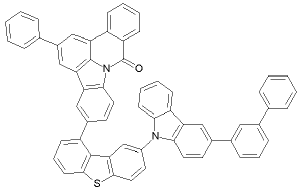 P265 |
| S126 | 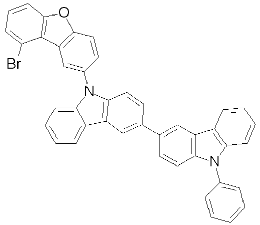 S193 | 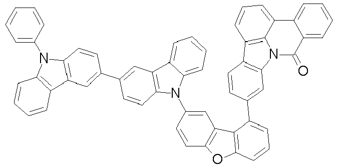 P266 |
| S127 | 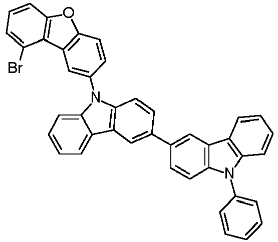 | 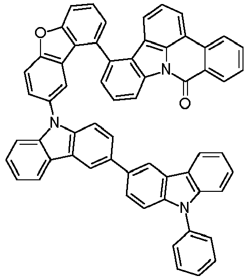 P267 |
| S128 | 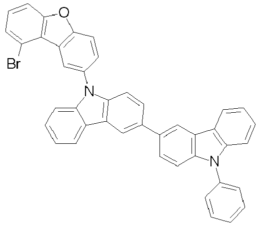 S193 | 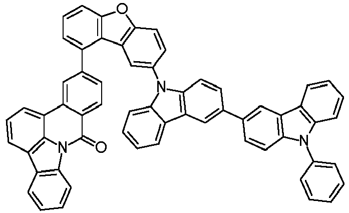 P268 |
| S129 | 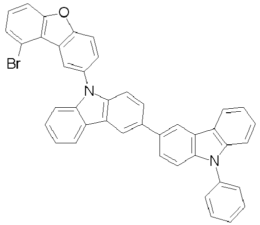 | 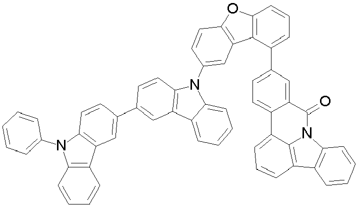 P269 |
| S130 | 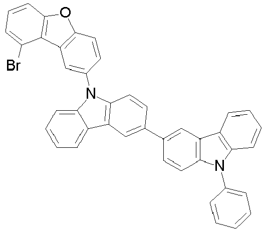 S193 | 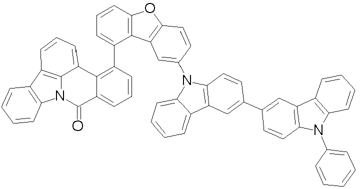 P270 |
| S131 | 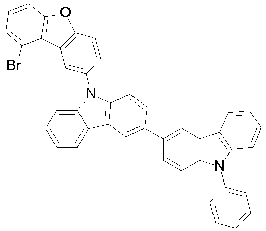 | 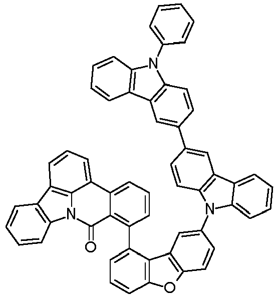 P271 |
| S132 | 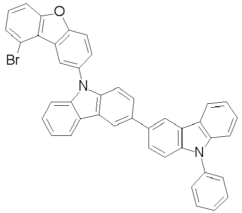 S193 | 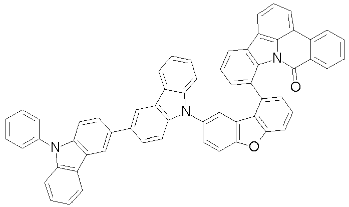 P272 |
| S133 | 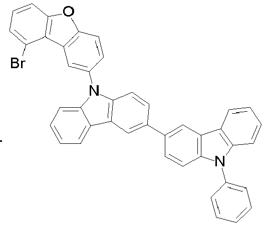 | 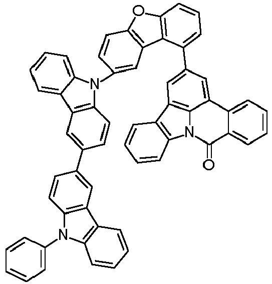 P273 |
| S134 | 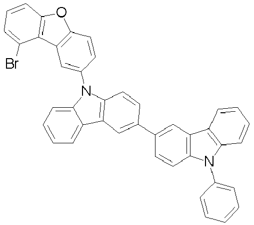 S193 | 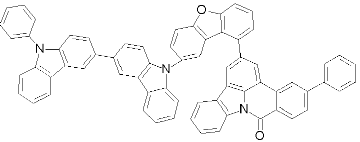 P274 |
| S135 | 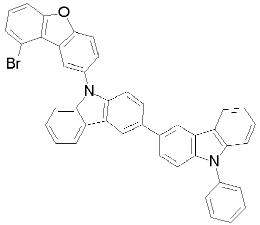 S193 | 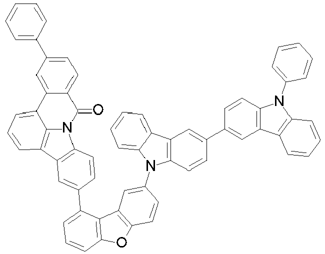 P275 |
| S136 | 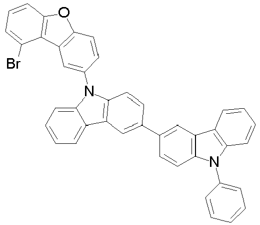 S193 | 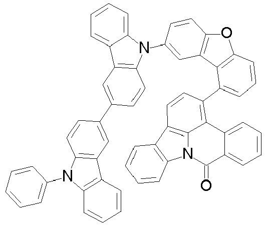 P276 |
| S137 | 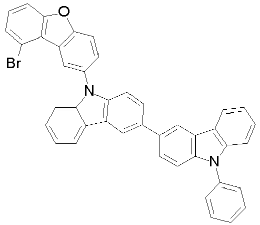 S193 | 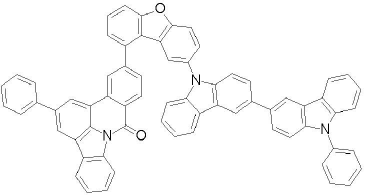 P277 |
| S138 | 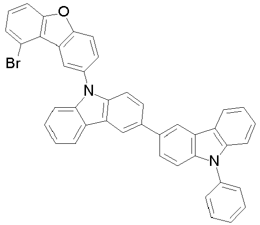 S193 | 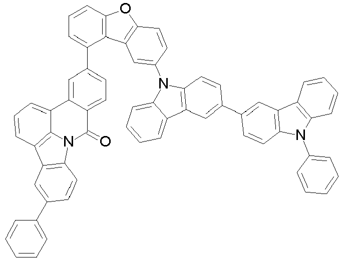 P278 |
| S139 | 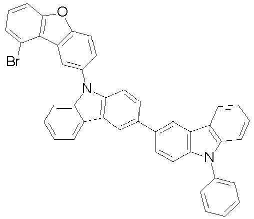 S193 | 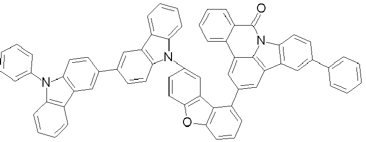 P279 |
| S140 | 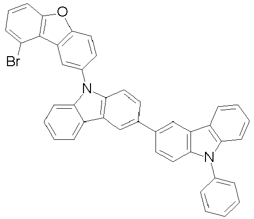 | 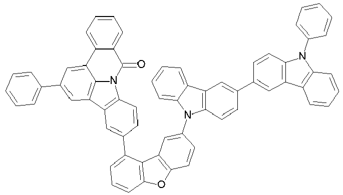 P280 |
| S128 | 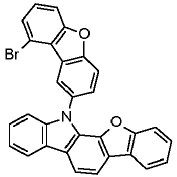 S153 | 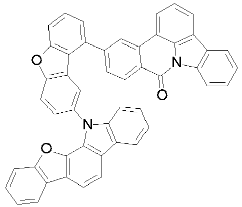 P281 |
| S128 | 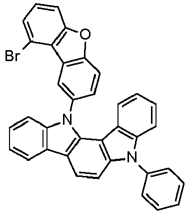 S155 | 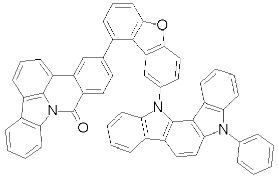 P282 |
| S128 | 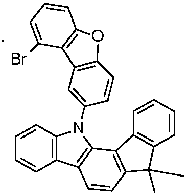 S156 | 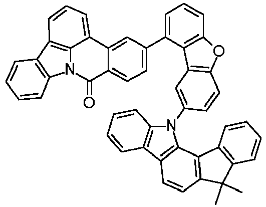 P283 |
| S128 | 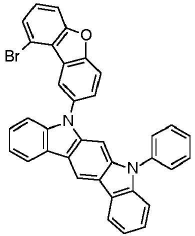 S160 | 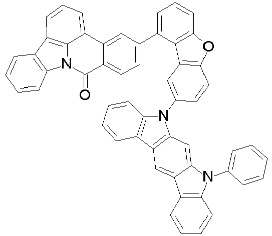 P284 |
| S128 | 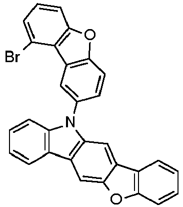 S165 | 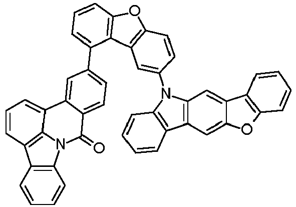 P285 |
| S128 | 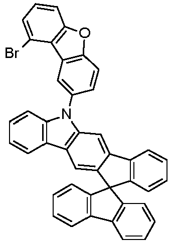 S168 | 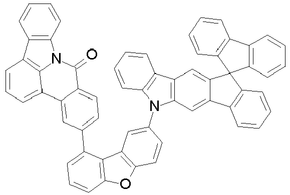 P286 |
| S128 | 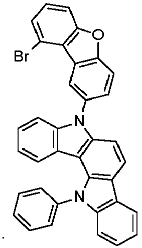 S174 | 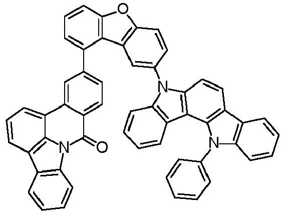 P287 |
| S128 | 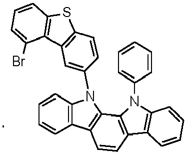 S182 | 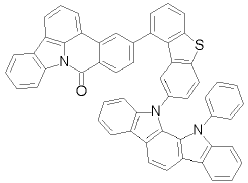 P288 |
| S128 | 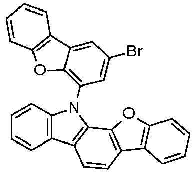 S185 | 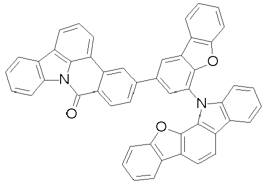 P289 |
| S128 | 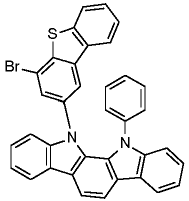 S188 | 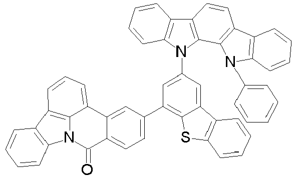 P290 |
| S128 | 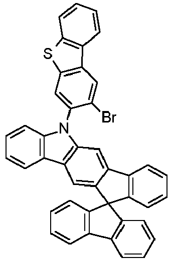 S190 | 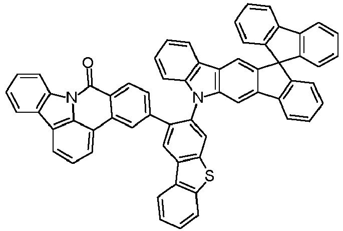 P291 |
| S128 | 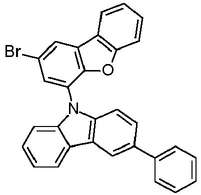 S194 | 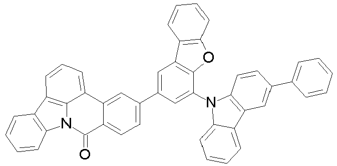 P292 |
| S133 | 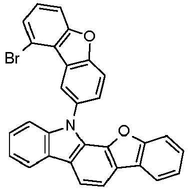 S153 | 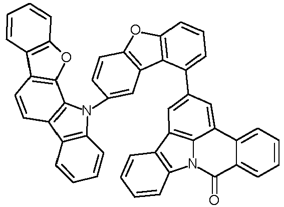 P293 |
| S133 | 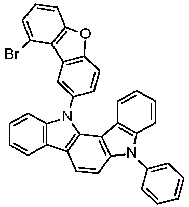 S155 | 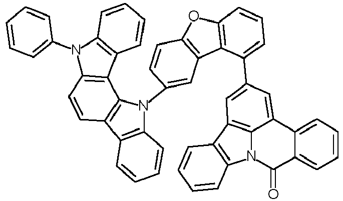 P295 |
| S133 | 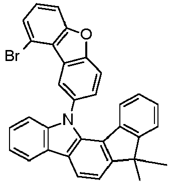 S156 | 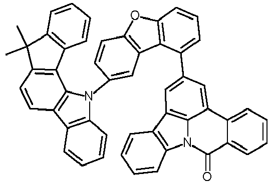 P296 |
| S133 | 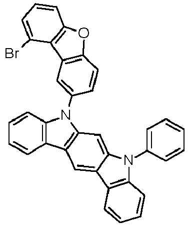 S160 | 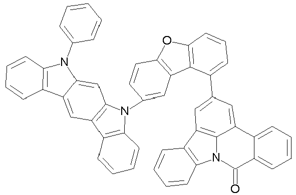 P297 |
| S133 | 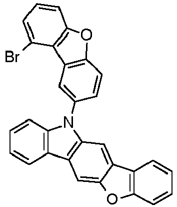 S165 | 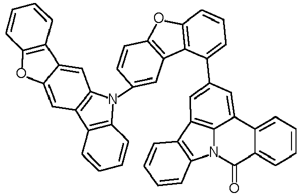 P298 |
| S133 | 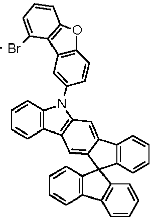 S168 | 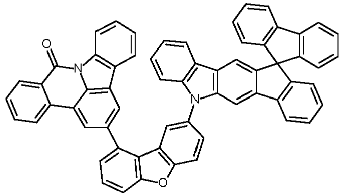 P299 |
| S133 | 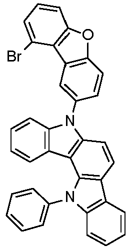 S174 | 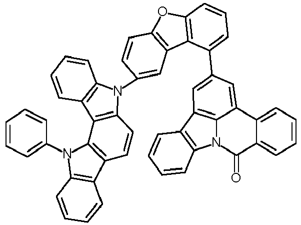 P300 |
| S133 | 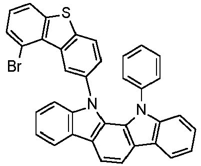 | 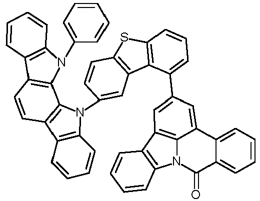 P301 |
| S133 | 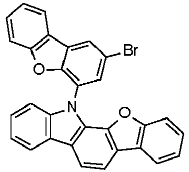 S185 | 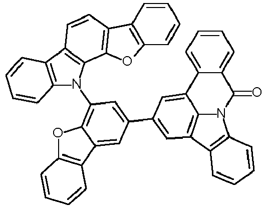 P302 |
| S133 | 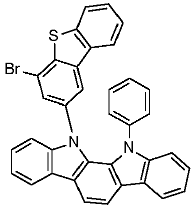 S188 | 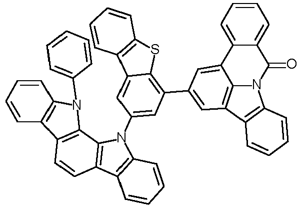 P303 |
| S133 | 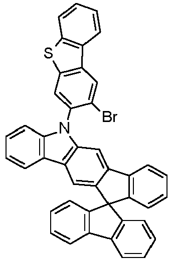 S190 | 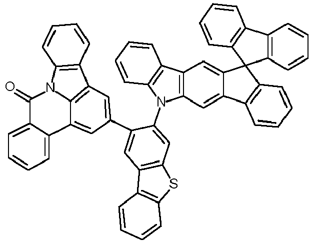 P304 |
| S133 | 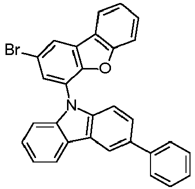 S194 | 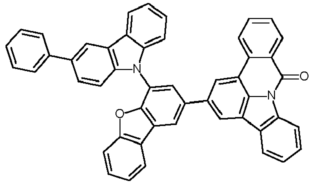 P305 |
| S135 | 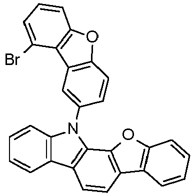 S153 | 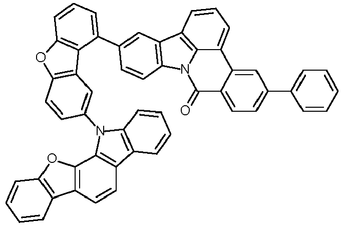 P306 |
| S135 | 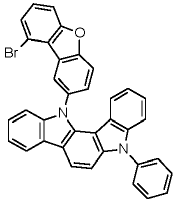 S155 | 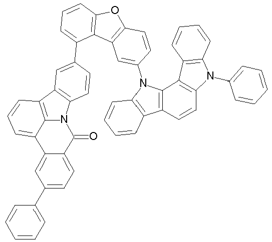 P307 |
| S135 | 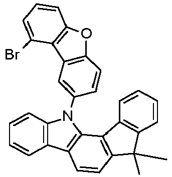 S156 | 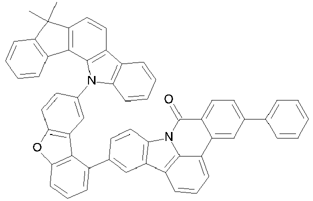 P308 |
| S135 | 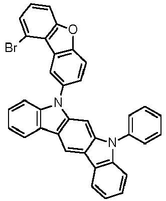 S160 | 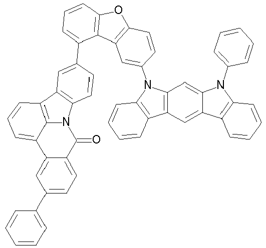 P309 |
| S135 | 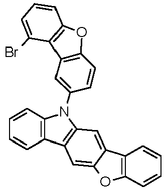 S165 | 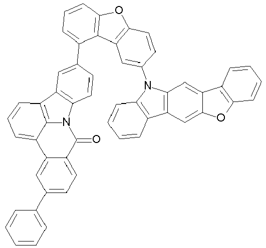 P310 |
| S135 | 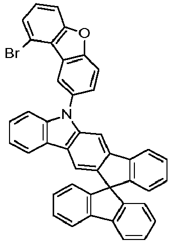 S168 | 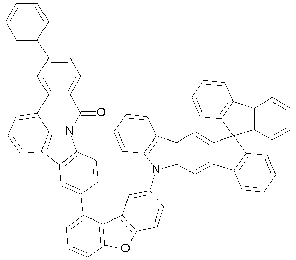 P311 |
| S135 | 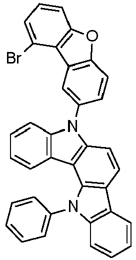 S174 | 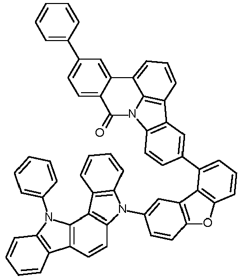 P312 |
| S135 | 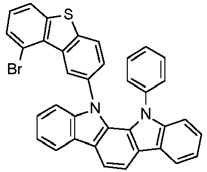 S182 | 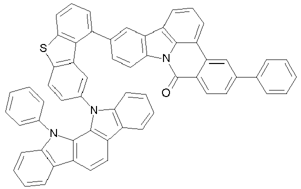 P313 |
| S135 | 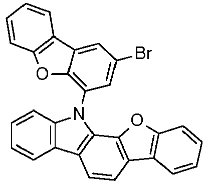 S185 | 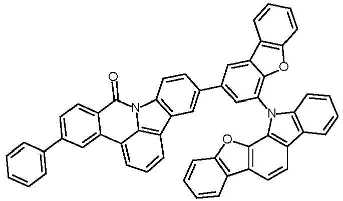 P314 |
| S135 | 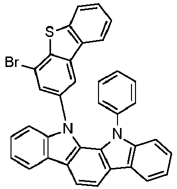 S188 | 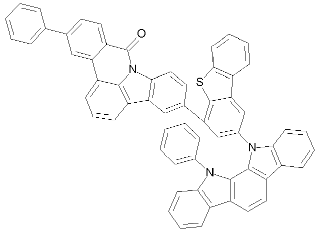 P315 |
| S135 | 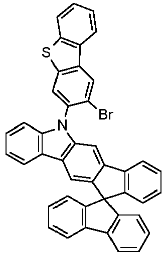 S190 | 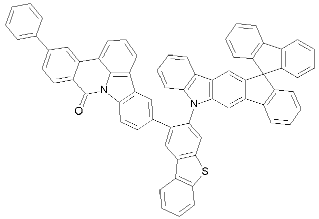 P316 |
| S135 | 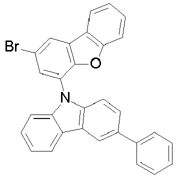 S194 | 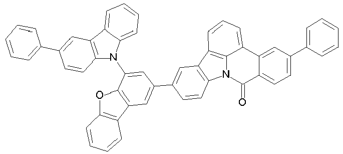 P317 |
| S139 | 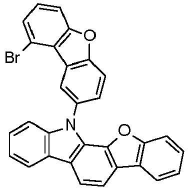 S153 | 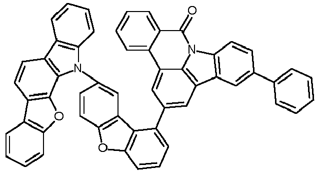 P318 |
| S139 | 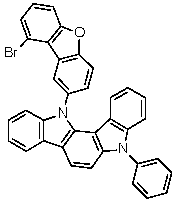 S155 | 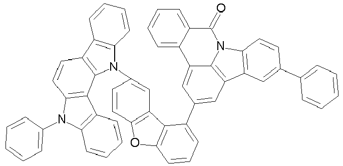 P319 |
| S139 | 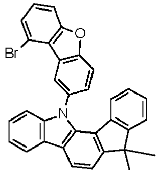 S156 | 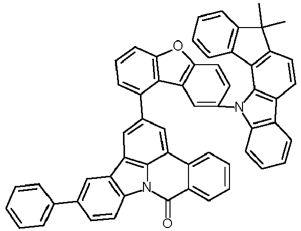 P320 |
| S139 | 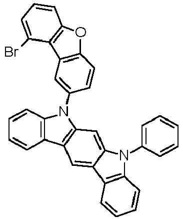 S160 | 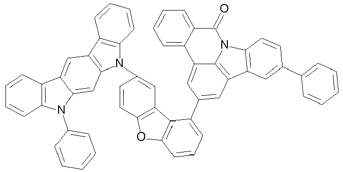 P321 |
| S139 | 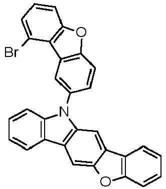 S165 | 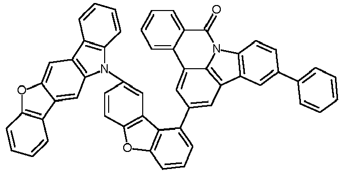 P322 |
| S139 | 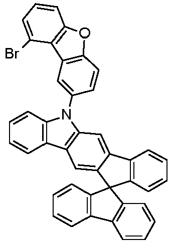 S168 | 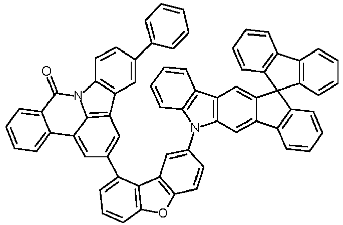 P323 |
| S139 | 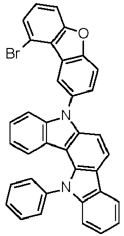 S174 | 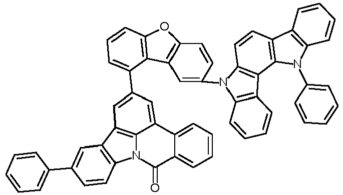 P324 |
| S139 | 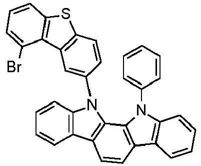 S182 | 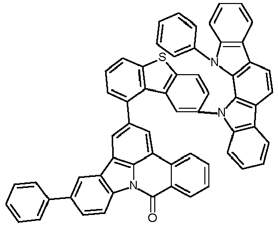 P325 |
| S139 | 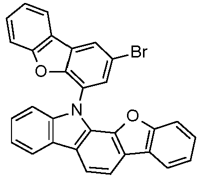 S185 | 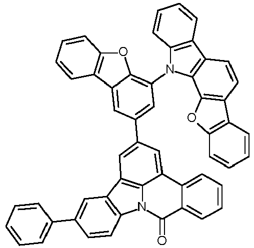 P326 |
| S139 | 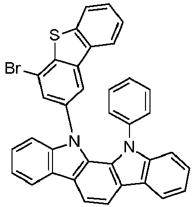 S188 | 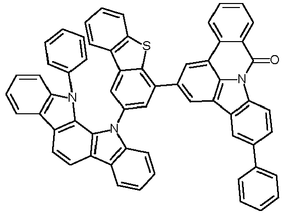 P327 |
| S139 | 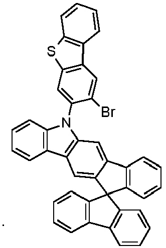 S190 | 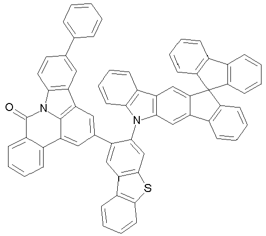 P328 |
| S139 | 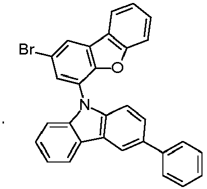 S194 | 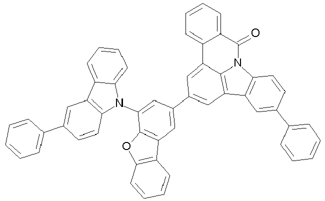 P329 |
| S140 | 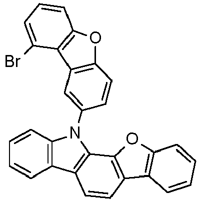 S153 | 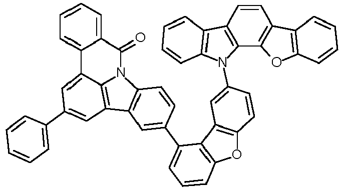 P330 |
| S140 | 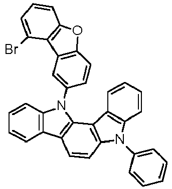 S155 | 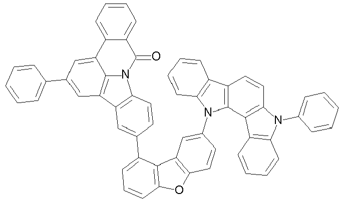 P331 |
| S140 | 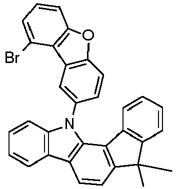 S156 | 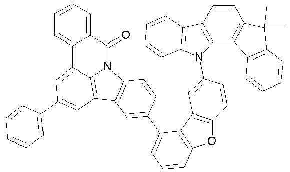 P332 |
| S140 | 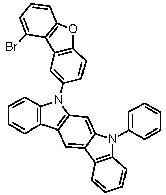 S160 | 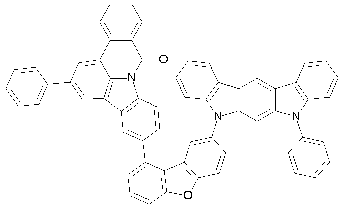 P333 |
| S140 | 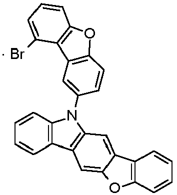 S165 | 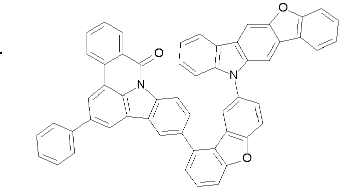 P334 |
| S140 | 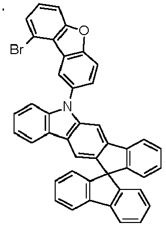 S168 | 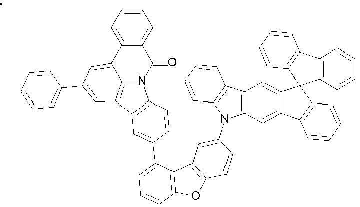 P335 |
| S140 | 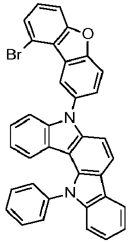 S174 | 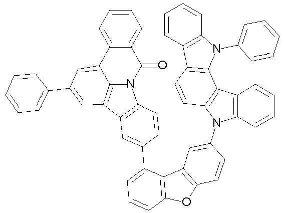 P336 |
| S140 | 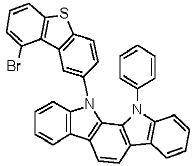 S182 | 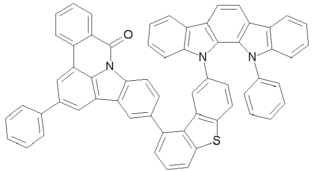 P337 |
| S140 | 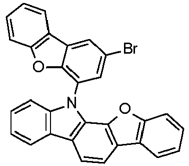 S185 | 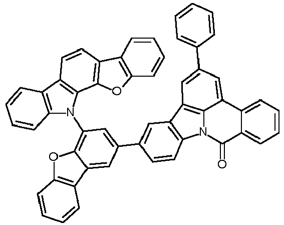 P338 |
| S140 | 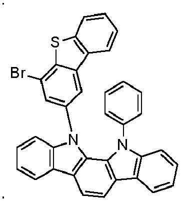 S188 | 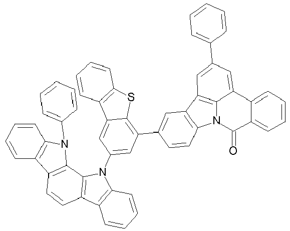 P339 |
| S140 | 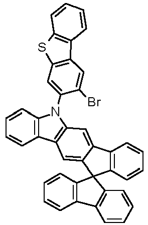 S190 | 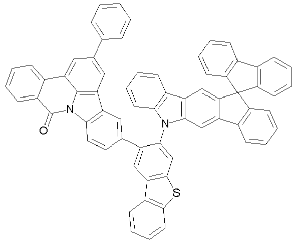 P340 |
| S140 | 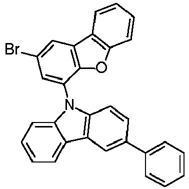 S194 | 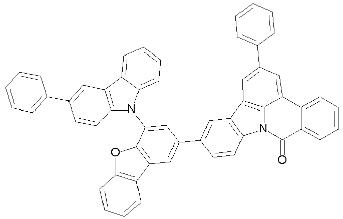 P341 |
| S128 | 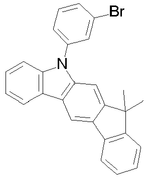 CAS-1369587-63-2 | 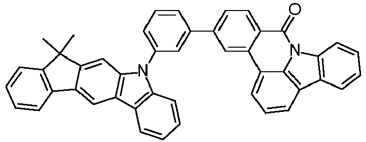 P342 |
| S128 | 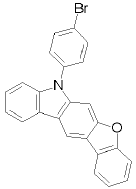 CAS-2079877-31-7 | 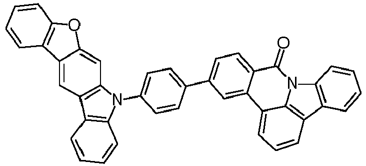 P343 |
| S128 | 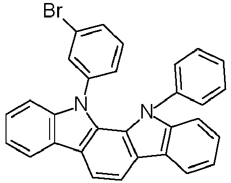 CAS-1260032-02-7 | 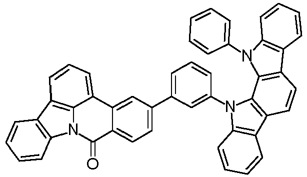 P344 |
| S128 | 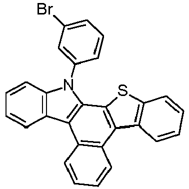 CAS-1644466-89-6 | 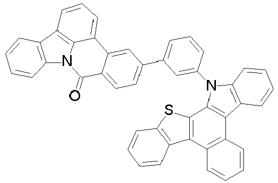 P345 |
| S128 | 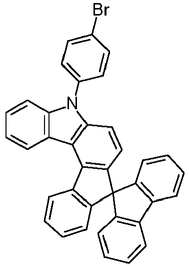 CAS-2095194-33-3 | 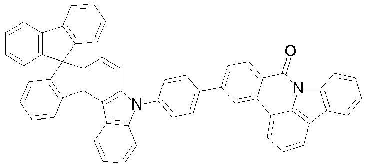 P346 |
| S128 | 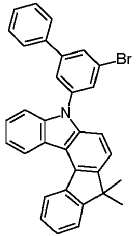 CAS-2134579-41-0 | 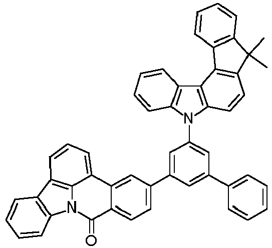 P347 |
| S128 | 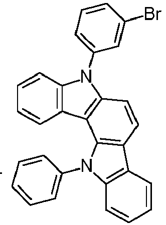 CAS-1782949-43-2 | 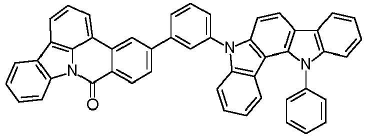 P348 |
| S128 | 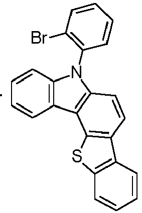 CAS-1622084-14-3 | 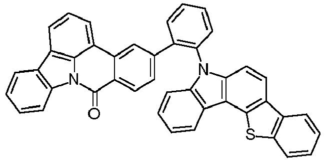 P349 |
| S128 | 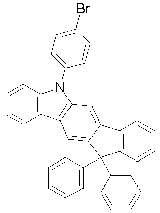 CAS-2249834-37-3 | 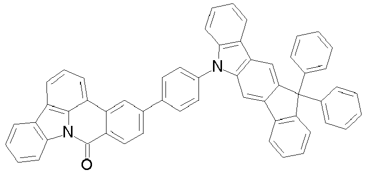 P350 |
| S128 | 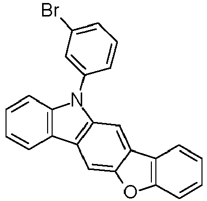 CAS-2109712-47-0 | 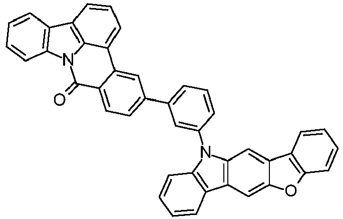 P351 |
| S128 | 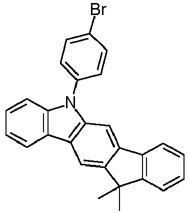 CAS-2249834-37-3 | 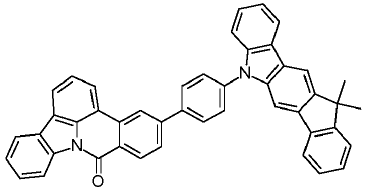 P352 |
| S128 | 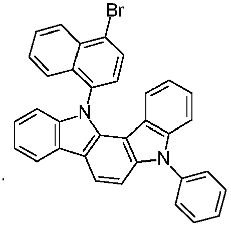 CAS-1632353-57-1 | 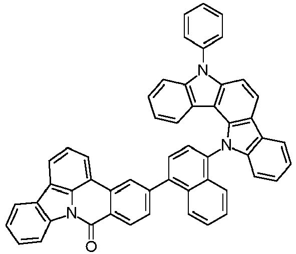 P353 |
| S128 | 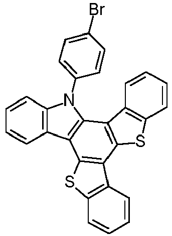 CAS-1534332-59-6 | 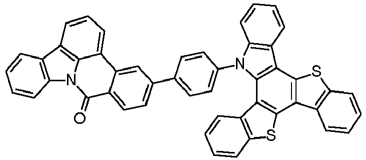 P354 |
| S128 | 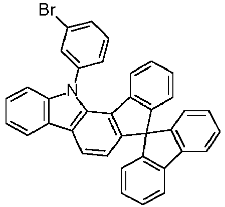 CAS-2101865-23-8 | 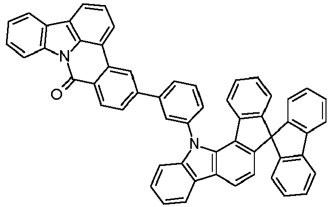 P355 |
| S133 | 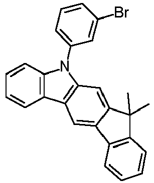 CAS-1369587-63-2 | 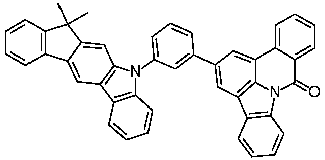 P356 |
| S133 | 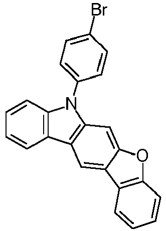 CAS-2079877-31-7 | 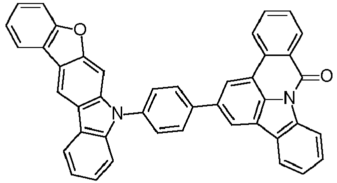 P357 |
| S133 | 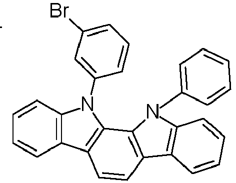 CAS-1260032-02-7 | 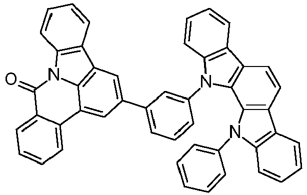 P358 |
| S133 | 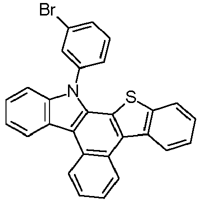 CAS-1644466-89-6 | 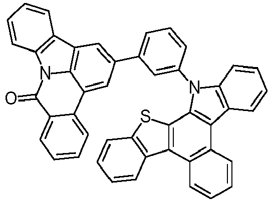 P359 |
| S133 | 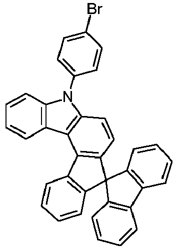 CAS-2095194-33-3 | 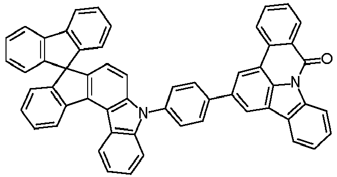 P360 |
| S133 | 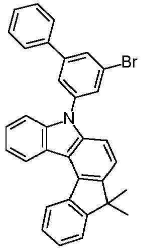 CAS-2134579-41-0 | 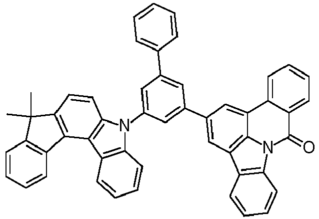 P361 |
| S133 | 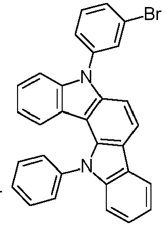 CAS-1782949-43-2 | 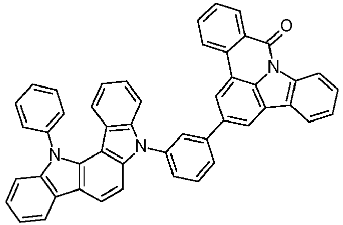 P362 |
| S133 | 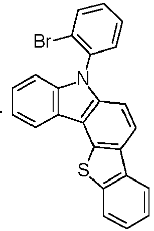 CAS-1622084-14-3 | 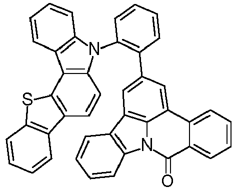 P363 |
| S133 | 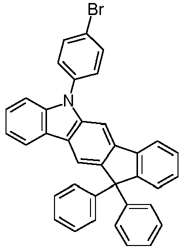 CAS-2249834-37-3 | 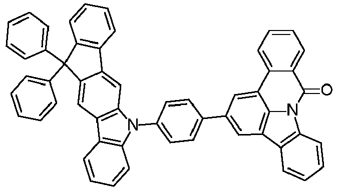 P364 |
| S133 | 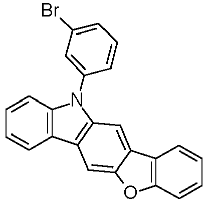 CAS-2109712-47-0 | 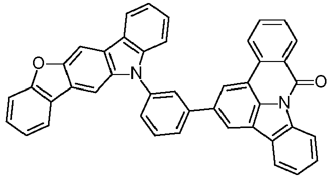 P365 |
| S133 | 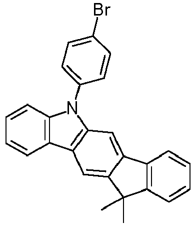 CAS-2249834-37-3 | 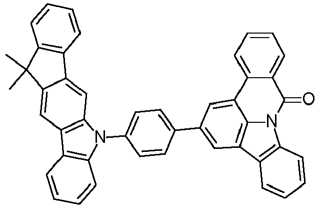 P366 |
| S133 | 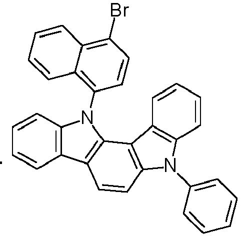 CAS-1632353-57-1 | 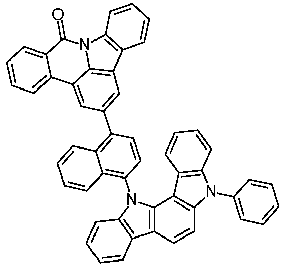 P367 |
| S133 | 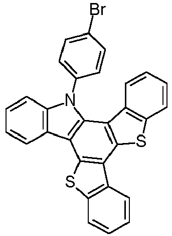 CAS-1534332-59-6 | 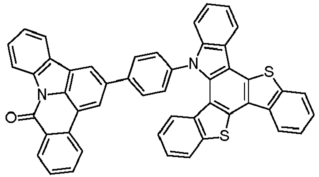 P368 |
| S133 | 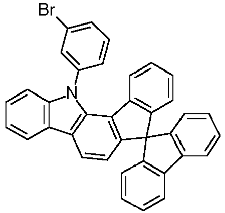 CAS-2101865-23-8 | 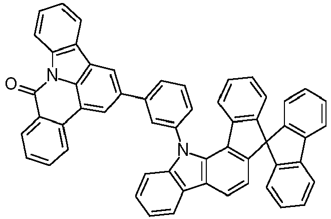 P369 |
| S135 | 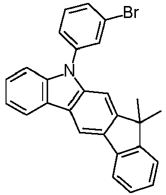 CAS-1369587-63-2 | 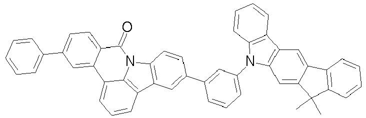 P370 |
| S135 | 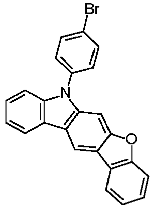 CAS-2079877-31-7 | 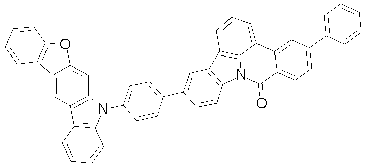 P371 |
| S135 | 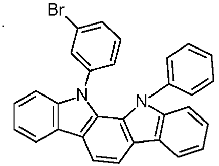 CAS-1260032-02-7 | 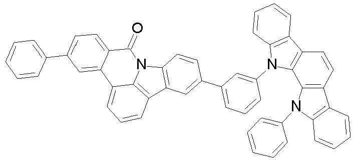 P372 |
| S135 | 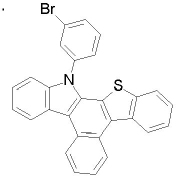 CAS-1644466-89-6 | 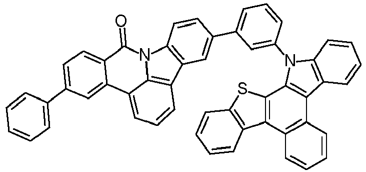 P373 |
| S135 | 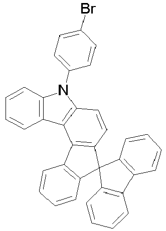 CAS-2095194-33-3 | 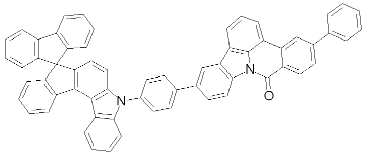 P374 |
| S135 | 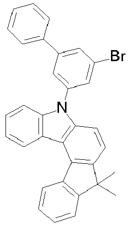 CAS-2134579-41-0 | 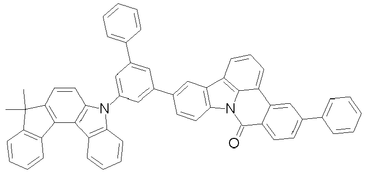 P375 |
| S135 | 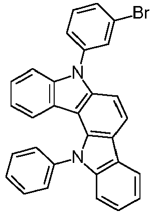 CAS-1782949-43-2 | 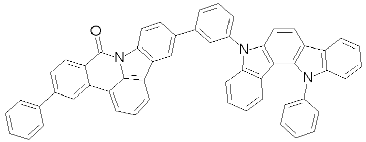 P376 |
| S135 | 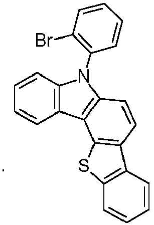 CAS-1622084-14-3 | 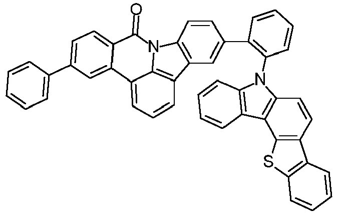 P377 |
| S135 | 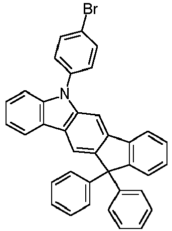 CAS-2249834-37-3 | 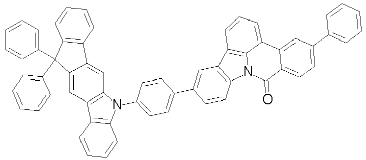 P378 |
| S135 | 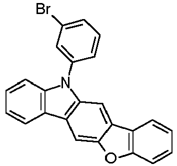 CAS-2109712-47-0 | 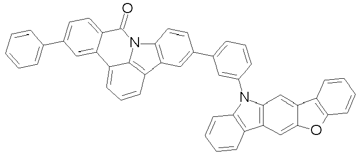 P379 |
| S135 | 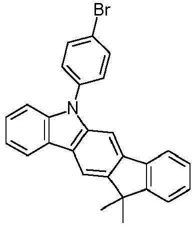 CAS-2249834-37-3 | 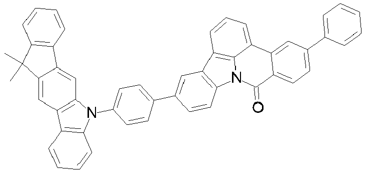 P380 |
| S135 | 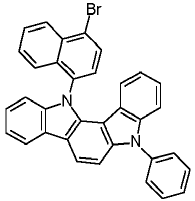 CAS-1632353-57-1 | 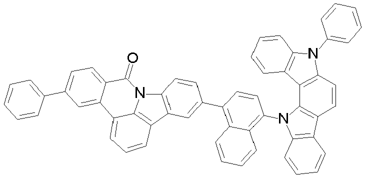 P381 |
| S135 | 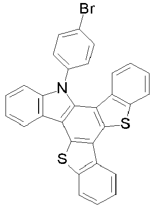 CAS-1534332-59-6 | 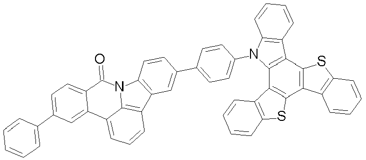 P382 |
| S135 | 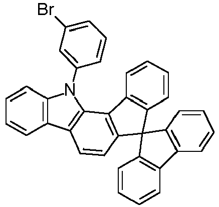 CAS-2101865-23-8 | 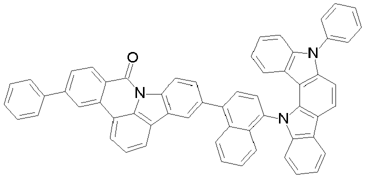 P383 |
| S125 | 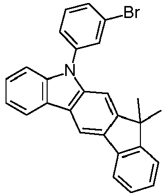 CAS-1369587-63-2 | 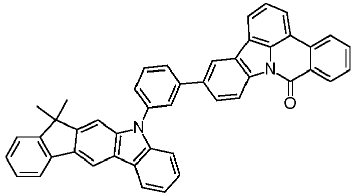 P384 |
| S125 | 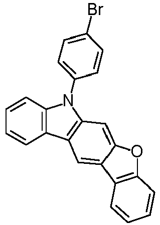 CAS-2079877-31-7 | 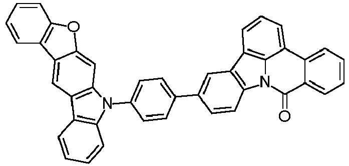 P385 |
| S125 | 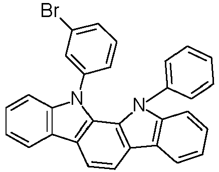 CAS-1260032-02-7 | 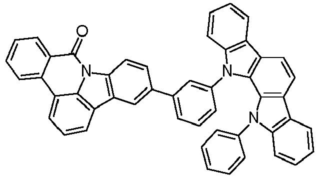 P386 |
| S125 | 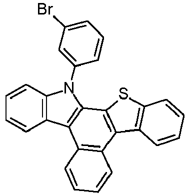 CAS-1644466-89-6 | 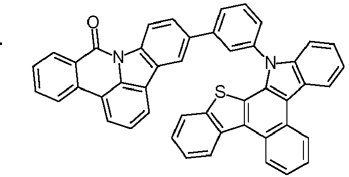 P387 |
| S125 | 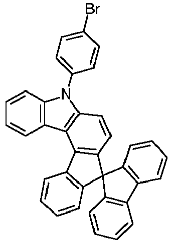 CAS-2095194-33-3 | 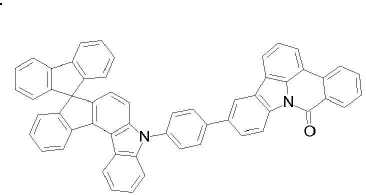 P388 |
| S125 | 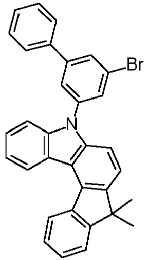 CAS-2134579-41-0 | 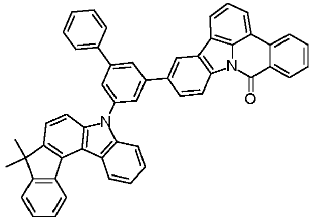 P389 |
| S125 | 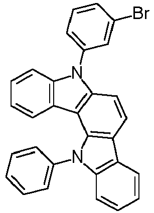 CAS-1782949-43-2 | 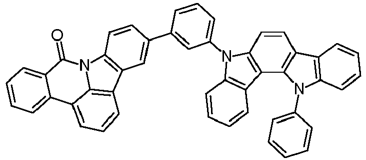 P390 |
| S125 | 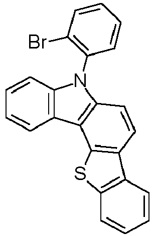 CAS-1622084-14-3 | 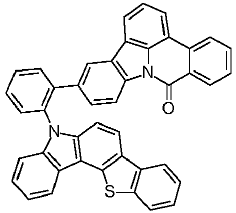 P391 |
| S125 | 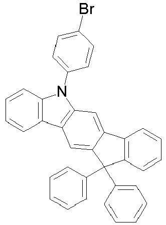 CAS-2249834-37-3 | 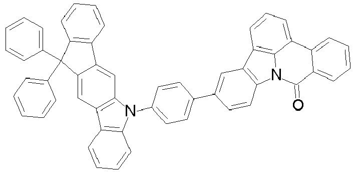 P392 |
| S125 | 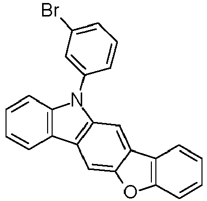 CAS-2109712-47-0 | 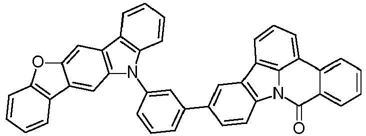 P393 |
| S125 | 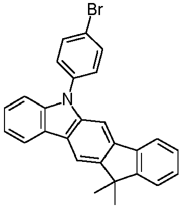 CAS-2249834-37-3 | 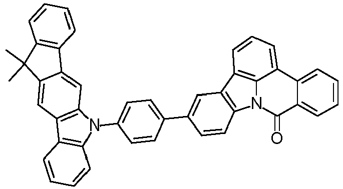 P394 |
| S125 | 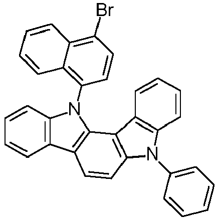 CAS-1632353-57-1 | 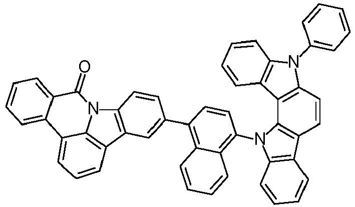 P395 |
| S125 | 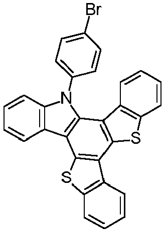 CAS-1534332-59-6 | 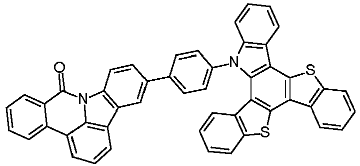 P396 |
| S125 | 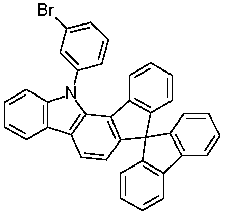 CAS-2101865-23-8 | 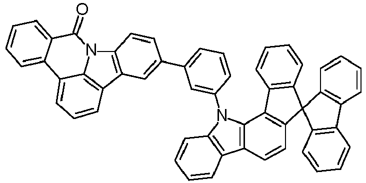 P397 |

### Herstellung der OLEDs

In den folgenden Beispielen V1 bis E18 (siehe Tabelle 1) wird der Einsatz der erfindungsgemäßen Materialien in OLEDs vorgestellt.

**Vorbehandlung für die Beispiele V1 - E18:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt. Die Devicedaten der OLEDs sind in Tabelle 2 aufgelistet. Die Beispiele V1 bis V4 sind Vergleichsbeispiele gemäß dem Stand der Technik, die Beispiele E1 bis E18 zeigen Daten von erfindungsgemäßen OLEDs.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie SoA1:CoH1:TEG1(45%:45%:10%) bedeutet hierbei, dass das Material SoA1 in einem Volumenanteil von 45%, CoH1 in einem Volumenanteil von 45% und TEG1 in einem Volumenanteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren und die Stromeffizienz (SE, gemessen in cd/A) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Stromdichte von 10 mA/cm² bestimmt und daraus die CIE 1931 x und y Farbkoordinaten berechnet. Als Lebensdauer LD wird die Zeit definiert, nach der die Leuchtdichte bei Betrieb mit konstanter Stromdichte j₀ von der Startleuchtdichte auf einen gewissen Anteil L1 absinkt. Eine Angabe L1=80% in Tabelle 2 bedeutet, dass die in Spalte LD angegebene Lebensdauer der Zeit entspricht, nach der die Leuchtdichte auf 80% ihres Anfangswertes absinkt.

### Verwendung von erfindungsgemäßen Materialien in OLEDs

Die erfindungsgemäßen Materialien können in der Emissionsschicht in phosphoreszierenden grünen OLEDs eingesetzt werden. Die erfindungsgemäßen Verbindungen P1, P9, P13, P28, P35, P67, P88, P205, P213, P218, P229, P247, P251, P332, P393 mit oder ohne CoH1 bzw. CoH2 werden in den Beispielen E1 bis E18 als Matrixmaterial in der Emissionsschicht eingesetzt. Im Folgenden werden die Beispiele näher erläutert, um die Vorteile der erfindungsgemäßen OLEDs zu verdeutlichen.

### Verwendung von erfindungsgemäßen Materialien in der Emissionsschicht phosphoreszenter OLEDs

Durch den Einsatz der erfindungsgemäßen Verbindungen P1 und P9 als Matrixmaterial in der Emissionsschicht (Beispiel E1 bis E4) kann eine deutliche Verbesserung der Lebensdauer im Vergleich zu den Verbindungen des Standes der Technik (Beispiele V1 bis V4) erreicht werden. Durch Kombination von P1, P9, P13, P28, P35, P67, P88, P205, P213, P218, P229, P247, P251, P332, P393 mit CoH1 bzw. mit CoH2 und TEG1 bzw. TEG2 kann die Lebendauer darüber hinaus noch weiter deutlich gesteigert werden.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 230nm | SpMA2 20nm | SoA1:TEG1 (88%:12%) 30nm | ST2 10nm | ST2:LiQ (50%:50%) 30nm | LiQ 1nm |
| V2 | HATCN 5nm | SpMA1 230nm | SpMA2 20nm | SoA2:TEG1 (88%:12%) 30nm | ST2 10nm | ST2:LiQ (50%:50%) 30nm | LiQ 1nm |
| E1 | HATCN 5nm | SpMA1 230nm | SpMA2 20nm | P1:TEG1 (88%:12%) 30nm | ST2 10nm | ST2:LiQ (50%:50%) 30nm | LiQ 1nm |
| E2 | HATCN 5nm | SpMA1 230nm | SpMA2 20nm | P9:TEG1 (88%:12%) 30nm | ST2 10nm | ST2:LiQ (50%:50%) 30nm | LiQ 1nm |
| V3 | HATCN 5nm | SpMA1 230nm | SpMA2 20nm | SoA1:CoH1:TEG1 (59%:29%:12%) 30nm | ST2 10nm | ST2:LiQ (50%:50%) 30nm | LiQ 1nm |
| V4 | HATCN 5nm | SpMA1 230nm | SpMA2 20nm | SoA2:CoH1:TEG1 (59%:29%:12%) 30nm | ST2 10nm | ST2:LiQ (50%:50%) 30nm | LiQ 1nm |
| E3 | HATCN | SpMA1 | SpMA2 | P1:CoH1:TEG1 | ST2 | ST2:LiQ (50%:50%) | LiQ 1nm |
| | 5nm | 230nm | 20nm | (59%:29%:12%) 30nm | 10nm | 30nm | |
| E4 | HATCN 5nm | SpMA1 230nm | SpMA2 20nm | P9:CoH1:TEG1 (59%:29%:12%) 30nm | ST2 10nm | ST2:LiQ (50%:50%) 30nm | LiQ 1nm |
| E5 | HATCN 5nm | SpMA1 230nm | SpMA2 20nm | P1:CoH2:TEG2 (44%:44%:12%) 30nm | ST2 10nm | ST2:LiQ (50%:50%) 30nm | LiQ 1nm |
| E6 | HATCN 5nm | SpMA1 230nm | SpMA2 20nm | P13:CoH1:TEG1 (59%:29%:12%) 30nm | ST2 10nm | ST2:LiQ (50%:50%) 30nm | LiQ 1nm |
| E7 | HATCN 5nm | SpMA1 230nm | SpMA2 20nm | P28:CoH2:TEG2 (44%:44%:12%) 30nm | ST2 10nm | ST2:LiQ (50%:50%) 30nm | LiQ 1nm |
| E8 | HATCN 5nm | SpMA1 230nm | SpMA2 20nm | P35:CoH2:TEG2 (44%:44%:12%) 30nm | ST2 10nm | ST2:LiQ (50%:50%) 30nm | LiQ 1nm |
| E9 | HATCN 5nm | SpMA1 230nm | SpMA2 20nm | P67:CoH1:TEG1 (59%:29%:12%) 30nm | ST2 10nm | ST2:LiQ (50%:50%) 30nm | LiQ 1nm |
| E10 | HATCN 5nm | SpMA1 230nm | SpMA2 20nm | P88:CoH1:TEG1 (59%:29%:12%) 30nm | ST2 10nm | ST2:LiQ (50%:50%) 30nm | LiQ 1nm |
| E11 | HATCN 5nm | SpMA1 230nm | SpMA2 20nm | P205:CoH2:TEG2 (44%:44%:12%) 30nm | ST2 10nm | ST2:LiQ (50%:50%) 30nm | LiQ 1nm |
| E12 | HATCN 5nm | SpMA1 230nm | SpMA2 20nm | P213:CoH1:TEG1 (59%:29%:12%) 30nm | ST2 10nm | ST2:LiQ (50%:50%) 30nm | LiQ 1nm |
| E13 | HATCN 5nm | SpMA1 230nm | SpMA2 20nm | P218:CoH2:TEG2 (44%:44%:12%) 30nm | ST2 10nm | ST2:LiQ (50%:50%) 30nm | LiQ 1nm |
| E14 | HATCN 5nm | SpMA1 230nm | SpMA2 20nm | P229:CoH1:TEG1 (59%:29%:12%) 30nm | ST2 10nm | ST2:LiQ (50%:50%) 30nm | LiQ 1nm |
| E15 | HATCN 5nm | SpMA1 230nm | SpMA2 20nm | P247:CoH2:TEG2 (44%:44%:12%) 30nm | ST2 10nm | ST2:LiQ (50%:50%) 30nm | LiQ 1nm |
| E16 | HATCN 5nm | SpMA1 230nm | SpMA2 20nm | P251:CoH1:TEG1 (59%:29%:12%) 30nm | ST2 10nm | ST2:LiQ (50%:50%) 30nm | LiQ 1nm |
| E17 | HATCN 5nm | SpMA1 230nm | SpMA2 20nm | P332:CoH2:TEG2 (44%:44%:12%) 30nm | ST2 10nm | ST2:LiQ (50%:50%) 30nm | LiQ 1nm |
| E18 | HATCN 5nm | SpMA1 230nm | SpMA2 20nm | P393:CoH1:TEG1 (59%:29%:12%) 30nm | ST2 10nm | ST2:LiQ (50%:50%) 30nm | LiQ 1nm |

**Tabelle 2: Devicedaten der OLEDs**

| Bsp. | CIE x/y bei 10 mA/cm² | j₀ (mA/cm²) | L1 (%) | LD (h) |
|---|---|---|---|---|
| V1 | 0.34/0.62 | 40 | 80 | 100 |
| V2 | 0.34/0.62 | 40 | 80 | 105 |
| E1 | 0.34/0.62 | 40 | 80 | 260 |
| E2 | 0.36/0.61 | 40 | 80 | 130 |
| V3 | 0.34/0.62 | 40 | 80 | 180 |
| V4 | 0.35/0.62 | 40 | 80 | 140 |
| E3 | 0.34/0.62 | 40 | 80 | 400 |
| E4 | 0.35/0.62 | 40 | 80 | 240 |
| E5 | 0.35/0.63 | 40 | 80 | 520 |
| E6 | 0.35/0.62 | 40 | 80 | 220 |
| E7 | 0.35/0.63 | 40 | 80 | 480 |
| E8 | 0.35/0.63 | 40 | 80 | 500 |
| E9 | 0.35/0.62 | 40 | 80 | 210 |
| E10 | 0.35/0.62 | 40 | 80 | 220 |
| E11 | 0.35/0.63 | 40 | 80 | 420 |
| E12 | 0.35/0.62 | 40 | 80 | 205 |
| E13 | 0.35/0.63 | 40 | 80 | 450 |
| E13 | 0.35/0.62 | 40 | 80 | 210 |
| E15 | 0.35/0.63 | 40 | 80 | 495 |
| E16 | 0.35/0.62 | 40 | 80 | 230 |
| E17 | 0.35/0.63 | 40 | 80 | 490 |
| E18 | 0.35/0.62 | 40 | 80 | 210 |

**Tabelle 3: Strukturformeln der Materialien für die OLEDs**

| | |
|---|---|
| 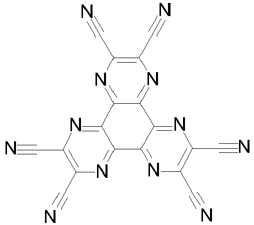 | 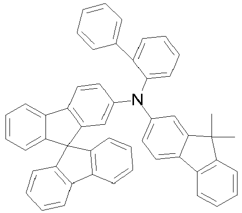 |
| HATCN | SpMA1 |
| 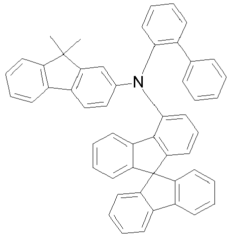 | 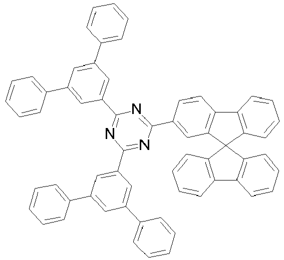 |
| SpMA2 | ST2 |
| 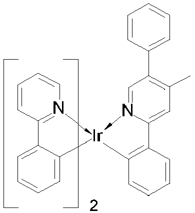 | 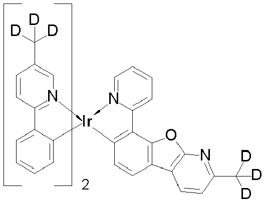 |
| TEG1 | TEG2 |
| 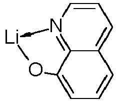 | |
| LiQ | |
| 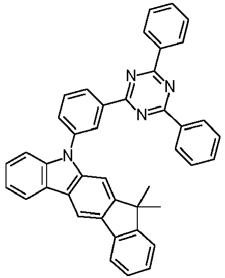 | 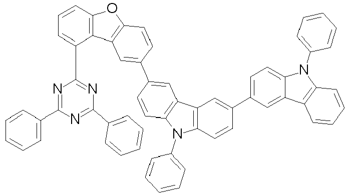 |
| CoH1 | CoH2 |
| 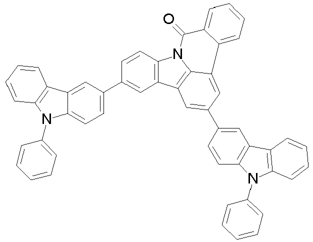 | 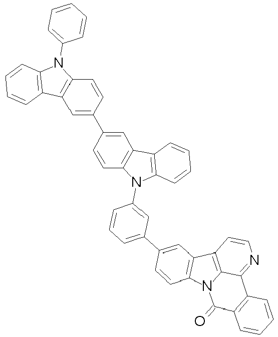 |
| SoA1 | SoA2 |
| 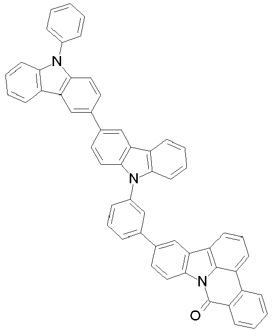 | 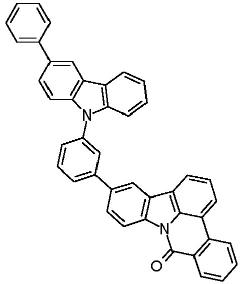 |
| P1 | P9 |
| 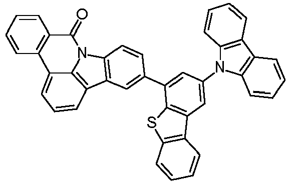 | 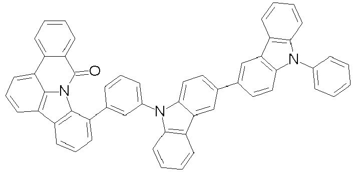 |
| P13 | P28 |
| 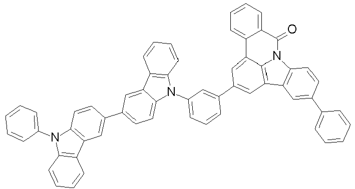 | 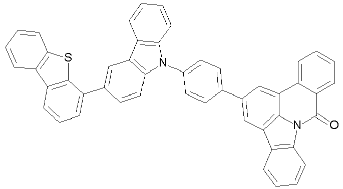 |
| P35 | P67 |
| 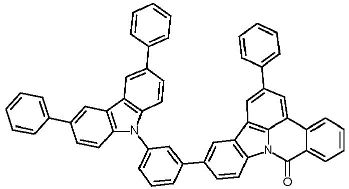 | 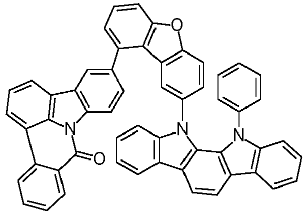 |
| P88 | P205 |
| 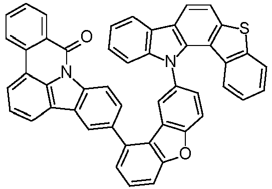 | 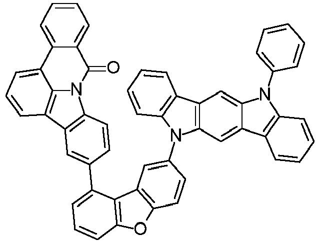 |
| P213 | P218 |
| 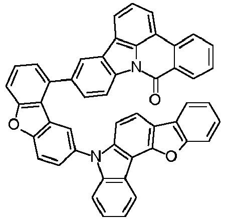 | 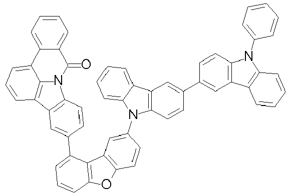 |
| P229 | P247 |
| 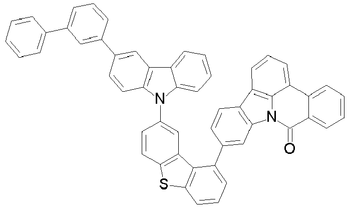 | 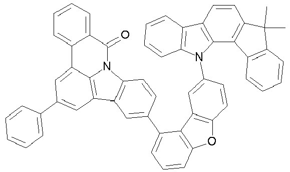 |
| P251 | P332 |
| 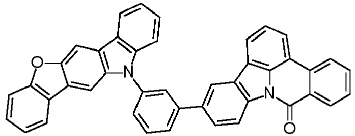 | |
| P393 | |

## Patentansprüche

1. Verbindung gemäß Formel (1), 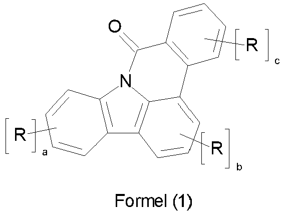 mit der Maßgabe, dass mindestens ein Rest R vorhanden ist, der für eine Gruppe Y steht, und wobei für die verwendeten Symbole und Indizes gilt:
Y ist bei jedem Auftreten gleich oder verschieden eine Gruppe der Formel (2), 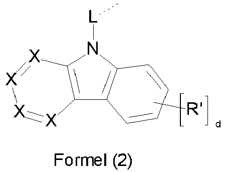 wobei die gestrichelte Bindung die Verknüpfung dieser Gruppe in der Formel (1) kennzeichnet;
X ist bei jedem Auftreten gleich oder verschieden CR' oder zwei benachbarte X stehen für eine Gruppe der Formel (3), und die verbleibenden X sind bei jedem Auftreten gleich oder verschieden CR', 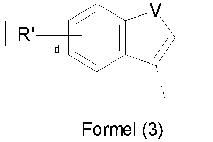 wobei die gestrichelten Bindungen die Verknüpfung dieser Gruppe in der Formel (2) kennzeichnen;
V ist NR', C(R')₂, O oder S;
L ist ein aromatisches oder heteroaromatisches Ringsystem mit 5 bis 30 aromatischen Ringatomen, das mit einem oder mehreren Resten R' substituiert sein kann;
R, R' ist bei jedem Auftreten gleich oder verschieden H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 60 aromatischen Ringatomen, bevorzugt mit 5 bis 40 aromatischen Ringatomen, das jeweils durch einen oder mehrere Reste R¹ substituiert sein kann; dabei können zwei Reste R auch miteinander ein aliphatisches, heteroaliphatisches, aromatisches oder heteroaromatisches Ringsystem bilden; weiterhin können zwei Reste R' auch miteinander ein aliphatisches, heteroaliphatisches, aromatisches oder heteroaromatisches Ringsystem bilden; mit der Maßgabe, dass mindestens ein Rest R für eine Gruppe Y steht;
Ar' ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, Cl, Br, I, N(R²)₂, CN, NO₂, OR², SR², Si(R¹)₃, B(OR²)₂, COOR², C(=O)R², P(=O)(R²)₂, S(=O)R², S(=O)₂R², OSO₂R², eine geradkettige Alkyl-gruppe mit 1 bis 20 C-Atomen oder eine Alkenyl- oder Alkinyl-gruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann, wobei eine oder mehrere nicht benachbarte CH₂-Gruppen durch Si(R²)₂, C=O, NR², O, S oder CONR² ersetzt sein können und wobei ein oder mehrere H-Atome in der Alkyl-, Alkenyl- oder Alkinylgruppe durch D, F, Cl, Br, I oder CN ersetzt sein können, oder ein aromatisches oder hetero-aromatisches Ringsystem mit 5 bis 40 aromatischen Ringatomen, das jeweils durch einen oder mehrere Reste R² substituiert sein kann; dabei können zwei oder mehrere Reste R¹ miteinander ein aliphatisches, heteroaliphatisches, aromatisches oder hetero-aromatisches Ringsystem bilden;
R² ist bei jedem Auftreten gleich oder verschieden H, D, F, CN oder ein aliphatischer, aromatischer oder heteroaromatischer organischer Rest, insbesondere ein Kohlenwasserstoffrest, mit 1 bis 20 C-Atomen, in dem auch ein oder mehrere H-Atome durch F ersetzt sein können;
a ist 0, 1, 2, 3 oder 4;
b ist bei jedem Auftreten gleich oder verschieden 0, 1, 2 oder 3;
c ist 0, 1, 2, 3 oder 4;
d ist bei jedem Auftreten gleich oder verschieden 0, 1, 2, 3 oder 4;
mit der Maßgabe, dass a + b + c ≥ 1 ist;
dabei ist die folgende Verbindung von der Erfindung ausgenommen:

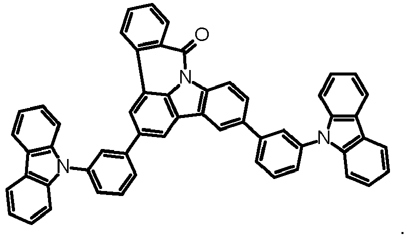

2. Verbindung nach Anspruch 1, ausgewählt aus den Verbindungen der Formeln (4) bis (6), 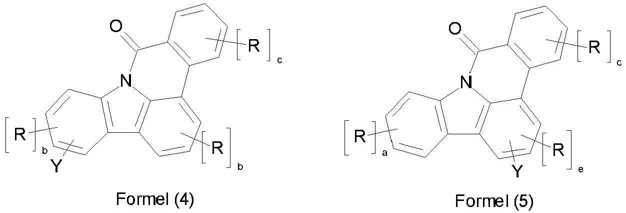 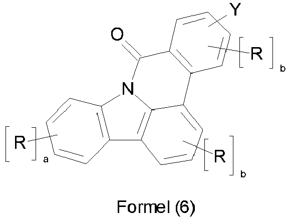 wobei die Symbole und Indizes die in Anspruch 1 genannten Bedeutungen aufweisen und weiterhin gilt:
e ist 0, 1 oder 2.

3. Verbindung nach Anspruch 1 oder 2, ausgewählt aus den Verbindungen der Formeln (4a) bis (6a), 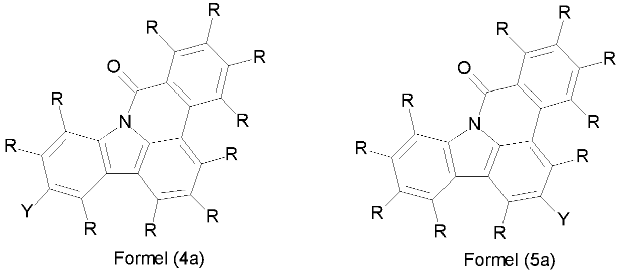 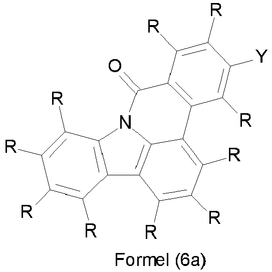 wobei die Symbole die in Anspruch 1 genannten Bedeutungen aufweisen.

4. Verbindung nach einem oder mehreren der Ansprüche 1 bis 3, ausgewählt aus den Verbindungen der Formeln (4b) bis (6b), 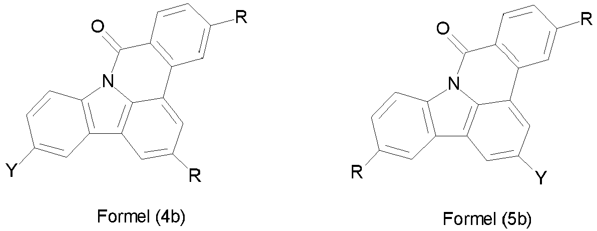 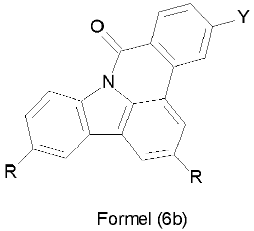 wobei die Symbole die in Anspruch 1 genannten Bedeutungen aufweisen.

5. Verbindung nach einem oder mehreren der Ansprüche 1 bis 4, **dadurch gekennzeichnet, dass** genau ein oder zwei Reste R für eine Gruppe Y stehen.

6. Verbindung nach einem oder mehreren der Ansprüche 1 bis 5, **dadurch gekennzeichnet, dass** L gleich oder verschieden bei jedem Auftreten für ein aromatisches oder heteroaromatisches Ringsystem mit 6 bis 24 aromatischen Ringatomen, welches durch einen oder mehrere Reste R' substituiert sein kann, wobei L, wenn L für ein heteroaromatisches Ringsystem steht, keine elektronenarmen Heteroarylgruppen enthält.

7. Verbindung nach einem oder mehreren der Ansprüche 1 bis 6, **dadurch gekennzeichnet, dass** L bei jedem Auftreten gleich oder verschieden ausgewählt ist aus der Gruppe bestehend aus Benzol, Biphenyl, Terphenyl, Quaterphenyl, Fluoren, Spirobifluoren, Naphthalin, Carbazol, Dibenzofuran, Dibenzothiophen, Indenocarbazol, Indolocarbazol, Phenanthren, Triphenylen oder einer Kombination aus zwei oder drei dieser Gruppen, wobei diese Gruppen jeweils mit einem oder mehreren Resten R' substituiert sein können.

8. Verbindung nach einem oder mehreren der Ansprüche 1 bis 7, **dadurch gekennzeichnet, dass** die Gruppe Y gleich oder verschieden bei jedem Auftreten gewählt ist aus den Gruppen der folgenden Formeln (Y-1) bis (Y-8), 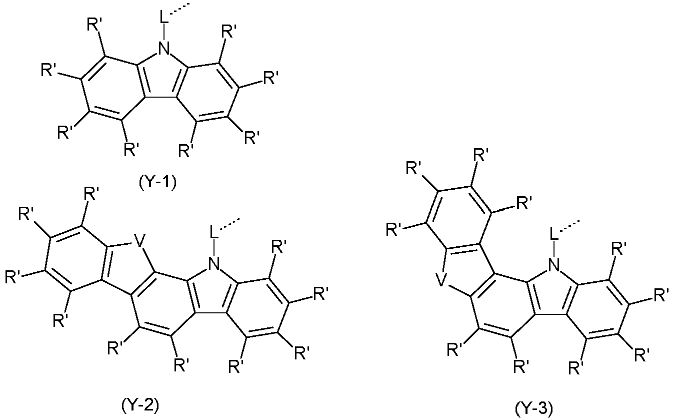 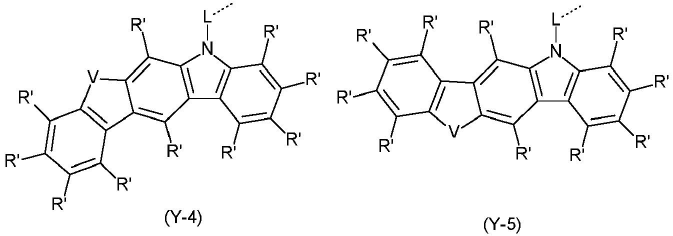 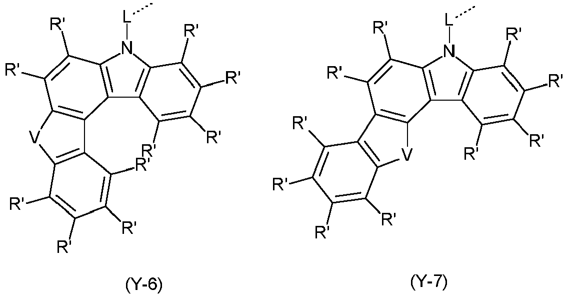 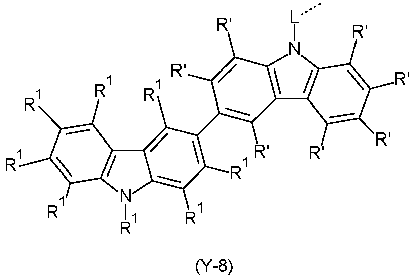 wobei die Symbole die in Anspruch 1 genannten Bedeutungen aufweisen.

9. Verbindung nach einem oder mehreren der Ansprüche 1 bis 8, **dadurch gekennzeichnet, dass** die Gruppe Y gleich oder verschieden bei jedem Auftreten ausgewählt ist aus den Strukturen der Formeln (Y-1a) bis (Y-8a), 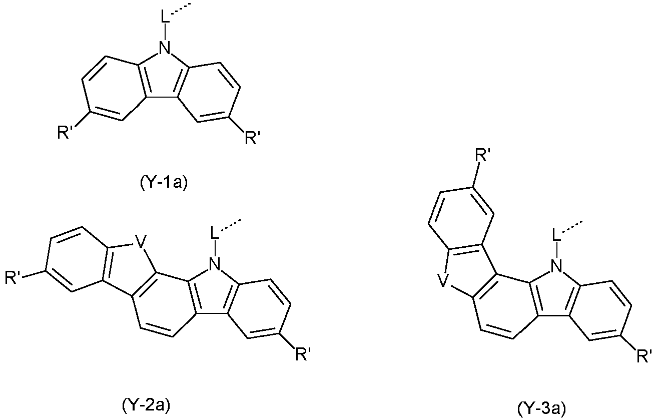 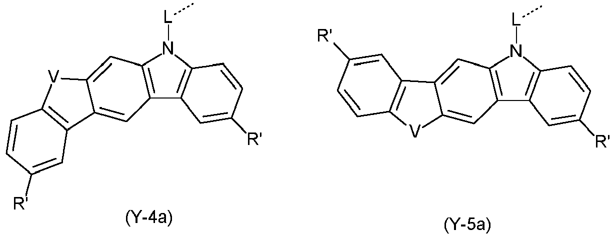 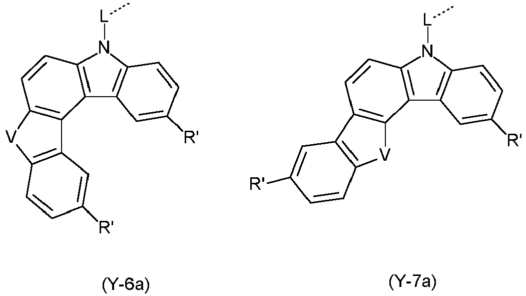 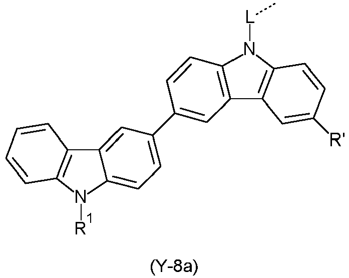 wobei die Symbole die in Anspruch 1 genannten Bedeutungen aufweisen.

10. Verbindung nach einem oder mehreren der Ansprüche 1 bis 9, **dadurch gekennzeichnet, dass** R und R' bei jedem Auftreten gleich oder verschieden ausgewählt sind aus der Gruppe bestehend aus H, D, F, N(Ar')₂, CN, OR¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl- bzw. Alkenylgruppe jeweils mit einem oder mehreren Resten R¹ substituiert sein kann, bevorzugt jedoch unsubstituiert ist, und wobei eine oder mehrere nicht benachbarte CH₂-Gruppen durch O ersetzt sein können, oder einem aromatischen oder heteroaromatischen Ringsystem mit 6 bis 30 aromatischen Ringatomen, das jeweils durch einen oder mehrere Reste R¹ substituiert sein kann; dabei können zwei Reste R auch miteinander ein aliphatisches Ringsystem bilden; weiterhin können zwei Reste R' auch miteinander ein aliphatisches oder aromatisches Ringsystem bilden.

11. Verfahren zur Herstellung einer Verbindung nach einem oder mehreren der Ansprüche 1 bis 10, **gekennzeichnet durch** die folgenden Syntheseschritte:
a) Synthese des Lactamgrundgerüsts, welches statt der Gruppe Y eine reaktive Abgangsgruppe trägt; und
b) Einführung der Gruppe Y durch eine Kupplungsreaktion.

12. Formulierung, enthaltend mindestens eine Verbindung nach einem oder mehreren der Ansprüche 1 bis 10 und mindestens eine weitere Verbindung und/oder mindestens ein Lösemittel.

13. Verwendung einer Verbindung nach einem oder mehreren der Ansprüche 1 bis 10 in einer elektronischen Vorrichtung.

14. Elektronische Vorrichtung enthaltend mindestens eine Verbindung nach einem oder mehreren der Ansprüche 1 bis 10.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 10 in einer emittierenden Schicht als Matrixmaterial für phosphoreszierende Emitter oder für Emitter, die TADF zeigen, und/oder in einer Elektronentransportschicht und/oder in einer Lochblockierschicht und/oder in einer Lochtransportschicht und/oder in einer Exzitonenblockierschicht eingesetzt wird.

## Claims

1. Compound of the formula (1), 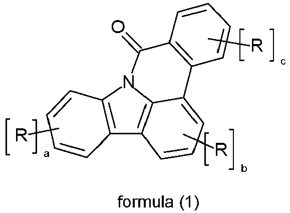 with the proviso that at least one radical R that stands for a group Y is present, and where the following applies to the symbols and indices used:
Y is on each occurrence, identically or differently, a group of the formula (2), 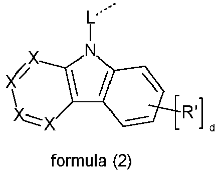 where the dashed bond denotes the linking of this group in the formula (1);
X is on each occurrence, identically or differently, CR' or two adjacent X stand for a group of the formula (3), and the remaining X are on each occurrence, identically or differently, CR', 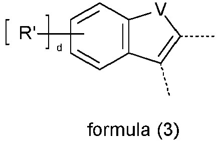 where the dashed bonds denote the linking of this group in the for-mula (2);
V is NR', C(R')₂, O or S;
L is an aromatic or heteroaromatic ring system having 5 to 30 aromatic ring atoms, which may be substituted by one or more radicals R';
R, R' are on each occurrence, identically or differently, H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R¹, where one or more non-adjacent CH₂ groups may be replaced by Si(R¹)₂, C=O, NR¹, O, S or CONR¹, or an aromatic or heteroaromatic ring system having 5 to 60 aromatic ring atoms, preferably having 5 to 40 aromatic ring atoms, which may in each case be substituted by one or more radicals R¹; two radicals R may also form an aliphatic, heteroaliphatic, aromatic or hetero-aromatic ring system with one another; furthermore, two radicals R' may also form an aliphatic, heteroaliphatic, aromatic or hetero-aromatic ring system with one another; with the proviso that at least one radical R stands for a group Y;
Ar' is on each occurrence, identically or differently, an aromatic or heteroaromatic ring system having 5 to 40 aromatic ring atoms, which may be substituted by one or more radicals R¹;
R¹ is on each occurrence, identically or differently, H, D, F, Cl, Br, I, N(R²)₂, CN, NO₂, OR², SR², Si(R²)₃, B(OR²)₂, COOR², C(=O)R², P(=O)(R²)₂, S(=O)R², S(=O)₂R², OSO₂R², a straight-chain alkyl group having 1 to 20 C atoms or an alkenyl or alkynyl group having 2 to 20 C atoms or a branched or cyclic alkyl group having 3 to 20 C atoms, where the alkyl, alkenyl or alkynyl group may in each case be substituted by one or more radicals R², where one or more non-adjacent CH₂ groups may be replaced by Si(R²)₂, C=O, NR², O, S or CONR² and where one or more H atoms in the alkyl, alkenyl or alkynyl group may be replaced by D, F, Cl, Br, I or CN, or an aromatic or heteroaromatic ring system having 5 to 40 aromatic ring atoms, which may in each case be substituted by one or more radicals R²; two or more radicals R¹ may form an aliphatic, heteroaliphatic, aromatic or heteroaromatic ring system with one another;
R² is on each occurrence, identically or differently, H, D, F, CN or an aliphatic, aromatic or heteroaromatic organic radical, in particular a hydrocarbon radical, having 1 to 20 C atoms, in which, in addition, one or more H atoms may be replaced by F;
a is 0, 1, 2, 3 or 4;
b is on each occurrence, identically or differently, 0, 1, 2 or 3;
c is 0, 1, 2, 3 or 4;
d is on each occurrence, identically or differently, 0, 1, 2, 3 or 4;
with the proviso that a + b + c ≥ 1;
the following compound is excluded from the invention:

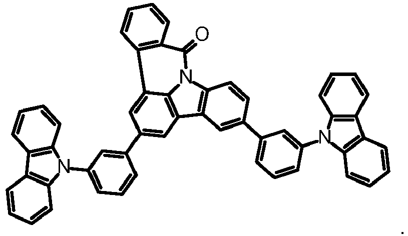

2. Compound according to Claim 1, selected from the compounds of the formulae (4) to (6), 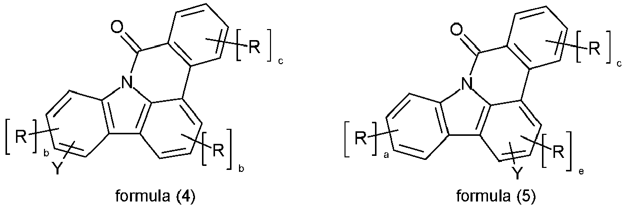 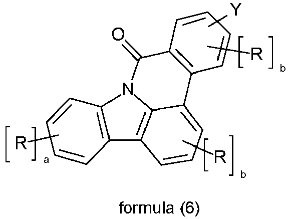 where the symbols and indices have the meanings given in Claim 1 and furthermore:
e is 0, 1 or 2.

3. Compound according to Claim 1 or 2, selected from the compounds of the formulae (4a) to (6a), 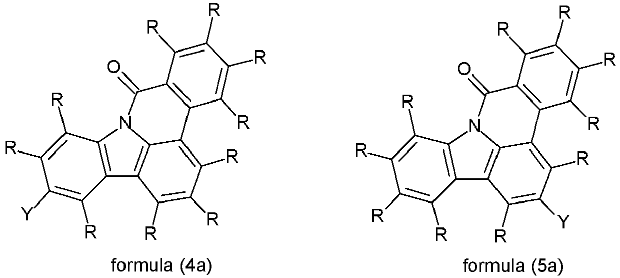 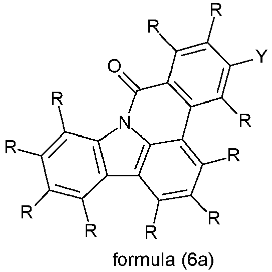 where the symbols have the meanings given in Claim 1.

4. Compound according to one or more of Claims 1 to 3, selected from the compounds of the formulae (4b) to (6b), 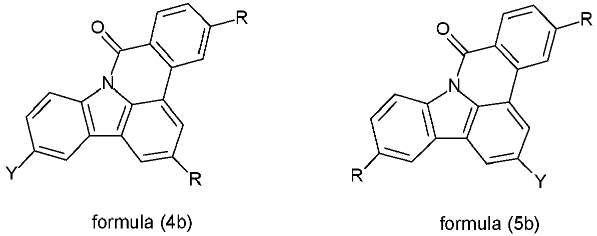 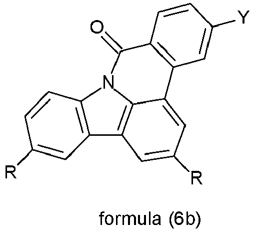 where the symbols have the meanings given in Claim 1.

5. Compound according to one or more of Claims 1 to 4, **characterised in that** precisely one or two radicals R stand for a group Y.

6. Compound according to one or more of Claims 1 to 5, **characterised in that** L stands, identically or differently on each occurrence, for an aromatic or heteroaromatic ring system having 6 to 24 aromatic ring atoms, which may be substituted by one or more radicals R', where L, if L stands for a heteroaromatic ring system, contains no electron-deficient heteroaryl groups.

7. Compound according to one or more of Claims 1 to 6, **characterised in that** L is selected on each occurrence, identically or differently, from the group consisting of benzene, biphenyl, terphenyl, quaterphenyl, fluorene, spirobifluorene, naphthalene, carbazole, dibenzofuran, dibenzothiophene, indenocarbazole, indolocarbazole, phenanthrene, triphenylene or a combination of two or three of these groups, where these groups may in each case be substituted by one or more radicals R'.

8. Compound according to one or more of Claims 1 to 7, **characterised in that** the group Y is selected, identically or differently on each occurrence, from the groups of the following formulae (Y-1) to (Y-8), 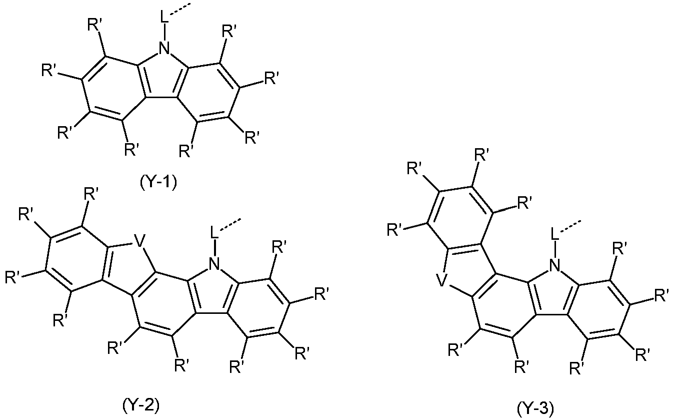 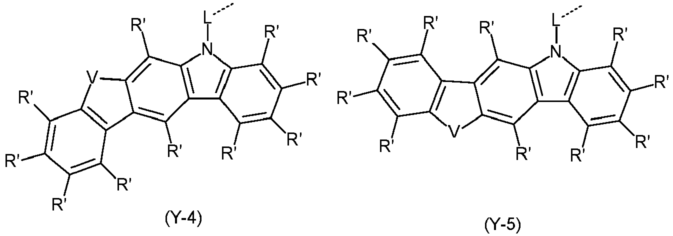 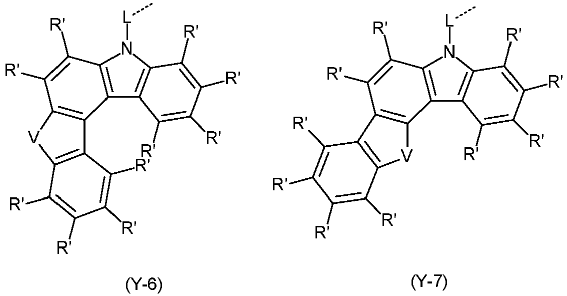 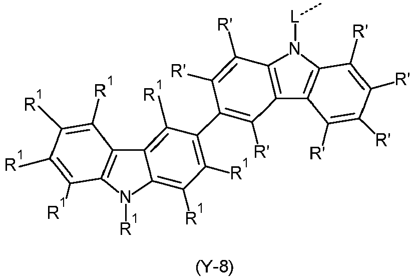 where the symbols have the meanings given in Claim 1.

9. Compound according to one or more of Claims 1 to 8, **characterised in that** the group Y is selected, identically or differently on each occurrence, from the structures of the formulae (Y-1a) to (Y-8a), 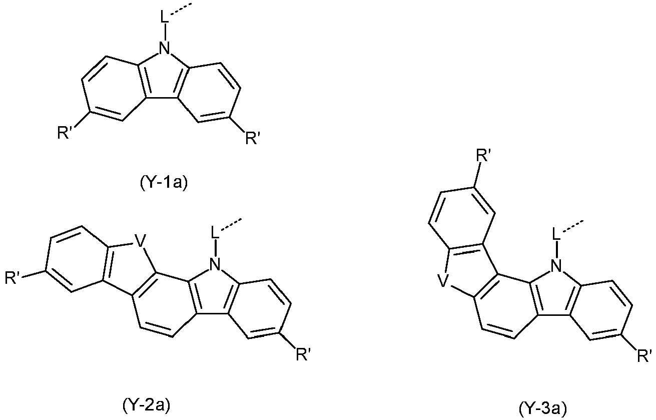 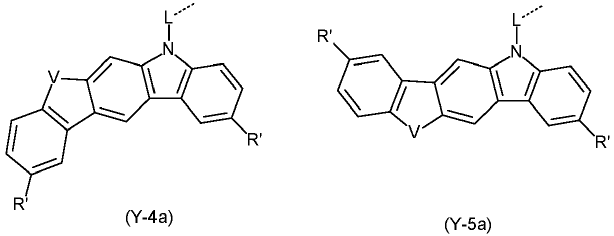 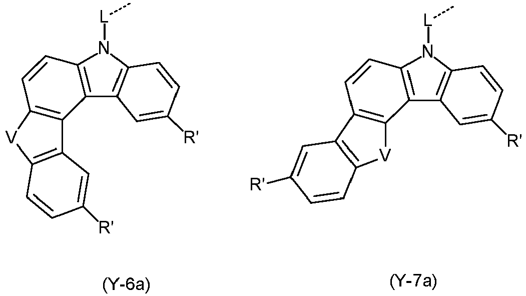 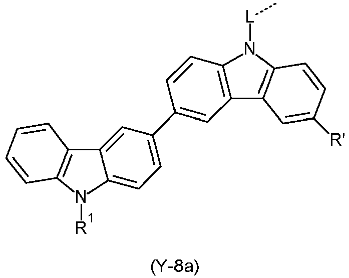 where the symbols have the meanings given in Claim 1.

10. Compound according to one or more of Claims 1 to 9, **characterised in that** R and R' are selected on each occurrence, identically or differently, from the group consisting of H, D, F, N(Ar')₂, CN, OR¹, a straight-chain alkyl group having 1 to 10 C atoms or an alkenyl group having 2 to 10 C atoms or a branched or cyclic alkyl group having 3 to 10 C atoms, where the alkyl or alkenyl group may in each case be substituted by one or more radicals R¹, but is preferably unsubstituted, and where one or more non-adjacent CH₂ groups may be replaced by O, or an aromatic or heteroaromatic ring system having 6 to 30 aromatic ring atoms, which may in each case be substituted by one or more radicals R¹; two radicals R may also form an aliphatic ring system with one another; furthermore, two radicals R' may also form an aliphatic or aromatic ring system with one another.

11. Process for the preparation of a compound according to one or more of Claims 1 to 10, **characterised by** the following synthesis steps:
a) synthesis of the lactam skeleton which carries a reactive leaving group instead of the group Y; and
b) introduction of the group Y by a coupling reaction.

12. Formulation comprising at least one compound according to one or more of Claims 1 to 10 and at least one further compound and/or at least one solvent.

13. Use of a compound according to one or more of Claims 1 to 10 in an electronic device.

14. Electronic device containing at least one compound according to one or more of Claims 1 to 10.

15. Electronic device according to Claim 14, which is an organic electroluminescent device, **characterised in that** the compound according to one or more of Claims 1 to 10 is employed in an emitting layer as matrix material for phosphorescent emitters or for emitters which exhibit TADF, and/or in an electron-transport layer and/or in a hole-blocking layer and/or in a hole-transport layer and/or in an exciton-blocking layer.

## Revendications

1. Composé de formule (1), 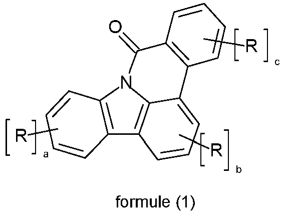 à condition qu'au moins un radical R qui représente un groupement Y soit présent, et où ce qui suit s'applique aux symboles et indices utilisés :
Y est à chaque occurrence, de manière identique ou différente, un groupement de formule (2), 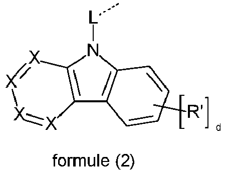 où la liaison en pointillés désigne la liaison de ce groupement dans la formule (1) ;
X est à chaque occurrence, de manière identique ou différente, CR' ou deux X adjacents représentent un groupement de formule (3), et les X restants sont à chaque occurrence, de manière identique ou différente, CR', 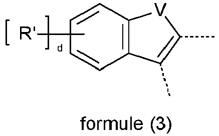 où les liaisons en pointillés dénotent la liaison de ce groupement dans la formule (2) ;
V est NR', C(R')₂, O ou S ;
L est un noyau aromatique ou hétéroaromatique ayant de 5 à 30 atomes de cycle aromatique, qui peut être substitué par un ou plusieurs radicaux R' ;
R, R' sont à chaque occurrence, de manière identique ou différente, H, D, F, Cl, Br, I, N(Ar')₂, N(R¹)₂, OAr', SAr', CN, NO₂, OR¹, SR¹, COOR¹, C(=O)N(R¹)₂, Si(R¹)₃, B(OR¹)₂, C(=O)R¹, P(=O)(R¹)₂, S(=O)R¹, S(=O)₂R¹, OSO₂R¹, un groupement alkyle à chaîne linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut dans chaque cas être substitué par un ou plusieurs radicaux R¹, où un ou plusieurs groupements CH₂ non adjacents peuvent être remplacés par Si(R¹)₂, C=O, NR¹, O, S ou CONR¹, ou un noyau aromatique ou hétéroaromatique ayant de 5 à 60 atomes de cycle aromatique, préférablement ayant de 5 à 40 atomes de cycle aromatique, qui peut dans chaque cas être substitué par un ou plusieurs radicaux R¹ ; deux radicaux R peuvent également former un noyau aliphatique, hétéroaliphatique, aromatique ou hétéroaromatique l'un avec l'autre ; de plus, deux radicaux R' peuvent également former un noyau aliphatique, hétéroaliphatique, aromatique ou hétéroaromatique l'un avec l'autre ; à condition qu'au moins un radical R représente un groupement Y ;
Ar' est à chaque occurrence, de manière identique ou différente, un noyau aromatique ou hétéroaromatique ayant de 5 à 40 atomes de cycle aromatique, qui peut être substitué par un ou plusieurs radicaux R¹ ;
R¹ est à chaque occurrence, de manière identique ou différente, H, D, F, Cl, Br, I, N(R²)₂, CN, NO₂, OR², SR², Si(R²)₃, B(OR²)₂, COOR², C(=O)R², P(=O)(R²)₂, S(=O)R², S(=O)₂R², OSO₂R², un groupement alkyle à chaîne linéaire ayant de 1 à 20 atomes de C ou un groupement alcényle ou alcynyle ayant de 2 à 20 atomes de C ou un groupement alkyle ramifié ou cyclique ayant de 3 à 20 atomes de C, où le groupement alkyle, alcényle ou alcynyle peut dans chaque cas être substitué par un ou plusieurs radicaux R², où un ou plusieurs groupements CH₂ non adjacents peuvent être remplacés par Si(R²)₂, C=O, NR², O, S ou CONR² et où un ou plusieurs atomes de H dans le groupement alkyle, alcényle ou alcynyle peuvent être remplacés par D, F, Cl, Br, I ou CN, ou un noyau aromatique ou hétéroaromatique ayant de 5 à 40 atomes de cycle aromatique, qui peut dans chaque cas être substitué par un ou plusieurs radicaux R² ; deux, ou plus, radicaux R¹ peuvent former un noyau aliphatique, hétéroaliphatique, aromatique ou hétéroaromatique l'un avec l'autre ;
R² est à chaque occurrence, de manière identique ou différente, H, D, F, CN ou un radical organique aliphatique, aromatique ou hétéroaromatique, en particulier un radical hydrocarboné, ayant de 1 à 20 atomes de C, dans lequel, de plus, un ou plusieurs atomes de H peuvent être remplacés par F ;
a vaut 0, 1, 2, 3 ou 4 ;
b vaut à chaque occurrence, de manière identique ou différente, 0, 1, 2 ou 3 ;
c vaut 0, 1, 2, 3 ou 4 ;
d vaut à chaque occurrence, de manière identique ou différente, 0, 1, 2, 3 ou 4 ;
à condition que a + b + c ≥ 1 ;
le composé suivant est exclu de l'invention :

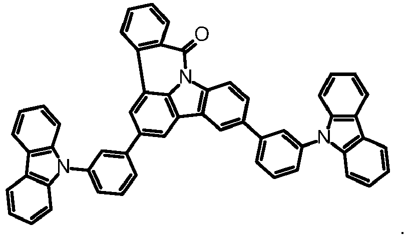

2. Composé selon la revendication 1, choisi parmi les composés de formules (4) à (6), 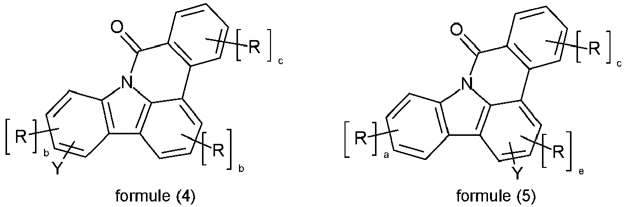 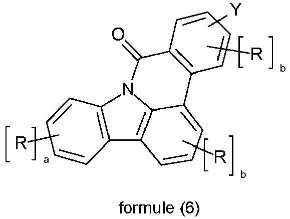 où les symboles et les indices revêtent les significations données selon la revendication 1 et de plus :
e vaut 0, 1 ou 2.

3. Composé selon la revendication 1 ou 2, choisi parmi les composés de formules (4a) à (6a), 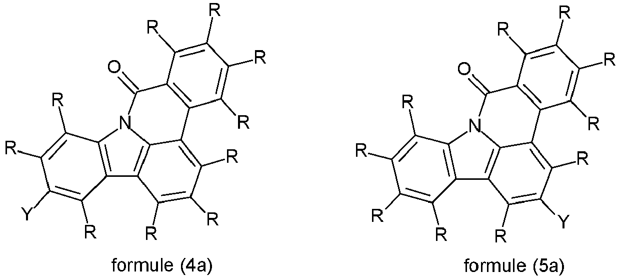 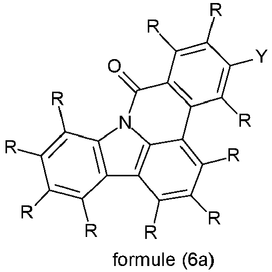 où les symboles revêtent les significations données selon la revendication 1.

4. Composé selon l'une ou plusieurs parmi les revendications 1 à 3, choisi parmi les composés de formules (4b) à (6b), 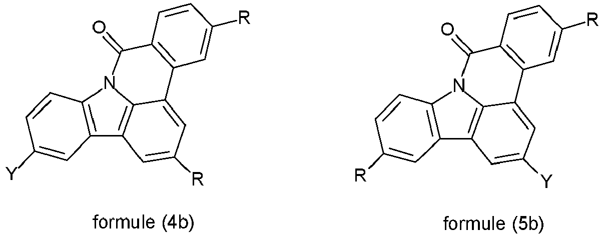 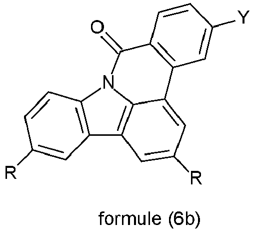 où les symboles revêtent les significations données selon la revendication 1.

5. Composé selon l'une ou plusieurs parmi les revendications 1 à 4, **caractérisé en ce que** précisément un ou deux radicaux R représentent un groupement Y.

6. Composé selon l'une ou plusieurs parmi les revendications 1 à 5, **caractérisé en ce que** L représente, de manière identique ou différente à chaque occurrence, un noyau aromatique ou hétéroaromatique ayant de 6 à 24 atomes de cycle aromatique, qui peut être substitué par un ou plusieurs radicaux R', où L, si L représente un noyau hétéroaromatique, ne contient aucun groupement hétéroaryle déficitaire en électrons.

7. Composé selon l'une ou plusieurs parmi les revendications 1 à 6, **caractérisé en ce que** L est choisi à chaque occurrence, de manière identique ou différente, dans le groupe constitué par benzène, biphényle, terphényle, quaterphényle, fluorène, spirobifluorène, naphtalène, carbazole, dibenzofurane, dibenzothiophène, indénocarbazole, indolocarbazole, phénanthrène, triphénylène ou une combinaison de deux ou trois parmi ces groupements, où ces groupements peuvent être dans chaque cas substitués par un ou plusieurs radicaux R'.

8. Composé selon l'une ou plusieurs parmi les revendications 1 à 7, **caractérisé en ce que** le groupement Y est choisi, de manière identique ou différente à chaque occurrence parmi les groupements de formules (Y-1) à (Y-8) suivantes, 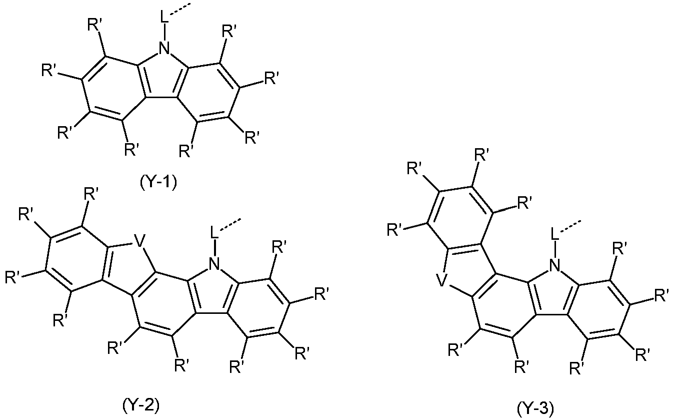 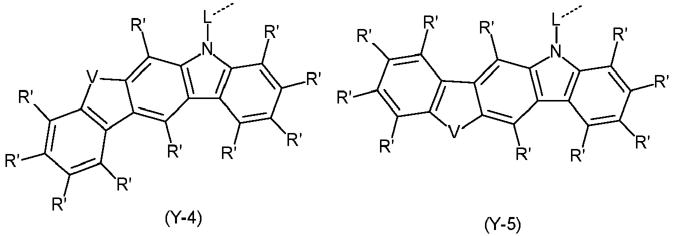 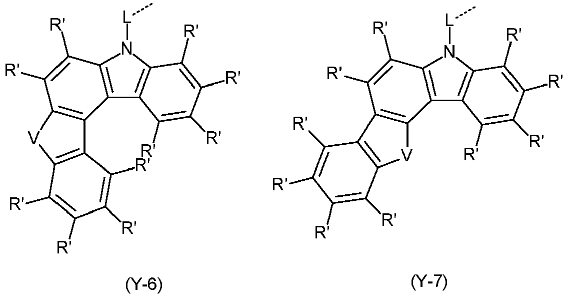 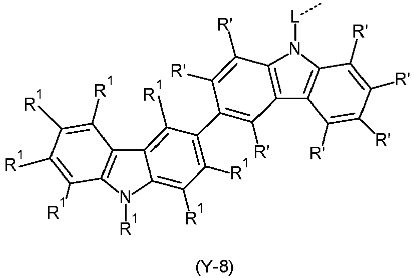 où les symboles revêtent les significations données selon la revendication 1.

9. Composé selon l'une ou plusieurs parmi les revendications 1 à 8, **caractérisé en ce que** le groupement Y est choisi, de manière identique ou différente à chaque occurrence, parmi les structures de formules (Y-1a) à (Y-8a), 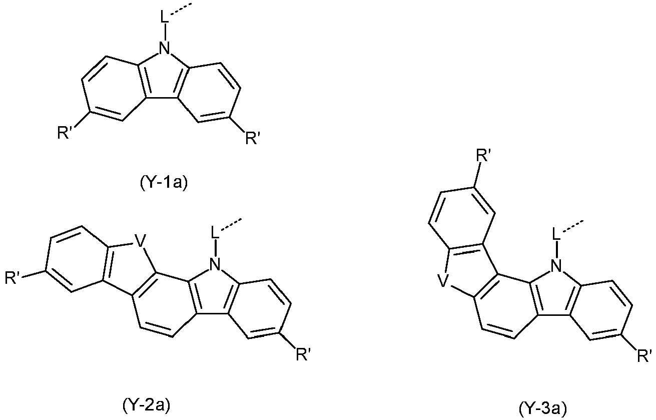 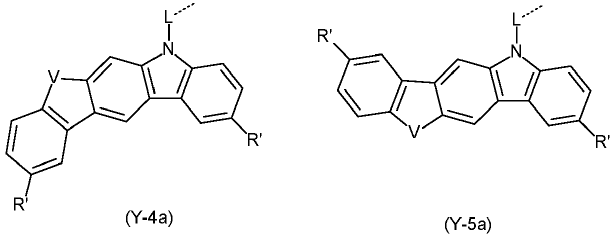 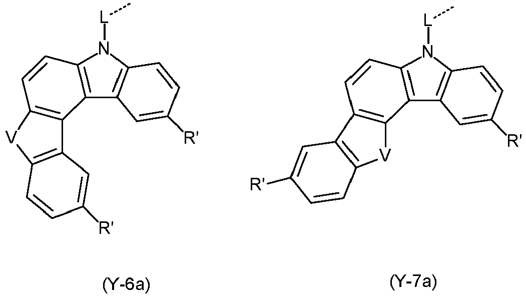 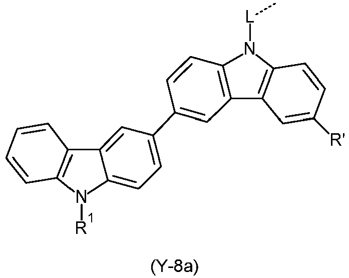 où les symboles revêtent les significations données selon la revendication 1.

10. Composé selon l'une ou plusieurs parmi les revendications 1 à 9, **caractérisé en ce que** R et R' sont choisis à chaque occurrence, de manière identique ou différente, dans le groupe constitué par H, D, F, N(Ar')₂, CN, OR¹, un groupement alkyle à chaîne linéaire ayant de 1 à 10 atomes de C ou un groupement alcényle ayant de 2 à 10 atomes de C ou un groupement alkyle ramifié ou cyclique ayant de 3 à 10 atomes de C, où le groupement alkyle ou alcényle peut dans chaque cas être substitué par un ou plusieurs radicaux R¹, mais est préférablement non substitué, et où un ou plusieurs groupements CH₂ non adjacents peuvent être remplacés par O, ou un noyau aromatique ou hétéroaromatique ayant de 6 à 30 atomes de cycle aromatique, qui peut dans chaque cas être substitué par un ou plusieurs radicaux R¹ ; deux radicaux R peuvent également former un noyau aliphatique l'un avec l'autre ; de plus, deux radicaux R' peuvent également former un noyau aliphatique ou aromatique l'un avec l'autre.

11. Procédé de préparation d'un composé selon l'une ou plusieurs parmi les revendications 1 à 10, **caractérisé par** les étapes de synthèse suivantes :
a) synthèse du squelette de lactame qui porte un groupement partant réactif au lieu du groupement Y ; et
b) introduction du groupement Y par une réaction de couplage.

12. Formulation comprenant au moins un composé selon l'une ou plusieurs parmi les revendications 1 à 10 et au moins un autre composé et/ou au moins un solvant.

13. Utilisation d'un composé selon l'une ou plusieurs parmi les revendications 1 à 10, dans un dispositif électronique.

14. Dispositif électronique contenant au moins un composé selon l'une ou plusieurs parmi les revendications 1 à 10.

15. Dispositif électronique selon la revendication 14, qui est un dispositif électroluminescent organique, **caractérisé en ce que** le composé selon l'une ou plusieurs parmi les revendications 1 à 10 est employé dans une couche émettrice comme matériau de matrice pour des émetteurs phosphorescents ou pour des émetteurs présentant une TADF, et/ou dans une couche de transport d'électrons et/ou dans une couche de blocage de trous et/ou dans une couche de transport de trous et/ou dans une couche de blocage d'excitons.
